(19) Europäisches Patentamt
European Patent Office
Office européen des brevets

(11) EP 4 089 096 A1

(12) EUROPEAN PATENT APPLICATION
published in accordance with Art. 153(4) EPC

(43) Date of publication:
**16.11.2022 Bulletin 2022/46**

(21) Application number: **21738769.5**

(22) Date of filing: **08.01.2021**

(51) International Patent Classification (IPC):
***C07D 519/00*** *(2006.01)* ***A01N 43/90*** *(2006.01)*
***A01P 7/02*** *(2006.01)* ***A01P 7/04*** *(2006.01)*
***A61K 31/519*** *(2006.01)* ***A61K 31/53*** *(2006.01)*
***A61P 33/14*** *(2006.01)*

(52) Cooperative Patent Classification (CPC):
**A01N 43/90; A01P 7/02; A01P 7/04; A61K 31/519; A61K 31/53; A61P 33/14; C07D 519/00**

(86) International application number:
**PCT/JP2021/000444**

(87) International publication number:
**WO 2021/141106 (15.07.2021 Gazette 2021/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.01.2020 JP 2020001899**
**05.08.2020 JP 2020132784**

(71) Applicant: **SUMITOMO CHEMICAL COMPANY, LIMITED**
**Tokyo 103-6020 (JP)**

(72) Inventors:
- **MAEHATA, Ryota**
**Takarazuka-shi, Hyogo 665-8555 (JP)**
- **TSURUDA, Takeshi**
**Takarazuka-shi, Hyogo 665-8555 (JP)**
- **SHIODA, Takayuki**
**Takarazuka-shi, Hyogo 665-8555 (JP)**
- **SAITO, Yasumasa**
**Takarazuka-shi, Hyogo 665-8555 (JP)**

(74) Representative: **Vossius & Partner**
**Patentanwälte Rechtsanwälte mbB**
**Siebertstraße 3**
**81675 München (DE)**

# (54) HETEROCYCLIC COMPOUND AND HARMFUL ARTHROPOD-CONTROLLING COMPOSITION INCLUDING SAME

(57) Provided is a compound having an excellent control effect on a harmful arthropod. A compound represented by Formula (I) [wherein Q represents a group represented by Q2 or the like, ● represents a site of binding with the rest of molecules, T1 represents a group represented by T1-1 or the like, Z represents an oxygen atom or the like, $A^2$ represents $CR^{4a}$ or the like, $B^1$ represents $CR^{38a}$ or the like, $B^2$ represents $CR^1$ or the like, $B^3$ represents a nitrogen atom or the like, $G^1$ represents a nitrogen atom or $CR^{3a}$, $G^2$ represents a nitrogen atom or $CR^{3b}$, $G^3$ represents a nitrogen atom or $CR^{3c}$, $G^4$ represents a nitrogen atom or $CR^{3d}$, $R^1$ represents a C1-C6 chain hydrocarbon group that is substituted with one or more substituents selected from the group consisting of a cyano group and a halogen atom, $R^2$ represents a C1-C6 alkyl group or the like, and $R^{3a}$, $R^{3b}$, $R^{3c}$, $R^{3d}$, $R^{4a}$, and $R^6$ are the same or different and represent a C1-C6 chain hydrocarbon group or the like, $R^{38a}$ represents a C1-C3 chain hydrocarbon group or the like, and n represents 0, 1, or 2.] or an N-oxide thereof has an excellent control effect on a harmful arthropod.

EP 4 089 096 A1

## Description

TECHNICAL FIELD

This application claims priority to and the benefit of Japanese Patent Application No. 2020-001899 filed on January 9, 2020 and Japanese Patent Application No. 2020-132784 filed on August 5, 2020, the entire contents of which are incorporated herein by reference.

The present invention relates to a heterocyclic compound and a harmful arthropod-controlling composition including same.

BACKGROUND ART

So far, for the purpose of harmful arthropod control, various compounds have been studied. For example, Patent Document 1 discloses that a certain compound has a pest control effect.

PRIOR ART DOCUMENTS

PATENT DOCUMENT

Patent Document 1: WO 2016/129684

SUMMARY OF THE INVENTION

PROBLEMS TO BE SOLVED BY THE INVENTION

An object of the present invention is to provide a compound having an excellent control effect on a harmful arthropod.

MEANS FOR SOLVING THE PROBLEMS

The present invention is as described below.

[1] A compound (hereinafter, referred to as a compound P of the present invention) represented by Formula (I),

[Chem. 1]

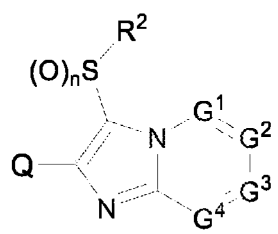

(I)

[wherein

$R^2$ represents a C1-C6 alkyl group that may be substituted with one or more halogen atoms, a cyclopropyl group, or a cyclopropylmethyl group,
n represents 0, 1, or 2,
$G^1$ represents a nitrogen atom or $CR^{3a}$,
$G^2$ represents a nitrogen atom or $CR^{3b}$,
$G^3$ represents a nitrogen atom or $CR^{3c}$,
$G^4$ represents a nitrogen atom or $CR^{3d}$,
$R^{3a}$, $R^{3b}$,$R^{3c}$, and $R^{3d}$ are the same or different and represent a C1-C6 chain hydrocarbon group that may be substituted with one or more substituents selected from a group B, a C3-C7 cycloalkyl group that may be substituted with one or more substituents selected from a group E, a phenyl group that may be substituted with one or more substituents selected from a group H, a 5- or 6-membered aromatic heterocyclic group that may

be substituted with one or more substituents selected from a group H, $OR^{12}$, $NR^{11}R^{12}$, $NR^{11a}R^{12a}$, $NR^{24}NR^{11}R^{12}$, $NR^{24}OR^{11}$, $NR^{11}C(O)R^{13}$, $NR^{24}NR^{11}C(O)R^{13}$, $NR^{11}C(O)OR^{14}$, $NR^{24}NR^{11}C(O)OR^{14}$, $NR^{11}C(O)NR^{31}R^{32}$, $NR^{24}NR^{11}C(O)NR^{31}R^{32}$, $N=CHNR^{31}R^{32}$, $N=S(O)_pR^{15}R^{16}$, $C(O)R^{13}$, $C(O)OR^{17}$, $C(O)NR^{31}R^{32}$, $C(O)NR^{11}S(O)_2R^{23}$, $CR^{30}=NOR^{17}$, $NR^{11}CR^{24}=NOR^{17}$, $S(O)_mR^{23}$, a cyano group, a nitro group, a hydrogen atom, or a halogen atom,

p represents 0 or 1,

m represents 0, 1, or 2,

$R^{30}$ represents a C1-C6 chain hydrocarbon group that may be substituted with one or more halogen atoms, a halogen atom, $OR^{35}$, $NR^{36}R^{37}$, or a hydrogen atom,

$R^{35}$ represents a C1-C6 chain hydrocarbon group that may be substituted with one or more halogen atoms,

$R^{17}$ represents a C1-C6 chain hydrocarbon group that may be substituted with one or more halogen atoms, a phenyl group that may be substituted with one or more substituents selected from a group D, or a hydrogen atom,

$R^{11}$, $R^{24}$, $R^{36}$, and $R^{37}$ are the same or different, and represent a C1-C6 chain hydrocarbon group that may be substituted with one or more halogen atoms, or a hydrogen atom,

$R^{12}$ represents a C1-C6 chain hydrocarbon group that may be substituted with one or more substituents selected from a group F, a C3-C7 cycloalkyl group that may be substituted with one or more substituents selected from a group J, a C3-C7 cycloalkenyl group that may be substituted with one or more substituents selected from a group J, a phenyl group that may be substituted with one or more substituents selected from a group D, a 6-membered aromatic heterocyclic group that may be substituted with one or more substituents selected from a group D, a hydrogen atom, or $S(O)_2R^{23}$,

$R^{23}$ represents a C1-C6 chain hydrocarbon group that may be substituted with one or more halogen atoms, or a phenyl group that may be substituted with one or more substituents selected from a group D,

$R^{11a}$ a and $R^{12a}$, together with the nitrogen atom to which they are attached, form a 3- to 7-membered non-aromatic heterocyclic group that may be substituted with one or more substituents selected from a group E,

$R^{13}$ represents a C1-C6 chain hydrocarbon group that may be substituted with one or more halogen atoms, a C3-C7 cycloalkyl group that may be substituted with one or more halogen atoms, a (C3-C6 cycloalkyl) C1-C3 alkyl group that may be substituted with one or more halogen atoms, a phenyl group that may be substituted with one or more substituents selected from a group D, a 5- or 6-membered aromatic heterocyclic group that may be substituted with one or more substituents selected from a group D, or a hydrogen atom,

$R^{14}$ represents a C1-C6 chain hydrocarbon group that may be substituted with one or more halogen atoms, a C3-C7 cycloalkyl group that may be substituted with one or more halogen atoms, a (C3-C6 cycloalkyl) C1-C3 alkyl group that may be substituted with one or more halogen atoms, or a phenyl C1-C3 alkyl group {a phenyl part in the phenyl C1-C3 alkyl group may be substituted with one or more substituents selected from a group D},

$R^{15}$ and $R^{16}$ are the same or different and represent a C1-C6 alkyl group that may be substituted with one or more halogen atoms,

$R^{31}$ represents a C1-C6 alkyl group that may be substituted with one or more halogen atoms or a hydrogen atom, and

$R^{32}$ represents a C1-C6 chain hydrocarbon group that may be substituted with one or more substituents selected from a group F, a C3-C7 cycloalkyl group that may be substituted with one or more substituents selected from a group J, $S(O)_2R^{23}$, or a hydrogen atom,

Q represents a group represented by Q2, a group represented by Q3, a group represented by Q4, a group represented by Q5, a group represented by Q6, or a group represented by Q7,

[Chem. 2]

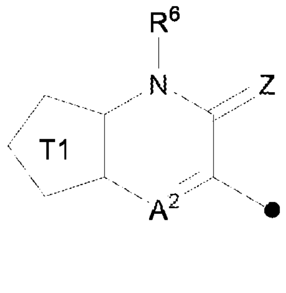

Q2

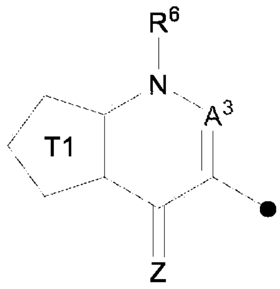

Q3

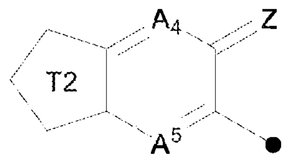

Q4

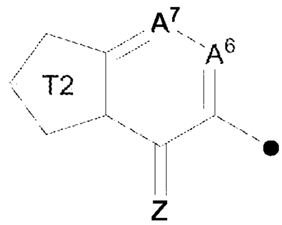

Q5

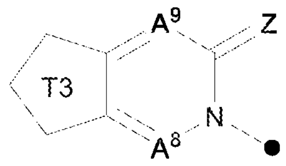

Q6

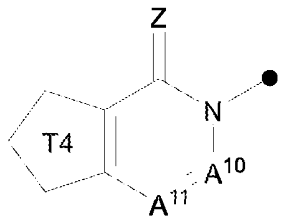

Q7

• represents a site of binding with the rest of molecules,
T1 represents a group represented by T1-1, a group represented by T1-2, or a group represented by T1-3,

[Chem. 3]

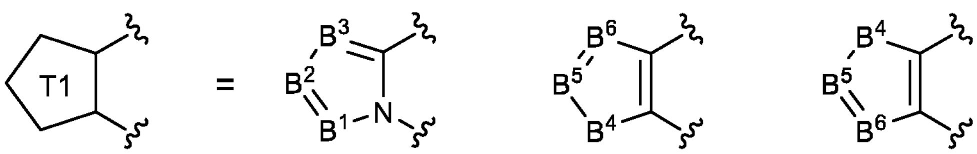

T1-1 T1-2 T1-3

T2 represents a group represented by T2-1, a group represented by T2-2, or a group represented by T2-3,

[Chem. 4]

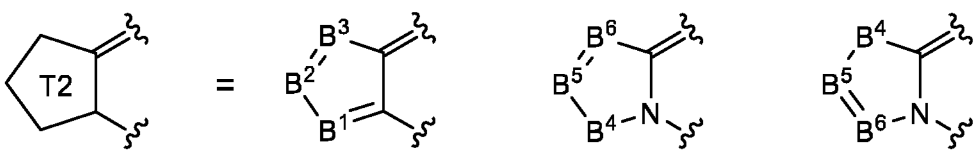

T2-1 T2-2 T2-3

T3 represents a group represented by T3-1 or a group represented by T3-2,

[Chem. 5]

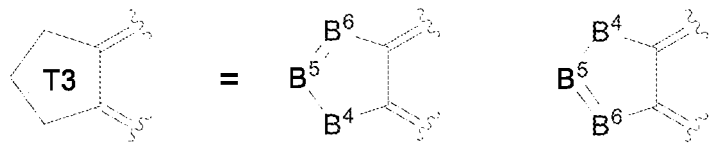

T3-1 T3-2

T4 represents a group represented by T4-1 or a group represented by T4-2,

[Chem. 6]

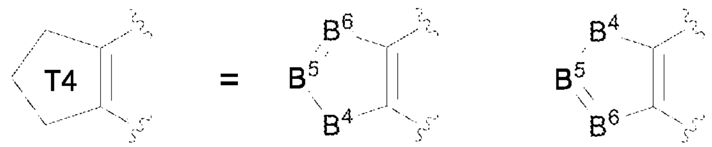

T4-1 T4-2

$A^2$ represents a nitrogen atom or $CR^{4a}$,
$A^3$ represents a nitrogen atom or $CR^{4b}$,
$A^4$ represents a nitrogen atom or $CR^{4c}$,
$A^5$ represents a nitrogen atom or $CR^{4d}$,
$A^6$ represents a nitrogen atom or $CR^{4e}$,
$A^7$ represents a nitrogen atom or $CR^{4f}$,
$A^8$ represents a nitrogen atom or $CR^{4g}$,
$A^9$ represents a nitrogen atom or $CR^{4h}$,
$A^{10}$ represents a nitrogen atom or $CR^{4i}$,
$A^{11}$ represents a nitrogen atom or $CR^{4j}$,
a combination of $B^1$, $B^2$, and $B^3$ represents
a combination in which $B^1$ is $CR^1$, $B^2$ is a nitrogen atom or $CR^{38b}$, and $B^3$ is a nitrogen atom or $CR^{38c}$,
a combination in which $B^1$ is a nitrogen atom or $CR^{38a}$, $B^2$ is $CR^1$, and $B^3$ is a nitrogen atom or $CR^{38c}$, or
a combination in which $B^1$ is a nitrogen atom or $CR^{38a}$, $B^2$ is a nitrogen atom or $CR^{38b}$, and $B^3$ is $CR^1$,
a combination of $B^4$, $B^5$, and $B^6$ represents
a combination in which $B^4$ is an oxygen atom, a sulfur atom, or $NR^5$, $B^5$ is $CR^1$, and $B^6$ is a nitrogen atom or $CR^{38c}$, r
a combination in which $B^4$ is an oxygen atom, a sulfur atom, or $NR^5$, $B^5$ is a nitrogen atom or $CR^{38b}$, and $B^6$ $CR^1$,
Z represents an oxygen atom or a sulfur atom,
$R^1$ represents a C1-C6 chain hydrocarbon group that is substituted with one or more substituents selected from the group consisting of a cyano group and a halogen atom, a C3-C4 cycloalkyl group that may be substituted with one or more substituents selected from the group consisting of a cyano group and a halogen atom, $SR^8$, $S(O)R^8$, $S(O)_2R^8$, $OR^8$, or $OS(O)_2R^8$,
$R^8$ represents a C1-C6 chain hydrocarbon group that is substituted with one or more substituents selected from the group consisting of a cyano group and a halogen atom or a C3-C4 cycloalkyl group that may be substituted with one or more substituents selected from the group consisting of a cyano group and a halogen atom,
$R^{4a}$, $R^{4b}$, $R^{4c}$, $R^{4d}$, $R^{4e}$, $R^{4f}$, $R^{4g}$, $R^{4h}$, $R^{4i}$, and $R^{4j}$ are the same or different and represent a C1-C6 chain hydrocarbon group that may be substituted with one or more halogen atoms, a nitro group, $OR^{18}$, $NR^{18}R^{19}$, a cyano group, an amino group, a halogen atom, or a hydrogen atom,
$R^{18}$ represents a C1-C6 chain hydrocarbon group that may be substituted with one or more halogen atoms,
$R^{19}$ represents a C1-C6 chain hydrocarbon group that may be substituted with one or more halogen atoms or a hydrogen atom,
$R^{38a}$, $R^{38b}$, and $R^{38c}$ are the same or different, and represent a C1-C3 chain hydrocarbon group that may be substituted with one or more halogen atoms, a halogen atom, or a hydrogen atom,
$R^5$ and $R^6$ are the same or different and represent a C1-C6 chain hydrocarbon group that may be substituted

with one or more substituents selected from a group F, a C3-C6 cycloalkyl group that may be substituted with one or more substituents selected from a group J, a phenyl group that may be substituted with one or more substituents selected from a group H, a 5- or 6-membered aromatic heterocyclic group that may be substituted with one or more substituents selected from a group H, or a hydrogen atom,

Group B: a group consisting of a C1-C6 alkoxy group that may be substituted with one or more halogen atoms, a C3-C6 alkenyloxy group that may be substituted with one or more halogen atoms, a C3-C6 alkynyloxy group that may be substituted with one or more halogen atoms, a C1-C6 alkylsulfanyl group that may be substituted with one or more halogen atoms, a C1-C6 alkylsulfinyl group that may be substituted with one or more halogen atoms, a C1-C6 alkylsulfonyl that may be substituted with one or more halogen atoms, a C3-C6 cycloalkyl group that may be substituted with one or more halogen atoms, a cyano group, a hydroxy group, and a halogen atom.

Group C: a group consisting of a C1-C6 chain hydrocarbon groups that may be substituted with one or more halogen atoms, a C1-C6 alkoxy group that may be substituted with one or more halogen atoms, a C3-C6 alkenyloxy group that may be substituted with one or more halogen atoms, a C3-C6 alkynyloxy group that may be substituted with one or more halogen atoms, and a halogen atom.

Group D: a group consisting of a C1-C6 chain hydrocarbon group that may be substituted with one or more halogen atoms, a hydroxy group, a C1-C6 alkoxy group that may be substituted with one or more halogen atoms, a C3-C6 alkenyloxy group that may be substituted with one or more halogen atoms, a C3-C6 alkynyloxy group that may be substituted with one or more halogen atoms, a sulfanyl group, a C1-C6 alkylsulfanyl group that may be substituted with one or more halogen atoms, a C1-C6 alkylsulfinyl group that may be substituted with one or more halogen atoms, a C1-C6 alkylsulfonyl that may be substituted with one or more halogen atoms, an amino group, $NHR^{21}$, $NR^{21}R^{22}$, $C(O)R^{21}$, $OC(O)R^{21}$, $C(O)OR^{21}$, a cyano group, a nitro group, and a halogen atom.

$R^{21}$ and $R^{22}$ are the same or different and represent a C1-C6 alkyl group that may be substituted with one or more halogen atoms.

Group E: a group consisting of a C1-C6 chain hydrocarbon groups that may be substituted with one or more halogen atoms, a C1-C6 alkoxy group that may be substituted with one or more halogen atoms, a C3-C6 alkenyloxy group that may be substituted with one or more halogen atoms, a C3-C6 alkynyloxy group that may be substituted with one or more halogen atoms, a halogen atom, an oxo group, a hydroxy group, a cyano group, and a nitro group.

Group F: a group consisting of a C1-C6 alkoxy group that may be substituted with one or more halogen atoms, a phenyl group that may be substituted with one or more substituents selected from a group D, a 5- or 6-membered aromatic heterocyclic group that may be substituted with one or more substituents selected from a group D, a C3-C7 cycloalkyl group that may be substituted with one or more halogen atoms, a 3- to 7-membered non-aromatic heterocyclic group that may be substituted with one or more substituents selected from a group C, an amino group, $NHR^{21}$, $NR^{21}R^{22}$, a halogen atom, and a cyano group.

Group H: a group consisting of a C1-C6 alkyl group that may be substituted with one or more halogen atoms, 5-or 6-membered aromatic heterocyclic group that may be substituted with one or more substituents selected from a group D, $OR^{10}$, $NR^9R^{10}$, $C(O)R^{10}$, $C(O)NR^9R^{18}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, and an amino group.

$R^9$ represents a C1-C6 alkyl group that may be substituted with one or more halogen atoms or a C3-C6 cycloalkyl group that may be substituted with one or more halogen atoms,

$R^{10}$ represents a C1-C6 alkyl group that may be substituted with one or more halogen atoms, a C3-C6 cycloalkyl group that may be substituted with one or more halogen atoms, or a hydrogen atom.

Group J: a group consisting of C1-C6 alkyl group that may be substituted with one or more halogen atoms, a halogen atom, and a cyano group.]

or an N-oxide thereof (hereinafter, the compound represented by Formula (I) and the N-oxide thereof is referred to as a compound X of the present invention).

[2] The compound or the N-oxide thereof according to [1], wherein Q represents a group represented by Q2, a group represented by Q3, a group represented by Q6, or a group represented by Q7,

$A^2$ is $CR^{4a}$, $A^3$ is $CR^{4b}$, $A^8$ is $CR^{4g}$, $A^9$ is a nitrogen atom or $CR^{4h}$, $A^{10}$ is $CR^{4i}$, and $A^{11}$ is a nitrogen atom or $CR^{4j}$, $B^1$ is $CR^{38a}$, $B^2$ is $CR^1$, $B^3$ is a nitrogen atom, $B^4$ is $NR^5$, $B^5$ is $CR^1$, and $B^6$ is a nitrogen atom.

[3] The compound or the N-oxide thereof according to [1], wherein Q represents a group represented by Q2 or a group represented by Q3.

[4] The compound or the N-oxide thereof according to [3], wherein $A^2$ is $CR^{4a}$ and $A^3$ is $CR^{4b}$,
$B^1$ is $CR^{38a}$, $B^2$ is $CR^1$, $B^3$ is a nitrogen atom, $B^4$ is $NR^5$, $B^5$ is $CR^1$, and $B^6$ is a nitrogen atom.

[5] The compound or the N-oxide thereof according to any one of [1] to [4], wherein $R^1$ represents a C1-C6 chain hydrocarbon group that is substituted with one or more halogen atoms,

$R^{4a}$, $R^{4b}$, $R^{4c}$, $R^{4d}$, $R^{4e}$, $R^{4f}$, $R^{4g}$, $R^{4h}$, $R^{4i}$, and $R^{4j}$ are the same or different and are a C1-C6 chain hydrocarbon group that may be substituted with one or more halogen atoms, a halogen atom, or a hydrogen atom, and
$R^5$ and $R^6$ are the same or different, and are a C1-C6 chain hydrocarbon group that may be substituted with one or more halogen atoms, or a hydrogen atom.

[6] The compound (hereinafter, referred to as a compound N of the present invention) or the N oxide thereof (hereinafter, the compound represented by Formula (I) or the N oxide thereof is referred to as a compound N of the present invention) according to [1], wherein Q is a group represented by Q1,

[Chem. 7]

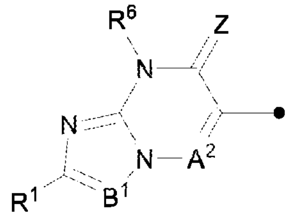

Q1

a combination of $A^2$ and $B^1$ represents
a combination in which $A^2$ is a nitrogen atom and $B^1$ is $CR^{38}$, or
a combination in which $A^2$ is $CR^{4a}$ and $B^1$ is a nitrogen atom or $CR^{38}$,
$R^{38}$ represents a C1-C3 chain hydrocarbon group that may be substituted with one or more halogen atoms, a halogen atom or a hydrogen atom, and
$R^6$ represents a C1-C6 chain hydrocarbon group that may be substituted with one or more substituents selected from a group F, a C3-C6 cycloalkyl group that may be substituted with one or more substituents selected from a group J, a phenyl group that may be substituted with one or more substituents selected from a group H, or a 5- or 6-membered aromatic heterocyclic group that may be substituted with one or more substituents selected from a group H.

[7] The compound or the N-oxide thereof according to any one of [1] to [6], wherein $G^1$ is CH, $G^2$ is $CR^{3b}$, $G^3$ is $CR^{3c}$, or $G^4$ is CH.
[8] The compound or the N-oxide thereof according to any one of [1] to [7], wherein $R^2$ is an ethyl group.
[9] The compound or the N-oxide thereof according to any one of [1] to [8], wherein Z is an oxygen atom.
[10] A harmful arthropod-controlling composition containing the compound and the N-oxide thereof according to any one of [1] to [9].
[11] A composition containing one or more ingredients selected from the group consisting of a group (a), a group (b), a group (c), and a group (d), and the compound or the N-oxide thereof according to any one of [1] to [9]:

Group (a): a group consisting of an insecticidal active ingredient, an acaricidal active ingredient, and a nematicidal active ingredient;
Group (b): a bactericidal active ingredient;
Group (c): a plant growth regulating ingredient; and
Group (d): a repellent ingredient.

[12] A method for controlling a harmful arthropod including: applying an effective amount of the compound or the N-oxide thereof according to any one of [1] to [9] or an effective amount of the composition according to [11] to a harmful arthropod or a habitat of the harmful arthropod.
[13] A seed or a vegetative reproductive organ retaining an effective amount of the compound or the N-oxide thereof according to any one of [1] to [9] or an effective amount of the composition according to [11].

EFFECT OF THE INVENTION

According to the present invention, the harmful arthropod can be controlled.

MODE FOR CARRYING OUT THE INVENTION

Substituents in the present invention will be described below.

A halogen atom means a fluorine atom, a chlorine atom, a bromine atom, or an iodine atom.

When the substituent is substituted with two or more halogen atoms or substituents, the halogen atoms or substituents may be the same or different.

The notation of "CX-CY" in the present specification means that the number of carbon atoms is X to Y. For example, the notation of "C1-C6" means that the number of carbon atoms is 1 to 6.

The chain hydrocarbon group represents an alkyl group, an alkenyl group, or an alkynyl group.

Examples of the alkyl group include a methyl group, an ethyl group, a propyl group, an isopropyl group, a 1,1-dimethylpropyl group, a 1,2-dimethylpropyl group, a 1-ethylpropyl group, a butyl group, an a sec-butyl group, a tert-butyl group, a pentyl group, and a hexyl group.

Examples of the alkenyl group include a vinyl group, a 1-propenyl group, a 2-propenyl group, a 1-methy-1-propenyl group, a 1-methy-2-propenyl group, a 1,2-dimethy-1-propenyl group, a 1-ethyl-2-propenyl group, a 3-butenyl group, a 4-pentenyl group, and a 5-hexenyl group.

Examples of the alkynyl group include an ethynyl group, a 1-propynyl group, a 2-propynyl group, a 1-methyl-2-propynyl group, a 1,1-dimethyl-2-propynyl group, a 1-ethyl-2-propynyl group, a 2-butynyl group, a 4-pentynyl group, and a 5-hexynyl group.

Examples of the alkoxy group include a methoxy group, an ethoxy group, a propoxy group, an isopropoxy group, a butoxy group, a tert-butoxy group, a pentyloxy group, and a hexyloxy group.

Examples of the alkenyloxy group include a 2-propenyloxy group, a 2-butenyloxy group, and a 5-hexenyloxy group.

Examples of the alkynyloxy group include a 2-propynyloxy group, a 2-butynyloxy group, and a 5-hexynyloxy group.

Examples of the perfluoroalkyl group include a trifluoromethyl group, a perfluoroethyl group, a perfluoropropyl group, and a perfluoroisopropyl group.

Examples of the alkylsulfanyl group include a methylsulfanyl group, an ethylsulfanyl group, a propylsulfanyl group, and an isopropylsulfanyl group.

Examples of the alkylsulfinyl group include a methylsulfinyl group, an ethylsulfinyl group, a propylsulfinyl group, and an isopropylsulfinyl group.

Examples of the alkylsulfonyl group include a methylsulfonyl group, an ethylsulfonyl group, a propylsulfonyl group, and an isopropylsulfonyl group.

Examples of the cycloalkyl group include a cyclopropyl group, a cyclobutyl group, a cyclopentyl group, a cyclohexyl group, and a cycloheptyl group.

Examples of the cycloalkenyl group include a cyclopropenyl group, a cyclobutenyl group, a cyclopentenyl group, a cyclohexenyl group, and a cycloheptenyl group.

The 3- to 7-membered non-aromatic heterocyclic group represents an aziridine ring, an azetidine ring, a pyrrolidine ring, an imidazoline ring, an imidazolidine ring, a piperidine ring, a tetrahydropyrimidine ring, a hexahydropyrimidine ring, a piperazine ring, an azepane ring, an oxazolidine ring, an isoxazolidine ring, a 1,3-oxazinane ring, a morpholine ring, a 1,4-oxazepane ring, a thiazolidine ring, an isothiazolidine ring, a 1,3-thiazinane ring, a thiomorpholine ring, or a 1,4-thiazepane ring.

Examples of the 3- to 7-membered non-aromatic heterocyclic group that may be substituted with one or more substituents selected from a group E include the groups described as follows.

[Chem. 8]

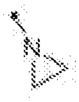
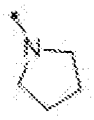
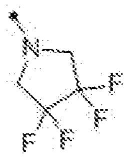
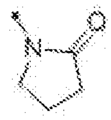
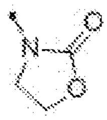
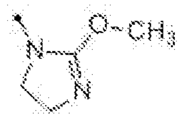
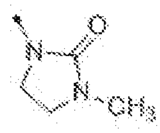
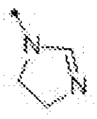
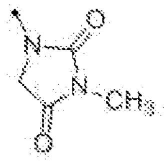
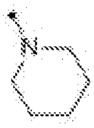
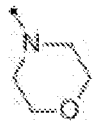
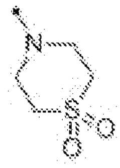

Examples of the 5- or 6-membered aromatic heterocyclic group represents a 5-membered aromatic heterocyclic group or a 6-membered aromatic heterocyclic group. Examples of the 5-membered aromatic heterocyclic groups include a pyrrolyl group, a furyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, and a thiadiazolyl group. The 6-membered aromatic heterocyclic group represents a pyridyl group, a pyridazinyl group, a pyrimidinyl group, a pyrazinyl group, a triazinyl group, or a tetrazinyl group.

Examples of the (C3-C6 cycloalkyl) C1-C3 alkyl group that may be substituted with one or more halogen atoms include a cyclopropylmethyl group, a (2-fluorocyclopropyl)methyl group, a cyclopropyl(fluoro)methyl group, and (2-fluorocyclopropyl)(fluoro)methyl group.

Examples of the phenyl C1-C3 alkyl group {a phenyl part in the phenyl C1-C3 alkyl group may be substituted with one or more substituents selected from a group D} include a benzyl group, a 2-fluorobenzyl group, a 4-chlorobenzyl group, a 4-(trifluoromethyl)benzyl group, and a 2-[4-(trifluoromethyl)phenyl]ethyl group

Q in the present invention will be described below.

In Q, for example, a group represented by Q2 in which T1 is T1-1 is a group represented by the following Q2-1.

[Chem. 9]

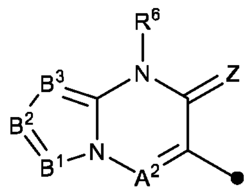

Q2-1

Examples of Q2 include a group represented by Q2-1, a group represented by Q2-2, and a group represented by Q2-3,

[Chem. 10]

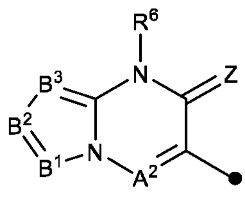
Q2-1

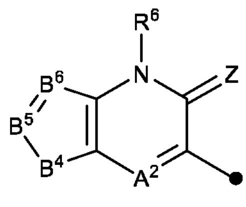
Q2-2

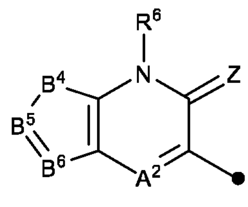
Q2-3

In Q, for example, a group represented by Q3 in which T1 is T1-1 is a group represented by the following Q3-1.

[Chem. 11]

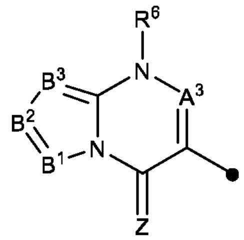

Q3-1

Examples of Q3 include a group represented by Q3-1, a group represented by Q3-2, and a group represented by Q3-3,

[Chem. 12]

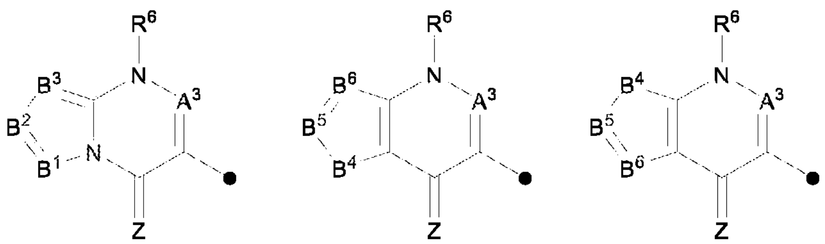

Q3-1 Q3-2 Q3-3

In Q, for example, a group represented by Q7 in which T4 is T4-1 is a group represented by the following Q7-1.

[Chem. 13]

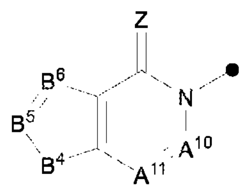

Q7-1

Examples of Q7 include a group represented by Q7-1 and a group represented by Q7-2.

[Chem. 14]

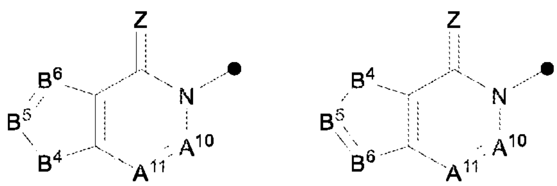

Q7-1 Q7-2

Examples of the N-oxide of the compound represented by Formula (I) include the compounds represented by the following formula:

[Chem. 15]

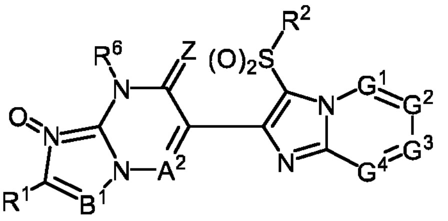

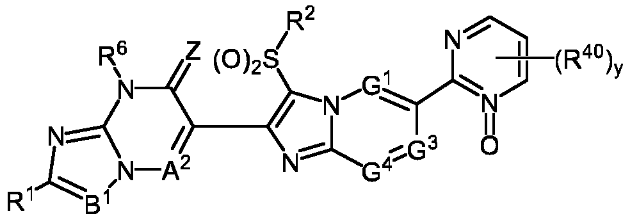

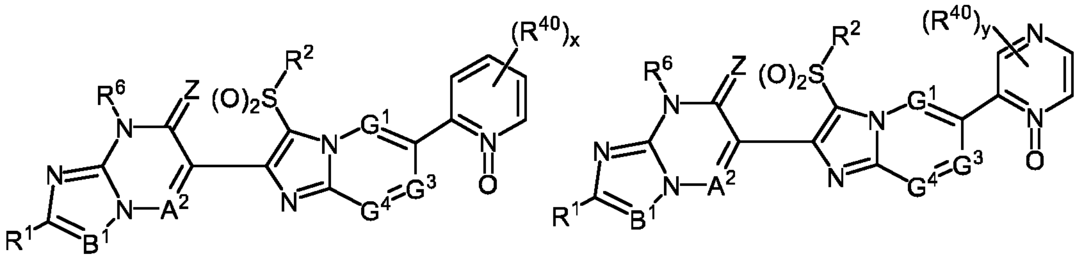

[wherein $R^{40}$ represents any substituent selected from a group H, x represents 0, 1, 2, 3, or 4, y represents 0, 1, 2, or 3, and other symbols represent the same meaning as described above.]

The compound X of the present invention may have one or more stereoisomers. Examples of the stereoisomer include an enantiomer, a diastereomer, and a geometric isomer. The compound X of present invention includes each stereoisomer and a stereoisomer mixture in an optional ratio.

The compound X of the present invention may form an acid addition salt. Examples of the acid forming the acid addition salt include inorganic acids such as hydrogen chloride, phosphoric acid, and sulfuric acid, and organic acids such as acetic acid, trifluoroacetic acid, benzoic acid, and p-toluenesulfonic acid. The acid addition salt is obtained by mixing the compound X of the present invention with acid.

Examples of the embodiments of the compound N of the present invention include the following compounds.

[Embodiment 1] In the compound N of the present invention, a compound in which $G^1$, $G^3$, and $G^4$ are CHs, $G^2$ is $CR^{3b}$, and $R^{3b}$ is a halogen atom or $CF_3$.

[Embodiment 2] In the compound N of the present invention, a compound in which $G^1$, $G^2$, and $G^4$ are CHs, $G^3$ is $CR^{3c}$, and $R^{3c}$ is a halogen atom or $CF_3$.

[Embodiment 3] In the compound N of the present invention, a compound in which $R^1$ is a C1-C3 chain hydrocarbon group that is substituted with one or more halogen atoms.

[Embodiment 4] In the compound N of the present invention, a compound in which $R^1$ is a C1-C3 perfluoroalkyl group.

[Embodiment 5] In Embodiment 1, a compound in which $R^1$ is a C1-C3 chain hydrocarbon group that is substituted with one or more halogen atoms.

[Embodiment 6] In Embodiment 2, a compound in which $R^1$ is a C1-C3 perfluoroalkyl group.

[Embodiment 7] In Embodiment 1, a compound in which $R^2$ is a C1-C6 alkyl group, and n is 2.

[Embodiment 8] In Embodiment 2, a compound in which $R^2$ is a C1-C6 alkyl group, and n is 2.

[Embodiment 9] In Embodiment 3, a compound in which $R^2$ is a C1-C6 alkyl group, and n is 2.

[Embodiment 10] In Embodiment 4, a compound in which $R^2$ is a C1-C6 alkyl group, and n is 2.

[Embodiment 11] In Embodiment 5, a compound in which $R^2$ is a C1-C6 alkyl group, and n is 2.

[Embodiment 12] In Embodiment 6, a compound in which $R^2$ is a C1-C6 alkyl group, and n is 2.

[Embodiment 13] In Embodiment 1, a compound in which $R^2$ is an ethyl group, and n is 2.

[Embodiment 14] In Embodiment 2, a compound in which $R^2$ is an ethyl group, and n is 2.

[Embodiment 15] In Embodiment 3, a compound in which $R^2$ is an ethyl group, and n is 2.

[Embodiment 16] In Embodiment 4, a compound in which $R^2$ is an ethyl group, and n is 2.

[Embodiment 17] In Embodiment 5, a compound in which $R^2$ is an ethyl group, and n is 2.

[Embodiment 18] In Embodiment 6, a compound in which $R^2$ is an ethyl group, and n is 2.

[Embodiment 19] In any of Embodiment 1 to Embodiment 18 and compound N of the present invention, a compound in which $A^2$ is $CR^{4a}$, $B^1$ is $CR^{38}$, Z is an oxygen atom, $R^6$ is a methyl group, an ethyl group, or a cyclopropyl group.

[Embodiment 20] In any of Embodiment 1 to Embodiment 18 and compound N of the present invention, a compound in which $A^2$ and $B^1$ is CH, Z is an oxygen atom, $R^6$ is a methyl group, an ethyl group, or a cyclopropyl group.

[Embodiment 21] In any of Embodiment 1 to Embodiment 18 and compound N of the present invention, a

compound in which $A^2$ is $CR^{4a}$, $B^1$ is $CR^{38}$, Z is an oxygen atom, and $R^6$ is a methyl group.

[Embodiment 22] In any of Embodiment 1 to Embodiment 18 and compound N of the present invention, a compound in which $A^2$ and $B^1$ are CHs, Z is an oxygen atom, and $R^6$ is a methyl group.

[Embodiment 23] In the compound N of the present invention, a compound in which $G^1$, $G^3$, and $G^4$ are CHs, $G^2$ is $CR^{3b}$, $R^{3b}$ is a halogen atom, $R^1$ is a C1-C3 perfluoroalkyl group, $R^2$ is an ethyl group, n is 2, $A^2$ and $B^1$ are CHs, Z is an oxygen atom, and $R^6$ is a methyl group.

Examples of the embodiments of the compound P of the present invention include the following compounds.

[Embodiment P1] In the compound P of the present invention, a compound in which $R^{3a}$ and $R^{3d}$ are hydrogen atom, $R^{3b}$ and $R^{3c}$ are the same or different, and are a C1-C6 alkyl group that may be substituted with one or more halogen atoms, a C3-C7 cycloalkyl group, a phenyl group, a pyridyl group {the phenyl group and the pyridyl group may be substituted with one or more substituents selected from a group J}, a hydrogen atom, or a halogen atom.

[Embodiment P2] In the compound P of the present invention, a compound in which $R^{3a}$ and $R^{3d}$ are hydrogen atom, $R^{3b}$ and $R^{3c}$ are the same or different, and are a C1-C3 alkyl group that may be substituted with one or more halogen atoms, a cyclopropyl group, a hydrogen atom, or a halogen atom.

[Embodiment P3] In the compound P of the present invention, a compound in which $G^1$ and $G^4$ are CHs, $G^2$ is $CR^{3b}$, and $G^3$ is $CR^{3c}$.

[Embodiment P4] In Embodiment P1, a compound in which $G^1$ and $G^4$ are CHs, $G^2$ is $CR^{3b}$, and $G^3$ is $CR^{3c}$.

[Embodiment P5] In Embodiment P2, a compound in which $G^1$ and $G^4$ are CHs, $G^2$ is $CR^{3b}$, and $G^3$ is $CR^{3c}$.

[Embodiment P6] In the compound P of the present invention, a compound in which $R^1$ is a C1-C6 alkyl group that is substituted with one or more halogen atoms.

[Embodiment P7] In the compound P of the present invention, a compound in which $R^1$ is a C1-C3 alkyl group that is substituted with one or more fluorine atoms.

[Embodiment P8] In Embodiment P1, a compound in which $R^1$ is a C1-C6 alkyl group that is substituted with one or more halogen atoms.

[Embodiment P9] In Embodiment P2, a compound in which $R^1$ is a C1-C6 alkyl group that is substituted with one or more halogen atoms.

[Embodiment P10] In Embodiment P3, a compound in which $R^1$ is a C1-C6 alkyl group that is substituted with one or more halogen atoms.

[Embodiment P11] In Embodiment P4, a compound in which $R^1$ is a C1-C6 alkyl group that is substituted with one or more halogen atoms.

[Embodiment P12] In Embodiment P5, a compound in which $R^1$ is a C1-C6 alkyl group that is substituted with one or more halogen atoms.

[Embodiment P13] In Embodiment P1, a compound in which $R^1$ is a C1-C3 alkyl group that is substituted with one or more fluorine atoms.

[Embodiment P14] In Embodiment P2, a compound in which $R^1$ is a C1-C3 alkyl group that is substituted with one or more fluorine atoms.

[Embodiment P15] In Embodiment P3, a compound in which $R^1$ is a C1-C3 alkyl group that is substituted with one or more fluorine atoms.

[Embodiment P16] In Embodiment P4, a compound in which $R^1$ is a C1-C3 alkyl group that is substituted with one or more fluorine atoms.

[Embodiment P17] In Embodiment P5, a compound in which $R^1$ is a C1-C3 alkyl group that is substituted with one or more fluorine atoms.

[Embodiment P18] In the compound P of the present invention, a compound in which $R^2$ is a C1-C6 alkyl group.

[Embodiment P19] In Embodiment P1, a compound in which $R^2$ is a C1-C6 alkyl group.

[Embodiment P20] In Embodiment P2, a compound in which $R^2$ is a C1-C6 alkyl group.

[Embodiment P21] In Embodiment P3, a compound in which $R^2$ is a C1-C6 alkyl group.

[Embodiment P22] In Embodiment P4, a compound in which $R^2$ is a C1-C6 alkyl group.

[Embodiment P23] In Embodiment P5, a compound in which $R^2$ is a C1-C6 alkyl group.

[Embodiment P24] In Embodiment P6, a compound in which $R^2$ is a C1-C6 alkyl group.

[Embodiment P25] In Embodiment P7, a compound in which $R^2$ is a C1-C6 alkyl group.

[Embodiment P26] In Embodiment P8, a compound in which $R^2$ is a C1-C6 alkyl group.

[Embodiment P27] In Embodiment P9, a compound in which $R^2$ is a C1-C6 alkyl group.

[Embodiment P28] In Embodiment P10, a compound in which $R^2$ is a C1-C6 alkyl group.

[Embodiment P29] In Embodiment P11, a compound in which $R^2$ is a C1-C6 alkyl group.

[Embodiment P30] In Embodiment P12, a compound in which $R^2$ is a C1-C6 alkyl group.

[Embodiment P31] In Embodiment P13, a compound in which $R^2$ is a C1-C6 alkyl group.

[Embodiment P32] In Embodiment P14, a compound in which $R^2$ is a C1-C6 alkyl group.

[Embodiment P33] In Embodiment P15, a compound in which $R^2$ is a C1-C6 alkyl group.

[Embodiment P34] In Embodiment P16, a compound in which $R^2$ is a C1-C6 alkyl group.
[Embodiment P35] In Embodiment P17, a compound in which $R^2$ is a C1-C6 alkyl group.
[Embodiment P36] In the compound P of the present invention, a compound in which n is 2.
[Embodiment P37] In Embodiment P1, a compound in which $R^2$ is a C1-C6 alkyl group and n is 2.
[Embodiment P38] In Embodiment P2, a compound in which $R^2$ is a C1-C6 alkyl group and n is 2.
[Embodiment P39] In Embodiment P3, a compound in which $R^2$ is a C1-C6 alkyl group and n is 2.
[Embodiment P40] In Embodiment P4, a compound in which $R^2$ is a C1-C6 alkyl group and n is 2.
[Embodiment P41] In Embodiment P5, a compound in which $R^2$ is a C1-C6 alkyl group and n is 2.
[Embodiment P42] In Embodiment P6, a compound in which $R^2$ is a C1-C6 alkyl group and n is 2.
[Embodiment P43] In Embodiment P7, a compound in which $R^2$ is a C1-C6 alkyl group and n is 2.
[Embodiment P44] In Embodiment P8, a compound in which $R^2$ is a C1-C6 alkyl group and n is 2.
[Embodiment P45] In Embodiment P9, a compound in which $R^2$ is a C1-C6 alkyl group and n is 2.
[Embodiment P46] In Embodiment P10, a compound in which $R^2$ is a C1-C6 alkyl group and n is 2.
[Embodiment P47] In Embodiment P11, a compound in which $R^2$ is a C1-C6 alkyl group and n is 2.
[Embodiment P48] In Embodiment P12, a compound in which $R^2$ is a C1-C6 alkyl group and n is 2.
[Embodiment P49] In Embodiment P13, a compound in which $R^2$ is a C1-C6 alkyl group and n is 2.
[Embodiment P50] In Embodiment P14, a compound in which $R^2$ is a C1-C6 alkyl group and n is 2.
[Embodiment P51] In Embodiment P15, a compound in which $R^2$ is a C1-C6 alkyl group and n is 2.
[Embodiment P52] In Embodiment P16, a compound in which $R^2$ is a C1-C6 alkyl group and n is 2.
[Embodiment P53] In Embodiment P17, a compound in which $R^2$ is a C1-C6 alkyl group and n is 2.
[Embodiment P54] In the compound P of the present invention, a compound in which $R^2$ is an ethyl group.
[Embodiment P55] In Embodiment P1, a compound in which $R^2$ is an ethyl group and n is 2.
[Embodiment P56] In Embodiment P2, a compound in which $R^2$ is an ethyl group and n is 2.
[Embodiment P57] In Embodiment P3, a compound in which $R^2$ is an ethyl group and n is 2.
[Embodiment P58] In Embodiment P4, a compound in which $R^2$ is an ethyl group and n is 2.
[Embodiment P59] In Embodiment P5, a compound in which $R^2$ is an ethyl group and n is 2.
[Embodiment P60] In Embodiment P6, a compound in which $R^2$ is an ethyl group and n is 2.
[Embodiment P61] In Embodiment P7, a compound in which $R^2$ is an ethyl group and n is 2.
[Embodiment P62] In Embodiment P8, a compound in which $R^2$ is an ethyl group and n is 2.
[Embodiment P63] In Embodiment P9, a compound in which $R^2$ is an ethyl group and n is 2.
[Embodiment P64] In Embodiment P10, a compound in which $R^2$ is an ethyl group and n is 2.
[Embodiment P65] In Embodiment P11, a compound in which $R^2$ is an ethyl group and n is 2.
[Embodiment P66] In Embodiment P12, a compound in which $R^2$ is an ethyl group and n is 2.
[Embodiment P67] In Embodiment P13, a compound in which $R^2$ is an ethyl group and n is 2.
[Embodiment P68] In Embodiment P14, a compound in which $R^2$ is an ethyl group and n is 2.
[Embodiment P69] In Embodiment P15, a compound in which $R^2$ is an ethyl group and n is 2.
[Embodiment P70] In Embodiment P16, a compound in which $R^2$ is an ethyl group and n is 2.
[Embodiment P71] In Embodiment P17, a compound in which $R^2$ is an ethyl group and n is 2.

[Embodiment P72] In any one of Embodiment P1 to Embodiment P71 and the compound P of the present invention, Q is a group represented by Q2, a group represented by Q3, a group represented by Q6, or a group represented by Q7, $B^1$ is a nitrogen atom or $CR^{38a}$, $B^2$ is $CR^1$, $B^3$ is a nitrogen atom, $B^4$ is $NCH_3$, $B^5$ is $CR^1$, $B^6$ is a nitrogen atom, $R^{4a}$, $R^{4b}$, $R^{4g}$, $R^{4h}$, $R^{4i}$, and $R^{4j}$ are the same or different and are a C1-C6 alkyl group that may be substituted with one or more halogen atoms or a hydrogen atom, $R^6$ is a C1-C6 alkyl group or a hydrogen atom, and Z is an oxygen atom.

[Embodiment P73] In any one of Embodiment P1 to Embodiment P71 and the compound P of the present invention, Q is a group represented by Q2, a group represented by Q3, a group represented by Q6, or a group represented by Q7, $A^2$, $A^3$, $A^8$, $A^9$, $A^{10}$, and $A^{11}$ are CHs, $B^1$ is a nitrogen atom or CH, $B^2$ is $CR^1$, $B^3$ is a nitrogen atom, $B^4$ is $NCH_3$, $B^5$ is $CR^1$, $B^6$ is a nitrogen atom, $R^6$ is a C1-C6 alkyl group or a hydrogen atom, and Z is an oxygen atom.

[Embodiment P74] In any one of Embodiment P1 to Embodiment P71 and the compound P of the present invention, a compound in which Q is a group represented by Q2, a group represented by Q3, a group represented by Q6, or a group represented by Q7, T1 is a group represented by T1-1, T3 is a group represented by T3-1, and T4 is a group represented by T4-1.

[Embodiment P75] In any one of Embodiment P1 to Embodiment P71 and the compound P of the present invention, Q is a group represented by Q2, a group represented by Q3, a group represented by Q6, or a group represented by Q7, T1 is a group represented by T1-1, T3 is a group represented by T3-1, and T4 is a group represented by T4-1, $B^1$ is a nitrogen atom or $CR^{38a}$, $B^2$ is $CR^1$, $B^3$ is a nitrogen atom, $B^4$ is $NCH_3$, $B^5$ is $CR^1$, $B^6$ is a nitrogen atom, $R^{4a}$, $R^{4b}$, $R^{4g}$, $R^{4h}$, $R^{4i}$, and $R^{4j}$ are the same or different and are a C1-C6 alkyl group that may be substituted with one or more halogen atoms or a hydrogen atom, $R^6$ is a C1-C6 alkyl group or a hydrogen atom, and Z is an oxygen atom.

[Embodiment P76] In any one of Embodiment P1 to Embodiment P71 and the compound P of the present

invention, Q is a group represented by Q2, a group represented by Q3, a group represented by Q6, or a group represented by Q7, T1 is a group represented by T1-1, T3 is a group represented by T3-1, and T4 is a group represented by T4-1, $A^2$ is $CR^{4a}$, $A^3$ is $CR^{4b}$, $A^8$ is $CR^{4g}$, $A^9$ is $CR^{4h}$, $A^{10}$ is $CR^{4i}$, and $A^{11}$ is $CR^{4j}$, $B^1$ is a nitrogen atom or $CR^{38a}$, $B^2$ is $CR^1$, $B^3$ is a nitrogen atom, $B^4$ is $NCH_3$, $B^5$ is $CR^1$, $B^6$ is a nitrogen atom, $R^{4a}$, $R^{4b}$, $R^{4g}$, $R^{4h}$, $R^{4i}$, and $R^{4j}$ are the same or different and are a C1-C6 alkyl group that may be substituted with one or more halogen atoms or a hydrogen atom, $R^6$ is a C1-C6 alkyl group or a hydrogen atom, and Z is an oxygen atom.

[Embodiment P77] In any one of Embodiment P1 to Embodiment P71 and the compound P of the present invention, Q is a group represented by Q2, a group represented by Q3, a group represented by Q6, or a group represented by Q7, T1 is a group represented by T1-1, T3 is a group represented by T3-1, and T4 is a group represented by T4-1, $A^2$, $A^3$, $A^8$, $A^9$, $A^{10}$, and $A^{11}$ are CHs, $B^1$ is a nitrogen atom or CH, $B^2$ is $CR^1$, $B^3$ is a nitrogen atom, $B^4$ is $NCH_3$, $B^5$ is $CR^1$, $B^6$ is a nitrogen atom, $R^6$ is a C1-C6 alkyl group or a hydrogen atom, and Z is an oxygen atom.

[Embodiment P78] In any one of Embodiment P1 to Embodiment P71 and the compound P of the present invention, a compound in which Q is a group represented by Q2, or a group represented by Q3, and T1 is a group represented by T1-1.

[Embodiment P79] In any one of Embodiment P1 to Embodiment P71 and the compound P of the present invention, Q is a group represented by Q2, or a group represented by Q3, T1 is a group represented by T1-1, $B^1$ is a nitrogen atom or $CR^{38a}$, $B^2$ is $CR^1$, $B^3$ is a nitrogen atom, $R^{4a}$ and $R^{4b}$ are the same or different and are a C1-C6 alkyl group that may be substituted with one or more halogen atoms or a hydrogen atom, $R^6$ is a C1-C6 alkyl group or a hydrogen atom, and Z is an oxygen atom.

[Embodiment P80] In Embodiment P79, a compound in which $R^{38a}$ is a hydrogen atom.

[Embodiment P81] In any one of Embodiment P1 to Embodiment P71 and the compound P of the present invention, Q is a group represented by Q2, or a group represented by Q3, T1 is a group represented by T1-1, $A^2$ is $CR^{4a}$, $A^3$ is $CR^{4b}$, $B^1$ is a nitrogen atom or CH, $B^2$ is $CR^1$ or CH, $B^3$ is a nitrogen atom, $R^{4a}$ and $R^{4b}$ are the same or different and are a C1-C6 alkyl group that may be substituted with one or more halogen atoms or a hydrogen atom, $R^6$ is a C1-C3 alkyl group or a hydrogen atom, and Z is an oxygen atom.

[Embodiment P82] In any one of Embodiment P1 to Embodiment P71 and the compound P of the present invention, Q is a group represented by Q2, or a group represented by Q3, $A^2$ and $A^3$ are CHs, $B^1$ is a nitrogen atom or CH, $B^2$ is $CR^1$, $B^3$ is a nitrogen atom, $R^6$ is a C1-C3 alkyl group or a hydrogen atom, and Z is an oxygen atom.

[Embodiment P83] In any one of Embodiment P1 to Embodiment P71 and the compound P of the present invention, a compound in which Q is a group represented by Q3, T1 is a group represented by T1-1.

[Embodiment P84] In any one of Embodiment P1 to Embodiment P71 and the compound P of the present invention, Q is a group represented by Q3, T1 is a group represented by T1-1, $B^1$ is a nitrogen atom or $CR^{38a}$, $B^2$ is $CR^1$, $B^3$ is a nitrogen atom, $R^{4b}$ is a C1-C6 alkyl group that may be substituted with one or more halogen atoms or a hydrogen atom, $R^6$ is a C1-C6 alkyl group or a hydrogen atom, and Z is an oxygen atom.

[Embodiment P85] In Embodiment P84, a compound in which $R^{38a}$ is a hydrogen atom.

[Embodiment P86] In any one of Embodiment P1 to Embodiment P71 and the compound P of the present invention, Q is a group represented by Q3, T1 is a group represented by T1-1, $A^3$ is $CR^{4b}$, $B^1$ is a nitrogen atom or CH, $B^2$ is $CR^1$ or CH, $B^3$ is a nitrogen atom, $R^{4b}$ is a C1-C6 alkyl group that may be substituted with one or more halogen atoms or a hydrogen atom, $R^6$ is a C1-C3 alkyl group or a hydrogen atom, Z is an oxygen atom, $R^{3a}$ and $R^{3d}$ are hydrogen atoms, $R^{3b}$ and $R^{3c}$ are the same or different, and a C1-C3 alkyl group that may be substituted with one or more halogen atoms, a hydrogen atom, or a halogen atom.

[Embodiment P87] In any one of Embodiment P1 to Embodiment P71 and the compound P of the present invention, Q is a group represented by Q3, T1 is a group represented by T1-1, $A^3$ is CH, $B^1$ is a nitrogen atom, $B^2$ is $CR^1$, $B^3$ is a nitrogen atom, $R^6$ is s C1-C3 alkyl group or a hydrogen atom, Z is an oxygen atom, $R^{3a}$ and $R^{3d}$ are hydrogen atoms, $R^{3b}$ and $R^{3c}$ are the same or different, and a C1-C3 alkyl group that may be substituted with one or more halogen atoms, a hydrogen atom, or a halogen atom.

[Embodiment P88] In any one of Embodiment P1 to Embodiment P71 and the compound P of the present invention, a compound in which Q is a group represented by Q2, T1 is a group represented by T1-1.

[Embodiment P89] In any one of Embodiment P1 to Embodiment P71 and the compound P of the present invention, Q is a group represented by Q2, T1 is a group represented by T1-1, $B^1$ is $CR^{38a}$, $B^2$ is $CR^1$, $B^3$ is a nitrogen atom, $R^{4a}$ is a C1-C6 alkyl group that may be substituted with one or more halogen atoms or a hydrogen atom, $R^6$ is a C1-C6 alkyl group, and Z is an oxygen atom.

[Embodiment P90] In Embodiment P89, a compound in which $R^{38a}$ is a hydrogen atom.

[Embodiment P91] In any one of Embodiment P1 to Embodiment P71 and the compound P of the present invention, Q is a group represented by Q2, T1 is a group represented by T1-1, $A^2$ is $CR^{4a}$, $B^1$ is CH, $B^2$ is $CR^1$, $B^3$ is a nitrogen atom, $R^{4a}$ is a C1-C6 alkyl group that may be substituted with one or more halogen atoms or a hydrogen atom, $R^6$ is a C1-C3 alkyl group, and Z is an oxygen atom.

[Embodiment P92] In any one of Embodiment P1 to Embodiment P71 and the compound P of the present

invention, Q is a group represented by Q2, $A^2$ and $B^1$ are CHs, $B^2$ is $CR^1$, $B^3$ is a nitrogen atom, $R^6$ is a C1-C3 alkyl group, and Z is an oxygen atom.

In the compound P of the present invention, a compound in which $R^2$ is an ethyl group and n is 2.

In the compound P of the present invention, a compound in which $R^2$ is an ethyl group and n is 2, $G^1$, $G^3$, and $G^4$ are CHs, $G^2$ is $R^{3b}$, $R^{3b}$ is a phenyl that may be substituted with one or more halogen atoms, a cyclopropyl group, or a halogen atom.

[Embodiment P101] In the compound P of the present invention, a compound in which Q is a group represented by Q2 or a group represented by Q3, T1 is a group represented by T1-1, and $B^2$ is a group represented by $CR^1$.

[Embodiment P102] In the compound P of the present invention, Q is a group represented by Q2 or Q3, T1 is a group represented by T1-1, $B^1$ is CH or a nitrogen atom, $B^2$ is a group represented by $CR^1$, $B^3$ is a nitrogen atom, $A^2$ and $A^3$ are CHs, $R^6$ is a C1-C3 alkyl group that may be substituted with one or more halogen atoms or a hydrogen atom, and Z is an oxygen atom.

[Embodiment P103] In the compound P of the present invention, Q is a group represented by Q2 or Q3, T1 is a group represented by T1-1, $B^1$ is CH or a nitrogen atom, $B^2$ is a group represented by $CR^1$, $B^3$ is a nitrogen atom, $A^2$ and $A^3$ are CHs, $R^6$ is a methyl group or a hydrogen atom, and Z is an oxygen atom.

[Embodiment P104] In the compound P of the present invention, Q is a group represented by Q2-1, and Z is an oxygen atom.

[Embodiment P105] In the compound P of the present invention, Q is a group represented by Q2-1, $B^1$ is CH, $B^2$ is $CR^1$, $B^3$ is a nitrogen atom, and Z is an oxygen atom.

[Embodiment P106] In the compound P of the present invention, Q is a group represented by Q2-1, $B^1$ is CH, $B^2$ is $CR^1$, $B^3$ is a nitrogen atom, $A^2$ is CH, $R^6$ is a C1-C3 alkyl group that may be substituted with one or more halogen atoms, and Z is an oxygen atom.

[Embodiment P107] In the compound P of the present invention, Q is a group represented by Q2-1, $B^1$ is CH, $B^2$ is $CR^1$, $B^3$ is a nitrogen atom, $A^2$ is CH, $R^6$ is a methyl group, and Z is an oxygen atom.

[Embodiment P108] In the compound P of the present invention, Q is a group represented by Q3-1, and Z is an oxygen atom.

[Embodiment P109] In the compound P of the present invention, Q is a group represented by Q3-1, $B^1$ and $B^3$ are nitrogen atoms, $B^2$ is $CR^1$, and Z is an oxygen atom.

[Embodiment P110] In the compound P of the present invention, Q is a group represented by Q3-1, $B^1$ and $B^3$ are nitrogen atoms, $B^2$ is $CR^1$, $A^3$ is CH, $R^6$ is a C1-C3 alkyl group that may be substituted with one or more halogen atoms or a hydrogen atom, and Z is an oxygen atom.

[Embodiment P111] In the compound P of the present invention, Q is a group represented by Q3-1, $B^1$ and $B^3$ are nitrogen atoms, $B^2$ is $CR^1$, $A^3$ is CH, $R^6$ is a methyl group or a hydrogen atom, and Z is an oxygen atom.

[Embodiment P112] In Embodiments P101 to 111, a compound in which $R^1$ is a C1-C3 alkyl group that is substituted with one or more halogen atoms.

[Embodiment P113] In Embodiments P101 to 111, a compound in which $R^1$ is a methyl group that is substituted with one or more halogen atoms.

[Embodiment P114] In Embodiments P101 to 111, a compound in which $R^1$ is a methyl group that is substituted with one or more fluorine atoms.

[Embodiment P115] In Embodiment P112, a compound in which $R^2$ is an ethyl group and n is 2.

[Embodiment P116] In Embodiment P113, a compound in which $R^2$ is an ethyl group and n is 2.

[Embodiment P117] In Embodiment P114, a compound in which $R^2$ is an ethyl group and n is 2.

[Embodiment P118] In Embodiment P112, a compound in which $R^2$ is an ethyl group and n is 2, $G^1$, $G^3$, and $G^4$ are CHs, $G^2$ is $R^{3b}$, $R^{3b}$ is a phenyl that may be substituted with one or more halogen atoms, a cyclopropyl group, or a halogen atom.

[Embodiment P119] In Embodiment P113, a compound in which $R^2$ is an ethyl group and n is 2, $G^1$, $G^3$, and $G^4$ are CHs, $G^2$ is $R^{3b}$, $R^{3b}$ is a phenyl that may be substituted with one or more halogen atoms, a cyclopropyl group, or a halogen atom.

[Embodiment P120] In Embodiment P114, a compound in which $R^2$ is an ethyl group and n is 2, $G^1$, $G^3$, and $G^4$ are CHs, $G^2$ is $R^{3b}$, $R^{3b}$ is a phenyl that may be substituted with one or more halogen atoms, a cyclopropyl group, or a halogen atom.

In the compound P of the present invention, Q is a group represented by Qf23-1.

[Chem. 16]

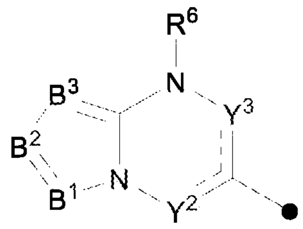

Qf23-1

[wherein a combination of $Y^2$ and $Y^3$ represents

a combination in which $Y^2$ is C=Z, and $Y^3$ is $A^3$; or
a combination in which $Y^2$ is $A^2$, and $Y^3$ is C=Z, and
other symbols represent the same meaning as described above.]

In the group represented by Qf23-1, a group in which $Y^2$ is C=Z and $Y^3$ is $A^3$ is a group represented by the following Qf23-1-a, and a group in which $Y^2$ is $A^2$ and $Y^3$ is C=Z is a group represented by the following Qf23-1-b.

[Chem. 17]

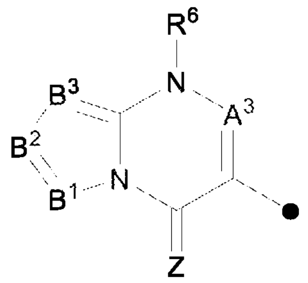

Qf23-1-a

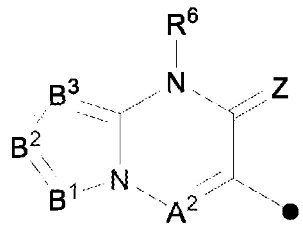

Qf23-1-b

In the compound P of the present invention, the compound in which Q is a group represented by Qf23-1 is a compound represented by Formula (I-Qf23) (hereinafter, referred to as a compound R of the present invention).

[Chem. 18]

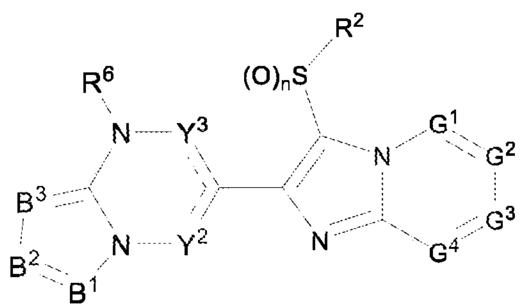

( I - Qf23 )

[wherein symbols represent the same meaning as described above.]

[Embodiment R1] In the compound R of the present invention, a compound in which $R^2$ is an ethyl group and n is 2.

[Embodiment R2] In the compound R of the present invention, a compound in which $R^2$ is an ethyl group and n is 2, $G^1$, $G^3$, and $G^4$ are CHs, $G^2$ is $R^{3b}$, $R^{3b}$ is a phenyl that may be substituted with one or more halogen atoms, a cyclopropyl group, or a halogen atom.

[Embodiment R3] In the compound R of the present invention, a compound in which $B^1$ is CH or a nitrogen atom, $B^2$ is a group represented by $CR^1$, and $B^3$ is a nitrogen atom.

[Embodiment R4] In Embodiment R3, $B^1$ is CH or a nitrogen atom, a compound in which $B^2$ is a group represented by $CR^1$, $B^3$ is a nitrogen atom, $A^2$ and $A^3$ are CHs, and Z is an oxygen atom.

[Embodiment R5] In Embodiment R4, a compound in which $R^6$ is a C1-C3 alkyl group that may be substituted with one or more halogen atoms or a hydrogen atom.

[Embodiment R6] In Embodiment R4, a compound in which $R^6$ is a methyl group or a hydrogen atom.

[Embodiment R7] In Embodiments R1 to R6, a compound in which $R^1$ is a C1-C3 alkyl group that is substituted with one or more halogen atoms.

[Embodiment R8] In Embodiments R1 to R6, a compound in which $R^1$ is a methyl group that is substituted with one or more halogen atoms.

[Embodiment R9] In Embodiments R1 to R6, a compound in which $R^1$ is a methyl group that is substituted with one or more fluorine atoms.

[Embodiment R10] In Embodiment R7, a compound in which $R^2$ is an ethyl group and n is 2.

[Embodiment R11] In Embodiment R8, a compound in which $R^2$ is an ethyl group and n is 2.

[Embodiment R12] In Embodiment R9, a compound in which $R^2$ is an ethyl group and n is 2.

[Embodiment R13] In Embodiment R7, a compound in which $R^2$ is an ethyl group and n is 2, $G^1$, $G^3$, and $G^4$ are CHs, $G^2$ is $R^{3b}$, $R^{3b}$ is a phenyl that may be substituted with one or more halogen atoms, a cyclopropyl group, or a halogen atom.

[Embodiment R14] In Embodiment R8, a compound in which $R^2$ is an ethyl group and n is 2, $G^1$, $G^3$, and $G^4$ are CHs, $G^2$ is $R^{3b}$, $R^{3b}$ is a phenyl that may be substituted with one or more halogen atoms, a cyclopropyl group, or a halogen atom.

[Embodiment R15] In Embodiment R9, a compound in which $R^2$ is an ethyl group and n is 2, $G^1$, $G^3$, and $G^4$ are CHs, $G^2$ is $R^{3b}$, $R^{3b}$ is a phenyl that may be substituted with one or more halogen atoms, a cyclopropyl group, or a halogen atom.

In the compound P of the present invention, Q is a group represented by Qf45-23.

[Chem. 19]

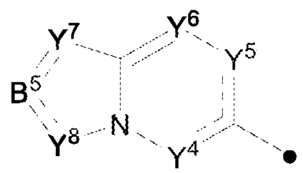

Qf45-23

[wherein a combination of $Y^4$, $Y^5$, and $Y^6$ represents

a combination in which $Y^4$ is C=Z, $Y^5$ is $A^6$, and $Y^6$ is $A^7$; or
a combination in which $Y^4$ is $A^5$, $Y^5$ is C=Z, and $Y^6$ is $A^4$,
a combination of $Y^7$ and $Y^8$ represents
a combination in which $Y^7$ is $B^4$, and $Y^8$ is $B^6$; or
a combination in which $Y^7$ is $B^6$, and $Y^8$ is $B^4$, and
other symbols represent the same meaning as described above.]

In the group represented by Qf45-23, for example, a group in which $Y^4$ is C=Z, $Y^5$ is $A^6$, $Y^6$ is $A^7$, $Y^7$ is $B^4$, and $Y^8$ is $B^6$ is a group represented by the following Qf45-23-a, a group in which $Y^4$ is C=Z, $Y^5$ is $A^6$, $Y^6$ is $A^7$, $Y^7$ is $B^6$, and $Y^8$ is $B^4$ is a group represented by the following Qf45-23-b, a group in which $Y^4$ is $A^5$, $Y^5$ is C=Z, $Y^6$ is $A^4$, $Y^7$ is $B^4$, and $Y^8$ is $B^6$ is a group represented by the following Qf45-23-c.

[Chem. 20]

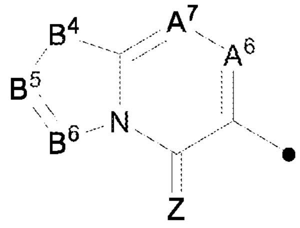

Qf45-23-a

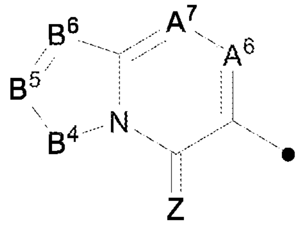

Qf45-23-b

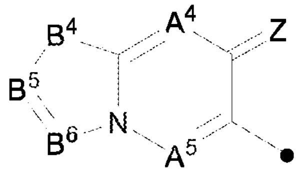

Qf45-23-c

In the compound P of the present invention, a compound in which Q is a group represented by Qf45-23 is a compound represented by Formula (I-Qf45).

[Chem. 21]

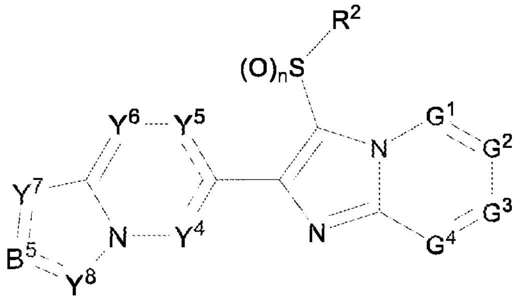

( I - Qf45 )

[wherein symbols represent the same meaning as described above.]

Next, the method for producing the compound X of the present invention will be described.

Production Method 1

A compound represented by Formula (II-b) (hereinafter, referred to as a compound (II-b) or a compound represented by Formula (II-c) (hereinafter, referred to as a compound (II-c) can be produced by reacting a compound represented by Formula (II-a) (hereinafter, referred to as a compound (II-1a) with an oxidizing agent.

[Chem. 22]

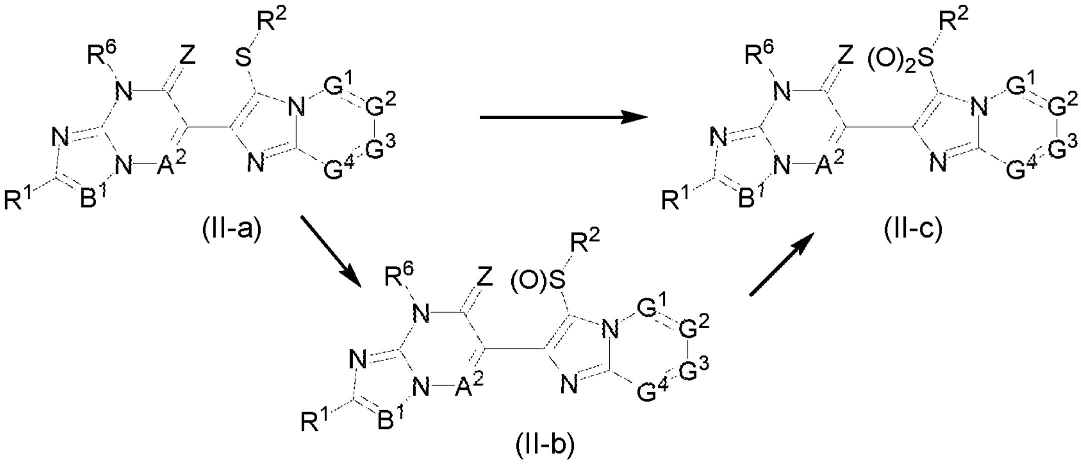

(II-a) (II-b) (II-c)

[wherein symbols represent the same meaning as described above.]

First, the method for producing the compound (II-a) from the compound (II-b) will be described.

The reaction is usually performed in a solvent. Examples of the solvent used in the reaction include halogenated hydrocarbon (hereinafter, referred to as halogenated hydrocarbons) such as dichloromethane and chloroform, nitrile

(hereinafter, referred to as nitriles) such as acetonitrile, alcohol (hereinafter, referred to as alcohols) such as methanol and ethanol, acetic acid, water, and mixtures of two or more kinds thereof.

Examples of the oxidizing agent used in the reaction include sodium periodate, m-chloroperbenzoic acid (hereinafter, referred to as mCPBA), and hydrogen peroxide.

When hydrogen peroxide is used as the oxidizing agent, a base or a catalyst may be added as necessary.

Examples of the base used in the reaction include sodium carbonate, and the like. In a case where the base is used in the reaction, it is usually used in a ratio of 0.01 to 1 mol, based on 1 mol of the compound (II-a).

Examples of the catalyst used in the reaction include sodium tungstate and sodium tungstate. In a case where the catalyst is used in the reaction, it is usually used in a ratio of 0.01 to 0.5 mol, based on 1 mol of the compound (II-a).

In the reaction, the oxidizing agent is usually used in a ratio of 1 to 1.2 mol, based on 1 mol of the compound (II-a).

The reaction temperature is usually in the range of - 20°C to 80°C. The reaction time is usually in the range of 0.1 to 12 hours.

After completion of the reaction, water is added to a reaction mixture, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate), as necessary. The compound (II-b) can be obtained by drying and concentrating the organic layer.

Next, a method for producing the compound (II-c) from the compound (II-b) will be described.

The reaction is usually performed in a solvent. Examples of the solvent used in the reaction include aliphatic halogenated hydrocarbons, nitriles, alcohols, acetic acid, water, and mixtures of two or more kinds thereof.

Examples of the oxidizing agent used in the reaction include mCPBA and a hydrogen peroxide.

When hydrogen peroxide is used as the oxidizing agent, a base or a catalyst may be added as necessary.

Examples of the base used in the reaction include sodium carbonate, and the like. In a case where the base is used in the reaction, it is usually used in a ratio of 0.01 to 1 mol, based on 1 mol of the compound (II-b).

Examples of the catalyst used in the reaction include sodium tungstate. In a case where the catalyst is used in the reaction, it is usually used in a ratio of 0.01 to 0.5 mol, based on 1 mol of the compound (II-b).

In the reaction, the oxidizing agent is usually used in a ratio of 1 to 2 mol, based on 1 mol of the compound (II-b) .

The reaction temperature is usually in the range of - 20°C to 120°C. The reaction time is usually in the range of 0.1 to 12 hours.

After completion of the reaction, water is added to a reaction mixture, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate), as necessary. The compound (II-c) can be obtained by drying and concentrating the organic layer.

Further, the compound (II-c) can be also produced by a one-step reaction (one pot) by reacting the compound (IIa) with an oxidizing agent.

The reaction can be performed in accordance with the method for producing the compound (II-c) from the compound (II-b), using the oxidizing agent in a ratio of usually 2 to 5 mol, based on 1 mol of the compound (II-a).

Production Method 2

A compound represented by Formula (II-1) (hereinafter, referred to as a compound (II-1) can be produced by reacting a compound represented by Formula (II-M1-1) (hereinafter, referred to as a compound (II-M1-1) with a compound represented by Formula (R-1) (hereinafter, referred to as a compound (R-1).

[Chem. 23]

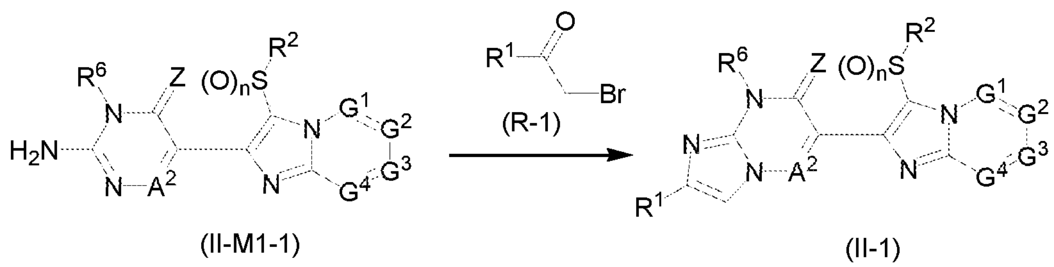

(II-M1-1) (R-1) (II-1)

[wherein symbols represent the same meaning as described above.]

The reaction is usually performed in a solvent. Examples of the solvent used in the reaction include ether (hereinafter, referred to as ethers) such as tetrahydrofuran (hereinafter, referred to as THF) and methyl tert-butyl ether

(hereinafter, referred to as MTBE); aromatic hydrocarbon (hereinafter, referred to as aromatic hydrocarbons) such as toluene and xylene; nitriles; a aprotic polar solvent (hereinafter, referred to as aprotic polar solvents) such as N-methylpyrrolidone (hereinafter, referred to as NMP), N,N-dimethylformamide (hereinafter, referred to as DMF), dimethyl sulfoxide (hereinafter, referred to as DMSO); alcohols; water; and a mixture of two or more kinds thereof.

The reaction can be also performed by using a base, as necessary. Examples of the base used in the reaction includes an organic base (hereinafter, referred to as organic bases) such as triethylamine, diisopropylethylamine, pyridine, and 4-(dimethylamino) pyridine, alkali metal carbonate (hereinafter, referred to as alkali metal carbonates) such as sodium carbonate and potassium carbonate. In a case where the base is used in the reaction, it is usually used in a ratio of 0.1 to 5 mol, based on 1 mol of the compound (II-M1-1).

In the reaction, the compound (R-1) is usually used in a ratio of 1 to 10 mol, based on 1 mol of the compound of the present invention (II-M1-1).

The reaction temperature is usually in the range of - 20°C to 200°C. The reaction time is usually in the range of 0.1 to 48 hours.

After completion of the reaction, water was added to the reaction mixture, the mixture is extracted with an organic solvent, and the organic layer is subjected to post-treatment operations such as drying and concentration, whereby the compound (II-1) can be obtained.

The compound (R-1) is a commercially available compound, or can be produced in accordance with a known method.

Production Method 3

A compound represented by Formula (II-2) (hereinafter, referred to as a compound (II-2) can be produced by reacting a compound represented by Formula (II-M2-1) (hereinafter, referred to as a compound (II-M2-1) with a compound represented by Formula (R-2) (hereinafter, referred to as a compound (R-2)).

[Chem. 24]

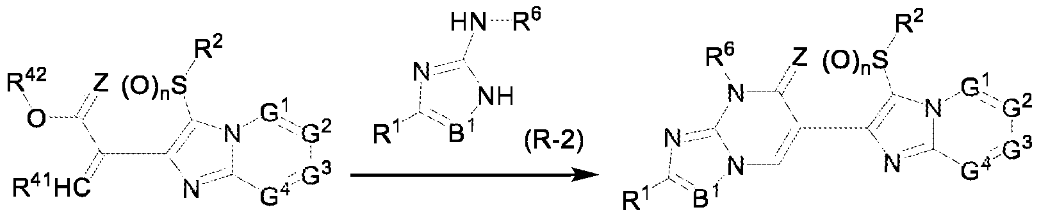

(II-M2-1) (R-2) (II-2)

[wherein $R^{41}$ represents a methoxy group, an ethoxy group, a dimethylamino group, or a hydroxy group, $R^{42}$ represents a methyl group or an ethyl group, and other symbols represent the same meaning as described above.]

The reaction is usually performed in a solvent. Examples of the solvent used in the reaction include ethers, aromatic hydrocarbons, nitriles, aprotic polar solvents, alcohols, water, and mixtures of two or more kinds thereof.

The reaction can be also performed by using a base, as necessary. Examples of the base used in the reaction include organic bases and alkali metal carbonates. In a case where the base is used in the reaction, it is usually used in a ratio of 0.1 to 5 mol, based on 1 mol of the compound (II-M2-1).

In the reaction, the compound (R-2) is usually used in a ratio of 1 to 10 mol, based on 1 mol of the compound (II-M2-1).

The reaction temperature is usually in the range of - 20°C to 200°C. The reaction time is usually in the range of 0.1 to 48 hours.

After completion of the reaction, water was added to the reaction mixture, the mixture is extracted with an organic solvent, and the organic layer is subjected to post-treatment operations such as drying and concentration, whereby the compound (II-2) can be obtained.

The compound (R-2) is a commercially available compound, or can be produced in accordance with a known method.

Production Method 4

N-oxide of the compound represented by Formula (I) can be produced by reacting the compound represented by Formula (I) with an oxidizing agent. The reaction can be performed, for example, according to the method described

in Production Method 1, US 2018/0009778, or WO 2016/121970.

Production Method 5

A compound represented by Formula (I-b) or a compound represented by Formula (I-c) can be produced by reacting the compound represented by Formula (I-a) with an oxidizing agent.

[Chem. 25]

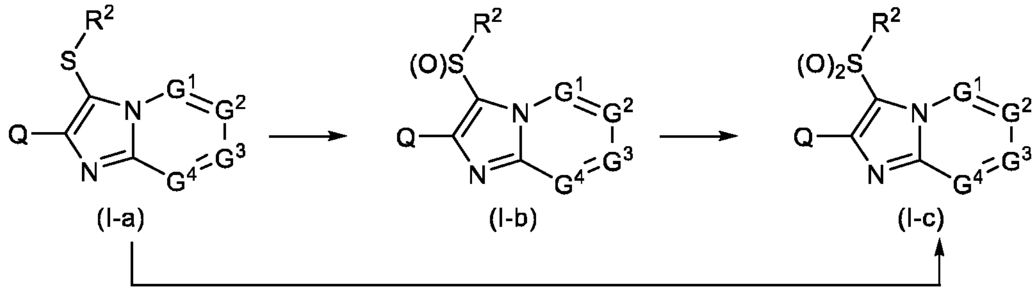

(I-a) (I-b) (I-c)

[wherein symbols represent the same meaning as described above.]

The reactions can be performed in accordance with Production Method 1.

Production Method 6

A compound represented by Formula (I-1-S) (hereinafter, referred to as a compound (I-1-S)) can be produced by reacting a compound represented by Formula (I-1-O) (hereinafter, referred to as a compound (I-1-O)) with a sulfurizing agent.

[Chem. 26]

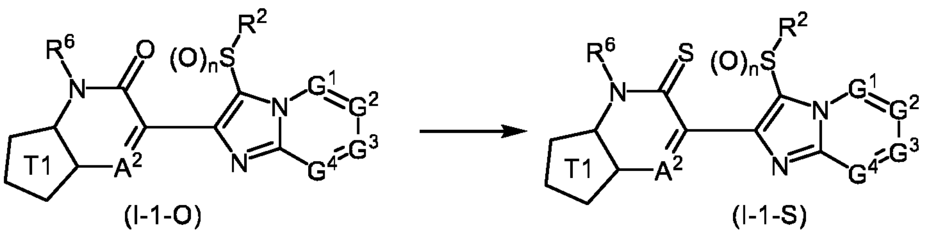

(I-1-O) (I-1-S)

[wherein symbols represent the same meaning as described above.]

The reaction is performed in a solvent or in the absence of a solvent. Examples of the solvent include ethers; halogenated hydrocarbons; aromatic hydrocarbons; nitriles; nitrogen-containing aromatic compounds such as pyridine, picoline, lutidine, and quinoline; and mixtures of two or more kinds thereof.

Examples of the sulfurizing agent include diphosphorus pentasulfide and Lawesson's reagent (2,4-bis-(4-methoxyphenyl)-1,3-dithia-2,4-diphosphetane-2,4-disulfide).

In the reaction, the sulfurizing agent is usually used in a ratio of 1 mol to 3 mol, based on 1 mol of the compound (I-1-O).

The reaction temperature is usually in the range of 0°C to 200°C. The reaction time is usually in the range of 1 to 24 hours.

After completion of the reaction, water was added to the reaction mixture, the mixture is extracted with an organic solvent, and the organic layer is subjected to post-treatment operations such as drying and concentration, whereby the compound (I-1-S) can be obtained.

Production Method 7

A compound represented by formula (I-2-S) can be produced by reacting a compound represented by formula (I-2-O) with a sulfurizing agent.

[Chem. 27]

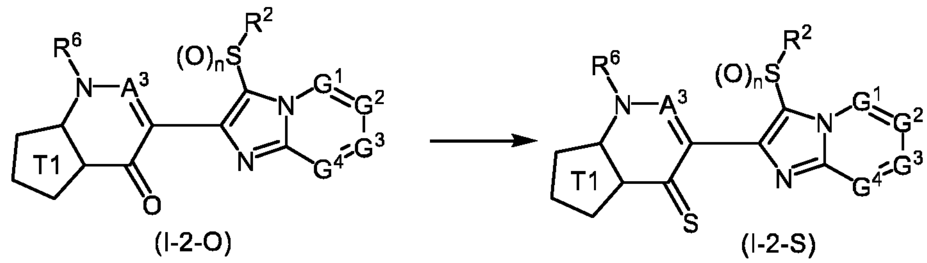

(I-2-O) (I-2-S)

[wherein symbols represent the same meaning as described above.]

The reactions can be performed in accordance with Production Method 6.

Production Method 8

A compound represented by formula (I-3-S) can be produced by reacting a compound represented by formula (I-3-O) with a sulfurizing agent.

[Chem. 28]

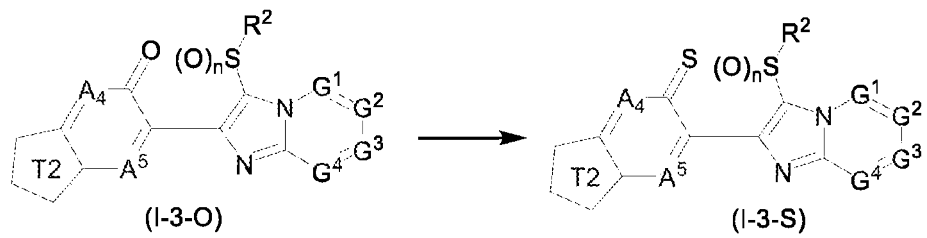

(I-3-O) (I-3-S)

[wherein symbols represent the same meaning as described above.]

The reactions can be performed in accordance with Production Method 6.

Production Method 9

A compound represented by formula (I-4-S) can be produced by reacting a compound represented by formula (1-4-O) with a sulfurizing agent.

[Chem. 29]

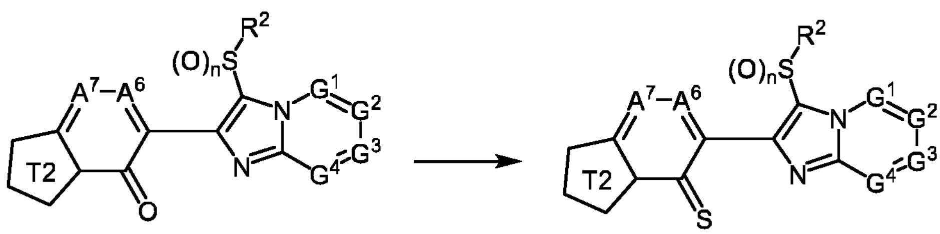

(I-4-O) (I-4-S)

[wherein symbols represent the same meaning as described above.]

The reactions can be performed in accordance with Production Method 6.

Production Method 10

A compound represented by formula (I-5-S) can be produced by reacting a compound represented by formula (I-5-O) with a sulfurizing agent.

[Chem. 30]

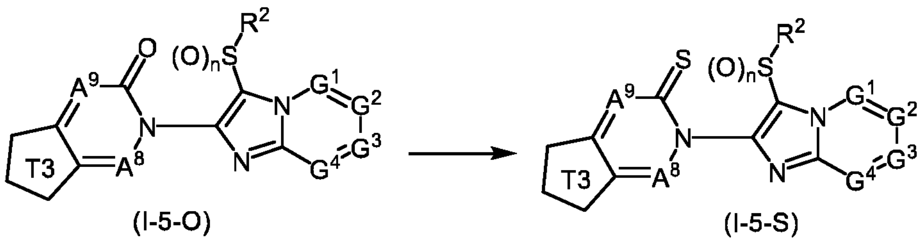

(I-5-O) (I-5-S)

[wherein symbols represent the same meaning as described above.]

The reactions can be performed in accordance with Production Method 6.

Production Method 11

A compound represented by formula (I-6-S) can be produced by reacting a compound represented by formula (I-6-O) with a sulfurizing agent.

[Chem. 31]

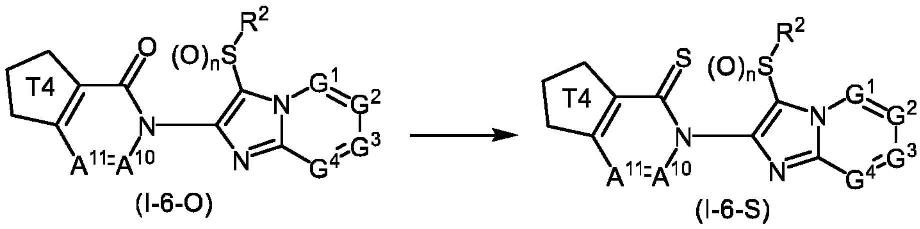

(I-6-O) (I-6-S)

[wherein symbols represent the same meaning as described above.]

The reactions can be performed in accordance with Production Method 6.

Production Method 12

A compound represented by Formula (II-2a) can be produced by reacting the compound (II-M2-1) with a compound represented by Formula (R-5) (hereinafter, referred to as a compound (R-5)).

[Chem. 32]

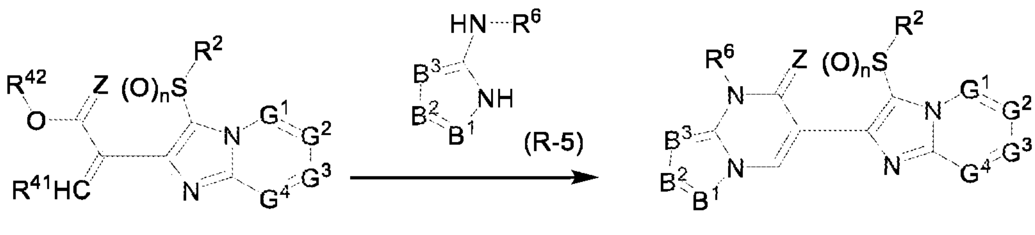

(R-5)

(II-M2-1) (II-2a)

[wherein symbols represent the same meaning as described above.]

The reaction can be performed in accordance with Production Method 3, using the compound (R-5) instead of the compound (R-2).

The compound (R-5) is a commercially available compound, or can be produced in accordance with a known method.

Production Method 13

A compound represented by Formula (II-3) (hereinafter, referred to as a compound (II-3)) can be produced according to the following scheme.

[Chem. 33]

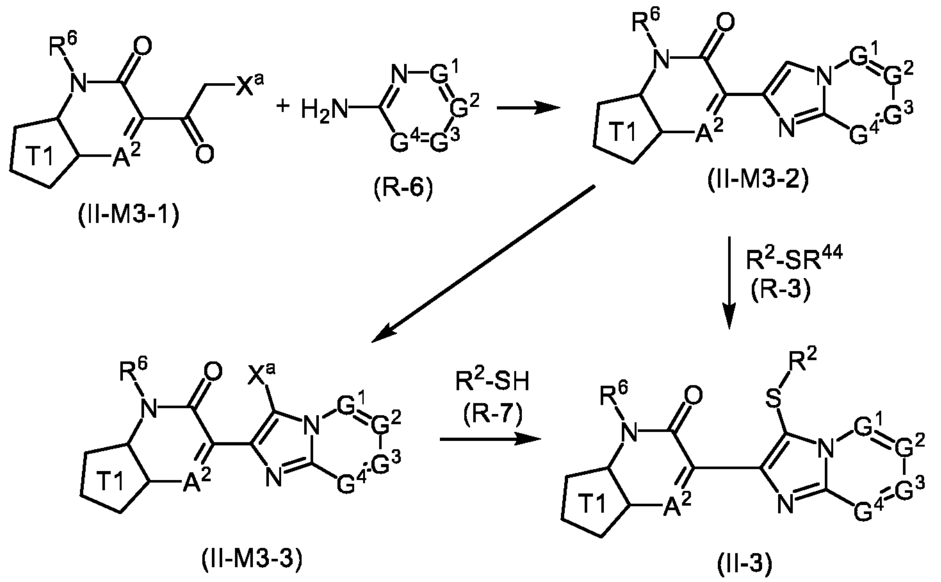


[wherein $X^a$ represents a chlorine atom, a bromine atom, or an iodine atom, $R^{44}$ represents $SR^2$ or a hydrogen atom, and other symbols represent the same meaning as described above.]

First, a method for producing a compound represented by Formula (II-M3-2) (hereinafter, referred to as a compound (II-M3-2)) will be described.

The compound (II-M3-2) can be produced by reacting a compound represented by Formula (II-M3-1) (hereinafter, referred to as a compound (II-M3-1)) with a compound represented by Formula (R-6) (hereinafter, referred to as a compound (R-6)).

The reaction is usually performed in a solvent. Examples of the solvent used in the reaction include ethers, aromatic hydrocarbons, aprotic polar solvents, alcohols, nitriles, water, and mixtures of two or more kinds thereof.

The reaction can be also performed by using a base, as necessary. Examples of the base used in the reaction include organic bases and alkali metal carbonates. In a case where the base is used in the reaction, the base is usually used in a ratio of 0.1 to 5 mol, based on 1 mol of the compound (II-M3-1).

In the reaction, the compound (R-6) is usually used in a ratio of 1 to 10 mol, based on 1 mol of the compound of the present invention (II-M3-1).

The reaction temperature is usually in the range of - 20°C to 200°C. The reaction time is usually in the range of 0.1 to 48 hours.

After completion of the reaction, water was added to the reaction mixture, the mixture is extracted with an organic solvent, and the organic layer is subjected to post-treatment operations such as drying and concentration, whereby the compound (II-M3-2) can be obtained.

The compound (R-6) is a commercially available compound, or can be produced in accordance with a known method.

Next, a method for producing a compound represented by Formula (II-M3-3) (hereinafter, referred to as a compound (II-M3-3)) will be described.

The compound (II-M3-3) can be produced by reacting the compound (II-M3-2) with a halogenating agent.

The reaction is usually performed in a solvent. Examples of the solvent used in the reaction include alcohols, nitriles, ethers, aromatic hydrocarbons, aprotic polar solvents, halogenated hydrocarbons, water, and mixtures of two or more kinds thereof.

Examples of the halogenating agent include chlorine, bromine, iodine, N-chlorosuccinimide, N-bromosuccinimide, and N-iodosuccinimide.

In the reaction, the halogenating agent is usually used in a ratio of 1 to 20 mol, based on 1 mol of the compound (II-M3-2).

The reaction temperature is usually in the range of - 20°C to 200°C. The reaction time is usually in the range of 0.1 to 72 hours.

After completion of the reaction, water was added to the reaction mixture, the mixture is extracted with an organic solvent, and the organic layer is subjected to post-treatment operations such as drying and concentration, whereby the compound (II-M3-3) can be obtained.

Next, a method for producing the compound (II-3) from the compound (II-M3-2) will be described.

The compound (II-3) can be produced by reacting a compound (II-M3-2) with a compound represented by Formula (R-3) (hereinafter, referred to as a compound (R-3)) under presence of the halogenating agent.

The reaction is usually performed in a solvent. Examples of the solvent used in the reaction include alcohols, nitriles, ethers, aromatic hydrocarbons, aprotic polar solvents, water, and mixtures of two or more kinds thereof.

Examples of the halogenating agent include bromine, iodine, sodium bromide, potassium bromide, sodium iodide, and potassium iodide.

An oxidizing agent may be used in the reaction as necessary. Examples of the oxidizing agent include hydrogen peroxide, tert-butyl hydroperoxide, and DMSO. In a case where the oxidizing agent is used in the reaction, the oxidizing agent is usually used in a ratio of 1 to 20 mol, based on 1 mol of the compound (II-M3-2).

In the reaction, the compound (R-3) is usually used in a ratio of 0.5 to 10 mol, and the halogenating agent is usually used in a ratio of 0.05 to 10 mol, based on 1 mol of the compound (II-M3-2).

The reaction temperature is usually in the range of 0°C to 200°C. The reaction time is usually in the range of 0.1 to 72 hours.

After completion of the reaction, water was added to the reaction mixture, the mixture is extracted with an organic solvent, and the organic layer is subjected to post-treatment operations such as drying and concentration, whereby the compound (II-3) can be obtained.

The compound (R-3) is known, or can be produced in accordance with a known method.

Next, a method for producing the compound (II-3) from the compound (II-M3-3) will be described.

The compound (II-3) can be produced by reacting a compound (II-M3-3) with a compound represented by Formula (R-7) (hereinafter, referred to as a compound (R-7)) under a metal catalyst and a base.

The reaction is usually performed in a solvent. Examples of the solvent used in the reaction include alcohols, nitriles, ethers, aromatic hydrocarbons, aprotic polar solvents, water, and mixtures of two or more kinds thereof.

Examples of the metal catalyst used in the reaction include palladium catalysts such as tetrakis(triphenylphosphine)palladium (0), [1,1'-bis(diphenylphosphino)ferrocene palladium (II) dichloride, tris(dibenzylideneacetone)dipalladium(0), and palladium acetate (II), nickel catalysts such as bis(cyclooctadiene) nickel (0) and nickel chloride (II), and copper catalysts such as copper (I) iodide and copper (I) chloride.

Examples of the base used in the reaction include alkali metal hydride (hereinafter, referred to as alkali metal hydrides) such as sodium hydride, alkali metal carbonates, and organic bases.

A ligand can also be used for the reaction. Examples of the ligand include triphenylphosphine, xantphos, 2,2'-bis(diphenylphosphino)-1,1'-binaphthyl, 1,1'-bis(diphenylphosphino) ferrocene, 2-dicyclohexylphosphino-2',4',6'-triisopropylbiphenyl, 2-dicyclohexylphosphino-2',6'-dimethoxybiphenyl, 1,2-bis(diphenylphosphino) ethane, 2,2'-bipyridine, 2-aminoethanol, 8-hydroxyquinoline, and 1,10-phenanthroline. In a case where the ligand is used in the reaction, the ligand is usually used in a ratio of 0.01 to 1 mol, based on 1 mol of the compound (II-M3-3).

In the reaction, the compound (R-7) is usually used in a ratio of 1 to 20 mol, the metal catalyst is usually used in a ratio of 0.01 to 0.5 mol, and the base is usually used in a ratio of 0.1 to 5 mol, based on 1 mol of the compound (II-M3-3).

The reaction temperature is usually in the range of - 20°C to 200°C. The reaction time is usually in the range of 0.1 to 72 hours.

After completion of the reaction, water was added to the reaction mixture, the mixture is extracted with an organic solvent, and the organic layer is subjected to post-treatment operations such as drying and concentration, whereby the compound (II-3) can be obtained.

Production Method 14

A compound represented by Formula (III-1) (hereinafter, referred to as a compound (III-1)) can be produced according to the following scheme.

[Chem. 34]

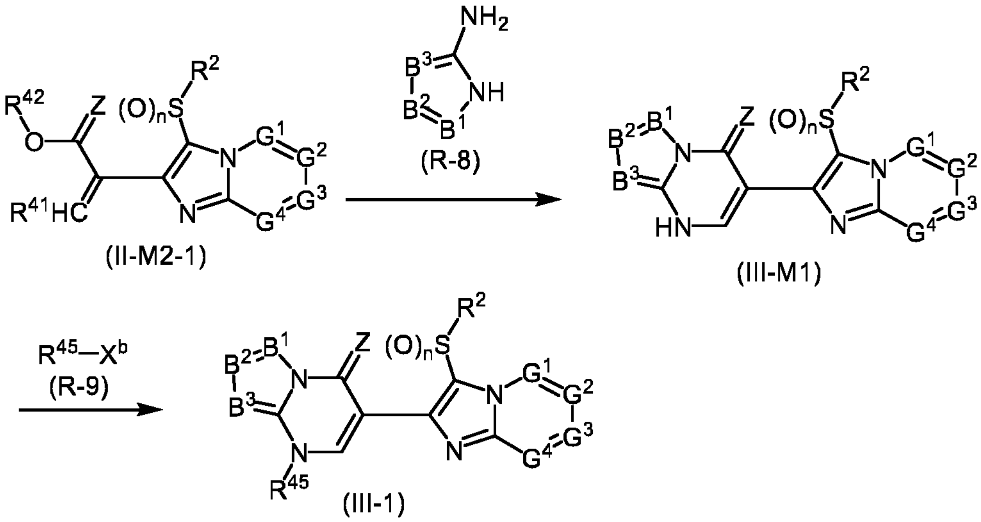


(II-M2-1) (III-M1) (III-1)

[wherein $R^{45}$ represents a C1-C6 chain hydrocarbon group that may be substituted with one or more substituents selected from a group F, a C3-C6 cycloalkyl group that may be substituted with one or more substituents selected from a group J, or a 5- or 6-membered aromatic heterocyclic group that may be substituted with one or more substituents selected from a group H, $X^b$ represents a leaving group such as a chlorine atom, a bromine atom, and an iodine atom, and other symbols represent the same meaning as described above.]

First, a method for producing a compound represented by Formula (III-M1) (hereinafter, referred to as a compound (III-M1)) will be described.

The compound (III-M1) can be produced by using a compound represented by Formula (R-8) (hereinafter, referred to as a compound (R-8)) instead of the compound (R-2), in accordance with Production Method 3.

The compound (R-8) is a commercially available compound, or can be produced in accordance with a known method.

Next, a method for producing the compound (III-1) will be described.

The compound (III-1) can be produced by reacting a compound (III-M1) and a compound represented by Formula (R-9) (hereinafter, referred to as a compound (R-9)) under the presence of the base.

The reaction is usually performed in a solvent. Examples of the solvent used in the reaction include ethers, aromatic hydrocarbons, aprotic polar solvents, and mixtures of two or more kinds thereof.

Examples of the base used in the reaction include organic bases, alkali metal hydrides and alkali metal carbonates.

In the reaction, the compound (R-9) is usually used in a ratio of 1 to 10 mol, and the base is usually used in a ratio of 0.1 to 5 mol, based on 1 mol of the compound (III-M1) .

The reaction temperature is usually in the range of - 20°C to 120°C. The reaction time is usually in the range of 0.1 to 24 hours.

After completion of the reaction, water was added to the reaction mixture, the mixture is extracted with an organic solvent, and the organic layer is subjected to post-treatment operations such as drying and concentration, whereby the compound (III-1) can be obtained.

The compound (R-9) is a commercially available compound, or can be produced in accordance with a known method.

Production Method 15

The compound represented by (III-3) (hereinafter, referred to as a compound (III-3)) can be produced by reacting a compound (III-M1) with a compound represented by Formula (R-13) (hereinafter, referred to as a compound (R-13) under the presence of a copper catalyst.

[Chem. 35]

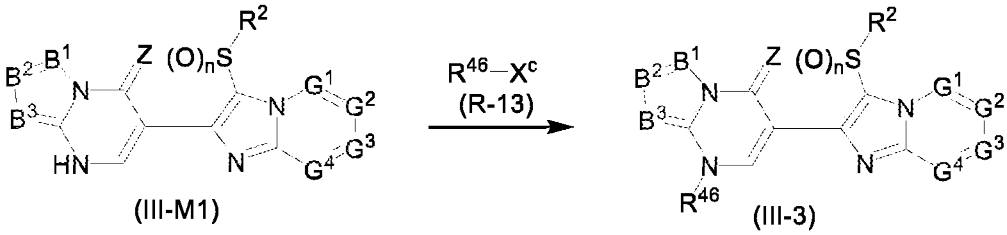


(III-M1) (III-3)

[wherein $R^{46}$ represents a phenyl group may be substituted with one or more substituents selected from a group H, $X^c$ represents a bromine atom or an iodine atom, and other symbols represent the same meaning as described above.]

The reaction is usually performed in a solvent. Examples of the solvent used in the reaction include ethers, aromatic hydrocarbons, aprotic polar solvents, water, and mixtures of two or more kinds thereof.

Examples of the copper catalyst used in the reaction include copper (I) iodide, copper (I) chloride, and copper (II) acetate.

In the reaction, a ligand and/or a base may be used as necessary.

Examples of the ligand include 2,2'-bipyridine, 2-aminoethanol, 8-hydroxyquinoline, picolinic acid, and 1,10-phenanthroline. In a case where the ligand is used in the reaction, the ligand is usually used in a ratio of 0.01 to 1 mol, based on 1 mol of the compound (III-M1).

Examples of the base include alkali metal hydrides, alkali metal carbonates, and organic bases. In a case where the base is used in the reaction, the base is usually used in a ratio of 0.1 to 5 mol, based on 1 mol of the compound (III-M1).

In the reaction, the compound (R-13) is usually used in a ratio of 1 to 10 mol, and the copper catalyst is usually used in a ratio of 0.01 to 0.5 mol, based on 1 mol of the compound (III-M1).

The reaction temperature is usually in the range of - 20°C to 200°C. The reaction time is usually in the range of 0.1 to 72 hours.

After completion of the reaction, water was added to the reaction mixture, the mixture is extracted with an organic solvent, and the organic layer is subjected to post-treatment operations such as drying and concentration, whereby the compound (III-3) can be obtained.

The compound (R-13) is a commercially available compound, or can be produced in accordance with a known method.

Production Method 16

A compound represented by Formula (III-2) can be produced according to the following scheme.

[Chem. 36]

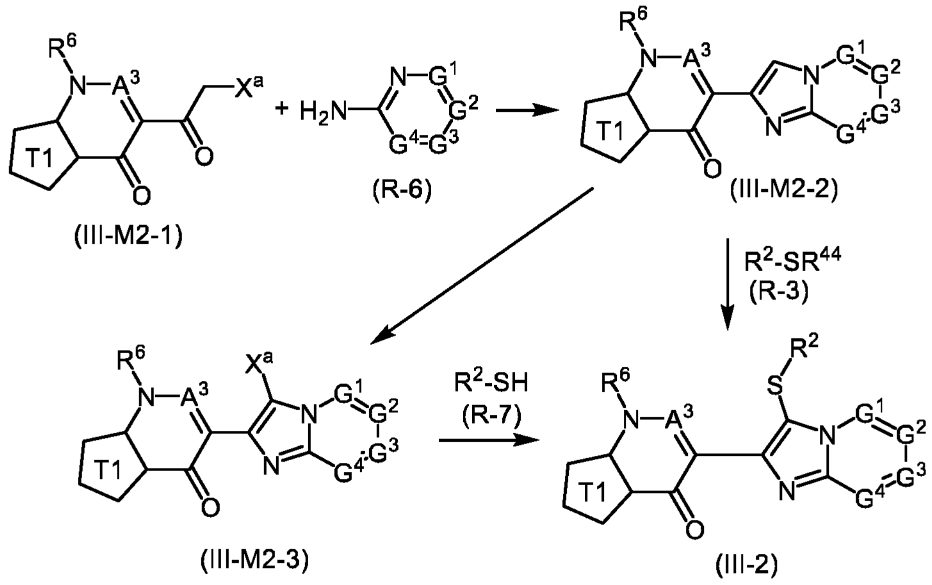


[wherein symbols represent the same meaning as described above.]

The reactions can be performed in accordance with Production Method 13.

Production Method 17

A compound represented by Formula (IV-2) can be produced according to the following scheme.

[Chem. 37]

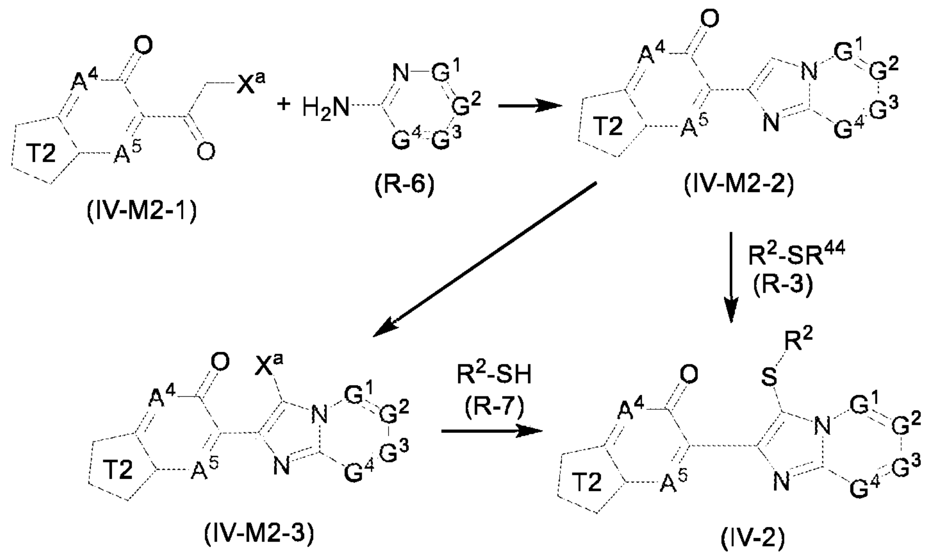


[wherein symbols represent the same meaning as described above.]

The reactions can be performed in accordance with Production Method 13.

Production Method 18

A compound represented by Formula (V-2) can be produced according to the following scheme.

[Chem. 38]

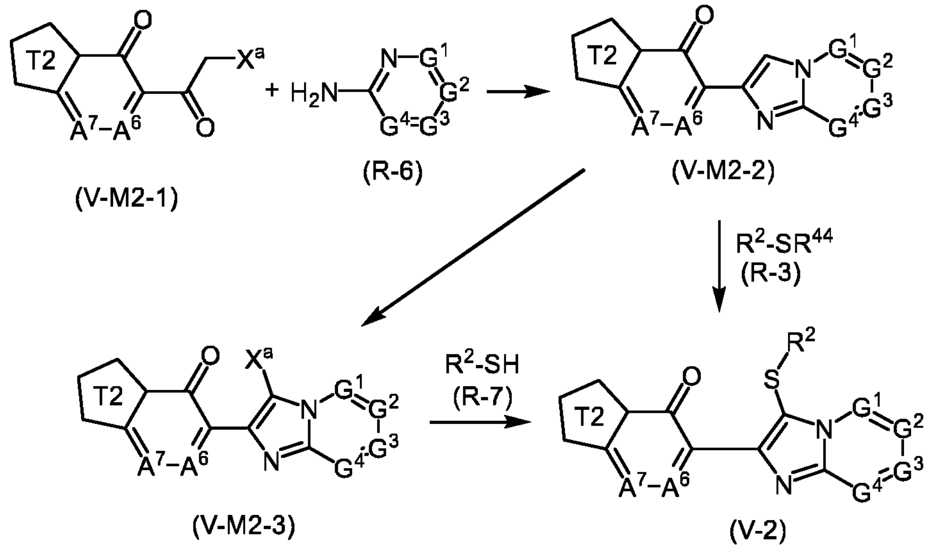


(V-M2-1) (R-6) (V-M2-2) (V-M2-3) (V-2)

[wherein symbols represent the same meaning as described above.]

The reactions can be performed in accordance with Production Method 13.

Production Method 19

A compound represented by Formula (VI-2) (hereinafter, referred to as a compound (VI-2) can be produced by reacting a compound represented by Formula (VIM2) (hereinafter, referred to as a compound (VI-M2) with a compound represented by Formula (VI-1) (hereinafter, referred to as a compound (VI-1)) under the presence of the base.

[Chem. 39]

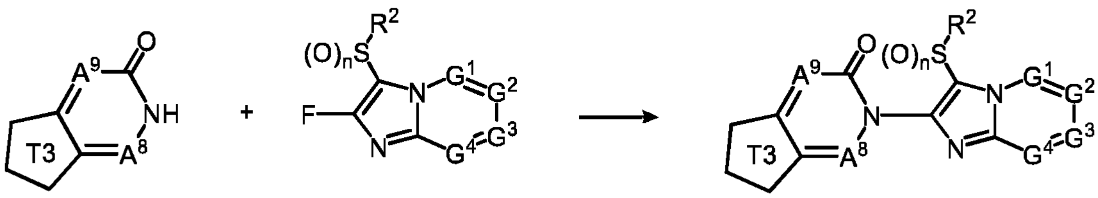

(VI-M2) (VI-1) (VI-2)

[wherein symbols represent the same meaning as described above.]

The reaction is usually performed in a solvent. Examples of the solvent used in the reaction include ethers, aromatic hydrocarbons, nitriles, aprotic polar solvents, and mixtures of two or more kinds thereof.

Examples of the base used in the reaction include alcohols, alkali metal carbonates and alkali metal hydrides.

In the reaction, the compound (VI-M2) is usually used in a ratio of 1 to 2 mol, and the base is usually used in a ratio of 1 to 10 mol, based on 1 mol of the compound (VI-1) .

The reaction temperature is usually in the range of - 20°C to 150°C. The reaction time is usually in the range of 0.5 to 24 hours.

After completion of the reaction, water was added to the reaction mixture, the mixture is extracted with an organic solvent, and the organic layer is subjected to post-treatment operations such as drying and concentration, whereby the compound (VI-2) can be obtained.

The compound (VI-M2) is a commercially available compound, or can be produced in accordance with a known method.

Production Method 20

The compound represented by Formula (VII-2) can be produced by reacting a compound represented by

Formula (VII-M2) (hereinafter, referred to as a compound (VII-M2)) and a compound (VI-1) under the presence of the base.

[Chem. 40]

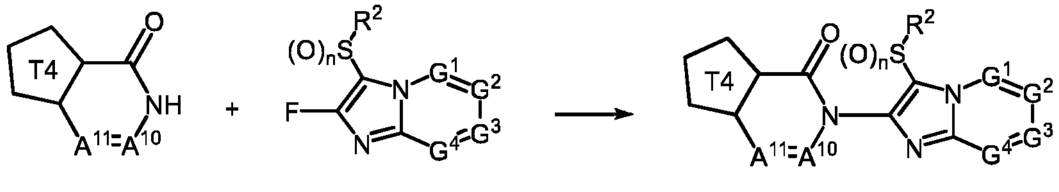

(VII-M2) (VI-1) (VII-2)

[wherein symbols represent the same meaning as described above.]

The reaction can be performed in accordance with Production Method 19, using the compound (VII-M2) instead of the compound (VI-M2).

The compound (VII-M2) is a commercially available compound, or can be produced in accordance with a known method.

The method for producing a production intermediate will be described.

Reference Production Method 1

The compound (II-M1-1) can be produced by reacting a compound represented by Formula (II-M1-2) (hereinafter, referred to as a compound (II-M1-2)) with ammonia.

[Chem. 41]

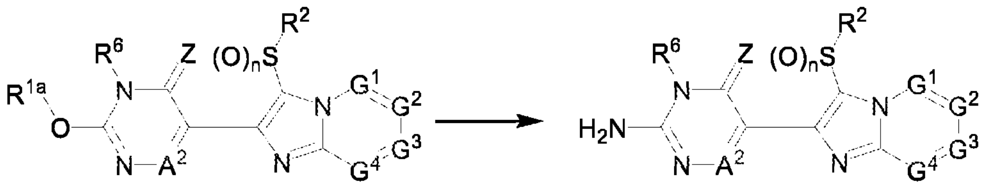

(II-M1-2) (II-M1-1)

[wherein $R^{1a}$ represents a C1-C10 chain hydrocarbon group that may be substituted, and other symbols represent the same meaning as described above.]

The reaction is usually performed in a solvent. Examples of the solvent used in the reaction include ethers, nitriles, aprotic polar solvents, alcohols, water, and mixtures of two or more kinds thereof.

The reaction can be also performed by using a base, as necessary. Examples of the base used in the reaction include organic bases and alkali metal carbonates. In a case where the base is used in the reaction, the base is usually used in a ratio of 0.1 to 5 mol, based on 1 mol of the compound (II-M1-2).

Ammonia can also be used as a solution such as ammonia water or an ammonia methanol solution.

In the reaction, the ammonia is usually used in a ratio of 1 to 100 mol, based on 1 mol of the compound (II-M1-2) .

The reaction temperature is usually in the range of - 20°C to 100°C. The reaction time is usually in the range of 0.1 to 48 hours.

After completion of the reaction, water was added to the reaction mixture, the mixture is extracted with an organic solvent, and the organic layer is subjected to post-treatment operations such as drying and concentration, whereby the compound (II-M1-1) can be obtained.

The compound (II-M1-2) can be produced by using a method disclosed in WO 2019/124548.

Reference Production Method 1-2

A compound represented by Formula (II-M1-1a) (hereinafter, referred to as a compound (II-M1-1a)) can be produced by reacting a compound represented by Formula (II-M1-3) (hereinafter, referred to as a compound (II-M1-3))

with ammonia.

[Chem. 42]

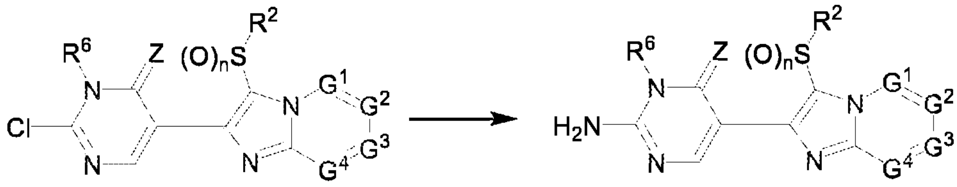

(II-M1-3)

(II-M1-1a)

[wherein symbols represent the same meaning as described above.]

The reaction can be performed in accordance with the method for producing the compound (II-M1-1) from the compound (II-M1-2), using a compound (II-M1-3) instead of the compound (II-M1-2).

Reference Production Method 2

A compound represented by Formula (II-M1-3c) (hereinafter, referred to as a compound (II-M1-3c) or a compound represented by Formula (II-M1-3b) (hereinafter, referred to as a compound (II-M1-3b) can be produced by reacting a compound represented by Formula (II-M1-3a) (hereinafter, referred to as a compound (II-M1-3a) with an oxidizing agent.

[Chem. 43]

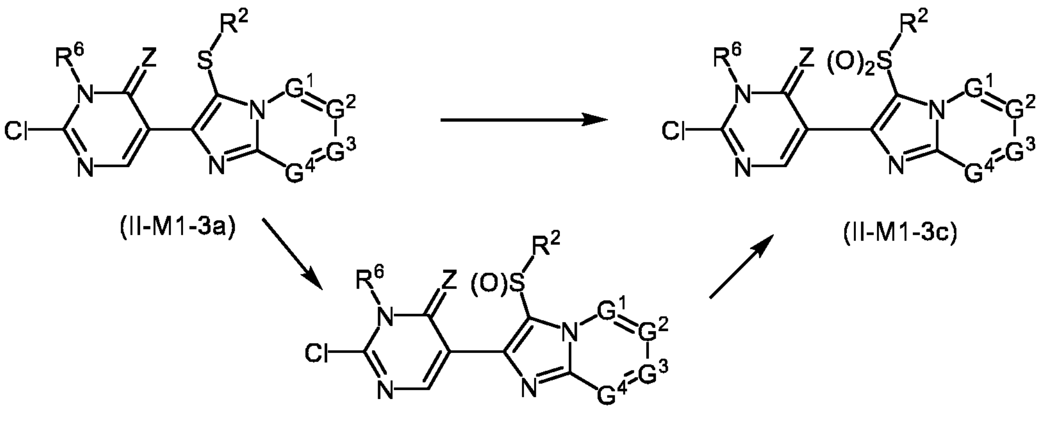

[wherein symbols represent the same meaning as described above.]

The reactions can be performed in accordance with Production Method 1.

Reference Production Method 3

The compound (II-M1-3a) can be produced according to the following scheme.

[Chem. 44]

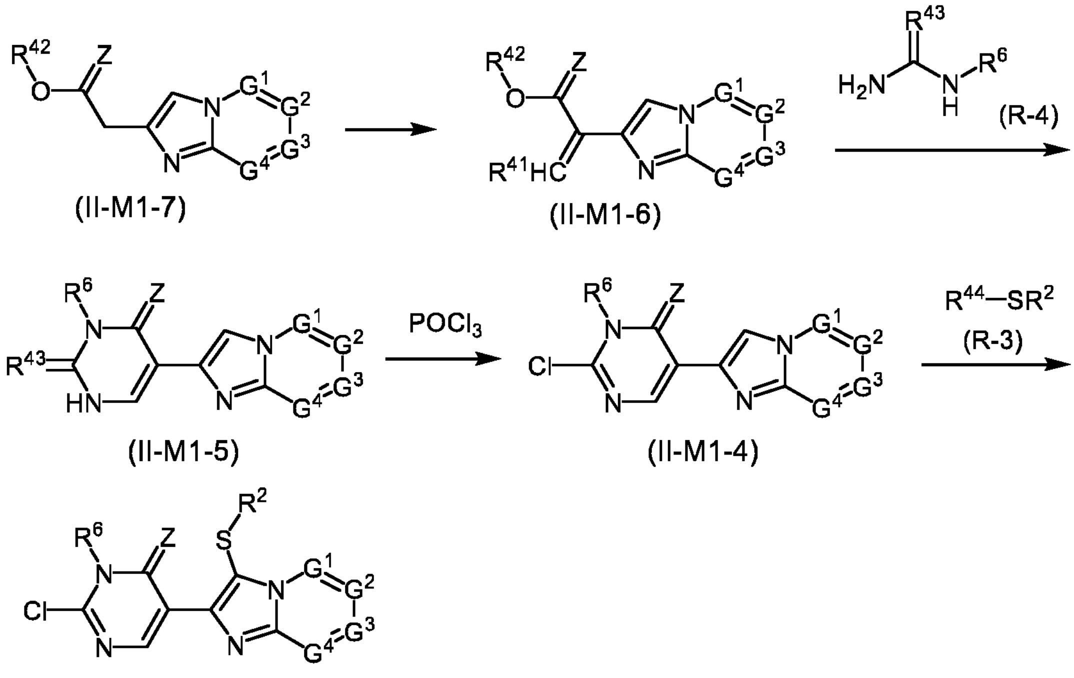


(II-M1-7) (II-M1-6) (II-M1-5) (II-M1-4) (II-M1-3a)

[wherein $R^{43}$ represents an oxygen atom or a sulfur atom, and other symbols represent the same meaning as described above.]

First, a method for producing a compound represented by Formula (II-M1-6) (hereinafter, referred to as a compound (II-M1-6)) from a compound represented by Formula (II-M1-7) (hereinafter, referred to as a compound (II-M1-7)).

The compound (II-M1-6) can be produced from the compound (II-M1-7).

The reaction can be performed according to a method described in, for example, Chem. Med. Chem., 2018, 13, 988., Org. Lett., 2016, 18, 6344., or Tetrahedron, 2017, 73, 3939.

The compound (II-M1-7) is known or can be produced according to a known method (for example, the method described in Bioorganic & Medicinal Chemistry, 2008, 16(6), 3125) .

Next, a method for producing a compound represented by Formula (II-M1-5) (hereinafter, referred to as a compound (II-M1-5)) from a compound represented by Formula (II-M1-6). The compound (II-M1-5) can be produced by reacting the compound (II-M1-6) with a compound represented by Formula (R-4) (hereinafter, referred to as a compound (R-4)) .

The reaction can be performed according to a method described in, for example, Tetrahedron, 2005, 61, 7508.

The compound (R-4) is known, or can be produced in accordance with a known method.

Next, a method for producing a compound represented by Formula (II-M1-4) (hereinafter, referred to as a compound (II-M1-4)) from a compound represented by Formula (II-M1-5). The compound (II-M1-4) can be produced by reacting the compound (II-M1-5) with phosphorus oxychloride.

The reaction can be performed according to a method described in, for example, Bioorg. Med. Chem. Lett., 2013, 23, 6928. or Bioorg. Med. Chem. Lett., 2016, 26, 5290.

Next, a method for producing the compound (II-M1-4) from the compound (II-M1-3a) will be described.

The compound (II-M1-3a) can be produced by reacting the compound (II-M1-4) with the compound (R-3).

The reaction can be performed according to a method described in, for example, Chem. Eur. J., 2016, 22, 11854.

Reference Production Method 4

The compound (II-M2-1) can be produced from a compound represented by Formula (II-M2-2) (hereinafter, referred to as a compound (II-M2-2)).

[Chem. 45]

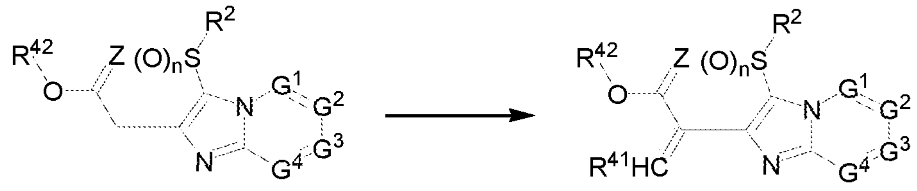

(II-M2-2) (II-M2-1)

[wherein symbols represent the same meaning as described above.]

The reaction can be performed according to a method described in, for example, Chem. Med. Chem., 2018, 13, 988., Org. Lett., 2016, 18, 6344., or Tetrahedron, 2017, 73, 3939.

Reference Production Method 5

A compound represented by Formula (II-M2-2c) and a compound represented by Formula (II-M2-2b) can be produced by reacting a compound represented by Formula (II-M2-2a) (hereinafter, referred to as a compound (II-M2-2a)) with an oxidizing agent.

[Chem. 46]

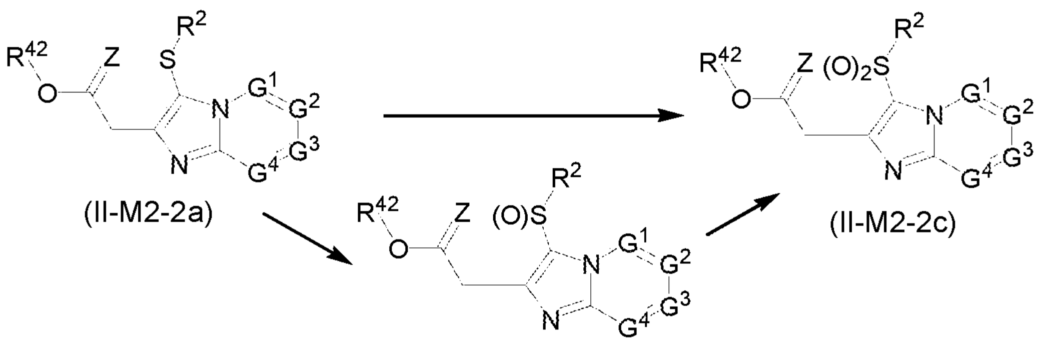

(II-M2-2a) (II-M2-2c)

(II-M2-2b)

[wherein symbols represent the same meaning as described above.]

These reactions can be carried out in accordance with the method described in Production Method 1.

Reference Production Method 6

The compound (II-M2-2a) can be produced by reacting the compound (II-M1-7) with the compound (R-3).

[Chem. 47]

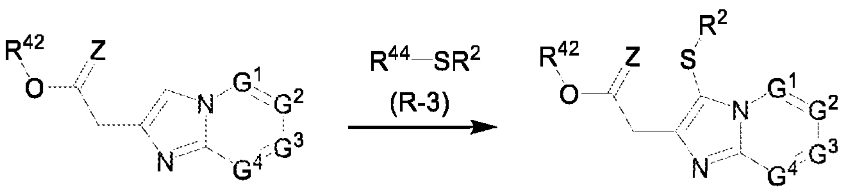


(II-M1-7) (II-M2-2a)

[wherein symbols represent the same meaning as described above.]

The reaction can be performed according to a method described in, for example, Chem. Eur. J., 2016, 22, 11854.

Reference Production Method 7

The compound (II-M3-1) can be produced according to the following scheme.

[Chem. 48]

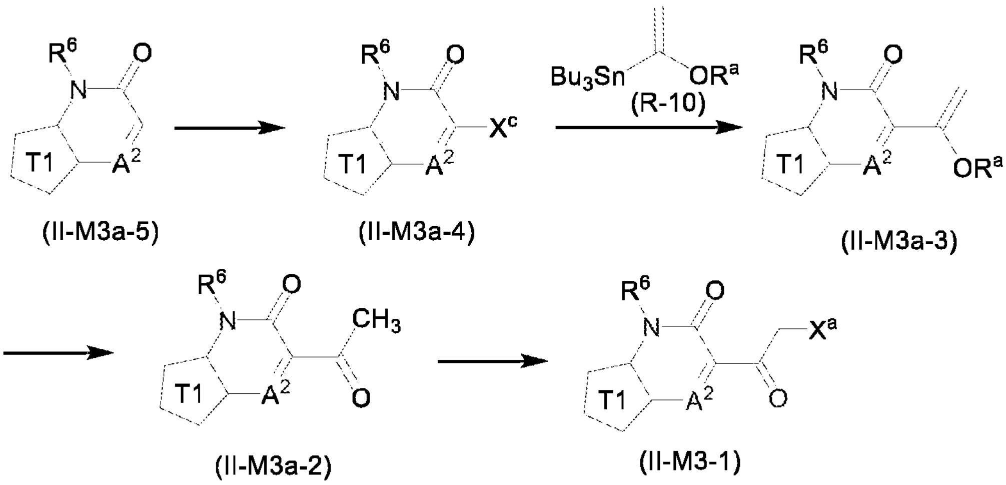

(R-10)

(II-M3a-5) (II-M3a-4) (II-M3a-3)

(II-M3a-2) (II-M3-1)

[wherein $R^a$ represents a methyl group or an ethyl groups, and other symbols represent the same meaning as described above.]

A compound represented by Formula (II-M3a-4) (hereinafter, referred to as a compound (II-M3a-4)) can be produced by reacting a compound represented by Formula (II-M3a-5) (hereinafter, referred to as a compound (II-M3a-5)) with a halogenating agent. The reaction can be performed according to a method described in, for example, WO 2012/020780 A or Chem. Commun., 2015, 51, 17744.

The compound (II-M3a-5) is a commercially available compound, or can be produced in accordance with a known method.

A compound represented by Formula (II-M3a-3) (hereinafter, referred to as a compound (II-M3a-3)) can be produced by reacting the compound (II-M3a-4) with a compound represented by Formula (R-10) (hereinafter, referred to as a compound (R-10)). The reaction can be performed, for example, in accordance with the method described in WO 2016/123253 A.

The compound (R-10) is known, or can be produced in accordance with a known method.

A compound represented by Formula (II-M3a-2) (hereinafter, referred to as a compound (II-M3a-2)) can be produced by reacting the compound (II-M3a-3) with acid. The reaction can be performed, for example, in accordance with the method described in WO 2016/123253 A.

The compound (II-M3-1) can be produced by reacting the compound (II-M3a-2) with a halogenating agent. The reaction can be performed in accordance with the method described in WO 2013/191113 A.

Reference Production Method 8

A compound represented by Formula (III-M2-1) (hereinafter, referred to as (III-M2-1)) can be produced according to the following scheme.

[Chem. 49]

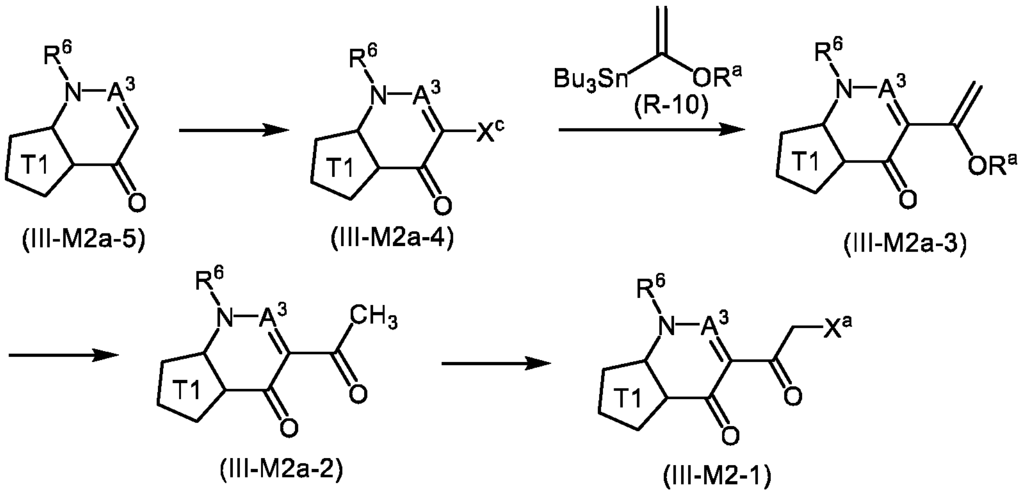
(R-10)

(III-M2a-5) (III-M2a-4) (III-M2a-3)

(III-M2a-2) (III-M2-1)

[wherein symbols represent the same meaning as described above.]

The reactions can be performed in accordance with Reference Production Method 7.

The compound represented by Formula (III-M2a-5) is a commercially available compound, or can be produced in accordance with a known method.

Reference Production Method 9

A compound represented by Formula (IV-M2-1) (hereinafter, referred to as (IV-M2-1)) can be produced according to the following scheme.

[Chem. 50]

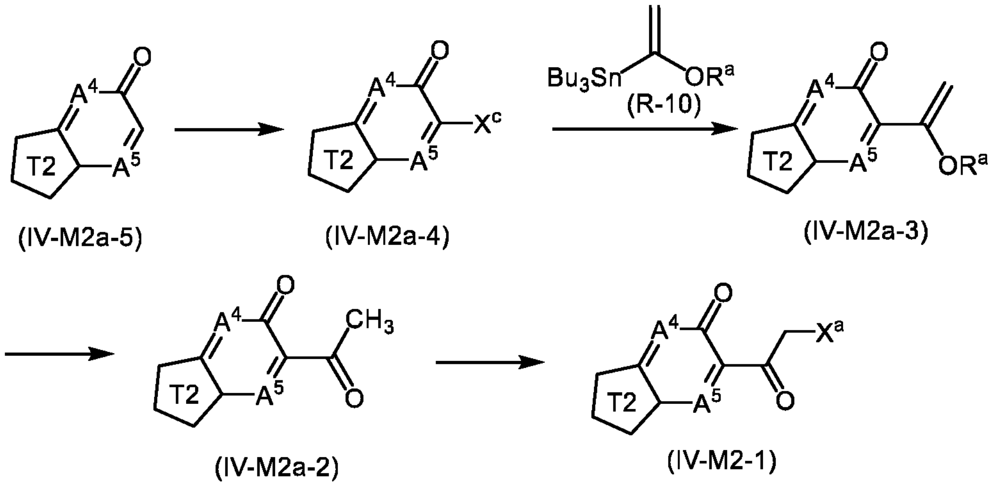
(R-10)

(IV-M2a-5) (IV-M2a-4) (IV-M2a-3)

(IV-M2a-2) (IV-M2-1)

[wherein symbols represent the same meaning as described above.]

The reactions can be performed in accordance with Reference Production Method 7.

The compound represented by Formula (IV-M2a-5) is a commercially available compound, or can be produced in accordance with a known method.

Reference Production Method 10

A compound represented by Formula (V-M2-1) (hereinafter, referred to as (V-M2-1)) can be produced according to the following scheme.

[Chem. 51]

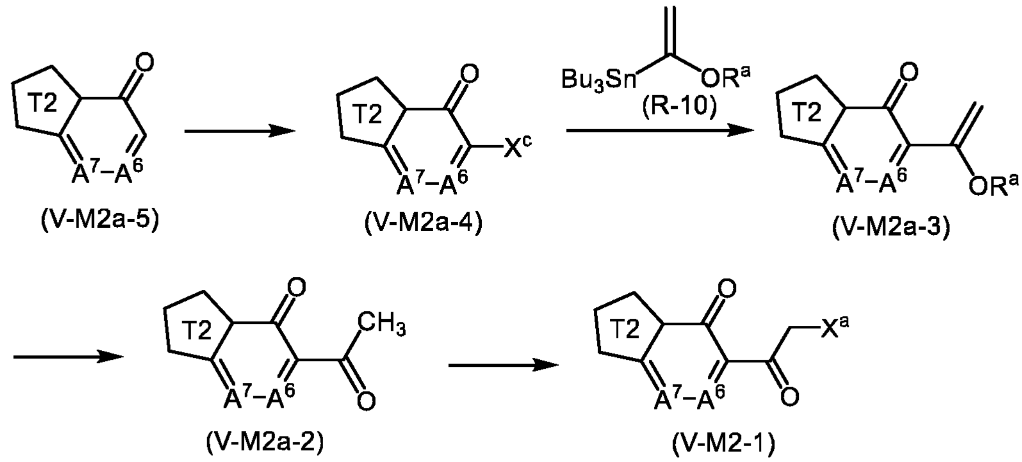


(V-M2a-5) (V-M2a-4) (V-M2a-3) (V-M2a-2) (V-M2-1)

[wherein symbols represent the same meaning as described above.]

The reactions can be performed in accordance with Reference Production Method 7.

The compound represented by Formula (V-M2a-5) is a commercially available compound, or can be produced in accordance with a known method.

Reference Production Method 11

A compound represented by Formula (VI-1-b) and a compound represented by Formula (VI-1-c) can be produced by reacting the compound represented by Formula (VI-1-a) with an oxidizing agent.

[Chem. 52]

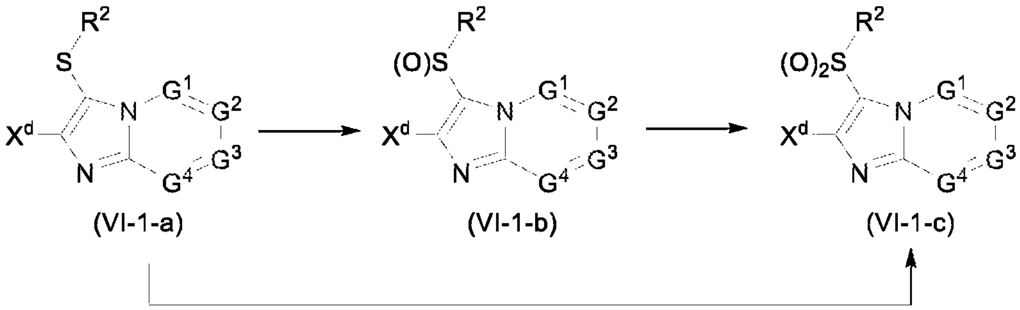

(VI-1-a) (VI-1-b) (VI-1-c)

[wherein $X^d$ represents a fluorine atom or a chlorine atom, and other symbols represent the same meaning as described above.]

The reactions can be performed in accordance with Production Method 1.

Reference Production Method 12

A compound represented by Formula (VI-1-0) (hereinafter, referred to as a compound (VI-1-0)) can be produced according to the following scheme.

[Chem. 53]

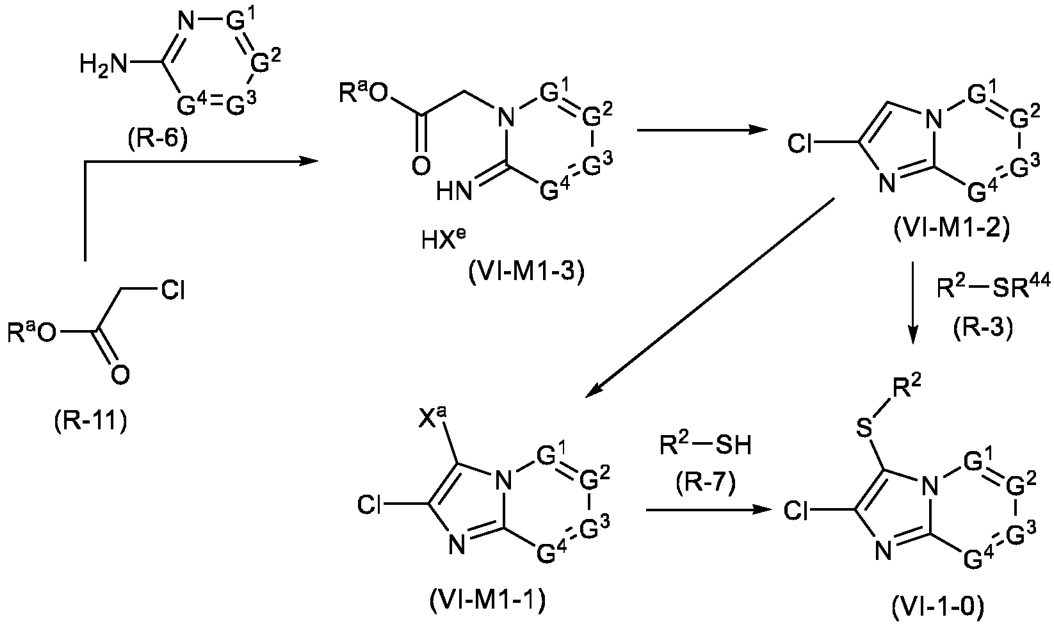


[wherein $X^e$ represents a chlorine atom, a bromine atom or an iodine atom, and other symbols represent the same meaning as described above.]

A compound represented by Formula (VI-M1-3) (hereinafter, referred to as a compound (VI-M1-3)) can be produced by reacting the compound (R-6) with a compound represented by Formula (R-11) (hereinafter, referred to as a compound (R-11)).

The reaction is usually performed in a solvent. Examples of the solvent include aromatic hydrocarbons, alcohols, nitriles, and mixtures of two or more kinds thereof.

In the reaction, the compound (R-6) is usually used in a ratio of 1 to 10 mol, based on 1 mol of the compound (R-11).

The reaction temperature is usually in the range of 0°C to 200°C. The reaction time is usually in the range of 0.1 to 48 hours.

After completion of the reaction, the compound (VIM1-3) can be obtained by subjecting it to normal post-process operations.

The compound (R-11) is a commercially available compound, or can be produced in accordance with a known method.

A compound Formula (VI-M1-2) (hereinafter, referred to as a compound (VI-M1-2)) can be produced by reacting the compound (VI-M1-3) with phosphorus oxychloride.

The reaction is usually performed in a solvent. Examples of the solvent include aromatic hydrocarbons, nitriles, and mixtures of two or more kinds thereof.

In the reaction, the phosphorus oxychloride is usually used in a ratio of 1 to 10 mol, based on 1 mol of the compound (VI-M1-3).

The reaction temperature is usually in the range of 60°C to 120°C. The reaction time is usually in the range of 0.1 to 48 hours.

After completion of the reaction, the compound (VIM1-2) can be obtained by subjecting it to normal post-process operations.

A compound Formula (VI-M1-1) (hereinafter, referred to as a compound (VI-M1-1)) can be produced by reacting the compound (VI-M1-2) with halogenating agent. The reaction can be performed in accordance with the method for producing the compound (II-M3-3) from the compound (II-M3-2) of Production Method 13.

The compound (VI-1-0) can be produced by reacting the compound (VI-M1-2) with the compound (R-3). The reaction can be performed in accordance with the method for producing the compound (II-3) from the compound (II-M3-2) of Production Method 13.

In addition, the compound (VI-1-0) can be produced in accordance with the method for producing the compound (II-3) from the compound (II-M3-3) of Production Method 13.

Reference Production Method 13

The compound (VI-1) can be produced by reacting a compound represented by formula (VI-1-d) with cesium

fluoride.

[Chem. 54]

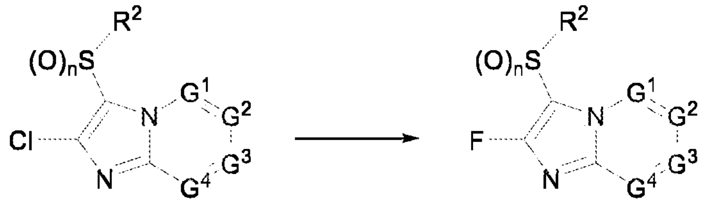

(VI-1-d) (VI-1)

[wherein symbols represent the same meaning as described above.]

The reaction can be performed according to a method described in, for example, Tetrahedron Lett., 2015, 56, 6043.

The compound of the present invention or the compound X of the present invention can be mixed with or used in combination with one or more ingredients (hereinafter, referred to as present ingredient) selected from the group consisting of a group (a), a group (b), a group (c), and a group (d).

The term "mixed with or used in combination" means that the compound of the present invention or the compound X of the present invention and the present ingredient are used simultaneously, separately, or at time intervals.

When the compound of the present invention or the compound X of the present invention and the present ingredient are used simultaneously, the compound of the present invention or the compound X of the present invention and the present ingredient may be contained in separate formulations, or may be contained in one formulation.

One aspect of the present invention relates to a composition containing one or more ingredients selected from the group consisting of a group (a), a group (b), a group (c), and a group (d), and the compound of the present invention.

One aspect of the present invention relates to a composition (hereinafter, referred to as a composition A) containing one or more ingredients selected from the group consisting of a group (a), a group (b), a group (c), and a group (d), and the compound X of the present invention.

The group (a) is a group consisting of an acetylcholinesterase inhibitor (for example, carbamate insecticide and organophosphorus insecticide), a GABAergic chloride channel blocker (for example, phenylpyrazole-based insecticide), a sodium channel modulator (for example, pyrethroid insecticide), a nicotinic acetylcholine receptor competitive modulator (for example, neonicotinoid insecticide), a nicotinic acetylcholine receptor allosteric modulator, a glutamatergic chloride channel allosteric modulator (for example, macrolide insecticide), a juvenile hormone mimic, a multisite inhibitor, a chordal organ TRPV channel modulator, a mite growth inhibitor, a microorganism-derived insect mid-intestinal membrane disruptor, a mitochondrial ATP synthase inhibitor, an oxidative phosphorylation uncoupler, a nicotinic acetylcholine receptor channel blocker (for example, nereistoxin-based insecticide), a chitin synthesis inhibitor, a moulting inhibitor, an ecdysone receptor agonist, an octopamine receptor agonist, inhibitors of mitochondrial electron transport chain complex I, II, III and IV, a voltage-gated sodium channel blocker, an acetyl-CoA carboxylase inhibitor, a ryanodine receptor modulator (for example, diamide insecticide), a chordotonal organ modulator, a microbial insecticide, and other insecticidal, miticidal, and nematicidal active ingredients. These are described in the classification based on the mechanism of action of IRAC.

The group (b) is a group consisting of a nucleic acid synthesis inhibitor (for example, phenylamide-based microbicide or acylamino acid-based microbicide), a cell division and cytoskeleton inhibitor (for example, MBC disinfectant), a respiratory inhibitor (for example, QoI fungicide and QiI fungicide), an amino acid and protein synthesis inhibitor (for example, anilinopyridine-based microbicide), a signal transduction inhibitor, a lipid and membrane synthesis inhibitor, a sterol biosynthesis inhibitor (for example, DMI fungicide such as a triazole fungicide), a cell wall synthesis inhibitor, a melanin synthesis inhibitor, a plant defense inducer, a multi-action point contact active bactericide, a microbial bactericide, and other bactericidal active ingredients. These are described in the classification based on the mechanism of action of FRAC.

The group (c) is a group of plant growth regulating ingredients (including mycorrhizae and rhizobia).

The group (d) is a group of repellent ingredients.

Hereinafter, examples of combinations of the present ingredient and the compound X of the present invention will be described. For example, alanycarb + SX means the combination of alanycarb and SX.

An abbreviation SX means any one compound X of the present invention selected from the compound groups SX1 to SX1491 described in Examples. The present ingredients described below are all known ingredients, and can be

obtained from a commercially available formulation or produced by a known method. When the present ingredient is a microorganism, it can also be obtained from a bacterial depository. The number in parentheses represents CAS RN (registered trademark).

Combinations of the present ingredients from the group (a) above and the compound X of the present invention: abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cycloprothrin + SX, cycloxaprid + SX, cyenopyrafen + SX, cyflumetofen + SX, cyfluthrin + SX, cyhalodiamide + SX, cyhalothrin + SX, cyhexatin + SX, cypermethrin + SX, cyphenothrin + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octoborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl 0-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Cassia nigricans + SX, extract of clitoria ternatea + SX, extract of Symphytum officinale + SX, extracts or simulated blend of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, flucycloxuron + SX, flucythrinate + SX, fluensulfone + SX, flufenoprox + SX, flufenoxuron + SX, flufiprole + SX, flumethrin + SX, flupyradifurone + SX, flupyrimin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hvla peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indoxacarb + SX, isocycloseram + SX, isofenphos + SX, isoprocarb + SX, isopropyl-O-(methoxyaminothiophosphoryl) salicylate + SX isoxathion + SX, ivermectin + SX, kadethrin + SX, kappa-tefluthrin + SX, kappa-bifenthrin + SX, kinoprene + SX, lambda-cyhalothrin + SX, lenoremycin + SX, lepimectin + SX, Calcium polysulfide (lime sulfur) + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, momfluorothrin + SX, monocrotophos + SX, moxidectin + SX, naled + SX, neem oil + SX, nicotine + SX, nicotine sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spinetoram + SX, spinosad + SX, spirodiclofen + SX, spiromesifen + SX, spiropidion + SX, spirotetramat + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tioxazafen + SX, tolfenpyrad + SX, tralomethrin + SX, transfluthrin + SX, triazamate + SX, triazophos + SX, trichlorfon + SX, triflumezopyrim + SX,

triflumuron + SX, trimethacarb + SX, tyclopyrazoflor + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, 2-chloro-4-fluoro-5-{[5-(trifluoromethylthio)pentyl]oxy}phenyl 2,2,2-trifluoroethyl sulfoxide (1472050-04-6) + SX, 4-chloro-5-[2,2-difluoro-2-(3,4,5-trifluorophenyl)ethoxy]-2-methylphenyl 2,2,2-trifluoroethyl sulfoxide (1632218-00-8) + SX, 4-fluoro-5-[2,2-difluoro-2-(3,4,5-trifluorophenyl)ethoxy]-2-methylphenyl 2,2,2-trifluoroethyl sulfoxide (1632217-98-1) + SX, 2-({2-fluoro-4-methyl-5-[(2,2,2-trifluoroethyl)sulfinyl]phenyl}imino)-3-(2,2,2-trifluoroethyl)-1,3-thiazolidin-4-one (1445683-71-5) + SX, (1Z)-2-(4-tert-butylphenyl)-2-cyano-1-(1-ethyl-3-methyl-1H-pyrazol-5-yl)ethenyl 2,2-dimethylpropanoate (1253429-01-4) + SX, N-[(1S,2S)-2-(2,4-dichlorophenyl)cyclobutyl]-2-(trifluoromethyl)pyridine-3-carboxamide (1644251-74-0) + SX, (3R)-3-(2-chlorothiazol-5-yl)-8-methyl-7-oxo-6-phenyl-2,3-dihydrothiazolo[3,2-a]pyrimidin-4-ium-5-olate (2249718-27-0) + SX, BT crop protein Cry1Ab + SX, BT crop protein Cry1Ac + SX, BT crop protein Cry1Fa + SX, BT crop protein Cry1A. 105 + SX, BT crop protein Cry2Ab + SX, BT crop protein Vip3A + SX, BT crop protein Cry3A + SX, BT crop protein Cry3Ab + SX, BT crop protein Cry3Bb + SX, BT crop protein Cry34Ab1/Cry35Ab1 + SX, Adoxophyes orana granulosis virus strain BV-0001 + SX, Anticarsia gemmatalis mNPV + SX, Autographa californica mNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV strain BV-0003 + SX, Helicoverpa zea NPV + SX, Lymantria dispar NPV + SX, Mamestra brassicae NPV + SX, Mamestra configurata NPV + SX, Neodiprion avietis NPV + SX, Neodiprion lcontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua mNPV) + SX, Spodoptera littoralis mNPV + SX, Spodoptera litura NPV + SX, Arthrobotrys dactyloides + SX, Bacillus firmus strain GB-126 + SX, Bacillus firmus strain 1-1582 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 + SX, Bacillus sphaericus strain ABTS1743 + SX, Bacillus sphaericus Serotype strain H5a5b + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain EG234 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thoynei + SX, Serratia entomophila + SX, Verticillium chlamydosporium + SX, Verticillium lecani strain NCIM1312 + SX, 2-chloro-N-cyclopropyl-5-{1-[2,6-dichloro-4-(1,1,1,2,3,3,3-heptafluoropropan-2-yl)phenyl]-1H-pyrazol-4-yl}-N-methylpyridine-3-carboxamide (1771741-86-6) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, 11-(4-chloro-2,6-dimethylphenyl)-12-hydroxy-1,4-dioxa-9-azadispiro[4.2.4.2]tetradec-11-en-10-one (907187-07-9) + SX, 3-(4'-fluoro-2,4-dimethyl[1,1'-biphenyl]-3-yl)-4-hydroxy-8-oxa-1-azaspiro[4.5]dec-3-en-2-one (1031385-91-7) + SX, N-[3-chloro-1-(pyridin-3-yl)-1H-pyrazol-4-yl]-2-(methanesulfonyl)propanamide (2396747-83-2) + SX, 2-isopropyl-5-[(3,4,4-trifluoro-3-buten-1-yl)sulfonyl]-1,3,4-thiadiazole (2058052-95-0) + SX, 1,4-dimethyl-2-[2-(3-pyridinyl)-2H-indazol-5-yl]-1,2,4-triazolidine-3,5-dione (2171099-09-3) + SX, cyproflanilide + SX.

Combinations of the present ingredients from the group (b) above and the compound X of the present invention: acibenzolar-S-methyl + SX, aldimorph + SX, ametoctradin + SX, aminopyrifen + SX, amisulbrom + SX, anilazine + SX,

azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroneb + SX, chlorothalonil + SX, chlozolinate + SX, colletochlorin B + SX, copper (II) acetate + SX, copper (II) hydroxide + SX, copper oxychloride + SX, copper (II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract from Melaleuca alternifolia + SX, extract from Reynoutria sachalinensis + SX, extract from the cotyledons of lupine plantlets "BLAD" + SX, extract of Allium sativum + SX, extract of Equisetum arvense + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, fludioxonil + SX, flufenoxystrobin + SX, fluindapyr + SX, flumorph + SX, fluopicolide + SX, fluopyram + SX, fluopimomide + SX, fluoroimide + SX, fluoxapiprolin + SX, fluoxastrobin + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin+ SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metconazole + SX, methasulfocarb + SX, metiram + SX, metominostrobin + SX, metrafenone + SX, metyltetraprole + SX, mineral oils + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-choro-4-(2-fluorophenoxy)-2-methylphenyl)-N-ethyl-N-methylmethanimidamide (2055589-28-9) + SX, N'-[2-choro-4-(2-fluorophenoxy)-5-methylphenyl)-N-ethyl-N-methylmethanimidamide (2055756-21-1) + SX, N'-[4-(1-hydroxy-1-phenyl-2,2,2-trifluoroethyl)-2-methyl-5-methoxyphenyl)-N-isopropyl-N-methylmethanimidamide (2101814-55-3) + SX, N'-[5-bromo-6-(1-methyl-2-propoxyethoxy)-2-methylpyridin-3-yl)-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, 5-fluoro-4-imino-3-methyl-1-tosyl-3,4-dihydropyrimidin-2(1H)-one (1616664-98-2) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R,2S,5S)-5-(4-chlorobenzyl)-2-(chloromethyl)-2-methyl-1-(1H-1,2,4-triazol-1-ylmethyl)cyclopentan-1-ol (1801930-06-2) + SX, (1S,2R,5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R,2S,5S)-2-(chloromethyl)-5-(4-fluorobenzyl)-2-methyl-1-(1H-1,2,4-triazol-1-ylmethyl)cyclopentan-1-ol (1801930-08-4) + SX, (1S,2R,5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 4-({6-[2-(2,4-difluorophenyl)-1,1-difluoro-2-hydroxy-3-(1H-1,2,4-triazol-1-yl)propyl]pyridin-3-yl}oxy)benzonitrile (2046300-61-0) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, Agrobacterium radiobactor strain K1026 + SX, Agrobacterium radiobactor strain K84 + SX, Bacillus amyloliquefaciens (Aveo (trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 +

SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX, flubeneteram + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, (2S,3S)-3-(2-methylphenyl)butan-2-yl N-[(3-acetoxy-4-methoxypyridin-2-yl)carbonyl]-L-alaninate (2376210-00-1) + SX, (2S,3S)-3-(4-fluoro-2-methylphenyl)butan-2-yl N-[(3-acetoxy-4-methoxypyridin-2-yl)carbonyl]-L-alaninate + SX, (2S,3S)-3-(4-methoxy-2-methylphenyl)butan-2-yl N-[(3-acetoxy-4-methoxypyridin-2-yl)carbonyl]-L-alaninate + SX, (2S,3S)-3-(2,4-dimethylphenyl)butan-2-yl N-[(3-acetoxy-4-methoxypyridin-2-yl)carbonyl]-L-alaninate (2376209-13-9) + SX, (2S,3S)-3-(2-methylphenyl)butan-2-yl N-({3-[(2-methylpropanoyl)oxy]-4-methoxypyridin-2-yl}carbonyl)-L-alaninate (2376210-02-3) + SX, (2S,3S)-3-(4-fluoro-2-methylphenyl)butan-2-yl N-({3-[(2-methylpropanoyl)oxy]-4-methoxypyridin-2-yl}carbonyl)-L-alaninate, (2S,3S)-3-(4-methoxy-2-methylphenyl)butan-2-yl N-({3-[(2-methylpropanoyl)oxy]-4-methoxypyridin-2-yl}carbonyl)-L-alaninate (2376209-40-2) + SX, (2S,3S)-3-(2,4-dimethylphenyl)butan-2-yl N-({3-[(2-methylpropanoyl)oxy]-4-methoxypyridin-2-yl}carbonyl)-L-alaninate (2376209-15-1) + SX, N'-(2-choro-4-phenoxy-5-methylphenyl)-N-ethyl-N-methylmethanimidamide (2062599-39-5) + SX.

Combinations of the present ingredients from the group (c) above and the compound X of the present invention: 1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, pacrobutrazol + SX, pendimethalin + SX, prohexandione-calcium + SX, prohydrojasmon + SX, pyraflufen-ethyl + SX, sintofen + SX, sodium 1-naphthaleneacetate + SX, sodium cyanate + SX, streptmycin + SX, thidiazuron + SX, triapenthenol + SX, Tribufos + SX, trinexapac-ethyl + SX, uniconazole-P + SX, 2-(naphthalen-1-yl)acetamide + SX, [4-oxo-4-(2-phenylethyl) amino] butyric acid + SX, 5-(trifluoromethyl) benzo [b] thiophene-2-methyl carboxylate + SX, 3-[(6-chloro-4-phenylquinazoline-2-yl) amino] propane-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combinations of the present ingredients from the group (d) above and the compound X of the present invention: anthraquinone + SX, deet + SX, and icaridin + SX.

The ratio of the compound X of the present invention to the present ingredient is not particularly limited, and examples thereof include 1000 : 1 to 1 : 1000, 500 : 1 to 1 : 500, 100 : 1 to 1 : 100, 50 : 1, 20 : 1, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, 1 : 10, 1 : 20, and 1 : 50 in terms of a weight ratio (the compound X of the present invention: the present ingredient).

The compound X of the present invention has an effect on harmful arthropods such as harmful insects and harmful mites, harmful nematodes, and harmful molluscs. Examples of the harmful arthropod, harmful nematodes, and harmful molluscs include those shown below.

Hemiptera:

from the family Delphacidae, for example, small brown planthopper (Laodelphax striatellus), brown planthopper (Nilaparvata lugens), white-backed planthopper (Sogatella furcifera), corn planthopper (Peregrinus maidis), cereal leafhopper (Javesella pellucida), sugarcane leafhopper (Perkinsiella saccharicida), and Tagosodes orizicolus;

from the family Cicadellidae, for example,
green rice leafhopper (Nephotettix cincticeps), green paddy leafhopper (Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidis), rice leafhopper (Cofana spectra), and Amrasca biguttula biguttula;
from the family Aphrophoridae, for example, European spittlebug (Philaenus spumarius);
from the family Cercopidae, for example, Mahanarva posticata and Mahanarva fimbriolata;
from the family Aphididae, for example,
bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), rosy apple aphid (Dysaphis plantaginea), false cabbage aphid (Lipaphis erysimi), potato aphid (Macrosiphum euphorbiae), foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), and English grain aphid (Sitobion avenae);
from the family Phylloxeridae, for example,
grapevine phylloxera (Daktulosphaira vitifoliae), Pecan phylloxera (Phylloxera devastatrix), Pecan leaf phylloxera (Phylloxera notabilis), and Southern pecan leaf phylloxera (Phylloxera russelae);
from the family Adelgidae, for example, hemlock woolly aphid (Adelges tsugae), balsam woolly aphid (Adelges piceae), and Aphrastasia pectinatae;
from the family Pentatomidae, for example, black rice bug (Scotinophara lurida), black paddy bug (Scotinophara coarctata), common green stink bug (Nezara antennata), white-spotted spined bug (Eysarcoris aeneus), lewis spined bug (Eysarcoris lewisi), white-spotted bug (Eysarcoris ventralis), Eysarcoris annamita, brown marmorated stink bug (Halyomorpha halys), green plant bug (Nezara viridula), brown stink bug (Euschistus heros), red banded stink bug (Piezodorus guildinii), Oebalus pugnax, and Dichelops melacanthus;
from the family Cydnidae, for example, Scaptocoris castanea;
from the family Alydidae, for example, bean bug (Riptortus clavatus), corbett rice bug (Leptocorisa chinensis), and rice bug (Leptocorisa acuta);
from the family Coreidae, for example, Cletus punctiger and Australian leaf-footed bug (Leptoglossus australis);
from the family Lygaeidae, for example, oriental chinch bug (Cavelerius saccharivorus), seed bug (Togo hemipterus), and chinch bug (Blissus leucopterus);
from the family Miridae, for example, rice leaf bug (Trigonotylus caelestialium), sorghum plant bug (Stenotus rubrovittatus), wheat leaf bug (Stenodema calcarata), and American tarnished plant bug (Lygus lineolaris);
from the family Aleyrodidae, for example, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), and Pealius euryae;
from the family Diaspididae, for example, Abgrallaspis cyanophylli, red scale (Aonidiella aurantii), San Jose scale (Diaspidiotus perniciosus), white peach scale (Pseudaulacaspis pentagona), arrowhead scale (Unaspis yanonensis), and citrus snow scale (Unaspis citri);
from the family Coccidae, for example, pink wax scale (Ceroplastes rubens);
from the family Margarodidae, for example, fluted scale (Icerya purchasi) and seychelles fluted scale (Icerya seychellarum);
from the family Pseudococcidae, for example, solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), currant mealybug (Pseudococcus calceolariae), long-tailed mealybug (Pseudococcus longispinus), and tuttle mealybug (Brevennia rehi);
from the family Psyllidae, for example, citrus psylla (Diaphorina citri), two-spotted citrus psyllid (Trioza erytreae), pear sucker (Cacopsylla pyrisuga), Cacopsylla chinensis, potato psyllid (Bactericera cockerelli), and Pear psylla (Cacopsylla pyricola);
from the family Tingidae, for example, sycamore lace bug (Corythucha ciliata), aster tingid (Corythucha marmorata), Japanese pear lace bug (Stephanitis nashi), and azalea lace bug (Stephanitis pyrioides);
from the family Cimicidae, for example, common bed bug (Cimex lectularius) and tropical bed bug (Cimex hemipterus);
from the family Cicadidae, for example, Quesada gigas;
from the family Reduviidae, for example, Triatoma infestans, large kissing bug (Triatoma rubrofasciata), Triatoma dimidiata, and Rhodonius prolixus;
and the others.

Lepidoptera:

from the family Crambidae, for example, rice stem borer (Chilo suppressalis), Dark-headed stem borer (Chilo polychrysus), white stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Parapediasia teterrellus), rice case-worm (Nymphula depunctalis), Sugarcane borer (Diatraea saccharalis), and eggplant fruit borer (Leucinodes orbonalis);

from the family Pyralidae, for example, lesser cornstalk borer (Elasmopalpus lignosellus), mealworm moth (Plodia interpunctella), persimmon bark borer (Euzophera batangensis), and fig moth (Cadra cautella);
from the family Noctuidae, for example, cotton worm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), Spodoptera frugiperda, true armyworm (Spodoptera exempta), semitropical armyworm (Spodoptera eridania), black cutworm (Agrotis ipsilon), beet worm (Autographa nigrisigna), rice looper (Plusia festucae), Soybean looper (Chrysodeixis includens), Trichoplusia spp., Heliothis spp. (such as tobacco budworm (Heliothis virescens)), Helicoverpa spp. (such as tobacco budworm (Helicoverpa armigera), corn earworm (Helicoverpa zea)), velvet-bean caterpillar (Anticarsia gemmatalis), cotton leafworm (Alabama argillacea), and hop vine borer (Hydraecia immanis);
from the family Pieridae, for example, common cabbage worm (Pieris rapae);
from the family Tortricidae, for example, oriental fruit moth (Grapholita molesta), Grapholita dimorpha, soybean moth (Leguminivora glycinivorella), Matsumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), bean shoot borer (Epinotia aporema), citrus fruit borer (Citripestis sagittiferella), and European grapevine moth (Lobesia botrana);
from the family Gracillariidae, for example, tea leaf roller (Caloptilia theivora) and Asiatic apple leaf miner (Phyllonorycter ringoniella);
from the family Carposinidae, for example, peach fruit moth (Carposina sasakii);
from the family Lyonetiidae, for example, coffee leaf miner (Leucoptera coffeella), peach leaf miner (Lyonetia clerkella), and Lyonetia prunifoliella;
from the family Lymantriidae, for example, Lymantria spp. (such as gypsy moth (Lymantria dispar)) and Euproctis spp. (such as tea lymantriid (Euproctis pseudoconspersa));
from the family Plutellidae, for example, diamondback moth (Plutella xylostella);
from the family Gelechiidae, for example, peach worm (Anarsia lineatella), sweetpotato leaf folder (Helcystogramma triannulella), pink bollworm (Pectinophora gossypiella), potato moth (Phthorimaea operculella), and Tuta absoluta;
from the family Arctiidae, for example, American white moth (Hyphantria cunea);
from the family Castniidae, for example, giant sugarcane borer (Telchin licus);
from the family Cossidae, for example, Cossus insularis;
from the family Geometridae, for example, Ascotis selenaria;
from the family Limacodidae, for example, blue-striped nettle grub (Parasa lepida);
from the family Stathmopodidae, for example, persimmon fruit moth (Stathmopoda masinissa);
from the family Sphingidae, for example, tobacco hornworm (Acherontia lachesis);
from the family Sesiidae, for example, Nokona feralis, cherry borer (Synanthedon hector), and Synanthedon tenuis:

from the family Hesperiidae, for example, rice skipper (Parnara guttata);
from the family Tineidae, for example, casemaking clothes moth (Tinea translucens), common clothes moth (Tineola bisselliella);

and the others.

Thysanoptera:

from the family Thripidae, for example, western flower thrips (Frankliniella occidentalis), oriental thrips (Thrips palmi), yellow tea thrips (Scirtothrips dorsalis), onion thrips (Thrips tabaci), eastern flower thrips (Frankliniella intonsa), rice thrips (Stenchaetothrips biformis), Echinothrips americanus, and avocado thrips (Scirtothrips perseae);

from the family Phlaeothripidae, for example, aculeated rice thrips (Haplothrips aculeatus);
and the others.

Diptera:

from the family Anthomyiidae, for example, seedcorn maggot (Delia platura), onion maggot (Delia antiqua), and beet leaf miner (Pegomya cunicularia);
from the family Ulidiidae, for example, sugarbeet root maggot (Tetanops myopaeformis);
from the family Agromyzidae, for example, rice leaf miner (Agromyza oryzae), tomato leaf miner (Liriomyza sativae), chrysanthemum leaf miner (Liriomyza trifolii), and pea leafminer (Chromatomyia horticola);
from the family Chloropidae, for example, rice stem maggot (Chlorops oryzae);
from the family Tephritidae, for example, melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), Mediterranean fruit fly (Ceratitis capitata), apple maggot (Rhagoletis pomonella), and Japanese cherry fruit fly (Rhacochlaena japonica);
from the family Ephydridae, for example, smaller rice leaf miner (Hydrellia griseola), whorl maggot (Hydrellia philippina), and paddy stem maggot (Hydrellia sasakii);
from the family Drosophilidae, for example, cherry drosophila (Drosophila suzukii), and common fruit fly (Drosophila melanogaster);
from the family Phoridae, for example, Megaselia spiracularis;
from the family Psychodidae, for example, Clogmia albipunctata;
from the family Sciaridae, for example, Bradysia difformis;
from the family Cecidomyiidae, for example, hessian fly (Mayetiola destructor) and paddy gall fly (Orseolia oryzae);
from the family Diopsidae, for example, Diopsis macrophthalma;
from the family Glossinidae, for example, Glossina palpalis and Glossina morsitans;
from the family Simuliidae, for example, Simulium japonicum and Simulium damnosum;
from the subfamily Phlebotominae;
from the family Tipulidae, for example, rice crane fly (Tipula aino), common cranefly (Tipula oleracea), and European cranefly (Tipula paludosa);
from the family Culicidae, for example, southern house mosquito (Culex pipiens pallens), Culex tritaeniorhynchus, Culex pipiens f. molestus, brown house mosquito (Culex quinquefasciatus), northern house mosquito (Culex pipiens pipiens), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, and Anopheles minimus;
from the family Simulidae, for example, Prosimulium yezoensis and Simulium ornatum;
from the family Tabanidae, for example, Tabanus trigonus;
from the family Muscidae, for example, house fly (Musca domestica), false stable fly (Muscina stabulans), biting house fly (Stomoxys calcitrans), and buffalo fly (Haematobia irritans);
from the family Calliphoridae;
from the family Sarcophagidae;
from the family Chironomidae, for example, Chironomus plumosus, Chironomus yoshimatsui, and Glyptotendipes tokunagai;
from the family Fannidae;
and the others.

Coleoptera:

from the family Chrysomelidae, for example, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), cucurbit beetle (Diabrotica speciosa)), bean leaf beetle (Cerotoma trifurcata), barley leaf beetle (Oulema melanopus), cucurbit leaf beetle (Aulacophora femoralis), striped flea beetle (Phyllotreta striolata), cabbage flea beetle (Phyllotreta cruciferae), western black flea beetle (Phyllotreta pusilla), cabbage stem flea beetle (Psylliodes chrysocephala), hop flea beetle (Psylliodes punctulata), Colorado potato beetle (Leptinotarsa decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice leaf beetle (Dicladispa armigera), southern corn

leaf beetle (Myochrous denticollis), Laccoptera quadrimaculata, and tobacco flea beetle (Epitrix hirtipennis);
from the family Carabidae, for example, Seedcorn beetle (Stenolophus lecontei) and slender seed-corn ground beetle (Clivina impressifrons);
from the family Scarabaeidae, for example, cupreus chafer (Anomala cuprea), soybean beetle (Anomala rufocuprea), Anomala albopilosa, Japanese beetle (Popillia japonica), yellowish elongate chafer (Heptophylla picea), European Chafer (Rhizotrogus majalis), Tomarus gibbosus, Holotrichia spp., Phyllophaga spp. (such as June beetle (Phyllophaga crinita)), and Diloboderus spp. (such as Diloboderus abderus);
from the family Anthriibidae, for example, coffee bean weevil (Araecerus coffeae);
from the family Aponidae, for example, sweet-potato weevil (Cylas formicarius);
from the family Bruchidae, for example, Mexican bean weevil (Zabrotes subfasciatus);
from the family Scolytidae, for example, pine beetle (Tomicus piniperda) and coffee berry borer (Hypothenemus hampei);
from the family Curculionidae, for example, West Indian sweet-potato weevil (Euscepes postfasciatus), alfalfa weevil (Hypera postica), maize wevil (Sitophilus zeamais), rice weevil (Sitophilus oryzae), grain weevil (Sitophilus granarius), rice plant weevil (Echinocnemus squameus), rice water weevil (Lissorhoptrus oryzophilus), Rhabdoscelus lineaticollis, boll weevil (Anthonomus grandis), nunting billbug (Sphenophorus venatus), southern corn billbug (Sphenophorus callosus), soybean stalk weevil (Sternechus subsignatus), sugarcane weevil (Sphenophorus levis), rusty gourd-shaped weevil (Scepticus griseus), brown gourd-shaped weevil (Scepticus uniformis), Aracanthus spp. (such as Aracanthus mourei), and cotton root borer (Eutinobothrus brasiliensis);
from the family Tenebrionidae, for example, red meal beetle (Tribolium castaneum), mason beetle (Tribolium confusum), and lesser mealworm (Alphitobius diaperinus);
from the family Coccinellidae, for example, twentyeight-spotted ladybird (Epilachna vigintioctopunctata);
from the family Bostrychidae, for example, common powder-post beetle (Lyctus brunneus), and lesser grain borer (Rhizopertha dominica);
from the family Ptinidae;
from the family Cerambycidae, for example, citrus long-horned beetle (Anoplophora malasiaca), Migdolus fryanus, and peach borer (Aromia bungii);
from the family Elateridae, for example, Melanotus okinawensis, barley wireworm (Agriotes fuscicollis), Melanotus legatus, Anchastus spp., Conoderus spp., Ctenicera spp., Limonius spp., and Aeolus spp.;
from the family Staphylinidae, for example, Paederus fuscipes;
from the family Dermestidae, for example, varied carpet beetle (Anthrenus verbasci), hide beetle (Dermestes maculates), and khapra beetle (Trogoderma granarium);
from the family Anobiidae, for example, tobacco beetle (Lasioderma serricorne), and biscuit beetle (Stegobium paniceum);
from the family Laemophloeidae, for example, flat grain beetle (Cryptolestes ferrugineus);
from the family Silvanidae, for example, saw-toothed grain beetle (Oryzaephilus surinamensis);
from the family Nitidulidae, for example, blossom beetle (Brassicogethes aeneus);
and the others.

Orthoptera:

from the family Acrididae, for example, oriental migratory locust (Locusta migratoria), Moroccan locust (Dociostaurus maroccanus), Australian plague locust (Chortoicetes terminifera), red locust (Nomadacris septemfasciata), brown locust (Locustana pardalina), tree locust (Anacridium melanorhodon), Italian locust (Calliptamus italicus), differential grasshopper (Melanoplus differentialis), two-striped grasshopper (Melanoplus bivittatus), migratory grasshopper (Melanoplus sanguinipes), red-legged grasshopper (Melanoplus femurrubrum), clear-winged grasshopper (Camnula pellucida), desert locust (Schistocerca gregaria), Yellow-winged locust (Gastrimargus musicus), spur-throated locust (Austracris guttulosa), Japanese grasshopper (Oxya yezoensis), rice grasshopper (Oxya japonica), and Bombay locust (Patanga succincta);
from the family Gryllotalpidae, for example, oriental mole cricket (Gryllotalpa orientalis);
from the family Gryllidae, for example, house cricket (Acheta domestica), and emma field cricket (Teleogryllus emma);
from the family Tettigoniidae, for example, mormon cricket (Anabrus simplex);
and the others.

Hymenoptera:

from the family Tenthredinidae, for example, beet sawfly (Athalia rosae) and nippon cabbage sawfly (Athalia japonica);
from the family Formicidae, for example, Solenopsis spp. (such as red imported fire ant (Solenopsis invicta), tropical fire ant (Solenopsis geminata)), Atta spp. (such as brown leaf-cutting ant (Atta capiguara), Acromyrmex spp., Paraponera clavata, black house ant (Ochetellus glaber), little red ant (Monomorium pharaonis), Argentine ant (Linepithema humile), Formica japonica, Pristomyrmex punctutus, Pheidole noda, big-headed ant (Pheidole megacephala), Camponotus spp. (such as Camponotus japonicus, Camponotus obscuripes), Pogonomyrmex spp. (such as western harvester ant (Pogonomyrmex occidentalis)), Wasmania spp. (such as Wasmania auropunctata), and longlegged ant (Anoplolepis gracilipes);
from the family Vespidae, for example, Asian giant hornet (Vespa mandarinia, Vespa simillima, Vespa analis, Asian hornet (Vespa velutina), and Polistes jokahamae;
from the family Siricidae, for example, pine wood wasp (Urocerus gigas);
from the family Bethylidae;
and the others.

Blattodea:

from the family Ectobiidae, for example, German cockroach (Blattella germanica);
from the family Blattidae, for example, smoky-brown cockroach (Periplaneta fuliginosa), American cockroach (Periplaneta americana), Australian cockroach (Periplaneta australasiae), brown cockroach (Periplaneta brunnea), and black cockroach (Blatta orientalis);
from the family Termitidae, for example, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, and Cornitermes cumulans;
and the others.

Siphonaptera:

from the family Pulicidae, for example, human flea (Pulex irritans), cat flea (Ctenocephalides felis), dog flea (Ctenocephalides canis), oriental rat flea (Xenopsylla cheopis), and chicken flea (Echidnophaga gallinacea);
from the family Pulicidae, for example, chigoe flea (Tunga penetrans);
from the family Ceratophyllidae, for example, European rat flea (Nosopsyllus fasciatus); and the others.

Psocodae:

from the family Pediculidae, for example, head louse (Pediculus humanus capitis);
from the family Pthiridae, for example, crab louse (Pthirus pubis);
from the family Haematopinidae, for example, shortnosed cattle louse (Haematopinus eurysternus) and pig louse (Haematopinus suis);
from the family Linognathidae, for example, blue cattle louse (Linognathus vituli), sheep face louse (Linognathus ovillus), and capillate louse (Solenopotes capillatus);
from the family Bovicoliidae, for example, cattle biting louse (Bovicola bovis), sheep biting louse (Bovicola ovis), Bovicola breviceps, Damalinia forficula, and Werneckiella spp.;
from the family Trichodectidae, for example, dog biting louse (Trichodectes canis) and cat louse (Felicola subrostratus);

from the family Menoponidae, for example, common chicken louse (Menopon gallinae), chicken body louse (Menacanthus stramineus), and Trinoton spp;
from the family Trimenoponidae, for example, Cummingsia spp.;
from the family Trogiidae, for example, death watch (Trogium pulsatorium);
from the family Liposcelidae or Liposcelididae, for example, book louse (Liposcelis corrodens), Liposcelis bostrychophila, Liposcelis pearmani and Liposcelis entomophila;
and the other.

Thysanura:

from the family Lepismatidae, for example, oriental silverfish (Ctenolepisma villosa) andmoth fish (Lepisma saccharina);
and the others.

Acari:

from the family Tetranychidae, for example, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), and Oligonychus spp.;
from the family Eriophyidae, for example, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes chibaensis, apple bud mite (Aculus schlechtendali), Aceria diospyri, Aceria tosichella, and Shevtchenkella sp.;
from the family Tarsonemidae, for example, broad mite (Polyphagotarsonemus latus);
from the family Tenuipalpidae, for example, Brevipalpus phoenicis;
from the family Tuckerellidae;
from the family Ixodidae, for example, Haemaphysalis longicornis, Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), netted tick (Dermacentor reticulatus), Ixodes ovatus, Ixodes persulcatus, blacklegged tick (Ixodes scapularis), Ixodes pacificus, Ixodes holocyclus, Ixodes ricinus, lone star tick (Amblyomma americanum), gulf coast tick (Amblyomma maculatum), Rhipicephalus microplus, cattle tick (Rhipicephalus annulatus), brown dog tick (Rhipicephalus sanguineus), Rhipicephalus appendiculatus, and Rhipicephalus decoloratus;
from the family Argasidae, for example, fowl tick (Argas persicus), Ornithodoros hermsi, and Ornithodoros turicata;
from the family Acaridae, for example, cereal mite (Tyrophagus putrescentiae) and grassland mite (Tyrophagus similis);
from the family Pyroglyphidae, for example, American house dust mite (Dermatophagoides farinae) and European house dust mite (Dermatophagoides pteronyssinus);
from the family Cheyletidae, for example, Cheyletus eruditus, Cheyletus malaccensis, Chelacaropsis moorei, and Cheyletiella yasguri;
from the family Psoroptidae, for example, sheep scab mite (Psoroptes ovis), horse psoroptic mange mite (Psoroptes equi), Knemidocoptes mutans, ear mange mite (Otodectes cynotis), and Chorioptes spp.;
from the family Sarcoptidae, for example, Notoedres cati, Notoedres muris, and itch mite (Sarcoptes scabiei);
from the family Listrophoridae, for example, Listrophorus gibbus;
from the family Dermanyssidae, for example,bird mite (Dermanyssus gallinae);
from the family Macronyssidae, for example, feather mite (Ornithonyssus sylviarum) and tropical rat mite (Ornithonyssus bacoti);
from the family Varroidae, for example, Varroa jacobsoni;
from the family Demodicidae, for example, dog follicle mite (Demodex canis) and cat follicle mite (Demodex cati);
from the family Trombiculidae, for example, Leptotrombidium akamushi, Leptotrombidium pallidum, and Leptotrombidium scutellare;
and the others.

Araneae:

from the family Eutichuridae, for example, Cheiracanthium japonicum;
from the family Theridiidae, for example, red-back spider (Latrodectus hasseltii);
and the others

Polydesmida:

from the family Paradoxosomatidae, for example, flatbacked millipede (Oxidus gracilis)and Nedyopus tambanus;
and the others.

Isopoda:

from the family Armadillidiidae, common pill bug (Armadillidium vulgare);
and the others.

Chilopoda:

from the family Scutigeridae, for example, Thereuonema hilgendorfi;
from the family Scolopendridae, giant tropical centipede (Scolopendra subspinipes);
from the family Ethopolyidae, Bothropolys rugosus;
and the others.

Gastropoda:

from the family Limacidae, for example, tree slug (Limax marginatus) and garden tawny slug (Limax flavus);
from the family Philomycidae, for example, Meghimatium bilineatum;
from the family Ampullariidae, for example, golden apple snail (Pomacea canaliculata);
from the family Lymnaeidae, for example, Austropeplea ollula;
and the others.

Nematoda:

from the family Aphelenchoididae, for example, rice white-tip nematode (Aphelenchoides besseyi);
from the family Pratylenchidae, for example, root lesion nematode (Pratylenchus coffeae), Pratylenchus brachyurus, California meadow nematode (Pratylenchus neglectus), and Radopholus similis;
from the family Heteroderidae, for example, javanese root-knot nematode (Meloidogyne javanica), southern root-knot nematode (Meloidogyne incognita), guava root-knot nematodes (Meloidogyne enterolobii), northern root-knot nematode (Meloidogyne hapla), soybean cyst nematode (Heterodera glycines), potato cyst nematode (Globodera rostochiensis), and white potato cyst nematode (Globodera pallida);
from the family Hoplolaimidae, for example, Rotylenchulus reniformis;
from the family Anguinidae, for example, strawberry bud nematode (Nothotylenchus acris), and stem nematode (Ditylenchus dipsaci);
from the family Tylenchulidae, for example, citrus nematode (Tylenchulus semipenetrans);
from the family Longidoridae, for example, dagger nematode (Xiphinema index);
from the family Trichodoridae;
from the family Parasitaphelenchidae, for example, pine wilt disease (Bursaphelenchus xylophilus);
and the others.

The harmful arthropods such as harmful insects and harmful mites, harmful molluscs, and harmful nematodes

may be harmful arthropods such as harmful insects and harmful mites, harmful molluscs, and harmful nematodes having reduced drug sensitivity to insecticides, acaricides, molluscicides, and nematicides, or having developed drug resistance. However, when the drug sensitivity is significantly reduced or the drug resistance is significantly developed, it is desirable to use a composition containing insecticide, acaricide, molluscicide, and nematicide components other than the target insecticides, acaricides, molluscicides, and nematicides.

The method for controlling a harmful arthropod of the present invention is performed by applying an effective amount of the compound of the present invention, the compound X of the present invention, and the composition A to a harmful arthropod directly and/or a habitat of the harmful arthropod (plants, soil, in-house, animal body, or the like). Examples of the method for controlling a harmful arthropod of the present invention include a foliage treatment, a soil treatment, a root treatment, a shower treatment, a fumigation treatment, a water surface treatment, and a seed treatment.

The compound of the present, the compound X of the present invention, the composition A are usually obtained and used by mixing the compound of the present invention or the present intermediate compound and an inert carrier such as a solid carrier, a liquid carrier or a gaseous carrier, and adding a surfactant or other auxiliaries for formulation as necessary, to be formulated into emulsifiable concentrates, oil formulations, dust formulations, granules, wettable powders, granule wettable powders, flowable agents, dry flowable agents, microcapsule formulations, aerosols, poisonous baits, resin formulations, shampoo agents, paste formulations, foam agents, carbon dioxide formulations, tablets, and the like. These formulations may be processed into mosquito repellent coil, electric mosquito repellent mat, mosquito repellent liquid formulation, smoking agent, fumigant, sheet formulation, spot-on agent, or oral treatment agent, and used. These formulations usually contain 0.0001% to 95% by weight of the compound of the present invention, the compound X of the present invention, or the composition A.

Examples of the solid carrier which is used in the formulation include fine powder and granules of clays (kaolin clay, diatomaceous earth, bentonite, acid clay, and the like), dry silica, wet silica, talc, ceramics, other inorganic minerals (sericite, quartz, sulfur, activated carbon, calcium carbonate, and the like), fine powder and granulated substances of chemical fertilizers (ammonium sulfate, ammonium phosphate, ammonium nitrate, urea, ammonium chloride, and the like) and the like, polyester resins such as synthetic resins (polyester resins such as polypropylene, polyacrylonitrile, polymethylmethacrylate and polyethylene terephthalate, nylon resins such as nylon-6, nylon-11 and nylon-66, polyamide resin, polyvinyl chloride, polyvinylidene chloride, vinyl chloride-propylene copolymer, and the like).

Examples of the liquid carrier include water, alcohols (methanol, ethanol, isopropyl alcohol, butanol, hexanol, benzyl alcohol, ethylene glycol, propylene glycol, phenoxyethanol, and the like), ketones (acetone, methyl ethyl ketone, cyclohexanone, and the like), aromatic hydrocarbons (toluene, xylene, ethylbenzene, dodecylbenzene, phenylxylylethane, methylnaphthalene, and the like), aliphatic hydrocarbons (hexane, cyclohexane, kerosene, light oil, and the like), esters (ethyl acetate, butyl acetate, isopropyl myristate, ethyl oleate, diisopropyl adipate, diisobutyl adipate, propylene glycol monomethyl ether acetate, and the like), nitriles (acetonitrile, isobutyronitrile, and the like), ethers (diisopropyl ether, 1,4-dioxane, 1,2-dimethoxyethane, diethylene glycol dimethyl ether, diethylene glycol monomethyl ether, propylene glycol monomethyl ether, dipropylene glycol monomethyl ether, 3-methoxy-3-methyl-1-butanol, and the like), amides (DMF, N,N-dimethylacetamide, and the like), sulfoxides (DMSO, and the like), and propylene carbonate and vegetable oils (soybean oil, cottonseed oil, and the like).

Examples of the gaseous carrier include fluorocarbon, butane gas, LPG (liquefied petroleum gas), dimethyl ether, and carbon dioxide.

Examples of the surfactant include nonionic surfactants such as polyoxyethylene alkyl ether, polyoxyethylene alkylaryl ether and polyethylene glycol fatty acid ester, and anionic surfactants such as alkyl sulfonates, alkylbenzene sulfonates and alkylsulfates.

The other auxiliaries for formulation include such as fixing agents, dispersants, colorants and stabilizers, specifically, for example, casein, gelatin, polysaccharides (starch, arabic gum, cellulose derivatives, alginic acid, and the like), lignin derivatives, bentonite, synthetic water-soluble polymers (polyvinyl alcohol, polyvinyl pyrrolidone, polyacrylic acids, and the like), isopropyl acid phosphate, 2,6-di-tert-butyl-4-methylphenol, and BHA (mixtures of 2-tert-butyl-4-methoxyphenol and 3-tert-butyl-4-methoxyphenol) .

Examples of a base material of the resin formulation include vinyl chloride polymer, polyurethane and the like, and a plasticizer such as ester phthalates (dimethyl phthalate, dioctyl phthalate, and the like), ester adipates or stearic acid may be added to these base materials as necessary. The resin formulation is obtained by kneading a compound into the base material using an ordinary kneading apparatus, then molding it by injection molding, extrusion molding, press molding or the like, and can be processed into a plate, film, taped, reticular or string resin formulation by further undergoing molding or cutting step as necessary. These resin formulation is processed into, for example, a collar for animal, an ear tag for animal, a sheet formulation, an induction cord, and a gardening pole.

Examples of a base material of the poison bait include grain powder, vegetable oil, sugar, crystalline cellulose and the like, and further, an antioxidizing agent such as dibutylhydroxytoluene and nordihydroguaiaretic acid, a preservative such as dehydroacetic acid, a substance for preventing accidental ingestion by children and pets such as hot pepper powder, a pest attractant such as cheese flavor, onion flavor and peanut oil or the like are added as necessary.

In the present invention, examples of the plant include entire plants, foliage, flowers, ears, fruits, trunks, branches, crowns, seeds, vegetative reproductive organs, and seedlings.

The vegetative reproductive organs mean roots, stems, leaves, and the like of plants that have the ability to grow when the parts are separated from the main body and provided in the soil. The vegetative reproductive organ specifically includes a tuberous root, a creeping root, a corm or a solid bulb, a tuber, a rhizome, a stolon, a rhizophore, a cane cutting, a propagule, and a vine cutting. The stolon is sometimes called as runner, and the propagule is sometimes called as broad bud or bulbil. The vine cutting means a shoot (collective term for leaves and stems, shoot) of sweet potato, yam, or the like. The bulb, corm, tuber, rhizome, cane cutting, rhizophore, or the tuberous root is also collectively called a bulb. Cultivation of potatoes is started by planting tubers in soil, and the tubers used are generally called seed tubers.

Examples of the method for controlling a harmful arthropod by applying an effective amount of the compound of the present invention, the compound X of the present invention, or the composition A to soil include a method for applying an effective amount of the compound of the present invention, the compound X of the present invention, or the composition A to soil before or after planting plants, a method for applying an effective amount of the compound of the present invention, the compound X of the present invention, or the composition A to the rhizosphere of a crop to be protected from damage such as eating by the harmful arthropod, and a method for controlling a harmful arthropod that eats plants by permeating and transferring an effective amount of the compound of the present invention, the compound X of the present invention, or the composition A from a root part or the like to the inside of the plant. More specific examples thereof include a planting hole treatment (plant hole spraying and plant hole treatment soil incorporation), a root treatment (root spraying, root soil incorporation, root irrigation, nursing seedling period second half root treatment), a panting groove treatment (planting groove spraying and planting groove soil incorporation), a row treatment (row spraying, row soil incorporation, and growth period row spraying), a sowing-time row treatment (sowing-time row spraying, sowing-time row soil incorporation), a full-scale treatment (full-scale soil spraying, full-scale soil incorporation), a side row treatment, a water surface treatment (water surface application, water surface application after flooding), other soil spraying treatments (growth period granule foliar spraying, under canopy or around main trunk spraying, soil surface spraying, soil surface incorporation, seed hole spraying, ridge surface spraying, and inter-strain spraying), other irrigation treatments (soil irrigation, nursing seedling irrigation, chemical injection treatment, ground irrigation, chemical drip irrigation, and chemigation), a nursing seedling box treatment (nursing seedling box spraying, nursing seedling box irrigation, and nursing seedling box chemical flooding), a nursing seedling tray treatment (nursing seedling tray spraying, nursing seedling tray irrigation, and nursing seedling tray chemical flooding), a nursery bed treatment (nursery bed spraying, nursery bed irrigation, water seedling nursery bed spraying, and seedling immersion), a bed soil incorporation treatment (bed soil incorporation, bed soil incorporation before sowing, soil covering spraying before sowing, soil covering spraying after sowing, and soil covering incorporation), and other treatments (soil incorporation, plow-in, topsoil incorporation, raindrop soil incorporation, planting position treatment, granule flower cluster spraying, and paste fertilizer incorporation)

Examples of the seed treatment include a treatment of a seed or a vegetative reproductive organ with the compound of the present invention, the compound X of the present invention, or the composition A, and specific examples thereof include a spray treatment in which a suspension of the compound of the present invention, the compound X of the present invention, or the composition A is atomized and sprayed onto a seed surface or a vegetative reproductive organ surface, a smear treatment in which the compound of the present invention, the compound X of the present invention, or the composition A is applied to a seed or a vegetative reproductive organ, an immersion treatment in which a seed is immersed in a chemical solution of the compound of the present invention, the compound X of the present invention, or the composition A for a certain period of time, and a method for coating a seed or a vegetative reproductive organ with a carrier containing the compound of the present invention, the compound X of the present invention, or the composition A (film coating treatment, pellet coating treatment, and the like). Examples of the vegetative reproductive organ include, in particular, seed tubers.

In a case of treating the composition A with respect to the seed or the vegetative reproductive organ, the composition A can be treated with respect to the seed or the vegetative reproductive organ as one formulation, or the composition A can be treated with respect to the seed or the vegetative reproductive organ in a plurality of divided times as a plurality of different formulations. Examples of the method for treating the composition A in a plurality of divided times as a plurality of different formulations include a method for treating a formulation containing only the compound of the present invention as an active ingredient, air-drying the seed or the vegetative reproductive organ, and then treating the formulation containing the present ingredient; and a method for treating a formulation containing the compound of the present invention and the present ingredient as active ingredients, air-drying the seed or vegetative reproductive organ, and then treating a formulation containing the present ingredient other than the treated present ingredient.

The seed or vegetative reproductive organ holding the compound of the present invention, the compound X of the present invention, or the composition A in the present invention means to be in a state in which the compound of the present invention, the compound X of the present invention, or the composition A is attached to the surface of the seed or the vegetative reproductive organ. In the seed or vegetative reproductive organ holding the compound of the

present invention, the compound X of the present invention, or the composition A, a material other than the compound of the present invention, the compound X of the present invention, or the composition A may be attached before or after the compound of the present invention, the compound X of the present invention, or the composition A is attached to the seed or vegetative reproductive organ.

In addition, when the composition A is attached as a layer to the surface of the seed or vegetative reproductive organ, the layer consists of one layer or a plurality of layers. In the case of a plurality of layers, each layer is a layer containing one or more active ingredients, or is formed of a layer containing one or more active ingredients and a layer not containing an active ingredient.

The seed or vegetative reproductive organ holding the compound of the present invention, the compound X of the present invention, or the composition A can be obtained, for example, by applying a formulation containing the compound of the present invention, the compound X of the present invention, or the composition A to the seed or vegetative reproductive organ by the method of seed treatment described above.

When the compound of the present invention, the compound X of the present invention, or the composition A are used in harmful arthropod controlling in the agricultural field, the application amount is usually 1 to 10000 g in the amount of the compound of the present invention or the compound X of the present invention per 10000 $m^2$. When the seed or the vegetative reproductive organ is treated, the amount of the compound of the present invention or the compound X of the present invention is usually applied in a range of 0.001 to 100 g with respect to 1 Kg of the seed or the vegetative reproductive organ. When being prepared into an emulsifiable concentrate, a wettable powder, a flowable or the like, the compound of the present invention, the compound X of the present invention, or the composition A are usually diluted with water for an application so as to have a concentration of the active ingredient of 0.01 to 10000 ppm, and dust formulations, granules and the like are usually applied as they are.

Also, as for the compound of the present invention, the compound X of the present invention, or the composition A, the resin formulation processed into a sheet or string can be also treated by a method such as winding it around crops, spreading it in the vicinity of crops, or spreading it to the soil around crop roots.

When the compound of the present invention, the compound X of the present invention, or the composition A are used in controlling the harmful arthropod that inhabits in the house, the application amount is usually 0.01 to 1000 mg in an amount of the compound of the present invention or the compound X of the present invention per 1 $m^2$ of an area to be treated, in the case of using it on a planar area, and is usually 0.01 to 500 mg in an amount of the compound of the present invention or the compound X of the present invention per 1 $m^3$ of a space to be treated, in the case of using it in a space. When being prepared into an emulsifiable concentrate, a wettable powder, a flowable or the like, the compound of the present invention, the compound X of the present invention, or the composition A are usually diluted with water for an application so as to have a concentration of the active ingredient of 0.1 to 10000 ppm, and oil formulations, aerosols, smoking agents, poisonous baits, and the like are usually applied as they are.

When the compound of the present invention, the compound X of the present invention, and the composition A are used in the control of external parasites on livestock such as cows, horses, pigs, sheep, goats and chickens, and small animals such as dogs, cats, rats and mice, veterinary known methods can be applied to the animals. As specific methods, the formulation is administered, for example, by way of a tablet, mixing in feed, a suppository and injection (intramuscular, subcutaneous, intravenous, intraperitoneal injections, or the like), when systemic control is intended, and the formulation is used, for example, by way of spraying an oil solution or aqueous solution, pour-on or spot-on treatment, washing an animal with a shampoo formulation, or putting a collar or ear tag made of a resin formulation on to an animal, when non-systemic control is intended. The amount of the compound of the present invention or the compound X of the present invention when administered to animals is usually in the range from 0.1 to 1000 mg per 1 kg of the weight of an animal.

The compound of the present invention, the compound X of the present invention, or the composition A can be used as a harmful arthropod controlling agent in agricultural lands such as cultivated lands, paddy fields, grass plot, and orchards. Examples of the plants include those shown below.

Corn, rice, wheat, barley, rye, oat, sorghum, cotton, soybean, peanut, sarrazin, sugar beet, rapeseed, sunflower, sugar cane, tobacco, solanaceae vegetables (eggplant, tomato, green pepper, hot pepper, potato, and the like), cucurbitaceae vegetables (cucumber, pumpkin, zucchini, watermelon, melon, and the like), cruciferae vegetables (Japanese radish, turnip, horseradish, kohlrabi, Chinese cabbage, cabbage, brown mustard, broccoli, cauliflower, and the like), compositae vegetables (burdock, garland chrysanthemum, artichoke, lettuce, and the like), liliaceae vegetables (Welsh onion, onion, garlic, asparagus, and the like), umbelliferae vegetables (carrot, parsley, celery, parsnip, and the like), chenopodiaceae vegetables (spinach, Swiss chard, and the like), labiatae vegetables (Japanese mint, mint, basil, and the like), strawberry, sweet potato, yam, aroid, pomaceous fruits (apples, common pears, Japanese pears, Chinese quince, quince, and the like), stone fleshy fruits (peaches, plums, nectarines, Japanese plums, cherries, apricots, prunes, and the like), citrus plants (satsuma mandarin, orange, lemon, lime, grapefruits, and the like), nuts (chestnuts, walnuts, hazel nuts, almonds, pistachios, cashew nuts, macadamia nuts, and the like), berry fruits (blueberries, cranberries, blackberries, raspberries, and the like), grapes, persimmons, olives, loquats, bananas, coffee, date, coconuts, tea,

mulberries, ornamental plants, forest plants, zoysias, and pastures

The plants also include plants that can be produced by natural crossbreeding, plants that can be generated by mutation, F1 hybrid plants, and genetically modified crops. Examples of the genetically modified crops include plants imparted with resistance to herbicides such as a HPPD (4-hydroxyphenylpyruvate dioxygenase enzyme) inhibitor such as isoxaflutole, an ALS (acetolactate synthetase) inhibitor such as imazethapyr or thifensulfuron-methyl, an EPSP (5-enolpyruvylshikimate-3-phosphate synthetase) inhibitor, a glutamine synthetase inhibitor, a PPO (protoporphyrinogen oxidase) inhibitor, bromoxynil, and dicamba; plants capable of synthesizing selective toxins and the like known in the genus Bacillus such as Bacillus thuringiensis; and plants capable of imparting specific insecticidal activity by synthesizing a gene fragment or the like that partially matches an endogenous gene derived from a harmful insect and inducing gene silencing (RNAi; RNA interference) in the target harmful insect.

The plant is not particularly limited as long as it is a cultivar that is generally cultivated.

EXAMPLES

Hereinbelow, the present invention will be further described in detail by production examples, formulation examples, test examples, and the like. However, the present invention is not limited to these examples.

In the present specification, Me represents a methyl group, Et represents an ethyl group, Pr represents a propyl group, i-Pr represents an isopropyl group, c-Pr represents a cyclopropyl group, c-Bu represents a cyclobutyl group, c-Pen represents a cyclopentyl group, c-Hex represents a cyclohexyl group, Ph represents a phenyl group, Py2 represents a 2-pyridyl group, Py3 represents a 3-pyridyl group, Py4 represents a 4-pyridyl group, and Bn represents a benzyl group. When c-Pr, c-Bu, c-Pen, c-Hex, Ph, Py2, Py3, and Py4 have a substituent, the substituent is described together with a substitution position before the symbol. For example, 1-CN-c-Pr represents a 1-cyanocyclopropyl group, 3,4-$F_2$-Ph represents a 3,4-difluorophenyl group, 4-$CF_3$-Py2 represents a 4-(trifluoromethyl)-2-pyridyl group, and 5-$OCH_2CF_2CF_3$-Py2 represents a 5-(2,2,3,3,3-pentafluoropropoxy)-2-pyridyl group.

First, production examples of the compound X of the present invention are described.

When the physical property value of the compound is measured by liquid chromatography/mass spectrometry (hereinafter, referred to as LCMS), the measured molecular ion value $[M + H]^+$ or $[M-H]^-$ and retention time (hereinafter, referred to as RT) are described. The conditions of the liquid chromatography (hereinafter, referred to as LC) are as follows.

[LC conditions]

Column: L-column2 ODS, inner diameter: 4.6 mm, length: 30 mm, particle diameter: 3 μm (Chemicals Evaluation and Research Institute, Japan)

UV measurement wavelength: 254 nm

Mobile phase: A solution: 0.1% formic acid aqueous solution, B solution: 0.1% formic acid acetonitrile Flow rate: 2.0 mL/min

Gradient conditions: Feeding is performed at the concentration gradient described in [Table LC1].

[Table 1]

[Table LC1]

| Time (Min) | A liquid (%) | B liquid (%) |
|---|---|---|
| 0.01 | 90 | 10 |
| 2.00 | 0 | 100 |
| 4.00 | 0 | 100 |
| 4.01 | 90 | 10 |

Reference Production Example 1

15 mL of 28% aqueous ammonia was added dropwise to a mixture of 1.0 g of 5-[3-(ethanesulfonyl)-6-iodoimidazo [1,2-a] pyridin-2 yl]-3-methyl -2-(2,2,3,3,3-pentafluoropropoxy) pyrimidine-4(3H)-one produced according to the method described in WO 2019/124548 and 10 mL of acetonitrile at 70°C, and the mixture was stirred at 70°C for 6 hours. The obtained mixture was cooled to room temperature, and the precipitated solid was filtered. The obtained solid was

washed with water and concentrated under reduced pressure to obtain 600 mg of intermediate 1 represented by the following formula.

[Chem. 55]

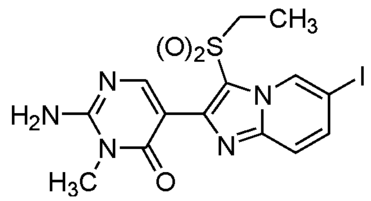

Intermediate 1: $^{1}$H-NMR (DMSO-$D_6$) δ: 8.98 (1H, d), 7.84 (1H, s), 7.78 (1H, dd), 7.62 (1H, d), 7.52 (2H, s), 3.79 (2H, q), 2.30 (3H, s), 1.26 (3H, t).

Reference Production Example 2

A mixture of 46.9 g of 2-amino-4-(trifluoromethyl) pyridine, 66.5 g of ethyl 4-chloroacetoacetate and 150 mL of ethanol was stirred at 85°C for 7 hours. The obtained mixture was concentrated under reduced pressure, 400 mL of 1 N hydrochloric acid was added to the obtained residue, and the mixture was washed with ethyl acetate. Sodium bicarbonate was added to the obtained aqueous layer for neutralization, and the mixture was extracted with ethyl acetate. The obtained organic layer was washed with saturated brine, dried over anhydrous sodium sulfate, and concentrated under reduced pressure. The obtained solid was washed with hexane to obtain 37.3 g of intermediate 2 represented by the following formula.

[Chem. 56]

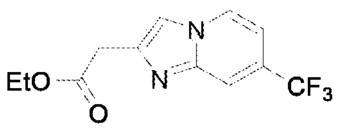

Intermediate 2: $^{1}$H-NMR ($CDCl_3$) δ: 8.20 (1H, d), 7.90 (1H, d), 7.76 (1H, s), 6.99 (1H, dd), 4.23 (2H, q), 3.93 (2H, s), 1.31 (3H, q).

Reference Production Example 2-1

The compounds produced in accordance with Reference Production Example 2 and physical property values thereof are shown below.

[Chem. 57]

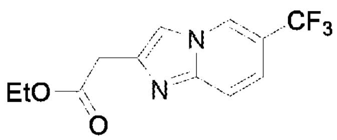

Intermediate 22: $^{1}$H-NMR ($CDCl_3$) δ: 8.46 (1H, s), 7.72 (1H, s), 7.65 (1H, d), 7.30 (1H, d), 4.22 (2H, q), 3.89 (2H, s), 1.30 (3H, t).

Reference Production Example 3

A mixture of 5.00 g of intermediate 2 and 10 mL of N,N-dimethylformamide dimethyl acetal was stirred at 80°C for 2 hours. The obtained mixture was concentrated under reduced pressure, 18 mL of sodium ethoxide (20% ethanol solution) and 2.48 g of N-methylthiourea were sequentially added thereto, and the mixture was stirred at 80°C for 7

hours. The obtained mixture was concentrated under reduced pressure, water was added thereto, and concentrated hydrochloric acid was added thereto for neutralization. The precipitated solid was filtered, and the obtained solid was washed with water and dried under reduced pressure to obtain 4.86 g of intermediate 3 represented by the following formula.

[Chem. 58]

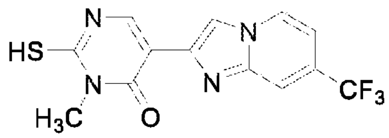

Intermediate 3: $^1$H-NMR (DMSO-$D_6$) δ: 8.84 (1H, d), 8.71 (1H, s), 8.16 (1H, s), 8.01 (1H, d), 7.18 (1H, dd), 3.67 (3H, s).

Reference Production Example 4

3.4 mL of oxalyl chloride was added dropwise to a mixture of 4.36 g of intermediate 3 and 63 mL of DMF under ice cooling. The obtained mixture was stirred at 40°C for 30 minutes, and then water was added under ice cooling. The precipitated solid was filtered, and the obtained solid was sequentially washed with water and MTBE and concentrated under reduced pressure to obtain 1.39 g of intermediate 4 represented by the following formula.

[Chem. 59]

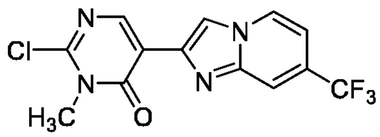

Intermediate 4: $^1$H-NMR ($CDCl_3$) δ: 8.87 (1H, s), 8.65 (1H, s), 8.25 (1H, d), 7.92-7.91 (1H, m), 6.98 (1H, d), 3.82 (3H, s).

Reference Production Example 5

An intermediate 5 represented by the following formula was obtained according to Reference Production Example 1, using the intermediate 4 instead of 5-[3-(ethanesulfonyl)-6-iodoimidazo [1,2-a] pyridin-2 yl]-3-methyl -2-(2,2,3,3,3-pentafluoropropoxy) pyrimidine-4(3H)-one.

[Chem. 60]

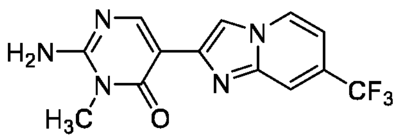

Intermediate 5: $^1$H-NMR (DMSO-$D_6$) δ: 8.79 (1H, d), 8.60 (1H, s), 8.58 (1H, s), 7.94 (1H, d), 7.45 (2H, br s), 7.10 (1H, dd), 3.39 (3H, s).

Reference Production Example 6

A mixture of 1.45 g of intermediate 5, 2.4 mL of 3-bromo-1,1,1-trifluoroacetone, and 5 mL of butanol was stirred in a pressure-resistant vessel at 100°C for 20 hours. The obtained mixture was allowed to cool to room temperature, water was added thereto, and the mixture was extracted with ethyl acetate. The obtained organic layer was dried over anhydrous sodium sulfate and concentrated under reduced pressure. The resulting residue was applied to a silica gel column chromatography to obtain 348 mg of intermediate 6 represented by the following formula.

[Chem. 61]

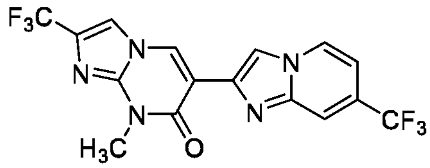

Intermediate 6: $^1$H-NMR ($CDCl_3$) δ: 8.96 (1H, s), 8.71 (1H, s), 8.27 (1H, d), 7.88-7.86 (1H, m), 7.53 (1H, s), 7.00 (1H, dd), 3.81 (3H, s).

Reference Production Example 7

204 mg of sodium hydride (60%, oil-based) was added to a mixture of 1.0 g of 4-chloro-1-methyl-2-(trifluoromethyl)-1H-imidazo [4,5-c] pyridine synthesized according to the method described in Tetrahedron Letters, 2013, 54, 201., 0.5 mL of benzyl alcohol and 14 mL of DMF at room temperature, and the mixture was stirred for 3 hours. A saturated aqueous ammonium chloride solution was added to the obtained mixture, and the mixture was extracted with ethyl acetate. The resulting organic layer was concentrated under reduced pressure. 7 mL of 6N hydrochloric acid was added to the obtained residue, and the mixture was stirred at room temperature for 2 hours. The obtained mixture was neutralized by adding sodium bicarbonate. The precipitated solid was filtered, and the obtained solid was washed with water and concentrated under reduced pressure to obtain 0.3 g of intermediate 7 represented by the following formula.

[Chem. 62]

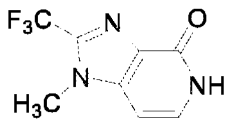

Intermediate 7: $^1$H-NMR (DMSO-$D_6$) δ: 11.50 (1H, br s), 7.37 (1H, d), 6.72 (1H, d), 3.88 (3H, s) .

Reference Production Example 8

16.7 mL of triethylamine was added to a mixture of 9.49 g of chloroacetic acid and 15 mL of water at 0°C over 30 minutes. 16.1 g of 2-amino-5-(trifluoromethyl) pyridine was added to the obtained mixture, and the mixture was stirred under reflux for 2 hours. The obtained mixture was allowed to cool to room temperature, and the precipitated solid was filtered. The obtained solid was washed with water and dried under reduced pressure to obtain 11.0 g of a crude product of intermediate 8 represented by the following formula.

[Chem. 63]

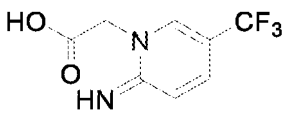

Intermediate 8: LCMS: 219 [M-H]$^-$, RT = 0.42 min

Reference Production Example 8-1

The compounds produced in accordance with Reference Production Example 8 and physical property values thereof are shown below.

[Chem. 64]

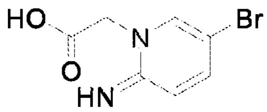

Intermediate 9: LCMS: 229 $[M-H]^-$, RT = 0.34 min

Reference Production Example 9

A mixture of 13.21 g of the crude product of intermediate 8 produced according to Reference Production Example 8, 18 mL of phosphorus oxychloride, and 150 mL of toluene was stirred under reflux for 6 hours. The obtained mixture was added dropwise to an aqueous sodium hydroxide solution and extracted with toluene. The obtained organic layer was dried over anhydrous sodium sulfate and concentrated under reduced pressure to obtain 13.2 g of intermediate 10 represented by the following formula.

[Chem. 65]

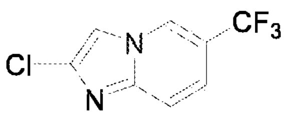

Intermediate 10: $^1H$-NMR ($CDCl_3$) δ: 8.44 (1H, s), 7.65 (1H, d), 7.62 (1H, s), 7.38 (1H, d).

Reference Production Example 9-1

The compounds produced in accordance with Reference Production Example 9 and physical property values thereof are shown below.

[Chem. 66]

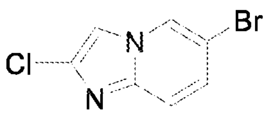

Intermediate 11: $^1H$-NMR ($CDCl_3$) δ: 8.21 (1H, s), 7.48 (1H, s), 7.44 (1H, d), 7.28 (1H, d) .

Reference Production Example 10

17.32 g of N-iodosuccinimide was added to a mixture of 15.44 g of intermediate 10 and 75 mL of DMF under ice cooling, and the mixture was stirred at 70°C for 5 hours. An aqueous sodium thiosulfate solution was added to the obtained mixture, and the precipitated solid was filtered. The obtained solid was washed with water and dried under reduced pressure to obtain 18.0 g of intermediate 12 represented by the following formula.

[Chem. 67]

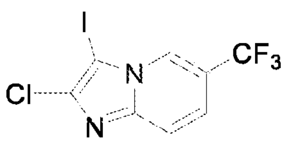

Intermediate 12: $^1H$-NMR ($CDCl_3$) δ: 8.41 (1H, s), 7.65 (1H, d), 7.44 (1H, d).

Reference Production Example 10-1

The compounds produced in accordance with Reference Production Example 10 and physical property values thereof are shown below.

[Chem. 68]

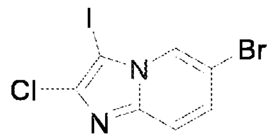

Intermediate 13: $^1$H-NMR ($CDCl_3$) $\delta$: 8.20 (1H, s), 7.43 (1H, d), 7.35 (1H, d).

[Chem. 69]

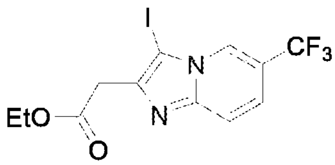

Intermediate 23: $^1$H-NMR ($CDCl_3$) $\delta$: 8.45 (1H, s), 7.67 (1H, d), 7.39 (1H, d), 4.21 (2H, q), 3.90 (2H, s), 1.29 (3H, t) .

Reference Production Example 11

A mixture of 18.0 g of intermediate 12, 140 mL of 1,4 dioxane, 2.38 g of tris(dibenzylideneacetone) dipalladium (0), 3.01 g of Xantphos, 27.2 mL of diisopropylethylamine, and 3.75 mL of ethanethiol was stirred under reflux for 3 hours. The obtained mixture was allowed to room temperature and concentrated under reduced pressure. The resulting residue was applied to a silica gel column chromatography to obtain 13.39 g of intermediate 14 represented by the following formula.

[Chem. 70]

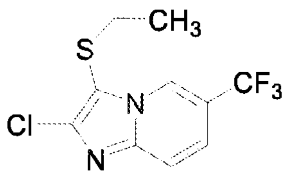

Intermediate 14: $^1$H-NMR ($CDCl_3$) $\delta$: 8.74 (1H, s), 7.67 (1H, d), 7.48 (1H, d), 2.78 (2H, d), 1.24 (3H, t)

Reference Production Example 11-1

The compounds produced in accordance with Reference Production Example 11 and physical property values thereof are shown below.

[Chem. 71]

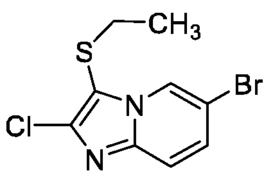

Intermediate 15: $^1$H-NMR ($CDCl_3$) δ: 8.51 (1H, s), 7.46 (1H, d), 7.38 (1H, d), 2.73 (2H, d), 1.23 (3H, t).

[Chem. 72]

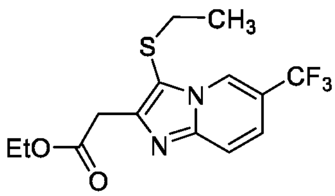

Intermediate 24: $^1$H-NMR ($CDCl_3$) δ: 8.76 (1H, s), 7.70 (1H, d), 7.42 (1H, d), 4.20 (2H, q), 4.00 (2H, s), 2.72 (2H, q), 1.28 (3H, t), 1.23 (3H, t).

Reference Production Example 12

5.16 g of mCPBA (purity 70%, 30% with water) was added to a mixture of 2.66 g of intermediate 15 and 10 mL of chloroform under ice cooling, and the mixture was stirred at room temperature for 2 hours. A saturated aqueous sodium bicarbonate solution and an aqueous sodium thiosulfate solution were sequentially added to the obtained mixture, and the mixture was extracted with chloroform. The obtained organic layer was dried over anhydrous sodium sulfate and concentrated under reduced pressure. The resulting residue was applied to a silica gel column chromatography to obtain 1.79 g of intermediate 16 represented by the following formula.

[Chem. 73]

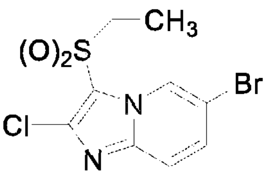

Intermediate 16: $^1$H-NMR ($CDCl_3$) δ: 9.15 (1H, s), 7.60 (1H, d), 7.57 (1H, d), 3.36 (2H, q), 1.36 (3H, t).

Reference Production Example 12-1

The compounds produced in accordance with Reference Production Example 12 and physical property values thereof are shown below.

[Chem. 74]

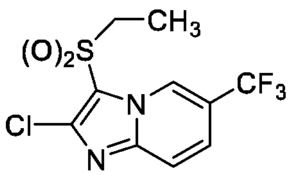

Intermediate 17: $^1$H-NMR ($CDCl_3$) δ: 9.39 (1H, s), 7.81 (1H, d), 7.67 (1H, d), 3.39 (2H, q), 1.37 (3H, t).

[Chem. 75]

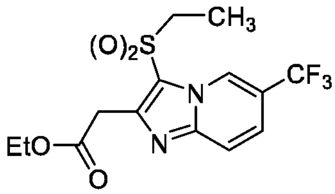

Intermediate 25: $^1$H-NMR ($CDCl_3$) δ: 9.22 (1H, s), 7.82 (1H, d), 7.61 (1H, d), 4.22 (2H, s), 4.21 (2H, q), 3.39 (2H, q), 1.39 (3H, t), 1.30 (3H, t).

Reference Production Example 13

A mixture of 324 mg of intermediate 16, 0.32 mL of trans-N,N'-dimethylcyclohexane-1,2-diamine, 225 mg of sodium iodide, 190 mg of copper (I) iodide, and 4 mL of toluene was stirred at 120°C for 21 hours. The obtained mixture was cooled to room temperature, and then filtered. Water was added to the obtained filtrate, and the mixture was extracted with chloroform. The obtained organic layer was dried over anhydrous sodium sulfate and concentrated under reduced pressure. The resulting residue was applied to a silica gel chromatography to obtain 70 mg of intermediate 18 represented by the following formula.

[Chem. 76]

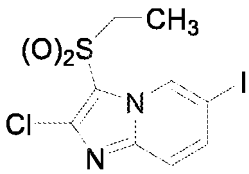

Intermediate 18: $^1$H-NMR ($CDCl_3$) δ: 9.23 (1H, s), 7.70 (1H, d), 7.46 (1H, d), 3.35 (2H, m), 1.35 (3H, t) .

Reference Production Example 14

A mixture of 936 mg of intermediate 17, 4.56 g of cesium fluoride and 10 mL of DMSO was stirred at 95°C for 2 hours. The obtained mixture was allowed to room temperature, then ethyl acetate and water were sequentially added thereto, and the mixture was filtered through Celite (registered trademark). The obtained filtrate was separated, and the obtained organic layer was dried over anhydrous sodium sulfate and concentrated under reduced pressure. The obtained residue was applied to a silica gel column chromatography to obtain 330 mg of crude of intermediate 19 represented by the following formula.

[Chem. 77]

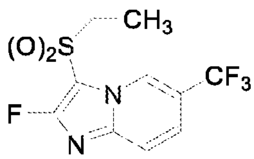

Intermediate 19: LCMS: 297 $[M+H]^+$, RT = 1.76 min

Reference Production Example 14-1

The compounds produced in accordance with Reference Production Example 14 and physical property values thereof are shown below.

[Chem. 78]

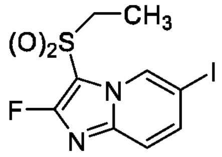

Intermediate 20: LCMS: 355 $[M+H]^+$, RT = 1.72 min

Reference Production Example 15

A mixture of 8.0 g of ethyl 2-(6-bromoimidazo [1,2-a] pyridine-2-yl) acetate produced according to the method described in WO 2015/086527 A and 20 mL of N,N-dimethylformamide dimethyl acetal was stirred at 90°C for 1 hour. The obtained mixture was concentrated under reduced pressure, 1.01 g of 5-(trifluoromethyl)-1,2,4-triazole-3-amine and 5 mL of propionic acid were sequentially added to the obtained residue, and the mixture was stirred at 120°C for 30 minutes. The obtained mixture was allowed to cool to room temperature, and a 12 N aqueous sodium hydroxide solution was added. The precipitated solid was filtered, and the obtained solid was washed with MTBE. The obtained solid was dried under reduced pressure to obtain 1.15 g of intermediate 21 represented by the following formula.

[Chem. 79]

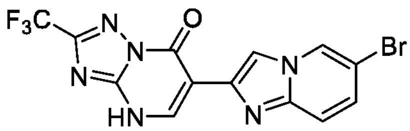

Intermediate 21: $^1$H-NMR (DMSO-$D_6$) δ: 8.85 (1H, s), 8.71 (1H, s), 8.43 (1H, s), 7.41 (1H, d), 7.30 (1H, d).

Production Example 1

A mixture of 300 mg of intermediate 1, 5 mL of NMP, and 1 mL of 3-bromo-1,1,1-trifluoroacetone was stirred at 130°C for 1 hour. Water was added to the obtained mixture, and the mixture was extracted with ethyl acetate. The obtained organic layer was dried over anhydrous sodium sulfate and concentrated under reduced pressure. The obtained residue was subjected to silica gel column chromatography (hexane : ethyl acetate = 1 : 4) to obtain 220 mg of a compound 1 of present invention represented by the following formula.

[Chem. 80]

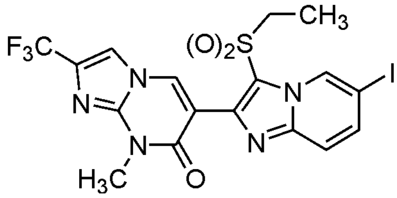

Compound 1 of the present invention: $^1$H-NMR ($CDCl_3$) δ: 9.15 (1H, s), 8.17 (1H, s), 7.70 (1H, d), 7.53 (1H, d), 7.46 (1H, s), 3.75 (3H, s), 3.65 (2H, q), 1.46 (3H, t).

Production Example 2

A mixture of 170 mg of intermediate 6, 105 μL of diethyl disulfide, 161 mg of iodine, and 1.5 mL of NMP was stirred at 170°C for 2 hour. The obtained mixture was cooled to room temperature, and a saturated aqueous sodium thiosulfate solution was added thereto, and the mixture was extracted with ethyl acetate. The obtained organic layer was dried over anhydrous sodium sulfate and concentrated under reduced pressure. The obtained residue was subjected

to silica gel column chromatography (hexane : ethyl acetate = 2 : 1) to obtain 105 mg of a compound 2 of present invention (containing 69 w/w% of intermediate 6) represented by the following formula.

[Chem. 81]

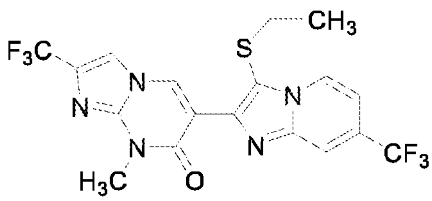

Compound 2 of the present invention: $^1$H-NMR ($CDCl_3$) δ: 8.60 (1H, d), 8.19 (1H, s), 7.99-7.96 (1H, m), 7.48 (1H, s), 7.16 (1H, dd), 3.79 (3H, s), 2.79 (2H, q), 1.17 (3H, t) .

Production Example 2-1

The compounds produced in accordance with Production Example 2 and physical property values thereof are shown below.

[Chem. 82]

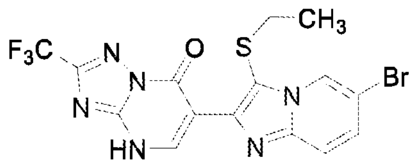

Compound 8 of the present invention: $^1$H-NMR ($CDCl_3$) δ: 9.59 (1H, s), 8.91 (1H, s), 8.77 (1H, d), 7.88 (1H, d), 2.89 (2H, q), 1.28 (3H, t).

Production Example 3

40 mg of mCPBA (purity 70%, 30% with water) was added to a mixture of 105 mg of the compound 2 of the present invention (containing 69 w/w% of intermediate 6) and 5 mL of chloroform under ice cooling, and the mixture was stirred at room temperature for 10 hours. A saturated aqueous sodium bicarbonate solution and a saturated aqueous sodium thiosulfate solution were sequentially added to the obtained mixture, and the mixture was extracted with chloroform. The obtained organic layer was dried over anhydrous sodium sulfate and concentrated under reduced pressure. The obtained residue was subjected to silica gel chromatography (hexane: ethyl acetate = 2: 1) to obtain 28 mg of a compound 3 of present invention.

[Chem. 83]

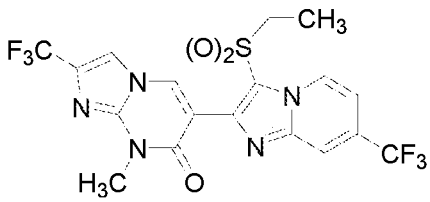

Compound 3 of the present invention: $^1$H-NMR ($CDCl_3$) δ: 9.03 (1H, d), 8.19 (1H, s), 8.05-8.04 (1H, m), 7.48 (1H, s), 7.28 (1H, dd), 3.76 (3H, s), 3.72 (2H, q), 1.48 (3H, t) .

Production Example 3-1

The compounds produced in accordance with Production Example 3 and physical property values thereof are shown below.

[Chem. 84]

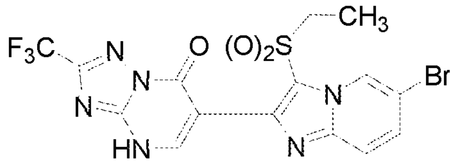

Compound 9 of the present invention: $^{1}$H-NMR (DMSO-$D_6$) δ: 8.91 (1H, s), 8.20 (1H, s), 7.74 (1H, d), 7.70 (1H, d), 3.29-3.27 (2H, m), 1.27 (3H, t).

Production Example 4

A mixture of 0.10 g of the compound 1 of present invention, 47 mg of cyclopropylboronic acid, 26 mg of [1,1'-bis(diphenylphosphino) ferrocene] dichloropalladium (II), 191 mg of tripotassium phosphate, 2 mL of toluene, and 0.3 mL of water was stirred at 100°C for 30 minutes. The obtained mixture was allowed to cool to room temperature, then water was added thereto, and the mixture was extracted with ethyl acetate. The obtained organic layer was dried over anhydrous sodium sulfate and concentrated under reduced pressure. The obtained residue was subjected to silica gel chromatography (ethyl acetate : hexane = 1 : 1) to obtain 72 mg of a compound 4 of present invention represented by the following formula.

[Chem. 85]

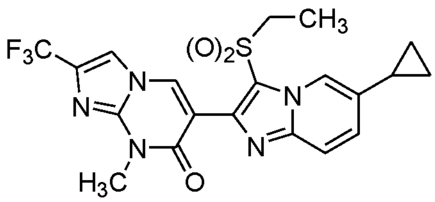

Compound 4 of the present invention: $^{1}$H-NMR ($CDCl_3$) δ: 8.67-8.66 (1H, m), 8.14 (1H, s), 7.64 (1H, d), 7.44-7.43 (1H, m), 7.20 (1H, d), 3.75 (3H, s), 3.65 (2H, q), 2.04-1.99 (1H, m), 1.46 (3H, t), 1.10-1.07 (2H, m), 0.79-0.75 (2H, m).

Production Example 4-1

The compounds produced in accordance with Production Example 4 and physical property values thereof are shown below.

[Chem. 86]

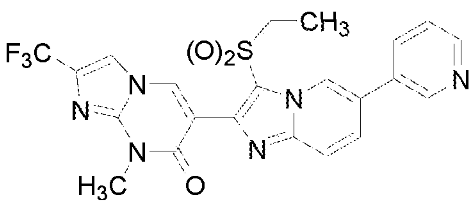

Compound 5 of the present invention: $^{1}$H-NMR ($CDCl_3$) δ: 9.09-9.07 (1H, m), 8.89 (1H, d), 8.73 (1H, dd), 8.21 (1H, s), 7.94-7.90 (1H, m), 7.88 (1H, d), 7.75 (1H, d), 7.50-7.48 (1H, m), 7.48-7.46 (1H, m), 3.77 (3H, s), 3.72 (2H, q), 1.50 (3H, t).

[Chem. 87]

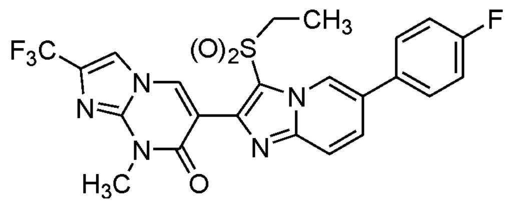

Compound 6 of the present invention: $^1$H-NMR ($CDCl_3$) $\delta$: 9.01-8.99 (1H, m), 8.19 (1H, s), 7.82 (1H, d), 7.72 (1H, d), 7.59-7.54 (2H, m), 7.47-7.45 (1H, m), 7.25-7.20 (2H, m), 3.77 (3H, s), 3.70 (2H, d), 1.48 (3H, t).

Production Example 5

35 mg of sodium hydride (60%, oil-based) was added to a mixture of 150 mg of intermediate 7, 380 mg of intermediate 20, and 2 mL of NMP, and the mixture was stirred at 80°C for 2 hours. The obtained mixture was allowed to cool to room temperature, water was added thereto, and the mixture was filtered. The obtained solid was sequentially washed with water and MTBE and dried under reduced pressure to obtain 148 mg of a compound 7 of the present invention represented by the following formula.

[Chem. 88]

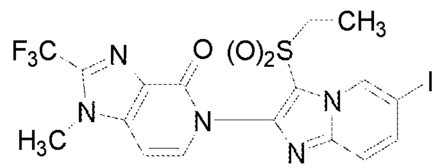

Compound 7 of the present invention: $^1$H-NMR ($CDCl_3$) $\delta$: 9.12 (1H, s), 7.74 (1H, d), 7.55 (1H, d), 7.49 (1H, d), 6.54 (1H, d), 3.92 (3H, s), 3.71-3.63 (2H, m), 1.44 (3H, t) .

Production Example 6

147 mg of cesium carbonate and 51 $\mu$L of methyl iodide were sequentially added to a mixture of 200 mg of a compound 9 of present invention and 5 mL of DMF at room temperature, and the mixture was stirred at 70°C for 30 minutes. The obtained mixture was allowed to cool to room temperature, water was added thereto, and the mixture was extracted with ethyl acetate. The obtained organic layer was dried over anhydrous sodium sulfate and concentrated under reduced pressure. The obtained residue was subjected to silica gel chromatography (ethyl acetate : hexane = 1 : 1) to obtain 64 mg of a compound 10 of present invention represented by the following formula.

[Chem. 89]

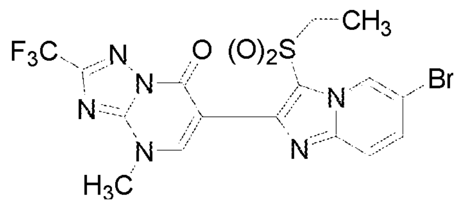

Compound 10 of the present invention: $^1$H-NMR ($CDCl_3$) $\delta$: 9.01 (1H, s), 8.02 (1H, s), 7.63 (1H, d), 7.60 (1H, d), 4.00 (3H, s), 3.79 (2H, q), 1.50 (3H, t).

Next, production examples described in examples and examples of the compound X of present invention produced in accordance with any of the production methods described in the present specification are shown below. The groups represented by Q11 to Q13 and the groups represented by Q34 to Q57 are groups shown below.

[Chem. 90]

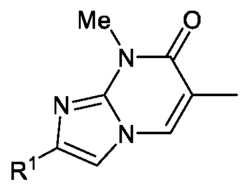
Q11

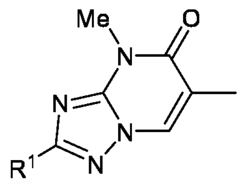
Q12

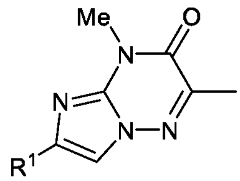
Q13

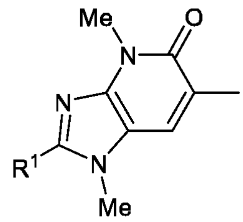
Q34

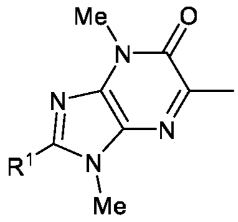
Q35

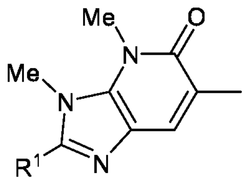
Q36

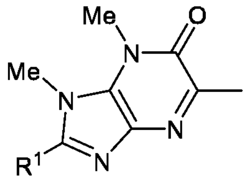
Q37

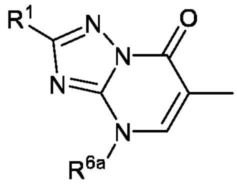
Q38

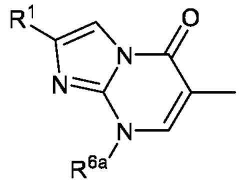
Q39

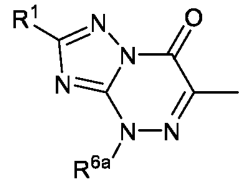
Q40

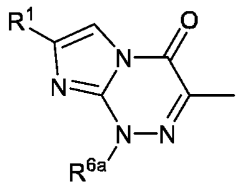
Q41

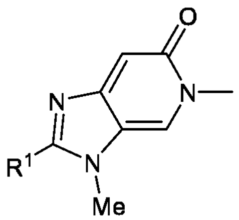
Q42

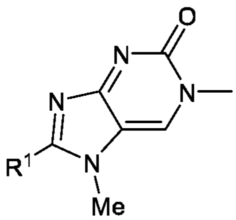
Q43

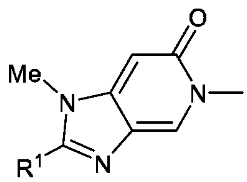
Q44

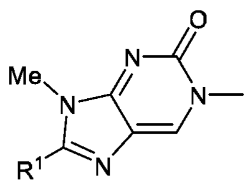
Q45

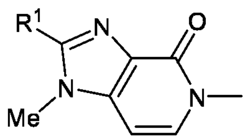
Q46

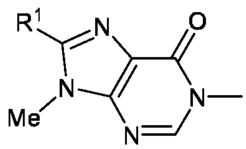
Q47

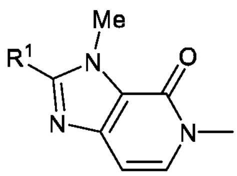
Q48

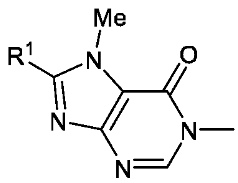
Q49

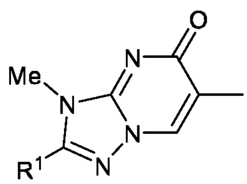
Q50

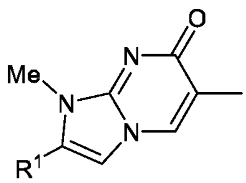
Q51

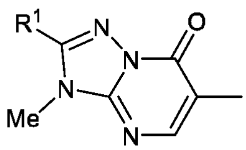
Q52

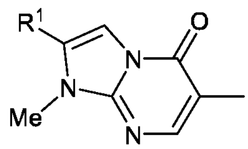
Q53

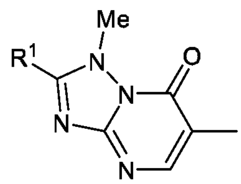
Q54

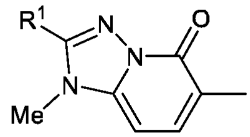
Q55

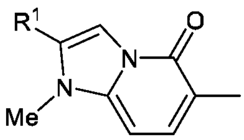
Q56

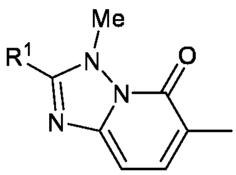
Q57

[Chem. 91]

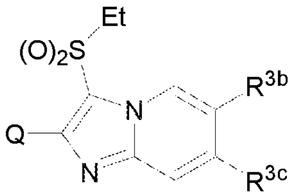

(L-1)

In a compound (hereinafter, referred to as a compound (L-1)) represented by Formula (L-1), a compound in which Q is a group represented by Q11, $R^{3b}$ and $R^{3c}$ are hydrogen atoms, and $R^1$ is any of the substituents shown in [Table L1] to [Table L3] (hereinafter, referred to as a compound group SX1).

[Table 2]

[Table L1]

| |
|---|
| $CF_3$ |
| $CHF_2$ |
| $CH_2CF_3$ |
| $CF_2CF_3$ |
| $CH_2CF_2CF_3$ |
| $CF_2CF_2CF_3$ |
| $CF_2CF_2CF_2CF_3$ |
| $CF_2CF_2CF_2CF_2CF_3$ |
| $OCF_3$ |
| $OCHF_2$ |
| $OCH_2CF_3$ |
| $OCH_2CHF_2$ |
| $OCF_2CF_3$ |
| $OCH(CH_3)CF_3$ |
| $OCH_2CF_2CHF_2$ |
| $OCH_2CF_2CF_3$ |
| $OCF_2CF_2CF_3$ |
| $OCH_2CF_2CHFCF_3$ |
| $OCH_2CF_2CF_2CF_3$ |
| $OCF_2CF_2CF_2CF_3$ |
| $OCH_2CF_2CF_2CF_2CF_3$ |
| $OCH_2C(Me)_2CN$ |
| $OCH_2$(1-CN-c-Pr) |
| $OCH_2$(2,2-$F_2$-c-Pr) |
| $OCH_2$(1-$CF_3$-c-Pr) |
| $OS(O)_2CF_3$ |
| $OS(O)_2CF_2CF_3$ |
| $OS(O)_2CF_2CF_2CF_3$ |
| 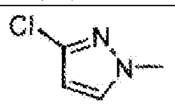 |
| 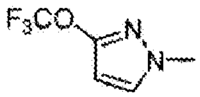 |

[Table L2]

| |
|---|
| $SCF_3$ |
| $SCH_2CF_3$ |
| $SCF_2CF_3$ |
| $SCH_2CF_2CF_3$ |
| $SCF_2CF_2CF_3$ |
| $SCH_2CF_2CF_2CF_3$ |
| $SCF_2CF_2CF_2CF_3$ |
| $S(O)CF_3$ |
| $S(O)CH_2CF_3$ |
| $S(O)CF_2CF_3$ |
| $S(O)CH_2CF_2CF_3$ |
| $S(O)CF_2CF_2CF_3$ |
| $S(O)CH_2CF_2CF_2CF_3$ |
| $S(O)CF_2CF_2CF_2CF_3$ |
| $S(O)_2CF_3$ |
| $S(O)_2CH_2CF_3$ |
| $S(O)_2CF_2CF_3$ |
| $S(O)_2CH_2CF_2CF_3$ |
| $S(O)_2CF_2CF_2CF_3$ |
| $S(O)_2CH_2CF_2CF_2CF_3$ |
| $S(O)_2CF_2CF_2CF_2CF_3$ |
| $NHCH_2CF_3$ |
| $NHCH_2CF_2CF_3$ |
| $NHCH_2CF_2CF_2CF_3$ |
| $NMeCH_2CF_3$ |
| $NMeCH_2CF_2CF_3$ |
| $NMeCH_2CF_2CF_2CF_3$ |
| $NEtCH_2CF_3$ |
| 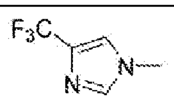 |
| 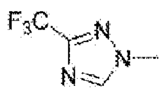 |

[Table L3]

| |
|---|
| 3-$CF_3$-Ph |
| 4-$CF_3$-Ph |
| 3,5-$(CF_3)_2$-Ph |
| 3-$SCF_3$-Ph |
| 3-$S(O)CF_3$-Ph |
| 3-$S(O)_2CF_3$-Ph |
| 4-$SCF_3$-Ph |
| 4-$S(O)CF_3$-Ph |
| 4-$S(O)_2CF_3$-Ph |
| 4-$CF_3$-Py2 |
| 5-$CF_3$-Py2 |
| 4-$SCF_3$-Py2 |
| 4-$S(O)CF_3$-Py2 |
| 4-$S(O)_2CF_3$-Py2 |
| 5-$SCF_3$-Py2 |
| 5-$S(O)CF_3$-Py2 |
| 5-$S(O)_2CF_3$-Py2 |
| 5-$NMeCH_2CF_3$-Py2 |
| 5-$CF_3$-Py3 |
| 6-$CF_3$-Py3 |
| 5-$SCF_3$-Py3 |
| 5-$S(O)CF_3$-Py3 |
| 5-$S(O)_2CF_3$-Py3 |
| 6-$SCF_3$-Py3 |
| 6-$S(O)CF_3$-Py3 |
| 6-$S(O)_2CF_3$-Py3 |
| 6-$NMeCH_2CF_3$-Py3 |
| $NEtCH_2CF_2CF_3$ |
| 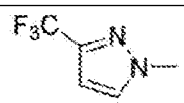 |
| 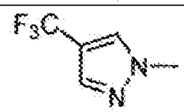 |

In the compound (L-1), a compound in which Q is a group represented by Q12, $R^{3b}$ and $R^{3c}$ are hydrogen atoms, and $R^1$ is any of the substituents shown in [Table L1] to [Table L3] (hereinafter, referred to as a compound group SX2) .

In the compound (L-1), a compound in which Q is a group represented by Q13, $R^{3b}$ and $R^{3c}$ are hydrogen atoms, and $R^1$ is any of the substituents shown in [Table L1] to [Table L3] (hereinafter, referred to as a compound group SX3).

In the compound (L-1), a compound in which Q is a group represented by Q11, $R^{3b}$ is a chlorine atom, $R^{3c}$ is a hydrogen atom, and $R^1$ is any of the substituents shown in [Table L1] to [Table L3] (hereinafter, referred to as a compound group SX4).

In the compound (L-1), a compound in which Q is a group represented by Q12, $R^{3b}$ is a chlorine atom, $R^{3c}$ is a hydrogen atom, and $R^1$ is any of the substituents shown in [Table L1] to [Table L3] (hereinafter, referred to as a compound group SX5).

In the compound (L-1), a compound in which Q is a group represented by Q13, $R^{3b}$ is a chlorine atom, $R^{3c}$ is a hydrogen atom, and $R^1$ is any of the substituents shown in [Table L1] to [Table L3] (hereinafter, referred to as a compound group SX6).

In the compound (L-1), a compound in which Q is a group represented by Q11, $R^{3b}$ is a bromine atom, $R^{3c}$ is a hydrogen atom, and $R^1$ is any of the substituents shown in [Table L1] to [Table L3] (hereinafter, referred to as a compound group SX7).

In the compound (L-1), a compound in which Q is a group represented by Q12, $R^{3b}$ is a bromine atom, $R^{3c}$ is a hydrogen atom, and $R^1$ is any of the substituents shown in [Table L1] to [Table L3] (hereinafter, referred to as a compound group SX8).

In the compound (L-1), a compound in which Q is a group represented by Q13, $R^{3b}$ is a bromine atom, $R^{3c}$ is a hydrogen atom, and $R^1$ is any of the substituents shown in [Table L1] to [Table L3] (hereinafter, referred to as a compound group SX9).

In the compound (L-1), a compound in which Q is a group represented by Q11, $R^{3b}$ is an iodine atom, $R^{3c}$ is a hydrogen atom, and $R^1$ is any of the substituents shown in [Table L1] to [Table L3] (hereinafter, referred to as a compound group SX10).

In the compound (L-1), a compound in which Q is a group represented by Q12, $R^{3b}$ is an iodine atom, $R^{3c}$ is a hydrogen atom, and $R^1$ is any of the substituents shown in [Table L1] to [Table L3] (hereinafter, referred to as a compound group SX11).

In the compound (L-1), a compound in which Q is a group represented by Q13, $R^{3b}$ is an iodine atom, $R^{3c}$ is a hydrogen atom, and $R^1$ is any of the substituents shown in [Table L1] to [Table L3] (hereinafter, referred to as a compound group SX12).

In the compound (L-1), a compound in which Q is a group represented by Q11, $R^{3b}$ is $CF_3$, $R^{3c}$ is a hydrogen atom, and $R^1$ is any of the substituents shown in [Table L1] to [Table L3] (hereinafter, referred to as a compound group SX13) .

In the compound (L-1), a compound in which Q is a group represented by Q12, $R^{3b}$ is $CF_3$, $R^{3c}$ is a hydrogen atom, and $R^1$ is any of the substituents shown in [Table L1] to [Table L3] (hereinafter, referred to as a compound group SX14) .

In the compound (L-1), a compound in which Q is a group represented by Q13, $R^{3b}$ is $CF_3$, $R^{3c}$ is a hydrogen atom, and $R^1$ is any of the substituents shown in [Table L1] to [Table L3] (hereinafter, referred to as a compound group SX15).

In the compound (L-1), a compound in which Q is a group represented by Q11, $R^{3b}$ is a hydrogen atom, $R^{3c}$ is a chlorine atom, and $R^1$ is any of the substituents shown in [Table L1] to [Table L3] (hereinafter, referred to as a compound group SX16).

In the compound (L-1), a compound in which Q is a group represented by Q12, $R^{3b}$ is a hydrogen atom, $R^{3c}$ is a chlorine atom, and $R^1$ is any of the substituents shown in [Table L1] to [Table L3] (hereinafter, referred to as a compound group SX17).

In the compound (L-1), a compound in which Q is a group represented by Q13, $R^{3b}$ is a hydrogen atom, $R^{3c}$ is a chlorine atom, and $R^1$ is any of the substituents shown in [Table L1] to [Table L3] (hereinafter, referred to as a compound group SX18).

In the compound (L-1), a compound in which Q is a group represented by Q11, $R^{3b}$ is a hydrogen atom, $R^{3c}$ is a bromine atom, and $R^1$ is any of the substituents shown in [Table L1] to [Table L3] (hereinafter, referred to as a compound group SX19).

In the compound (L-1), a compound in which Q is a group represented by Q12, $R^{3b}$ is a hydrogen atom, $R^{3c}$ is a bromine atom, and $R^1$ is any of the substituents shown in [Table L1] to [Table L3] (hereinafter, referred to as a compound group SX20).

In the compound (L-1), a compound in which Q is a group represented by Q13, $R^{3b}$ is a hydrogen atom, $R^{3c}$ is a bromine atom, and $R^1$ is any of the substituents shown in [Table L1] to [Table L3] (hereinafter, referred to as a

compound group SX21).

In the compound (L-1), a compound in which Q is a group represented by Q11, $R^{3b}$ is a hydrogen atom, $R^{3c}$ is an iodine atom, and $R^1$ is any of the substituents shown in [Table L1] to [Table L3] (hereinafter, referred to as a compound group SX22).

In the compound (L-1), a compound in which Q is a group represented by Q12, $R^{3b}$ is a hydrogen atom, $R^{3c}$ is an iodine atom, and $R^1$ is any of the substituents shown in [Table L1] to [Table L3] (hereinafter, referred to as a compound group SX23).

In the compound (L-1), a compound in which Q is a group represented by Q13, $R^{3b}$ is a hydrogen atom, $R^{3c}$ is an iodine atom, and $R^1$ is any of the substituents shown in [Table L1] to [Table L3] (hereinafter, referred to as a compound group SX24).

In the compound (L-1), a compound in which Q is a group represented by Q11, $R^{3b}$ is a hydrogen atom, $R^{3c}$ is $CF_3$, and $R^1$ is any of the substituents shown in [Table L1] to [Table L3] (hereinafter, referred to as a compound group SX25) .

In the compound (L-1), a compound in which Q is a group represented by Q12, $R^{3b}$ is a hydrogen atom, $R^{3c}$ is $CF_3$, and $R^1$ is any of the substituents shown in [Table L1] to [Table L3] (hereinafter, referred to as a compound group SX26) .

In the compound (L-1), a compound in which Q is a group represented by Q13, $R^{3b}$ is a hydrogen atom, $R^{3c}$ is $CF_3$, and $R^1$ is any of the substituents shown in [Table L1] to [Table L3] (hereinafter, referred to as a compound group SX27) .

In the compound (L-1), a compound in which Q is a group represented by Q34, $R^{3b}$ and $R^{3c}$ are hydrogen atoms, and $R^1$ is any of the substituents shown in [Table L1] to [Table L3] (hereinafter, referred to as a compound group SX184).

In the compound (L-1), a compound in which Q is a group represented by Q34, $R^{3b}$ is a chlorine atom, $R^{3c}$ is a hydrogen atom, and $R^1$ is any of the substituents shown in [Table L1] to [Table L3] (hereinafter, referred to as a compound group SX185).

In the compound (L-1), a compound in which Q is a group represented by Q34, $R^{3b}$ is a bromine atom, $R^{3c}$ is a hydrogen atom, and $R^1$ is any of the substituents shown in [Table L1] to [Table L3] (hereinafter, referred to as a compound group SX186).

In the compound (L-1), a compound in which Q is a group represented by Q34, $R^{3b}$ is an iodine atom, $R^{3c}$ is a hydrogen atom, and $R^1$ is any of the substituents shown in [Table L1] to [Table L3] (hereinafter, referred to as a compound group SX187).

In the compound (L-1), a compound in which Q is a group represented by Q34, $R^{3b}$ is $CF_3$, $R^{3c}$ is a hydrogen atom, and $R^1$ is any of the substituents shown in [Table L1] to [Table L3] (hereinafter, referred to as a compound group SX188).

In the compound (L-1), a compound in which Q is a group represented by Q34, $R^{3b}$ is a hydrogen atom, $R^{3c}$ is a chlorine atom, and $R^1$ is any of the substituents shown in [Table L1] to [Table L3] (hereinafter, referred to as a compound group SX189).

In the compound (L-1), a compound in which Q is a group represented by Q34, $R^{3b}$ is a hydrogen atom, $R^{3c}$ is a bromine atom, and $R^1$ is any of the substituents shown in [Table L1] to [Table L3] (hereinafter, referred to as a compound group SX190).

In the compound (L-1), a compound in which Q is a group represented by Q34, $R^{3b}$ is a hydrogen atom, $R^{3c}$ is an iodine atom, and $R^1$ is any of the substituents shown in [Table L1] to [Table L3] (hereinafter, referred to as a compound group SX191).

In the compound (L-1), a compound in which Q is a group represented by Q34, $R^{3b}$ is a hydrogen atom, $R^{3c}$ is $CF_3$, and $R^1$ is any of the substituents shown in [Table L1] to [Table L3] (hereinafter, referred to as a compound group SX192).

In the compound (L-1), a compound in which Q is a group represented by Q35, $R^{3b}$ and $R^{3c}$ are hydrogen atoms, and $R^1$ is any of the substituents shown in [Table L1] to [Table L3] (hereinafter, referred to as a compound group SX193).

In the compound (L-1), a compound in which Q is a group represented by Q35, $R^{3b}$ is a chlorine atom, $R^{3c}$ is a hydrogen atom, and $R^1$ is any of the substituents shown in [Table L1] to [Table L3] (hereinafter, referred to as a compound group SX194).

In the compound (L-1), a compound in which Q is a group represented by Q35, $R^{3b}$ is a bromine atom, $R^{3c}$ is a hydrogen atom, and $R^1$ is any of the substituents shown in [Table L1] to [Table L3] (hereinafter, referred to as a compound group SX195).

In the compound (L-1), a compound in which Q is a group represented by Q35, $R^{3b}$ is an iodine atom, $R^{3c}$ is a hydrogen atom, and $R^1$ is any of the substituents shown in [Table L1] to [Table L3] (hereinafter, referred to as a compound group SX196).

In the compound (L-1), a compound in which Q is a group represented by Q35, $R^{3b}$ is $CF_3$, $R^{3c}$ is a hydrogen atom, and $R^1$ is any of the substituents shown in [Table L1] to [Table L3] (hereinafter, referred to as a compound group SX197).

In the compound (L-1), a compound in which Q is a group represented by Q35, $R^{3b}$ is a hydrogen atom, $R^{3c}$ is a chlorine atom, and $R^1$ is any of the substituents shown in [Table L1] to [Table L3] (hereinafter, referred to as a compound group SX198).

In the compound (L-1), a compound in which Q is a group represented by Q35, $R^{3b}$ is a hydrogen atom, $R^{3c}$ is a bromine atom, and $R^1$ is any of the substituents shown in [Table L1] to [Table L3] (hereinafter, referred to as a compound group SX199).

In the compound (L-1), a compound in which Q is a group represented by Q35, $R^{3b}$ is a hydrogen atom, $R^{3c}$ is an iodine atom, and $R^1$ is any of the substituents shown in [Table L1] to [Table L3] (hereinafter, referred to as a compound group SX200).

In the compound (L-1), a compound in which Q is a group represented by Q35, $R^{3b}$ is a hydrogen atom, $R^{3c}$ is $CF_3$, and $R^1$ is any of the substituents shown in [Table L1] to [Table L3] (hereinafter, referred to as a compound group SX201).

In the compound (L-1), a compound in which Q is a group represented by Q36, $R^{3b}$ and $R^{3c}$ are hydrogen atoms, and $R^1$ is any of the substituents shown in [Table L1] to [Table L3] (hereinafter, referred to as a compound group SX202).

In the compound (L-1), a compound in which Q is a group represented by Q36, $R^{3b}$ is a chlorine atom, $R^{3c}$ is a hydrogen atom, and $R^1$ is any of the substituents shown in [Table L1] to [Table L3] (hereinafter, referred to as a compound group SX203).

In the compound (L-1), a compound in which Q is a group represented by Q36, $R^{3b}$ is a bromine atom, $R^{3c}$ is a hydrogen atom, and $R^1$ is any of the substituents shown in [Table L1] to [Table L3] (hereinafter, referred to as a compound group SX204).

In the compound (L-1), a compound in which Q is a group represented by Q36, $R^{3b}$ is an iodine atom, $R^{3c}$ is a hydrogen atom, and $R^1$ is any of the substituents shown in [Table L1] to [Table L3] (hereinafter, referred to as a compound group SX205).

In the compound (L-1), a compound in which Q is a group represented by Q36, $R^{3b}$ is $CF_3$, $R^{3c}$ is a hydrogen atom, and $R^1$ is any of the substituents shown in [Table L1] to [Table L3] (hereinafter, referred to as a compound group SX206).

In the compound (L-1), a compound in which Q is a group represented by Q36, $R^{3b}$ is a hydrogen atom, $R^{3c}$ is a chlorine atom, and $R^1$ is any of the substituents shown in [Table L1] to [Table L3] (hereinafter, referred to as a compound group SX207).

In the compound (L-1), a compound in which Q is a group represented by Q36, $R^{3b}$ is a hydrogen atom, $R^{3c}$ is a bromine atom, and $R^1$ is any of the substituents shown in [Table L1] to [Table L3] (hereinafter, referred to as a compound group SX208).

In the compound (L-1), a compound in which Q is a group represented by Q36, $R^{3b}$ is a hydrogen atom, $R^{3c}$ is an iodine atom, and $R^1$ is any of the substituents shown in [Table L1] to [Table L3] (hereinafter, referred to as a compound group SX209).

In the compound (L-1), a compound in which Q is a group represented by Q36, $R^{3b}$ is a hydrogen atom, $R^{3c}$ is $CF_3$, and $R^1$ is any of the substituents shown in [Table L1] to [Table L3] (hereinafter, referred to as a compound group SX210).

In the compound (L-1), a compound in which Q is a group represented by Q37, $R^{3b}$ and $R^{3c}$ are hydrogen atoms, and $R^1$ is any of the substituents shown in [Table L1] to [Table L3] (hereinafter, referred to as a compound group SX211).

In the compound (L-1), a compound in which Q is a group represented by Q37, $R^{3b}$ is a chlorine atom, $R^{3c}$ is a hydrogen atom, and $R^1$ is any of the substituents shown in [Table L1] to [Table L3] (hereinafter, referred to as a compound group SX212).

In the compound (L-1), a compound in which Q is a group represented by Q37, $R^{3b}$ is a bromine atom, $R^{3c}$ is a hydrogen atom, and $R^1$ is any of the substituents shown in [Table L1] to [Table L3] (hereinafter, referred to as a compound group SX213).

In the compound (L-1), a compound in which Q is a group represented by Q37, $R^{3b}$ is an iodine atom, $R^{3c}$ is a hydrogen atom, and $R^1$ is any of the substituents shown in [Table L1] to [Table L3] (hereinafter, referred to as a compound group SX214).

In the compound (L-1), a compound in which Q is a group represented by Q37, $R^{3b}$ is $CF_3$, $R^{3c}$ is a hydrogen atom, and $R^1$ is any of the substituents shown in [Table L1] to [Table L3] (hereinafter, referred to as a compound group SX215).

In the compound (L-1), a compound in which Q is a group represented by Q37, $R^{3b}$ is a hydrogen atom, $R^{3c}$

is a chlorine atom, and $R^1$ is any of the substituents shown in [Table L1] to [Table L3] (hereinafter, referred to as a compound group SX216).

In the compound (L-1), a compound in which Q is a group represented by Q37, $R^{3b}$ is a hydrogen atom, $R^{3c}$ is a bromine atom, and $R^1$ is any of the substituents shown in [Table L1] to [Table L3] (hereinafter, referred to as a compound group SX217).

In the compound (L-1), a compound in which Q is a group represented by Q37, $R^{3b}$ is a hydrogen atom, $R^{3c}$ is an iodine atom, and $R^1$ is any of the substituents shown in [Table L1] to [Table L3] (hereinafter, referred to as a compound group SX218).

In the compound (L-1), a compound in which Q is a group represented by Q37, $R^{3b}$ is a hydrogen atom, $R^{3c}$ is $CF_3$, and $R^1$ is any of the substituents shown in [Table L1] to [Table L3] (hereinafter, referred to as a compound group SX219).

In the compound (L-1), a compound in which Q is a group represented by Q38, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX220).

In the compound (L-1), a compound in which Q is a group represented by Q38, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX221).

In the compound (L-1), a compound in which Q is a group represented by Q38, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX222).

In the compound (L-1), a compound in which Q is a group represented by Q38, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX223).

In the compound (L-1), a compound in which Q is a group represented by Q38, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX224).

In the compound (L-1), a compound in which Q is a group represented by Q38, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX225).

In the compound (L-1), a compound in which Q is a group represented by Q38, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX226).

In the compound (L-1), a compound in which Q is a group represented by Q38, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX227).

In the compound (L-1), a compound in which Q is a group represented by Q38, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX228).

In the compound (L-1), a compound in which Q is a group represented by Q38, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX229).

In the compound (L-1), a compound in which Q is a group represented by Q38, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX230).

In the compound (L-1), a compound in which Q is a group represented by Q38, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX231).

In the compound (L-1), a compound in which Q is a group represented by Q38, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX232).

In the compound (L-1), a compound in which Q is a group represented by Q38, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX233).

In the compound (L-1), a compound in which Q is a group represented by Q38, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX234).

In the compound (L-1), a compound in which Q is a group represented by Q38, $R^{3b}$ is a hydrogen atom, $R^{3c}$ is a bromine atom, $R^{6a}$ is a methyl group, and $R^1$ is any of the substituents shown in [Table L1] to [Table L3] (hereinafter,

referred to as a compound group SX235).

In the compound (L-1), a compound in which Q is a group represented by Q38, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX236).

In the compound (L-1), a compound in which Q is a group represented by Q38, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX237).

In the compound (L-1), a compound in which Q is a group represented by Q39, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX238).

In the compound (L-1), a compound in which Q is a group represented by Q39, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX239).

In the compound (L-1), a compound in which Q is a group represented by Q39, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX240).

In the compound (L-1), a compound in which Q is a group represented by Q39, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX241).

In the compound (L-1), a compound in which Q is a group represented by Q39, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX242).

In the compound (L-1), a compound in which Q is a group represented by Q39, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX243).

In the compound (L-1), a compound in which Q is a group represented by Q39, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX244).

In the compound (L-1), a compound in which Q is a group represented by Q39, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX245).

In the compound (L-1), a compound in which Q is a group represented by Q39, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX246).

In the compound (L-1), a compound in which Q is a group represented by Q39, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX247).

In the compound (L-1), a compound in which Q is a group represented by Q39, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX248).

In the compound (L-1), a compound in which Q is a group represented by Q39, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX249).

In the compound (L-1), a compound in which Q is a group represented by Q39, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX250).

In the compound (L-1), a compound in which Q is a group represented by Q39, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX251).

In the compound (L-1), a compound in which Q is a group represented by Q39, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX252).

In the compound (L-1), a compound in which Q is a group represented by Q39, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX253).

In the compound (L-1), a compound in which Q is a group represented by Q39, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX254).

In the compound (L-1), a compound in which Q is a group represented by Q39, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX255).

In the compound (L-1), a compound in which Q is a group represented by Q40, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX256).

In the compound (L-1), a compound in which Q is a group represented by Q40, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX257).

In the compound (L-1), a compound in which Q is a group represented by Q40, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX258).

In the compound (L-1), a compound in which Q is a group represented by Q40, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX259).

In the compound (L-1), a compound in which Q is a group represented by Q40, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX260).

In the compound (L-1), a compound in which Q is a group represented by Q40, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX261).

In the compound (L-1), a compound in which Q is a group represented by Q40, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX262).

In the compound (L-1), a compound in which Q is a group represented by Q40, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX263).

In the compound (L-1), a compound in which Q is a group represented by Q40, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX264).

In the compound (L-1), a compound in which Q is a group represented by Q40, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX265).

In the compound (L-1), a compound in which Q is a group represented by Q40, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX266).

In the compound (L-1), a compound in which Q is a group represented by Q40, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX267).

In the compound (L-1), a compound in which Q is a group represented by Q40, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX268).

In the compound (L-1), a compound in which Q is a group represented by Q40, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX269).

In the compound (L-1), a compound in which Q is a group represented by Q40, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX270).

In the compound (L-1), a compound in which Q is a group represented by Q40, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX271).

In the compound (L-1), a compound in which Q is a group represented by Q40, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX272).

In the compound (L-1), a compound in which Q is a group represented by Q40, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX273).

In the compound (L-1), a compound in which Q is a group represented by Q41, $R^{3b}$ and $R^{3c}$ are hydrogen

atoms, $R^{6a}$ is a hydrogen atom, and $R^1$ is any of the substituents shown in [Table L1] to [Table L3] (hereinafter, referred to as a compound group SX274).

In the compound (L-1), a compound in which Q is a group represented by Q41, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX275).

In the compound (L-1), a compound in which Q is a group represented by Q41, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX276).

In the compound (L-1), a compound in which Q is a group represented by Q41, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX277).

In the compound (L-1), a compound in which Q is a group represented by Q41, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX278).

In the compound (L-1), a compound in which Q is a group represented by Q41, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX279).

In the compound (L-1), a compound in which Q is a group represented by Q41, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX280).

In the compound (L-1), a compound in which Q is a group represented by Q41, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX281).

In the compound (L-1), a compound in which Q is a group represented by Q41, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX282).

In the compound (L-1), a compound in which Q is a group represented by Q41, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX283).

In the compound (L-1), a compound in which Q is a group represented by Q41, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX284).

In the compound (L-1), a compound in which Q is a group represented by Q41, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX285).

In the compound (L-1), a compound in which Q is a group represented by Q41, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX286).

In the compound (L-1), a compound in which Q is a group represented by Q41, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX287).

In the compound (L-1), a compound in which Q is a group represented by Q41, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX288).

In the compound (L-1), a compound in which Q is a group represented by Q41, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX289).

In the compound (L-1), a compound in which Q is a group represented by Q41, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX290).

In the compound (L-1), a compound in which Q is a group represented by Q41, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX291).

In the compound (L-1), a compound in which Q is a group represented by Q42, $R^{3b}$ and $R^{3c}$ are hydrogen atoms, and $R^1$ is any of the substituents shown in [Table L1] to [Table L3] (hereinafter, referred to as a compound group SX292).

In the compound (L-1), a compound in which Q is a group represented by Q42, $R^{3b}$ is a chlorine atom, $R^{3c}$ is a hydrogen atom, and $R^1$ is any of the substituents shown in [Table L1] to [Table L3] (hereinafter, referred to as a

compound group SX293).

In the compound (L-1), a compound in which Q is a group represented by Q42, $R^{3b}$ is a bromine atom, $R^{3c}$ is a hydrogen atom, and $R^1$ is any of the substituents shown in [Table L1] to [Table L3] (hereinafter, referred to as a compound group SX294).

In the compound (L-1), a compound in which Q is a group represented by Q42, $R^{3b}$ is an iodine atom, $R^{3c}$ is a hydrogen atom, and $R^1$ is any of the substituents shown in [Table L1] to [Table L3] (hereinafter, referred to as a compound group SX295).

In the compound (L-1), a compound in which Q is a group represented by Q42, $R^{3b}$ is $CF_3$, $R^{3c}$ is a hydrogen atom, and $R^1$ is any of the substituents shown in [Table L1] to [Table L3] (hereinafter, referred to as a compound group SX296) .

In the compound (L-1), a compound in which Q is a group represented by Q42, $R^{3b}$ is a hydrogen atom, $R^{3c}$ is a chlorine atom, and $R^1$ is any of the substituents shown in [Table L1] to [Table L3] (hereinafter, referred to as a compound group SX297).

In the compound (L-1), a compound in which Q is a group represented by Q42, $R^{3b}$ is a hydrogen atom, $R^{3c}$ is a bromine atom, and $R^1$ is any of the substituents shown in [Table L1] to [Table L3] (hereinafter, referred to as a compound group SX298).

In the compound (L-1), a compound in which Q is a group represented by Q42, $R^{3b}$ is a hydrogen atom, $R^{3c}$ is an iodine atom, and $R^1$ is any of the substituents shown in [Table L1] to [Table L3] (hereinafter, referred to as a compound group SX299).

In the compound (L-1), a compound in which Q is a group represented by Q42, $R^{3b}$ is a hydrogen atom, $R^{3c}$ is $CF_3$, and $R^1$ is any of the substituents shown in [Table L1] to [Table L3] (hereinafter, referred to as a compound group SX300) .

In the compound (L-1), a compound in which Q is a group represented by Q43, $R^{3b}$ and $R^{3c}$ are hydrogen atoms, and $R^1$ is any of the substituents shown in [Table L1] to [Table L3] (hereinafter, referred to as a compound group SX301) .

In the compound (L-1), a compound in which Q is a group represented by Q43, $R^{3b}$ is a chlorine atom, $R^{3c}$ is a hydrogen atom, and $R^1$ is any of the substituents shown in [Table L1] to [Table L3] (hereinafter, referred to as a compound group SX302).

In the compound (L-1), a compound in which Q is a group represented by Q43, $R^{3b}$ is a bromine atom, $R^{3c}$ is a hydrogen atom, and $R^1$ is any of the substituents shown in [Table L1] to [Table L3] (hereinafter, referred to as a compound group SX303).

In the compound (L-1), a compound in which Q is a group represented by Q43, $R^{3b}$ is an iodine atom, $R^{3c}$ is a hydrogen atom, and $R^1$ is any of the substituents shown in [Table L1] to [Table L3] (hereinafter, referred to as a compound group SX304).

In the compound (L-1), a compound in which Q is a group represented by Q43, $R^{3b}$ is $CF_3$, $R^{3c}$ is a hydrogen atom, and $R^1$ is any of the substituents shown in [Table L1] to [Table L3] (hereinafter, referred to as a compound group SX305) .

In the compound (L-1), a compound in which Q is a group represented by Q43, $R^{3b}$ is a hydrogen atom, $R^{3c}$ is a chlorine atom, and $R^1$ is any of the substituents shown in [Table L1] to [Table L3] (hereinafter, referred to as a compound group SX306).

In the compound (L-1), a compound in which Q is a group represented by Q43, $R^{3b}$ is a hydrogen atom, $R^{3c}$ is a bromine atom, and $R^1$ is any of the substituents shown in [Table L1] to [Table L3] (hereinafter, referred to as a compound group SX307).

In the compound (L-1), a compound in which Q is a group represented by Q43, $R^{3b}$ is a hydrogen atom, $R^{3c}$ is an iodine atom, and $R^1$ is any of the substituents shown in [Table L1] to [Table L3] (hereinafter, referred to as a compound group SX308).

In the compound (L-1), a compound in which Q is a group represented by Q43, $R^{3b}$ is a hydrogen atom, $R^{3c}$ is $CF_3$, and $R^1$ is any of the substituents shown in [Table L1] to [Table L3] (hereinafter, referred to as a compound group SX309) .

In the compound (L-1), a compound in which Q is a group represented by Q44, $R^{3b}$ and $R^{3c}$ are hydrogen atoms, and $R^1$ is any of the substituents shown in [Table L1] to [Table L3] (hereinafter, referred to as a compound group SX310) .

In the compound (L-1), a compound in which Q is a group represented by Q44, $R^{3b}$ is a chlorine atom, $R^{3c}$ is a hydrogen atom, and $R^1$ is any of the substituents shown in [Table L1] to [Table L3] (hereinafter, referred to as a compound group SX311).

In the compound (L-1), a compound in which Q is a group represented by Q44, $R^{3b}$ is a bromine atom, $R^{3c}$ is a hydrogen atom, and $R^1$ is any of the substituents shown in [Table L1] to [Table L3] (hereinafter, referred to as a compound group SX312).

In the compound (L-1), a compound in which Q is a group represented by Q44, $R^{3b}$ is an iodine atom, $R^{3c}$ is a hydrogen atom, and $R^1$ is any of the substituents shown in [Table L1] to [Table L3] (hereinafter, referred to as a compound group SX313).

In the compound (L-1), a compound in which Q is a group represented by Q44, $R^{3b}$ is $CF_3$, $R^{3c}$ is a hydrogen atom, and $R^1$ is any of the substituents shown in [Table L1] to [Table L3] (hereinafter, referred to as a compound group SX314) .

In the compound (L-1), a compound in which Q is a group represented by Q44, $R^{3b}$ is a hydrogen atom, $R^{3c}$ is a chlorine atom, and $R^1$ is any of the substituents shown in [Table L1] to [Table L3] (hereinafter, referred to as a compound group SX315).

In the compound (L-1), a compound in which Q is a group represented by Q44, $R^{3b}$ is a hydrogen atom, $R^{3c}$ is a bromine atom, and $R^1$ is any of the substituents shown in [Table L1] to [Table L3] (hereinafter, referred to as a compound group SX316).

In the compound (L-1), a compound in which Q is a group represented by Q44, $R^{3b}$ is a hydrogen atom, $R^{3c}$ is an iodine atom, and $R^1$ is any of the substituents shown in [Table L1] to [Table L3] (hereinafter, referred to as a compound group SX317).

In the compound (L-1), a compound in which Q is a group represented by Q44, $R^{3b}$ is a hydrogen atom, $R^{3c}$ is $CF_3$, and $R^1$ is any of the substituents shown in [Table L1] to [Table L3] (hereinafter, referred to as a compound group SX318) .

In the compound (L-1), a compound in which Q is a group represented by Q45, $R^{3b}$ and $R^{3c}$ are hydrogen atoms, and $R^1$ is any of the substituents shown in [Table L1] to [Table L3] (hereinafter, referred to as a compound group SX319) .

In the compound (L-1), a compound in which Q is a group represented by Q45, $R^{3b}$ is a chlorine atom, $R^{3c}$ is a hydrogen atom, and $R^1$ is any of the substituents shown in [Table L1] to [Table L3] (hereinafter, referred to as a compound group SX320).

In the compound (L-1), a compound in which Q is a group represented by Q45, $R^{3b}$ is a bromine atom, $R^{3c}$ is a hydrogen atom, and $R^1$ is any of the substituents shown in [Table L1] to [Table L3] (hereinafter, referred to as a compound group SX321).

In the compound (L-1), a compound in which Q is a group represented by Q45, $R^{3b}$ is an iodine atom, $R^{3c}$ is a hydrogen atom, and $R^1$ is any of the substituents shown in [Table L1] to [Table L3] (hereinafter, referred to as a compound group SX322).

In the compound (L-1), a compound in which Q is a group represented by Q45, $R^{3b}$ is $CF_3$, $R^{3c}$ is a hydrogen atom, and $R^1$ is any of the substituents shown in [Table L1] to [Table L3] (hereinafter, referred to as a compound group SX323) .

In the compound (L-1), a compound in which Q is a group represented by Q45, $R^{3b}$ is a hydrogen atom, $R^{3c}$ is a chlorine atom, and $R^1$ is any of the substituents shown in [Table L1] to [Table L3] (hereinafter, referred to as a compound group SX324).

In the compound (L-1), a compound in which Q is a group represented by Q45, $R^{3b}$ is a hydrogen atom, $R^{3c}$ is a bromine atom, and $R^1$ is any of the substituents shown in [Table L1] to [Table L3] (hereinafter, referred to as a compound group SX325).

In the compound (L-1), a compound in which Q is a group represented by Q45, $R^{3b}$ is a hydrogen atom, $R^{3c}$ is an iodine atom, and $R^1$ is any of the substituents shown in [Table L1] to [Table L3] (hereinafter, referred to as a compound group SX326).

In the compound (L-1), a compound in which Q is a group represented by Q45, $R^{3b}$ is a hydrogen atom, $R^{3c}$ is $CF_3$, and $R^1$ is any of the substituents shown in [Table L1] to [Table L3] (hereinafter, referred to as a compound group SX327) .

In the compound (L-1), a compound in which Q is a group represented by Q46, $R^{3b}$ and $R^{3c}$ are hydrogen atoms, and $R^1$ is any of the substituents shown in [Table L1] to [Table L3] (hereinafter, referred to as a compound group SX328) .

In the compound (L-1), a compound in which Q is a group represented by Q46, $R^{3b}$ is a chlorine atom, $R^{3c}$ is a hydrogen atom, and $R^1$ is any of the substituents shown in [Table L1] to [Table L3] (hereinafter, referred to as a compound group SX329).

In the compound (L-1), a compound in which Q is a group represented by Q46, $R^{3b}$ is a bromine atom, $R^{3c}$ is a hydrogen atom, and $R^1$ is any of the substituents shown in [Table L1] to [Table L3] (hereinafter, referred to as a compound group SX330).

In the compound (L-1), a compound in which Q is a group represented by Q46, $R^{3b}$ is an iodine atom, $R^{3c}$ is a hydrogen atom, and $R^1$ is any of the substituents shown in [Table L1] to [Table L3] (hereinafter, referred to as a compound group SX331).

In the compound (L-1), a compound in which Q is a group represented by Q46, $R^{3b}$ is $CF_3$, $R^{3c}$ is a hydrogen

atom, and $R^1$ is any of the substituents shown in [Table L1] to [Table L3] (hereinafter, referred to as a compound group SX332) .

In the compound (L-1), a compound in which Q is a group represented by Q46, $R^{3b}$ is a hydrogen atom, $R^{3c}$ is a chlorine atom, and $R^1$ is any of the substituents shown in [Table L1] to [Table L3] (hereinafter, referred to as a compound group SX333).

In the compound (L-1), a compound in which Q is a group represented by Q46, $R^{3b}$ is a hydrogen atom, $R^{3c}$ is a bromine atom, and $R^1$ is any of the substituents shown in [Table L1] to [Table L3] (hereinafter, referred to as a compound group SX334).

In the compound (L-1), a compound in which Q is a group represented by Q46, $R^{3b}$ is a hydrogen atom, $R^{3c}$ is an iodine atom, and $R^1$ is any of the substituents shown in [Table L1] to [Table L3] (hereinafter, referred to as a compound group SX335).

In the compound (L-1), a compound in which Q is a group represented by Q46, $R^{3b}$ is a hydrogen atom, $R^{3c}$ is $CF_3$, and $R^1$ is any of the substituents shown in [Table L1] to [Table L3] (hereinafter, referred to as a compound group SX336) .

In the compound (L-1), a compound in which Q is a group represented by Q47, $R^{3b}$ and $R^{3c}$ are hydrogen atoms, and $R^1$ is any of the substituents shown in [Table L1] to [Table L3] (hereinafter, referred to as a compound group SX337) .

In the compound (L-1), a compound in which Q is a group represented by Q47, $R^{3b}$ is a chlorine atom, $R^{3c}$ is a hydrogen atom, and $R^1$ is any of the substituents shown in [Table L1] to [Table L3] (hereinafter, referred to as a compound group SX338).

In the compound (L-1), a compound in which Q is a group represented by Q47, $R^{3b}$ is a bromine atom, $R^{3c}$ is a hydrogen atom, and $R^1$ is any of the substituents shown in [Table L1] to [Table L3] (hereinafter, referred to as a compound group SX339).

In the compound (L-1), a compound in which Q is a group represented by Q47, $R^{3b}$ is an iodine atom, $R^{3c}$ is a hydrogen atom, and $R^1$ is any of the substituents shown in [Table L1] to [Table L3] (hereinafter, referred to as a compound group SX340).

In the compound (L-1), a compound in which Q is a group represented by Q47, $R^{3b}$ is $CF_3$, $R^{3c}$ is a hydrogen atom, and $R^1$ is any of the substituents shown in [Table L1] to [Table L3] (hereinafter, referred to as a compound group SX341).

In the compound (L-1), a compound in which Q is a group represented by Q47, $R^{3b}$ is a hydrogen atom, $R^{3c}$ is a chlorine atom, and $R^1$ is any of the substituents shown in [Table L1] to [Table L3] (hereinafter, referred to as a compound group SX342).

In the compound (L-1), a compound in which Q is a group represented by Q47, $R^{3b}$ is a hydrogen atom, $R^{3c}$ is a bromine atom, and $R^1$ is any of the substituents shown in [Table L1] to [Table L3] (hereinafter, referred to as a compound group SX343).

In the compound (L-1), a compound in which Q is a group represented by Q47, $R^{3b}$ is a hydrogen atom, $R^{3c}$ is an iodine atom, and $R^1$ is any of the substituents shown in [Table L1] to [Table L3] (hereinafter, referred to as a compound group SX344).

In the compound (L-1), a compound in which Q is a group represented by Q47, $R^{3b}$ is a hydrogen atom, $R^{3c}$ is $CF_3$, and $R^1$ is any of the substituents shown in [Table L1] to [Table L3] (hereinafter, referred to as a compound group SX345) .

In the compound (L-1), a compound in which Q is a group represented by Q48, $R^{3b}$ and $R^{3c}$ are hydrogen atoms, and $R^1$ is any of the substituents shown in [Table L1] to [Table L3] (hereinafter, referred to as a compound group SX346) .

In the compound (L-1), a compound in which Q is a group represented by Q48, $R^{3b}$ is a chlorine atom, $R^{3c}$ is a hydrogen atom, and $R^1$ is any of the substituents shown in [Table L1] to [Table L3] (hereinafter, referred to as a compound group SX347).

In the compound (L-1), a compound in which Q is a group represented by Q48, $R^{3b}$ is a bromine atom, $R^{3c}$ is a hydrogen atom, and $R^1$ is any of the substituents shown in [Table L1] to [Table L3] (hereinafter, referred to as a compound group SX348).

In the compound (L-1), a compound in which Q is a group represented by Q48, $R^{3b}$ is an iodine atom, $R^{3c}$ is a hydrogen atom, and $R^1$ is any of the substituents shown in [Table L1] to [Table L3] (hereinafter, referred to as a compound group SX349).

In the compound (L-1), a compound in which Q is a group represented by Q48, $R^{3b}$ is $CF_3$, $R^{3c}$ is a hydrogen atom, and $R^1$ is any of the substituents shown in [Table L1] to [Table L3] (hereinafter, referred to as a compound group SX350).

In the compound (L-1), a compound in which Q is a group represented by Q48, $R^{3b}$ is a hydrogen atom, $R^{3c}$ is a chlorine atom, and $R^1$ is any of the substituents shown in [Table L1] to [Table L3] (hereinafter, referred to as a

compound group SX351).

In the compound (L-1), a compound in which Q is a group represented by Q48, $R^{3b}$ is a hydrogen atom, $R^{3c}$ is a bromine atom, and $R^1$ is any of the substituents shown in [Table L1] to [Table L3] (hereinafter, referred to as a compound group SX352).

In the compound (L-1), a compound in which Q is a group represented by Q48, $R^{3b}$ is a hydrogen atom, $R^{3c}$ is an iodine atom, and $R^1$ is any of the substituents shown in [Table L1] to [Table L3] (hereinafter, referred to as a compound group SX353).

In the compound (L-1), a compound in which Q is a group represented by Q48, $R^{3b}$ is a hydrogen atom, $R^{3c}$ is $CF_3$, and $R^1$ is any of the substituents shown in [Table L1] to [Table L3] (hereinafter, referred to as a compound group SX354) .

In the compound (L-1), a compound in which Q is a group represented by Q49, $R^{3b}$ and $R^{3c}$ are hydrogen atoms, and $R^1$ is any of the substituents shown in [Table L1] to [Table L3] (hereinafter, referred to as a compound group SX355) .

In the compound (L-1), a compound in which Q is a group represented by Q49, $R^{3b}$ is a chlorine atom, $R^{3c}$ is a hydrogen atom, and $R^1$ is any of the substituents shown in [Table L1] to [Table L3] (hereinafter, referred to as a compound group SX356).

In the compound (L-1), a compound in which Q is a group represented by Q49, $R^{3b}$ is a bromine atom, $R^{3c}$ is a hydrogen atom, and $R^1$ is any of the substituents shown in [Table L1] to [Table L3] (hereinafter, referred to as a compound group SX357).

In the compound (L-1), a compound in which Q is a group represented by Q49, $R^{3b}$ is an iodine atom, $R^{3c}$ is a hydrogen atom, and $R^1$ is any of the substituents shown in [Table L1] to [Table L3] (hereinafter, referred to as a compound group SX358).

In the compound (L-1), a compound in which Q is a group represented by Q49, $R^{3b}$ is $CF_3$, $R^{3c}$ is a hydrogen atom, and $R^1$ is any of the substituents shown in [Table L1] to [Table L3] (hereinafter, referred to as a compound group SX359) .

In the compound (L-1), a compound in which Q is a group represented by Q49, $R^{3b}$ is a hydrogen atom, $R^{3c}$ is a chlorine atom, and $R^1$ is any of the substituents shown in [Table L1] to [Table L3] (hereinafter, referred to as a compound group SX360).

In the compound (L-1), a compound in which Q is a group represented by Q49, $R^{3b}$ is a hydrogen atom, $R^{3c}$ is a bromine atom, and $R^1$ is any of the substituents shown in [Table L1] to [Table L3] (hereinafter, referred to as a compound group SX361).

In the compound (L-1), a compound in which Q is a group represented by Q49, $R^{3b}$ is a hydrogen atom, $R^{3c}$ is an iodine atom, and $R^1$ is any of the substituents shown in [Table L1] to [Table L3] (hereinafter, referred to as a compound group SX362).

In the compound (L-1), a compound in which Q is a group represented by Q49, $R^{3b}$ is a hydrogen atom, $R^{3c}$ is $CF_3$, and $R^1$ is any of the substituents shown in [Table L1] to [Table L3] (hereinafter, referred to as a compound group SX363) .

In the compound (L-1), a compound in which Q is a group represented by Q50, $R^{3b}$ and $R^{3c}$ are hydrogen atoms, and $R^1$ is any of the substituents shown in [Table L1] to [Table L3] (hereinafter, referred to as a compound group SX364) .

In the compound (L-1), a compound in which Q is a group represented by Q50, $R^{3b}$ is a chlorine atom, $R^{3c}$ is a hydrogen atom, and $R^1$ is any of the substituents shown in [Table L1] to [Table L3] (hereinafter, referred to as a compound group SX365).

In the compound (L-1), a compound in which Q is a group represented by Q50, $R^{3b}$ is a bromine atom, $R^{3c}$ is a hydrogen atom, and $R^1$ is any of the substituents shown in [Table L1] to [Table L3] (hereinafter, referred to as a compound group SX366).

In the compound (L-1), a compound in which Q is a group represented by Q50, $R^{3b}$ is an iodine atom, $R^{3c}$ is a hydrogen atom, and $R^1$ is any of the substituents shown in [Table L1] to [Table L3] (hereinafter, referred to as a compound group SX367).

In the compound (L-1), a compound in which Q is a group represented by Q50, $R^{3b}$ is $CF_3$, $R^{3c}$ is a hydrogen atom, and $R^1$ is any of the substituents shown in [Table L1] to [Table L3] (hereinafter, referred to as a compound group SX368) .

In the compound (L-1), a compound in which Q is a group represented by Q50, $R^{3b}$ is a hydrogen atom, $R^{3c}$ is a chlorine atom, and $R^1$ is any of the substituents shown in [Table L1] to [Table L3] (hereinafter, referred to as a compound group SX369).

In the compound (L-1), a compound in which Q is a group represented by Q50, $R^{3b}$ is a hydrogen atom, $R^{3c}$ is a bromine atom, and $R^1$ is any of the substituents shown in [Table L1] to [Table L3] (hereinafter, referred to as a compound group SX370).

In the compound (L-1), a compound in which Q is a group represented by Q50, $R^{3b}$ is a hydrogen atom, $R^{3c}$ is an iodine atom, and $R^1$ is any of the substituents shown in [Table L1] to [Table L3] (hereinafter, referred to as a compound group SX371).

In the compound (L-1), a compound in which Q is a group represented by Q50, $R^{3b}$ is a hydrogen atom, $R^{3c}$ is $CF_3$, and $R^1$ is any of the substituents shown in [Table L1] to [Table L3] (hereinafter, referred to as a compound group SX372) .

In the compound (L-1), a compound in which Q is a group represented by Q51, $R^{3b}$ and $R^{3c}$ are hydrogen atoms, and $R^1$ is any of the substituents shown in [Table L1] to [Table L3] (hereinafter, referred to as a compound group SX373) .

In the compound (L-1), a compound in which Q is a group represented by Q51, $R^{3b}$ is a chlorine atom, $R^{3c}$ is a hydrogen atom, and $R^1$ is any of the substituents shown in [Table L1] to [Table L3] (hereinafter, referred to as a compound group SX374).

In the compound (L-1), a compound in which Q is a group represented by Q51, $R^{3b}$ is a bromine atom, $R^{3c}$ is a hydrogen atom, and $R^1$ is any of the substituents shown in [Table L1] to [Table L3] (hereinafter, referred to as a compound group SX375).

In the compound (L-1), a compound in which Q is a group represented by Q51, $R^{3b}$ is an iodine atom, $R^{3c}$ is a hydrogen atom, and $R^1$ is any of the substituents shown in [Table L1] to [Table L3] (hereinafter, referred to as a compound group SX376).

In the compound (L-1), a compound in which Q is a group represented by Q51, $R^{3b}$ is $CF_3$, $R^{3c}$ is a hydrogen atom, and $R^1$ is any of the substituents shown in [Table L1] to [Table L3] (hereinafter, referred to as a compound group SX377) .

In the compound (L-1), a compound in which Q is a group represented by Q51, $R^{3b}$ is a hydrogen atom, $R^{3c}$ is a chlorine atom, and $R^1$ is any of the substituents shown in [Table L1] to [Table L3] (hereinafter, referred to as a compound group SX378).

In the compound (L-1), a compound in which Q is a group represented by Q51, $R^{3b}$ is a hydrogen atom, $R^{3c}$ is a bromine atom, and $R^1$ is any of the substituents shown in [Table L1] to [Table L3] (hereinafter, referred to as a compound group SX379).

In the compound (L-1), a compound in which Q is a group represented by Q51, $R^{3b}$ is a hydrogen atom, $R^{3c}$ is an iodine atom, and $R^1$ is any of the substituents shown in [Table L1] to [Table L3] (hereinafter, referred to as a compound group SX380).

In the compound (L-1), a compound in which Q is a group represented by Q51, $R^{3b}$ is a hydrogen atom, $R^{3c}$ is $CF_3$, and $R^1$ is any of the substituents shown in [Table L1] to [Table L3] (hereinafter, referred to as a compound group SX381).

In the compound (L-1), a compound in which Q is a group represented by Q52, $R^{3b}$ and $R^{3c}$ are hydrogen atoms, and $R^1$ is any of the substituents shown in [Table L1] to [Table L3] (hereinafter, referred to as a compound group SX382) .

In the compound (L-1), a compound in which Q is a group represented by Q52, $R^{3b}$ is a chlorine atom, $R^{3c}$ is a hydrogen atom, and $R^1$ is any of the substituents shown in [Table L1] to [Table L3] (hereinafter, referred to as a compound group SX383).

In the compound (L-1), a compound in which Q is a group represented by Q52, $R^{3b}$ is a bromine atom, $R^{3c}$ is a hydrogen atom, and $R^1$ is any of the substituents shown in [Table L1] to [Table L3] (hereinafter, referred to as a compound group SX384).

In the compound (L-1), a compound in which Q is a group represented by Q52, $R^{3b}$ is an iodine atom, $R^{3c}$ is a hydrogen atom, and $R^1$ is any of the substituents shown in [Table L1] to [Table L3] (hereinafter, referred to as a compound group SX385).

In the compound (L-1), a compound in which Q is a group represented by Q52, $R^{3b}$ is $CF_3$, $R^{3c}$ is a hydrogen atom, and $R^1$ is any of the substituents shown in [Table L1] to [Table L3] (hereinafter, referred to as a compound group SX386) .

In the compound (L-1), a compound in which Q is a group represented by Q52, $R^{3b}$ is a hydrogen atom, $R^{3c}$ is a chlorine atom, and $R^1$ is any of the substituents shown in [Table L1] to [Table L3] (hereinafter, referred to as a compound group SX387).

In the compound (L-1), a compound in which Q is a group represented by Q52, $R^{3b}$ is a hydrogen atom, $R^{3c}$ is a bromine atom, and $R^1$ is any of the substituents shown in [Table L1] to [Table L3] (hereinafter, referred to as a compound group SX388).

In the compound (L-1), a compound in which Q is a group represented by Q52, $R^{3b}$ is a hydrogen atom, $R^{3c}$ is an iodine atom, and $R^1$ is any of the substituents shown in [Table L1] to [Table L3] (hereinafter, referred to as a compound group SX389).

In the compound (L-1), a compound in which Q is a group represented by Q52, $R^{3b}$ is a hydrogen atom, $R^{3c}$

is $CF_3$, and $R^1$ is any of the substituents shown in [Table L1] to [Table L3] (hereinafter, referred to as a compound group SX390) .

In the compound (L-1), a compound in which Q is a group represented by Q53, $R^{3b}$ and $R^{3c}$ are hydrogen atoms, and $R^1$ is any of the substituents shown in [Table L1] to [Table L3] (hereinafter, referred to as a compound group SX391).

In the compound (L-1), a compound in which Q is a group represented by Q53, $R^{3b}$ is a chlorine atom, $R^{3c}$ is a hydrogen atom, and $R^1$ is any of the substituents shown in [Table L1] to [Table L3] (hereinafter, referred to as a compound group SX392).

In the compound (L-1), a compound in which Q is a group represented by Q53, $R^{3b}$ is a bromine atom, $R^{3c}$ is a hydrogen atom, and $R^1$ is any of the substituents shown in [Table L1] to [Table L3] (hereinafter, referred to as a compound group SX393).

In the compound (L-1), a compound in which Q is a group represented by Q53, $R^{3b}$ is an iodine atom, $R^{3c}$ is a hydrogen atom, and $R^1$ is any of the substituents shown in [Table L1] to [Table L3] (hereinafter, referred to as a compound group SX394).

In the compound (L-1), a compound in which Q is a group represented by Q53, $R^{3b}$ is $CF_3$, $R^{3c}$ is a hydrogen atom, and $R^1$ is any of the substituents shown in [Table L1] to [Table L3] (hereinafter, referred to as a compound group SX395) .

In the compound (L-1), a compound in which Q is a group represented by Q53, $R^{3b}$ is a hydrogen atom, $R^{3c}$ is a chlorine atom, and $R^1$ is any of the substituents shown in [Table L1] to [Table L3] (hereinafter, referred to as a compound group SX396).

In the compound (L-1), a compound in which Q is a group represented by Q53, $R^{3b}$ is a hydrogen atom, $R^{3c}$ is a bromine atom, and $R^1$ is any of the substituents shown in [Table L1] to [Table L3] (hereinafter, referred to as a compound group SX397).

In the compound (L-1), a compound in which Q is a group represented by Q53, $R^{3b}$ is a hydrogen atom, $R^{3c}$ is an iodine atom, and $R^1$ is any of the substituents shown in [Table L1] to [Table L3] (hereinafter, referred to as a compound group SX398).

In the compound (L-1), a compound in which Q is a group represented by Q53, $R^{3b}$ is a hydrogen atom, $R^{3c}$ is $CF_3$, and $R^1$ is any of the substituents shown in [Table L1] to [Table L3] (hereinafter, referred to as a compound group SX399).

In the compound (L-1), a compound in which Q is a group represented by Q54, $R^{3b}$ and $R^{3c}$ are hydrogen atoms, and $R^1$ is any of the substituents shown in [Table L1] to [Table L3] (hereinafter, referred to as a compound group SX400).

In the compound (L-1), a compound in which Q is a group represented by Q54, $R^{3b}$ is a chlorine atom, $R^{3c}$ is a hydrogen atom, and $R^1$ is any of the substituents shown in [Table L1] to [Table L3] (hereinafter, referred to as a compound group SX401).

In the compound (L-1), a compound in which Q is a group represented by Q54, $R^{3b}$ is a bromine atom, $R^{3c}$ is a hydrogen atom, and $R^1$ is any of the substituents shown in [Table L1] to [Table L3] (hereinafter, referred to as a compound group SX402).

In the compound (L-1), a compound in which Q is a group represented by Q54, $R^{3b}$ is an iodine atom, $R^{3c}$ is a hydrogen atom, and $R^1$ is any of the substituents shown in [Table L1] to [Table L3] (hereinafter, referred to as a compound group SX403).

In the compound (L-1), a compound in which Q is a group represented by Q54, $R^{3b}$ is $CF_3$, $R^{3c}$ is a hydrogen atom, and $R^1$ is any of the substituents shown in [Table L1] to [Table L3] (hereinafter, referred to as a compound group SX4040.

In the compound (L-1), a compound in which Q is a group represented by Q54, $R^{3b}$ is a hydrogen atom, $R^{3c}$ is a chlorine atom, and $R^1$ is any of the substituents shown in [Table L1] to [Table L3] (hereinafter, referred to as a compound group SX405).

In the compound (L-1), a compound in which Q is a group represented by Q54, $R^{3b}$ is a hydrogen atom, $R^{3c}$ is a bromine atom, and $R^1$ is any of the substituents shown in [Table L1] to [Table L3] (hereinafter, referred to as a compound group SX406).

In the compound (L-1), a compound in which Q is a group represented by Q54, $R^{3b}$ is a hydrogen atom, $R^{3c}$ is an iodine atom, and $R^1$ is any of the substituents shown in [Table L1] to [Table L3] (hereinafter, referred to as a compound group SX407).

In the compound (L-1), a compound in which Q is a group represented by Q54, $R^{3b}$ is a hydrogen atom, $R^{3c}$ is $CF_3$, and $R^1$ is any of the substituents shown in [Table L1] to [Table L3] (hereinafter, referred to as a compound group SX408) .

In the compound (L-1), a compound in which Q is a group represented by Q55, $R^{3b}$ and $R^{3c}$ are hydrogen atoms, and $R^1$ is any of the substituents shown in [Table L1] to [Table L3] (hereinafter, referred to as a compound group

SX409).

In the compound (L-1), a compound in which Q is a group represented by Q55, $R^{3b}$ is a chlorine atom, $R^{3c}$ is a hydrogen atom, and $R^1$ is any of the substituents shown in [Table L1] to [Table L3] (hereinafter, referred to as a compound group SX410).

In the compound (L-1), a compound in which Q is a group represented by Q55, $R^{3b}$ is a bromine atom, $R^{3c}$ is a hydrogen atom, and $R^1$ is any of the substituents shown in [Table L1] to [Table L3] (hereinafter, referred to as a compound group SX411).

In the compound (L-1), a compound in which Q is a group represented by Q55, $R^{3b}$ is an iodine atom, $R^{3c}$ is a hydrogen atom, and $R^1$ is any of the substituents shown in [Table L1] to [Table L3] (hereinafter, referred to as a compound group SX412).

In the compound (L-1), a compound in which Q is a group represented by Q55, $R^{3b}$ is $CF_3$, $R^{3c}$ is a hydrogen atom, and $R^1$ is any of the substituents shown in [Table L1] to [Table L3] (hereinafter, referred to as a compound group SX413).

In the compound (L-1), a compound in which Q is a group represented by Q55, $R^{3b}$ is a hydrogen atom, $R^{3c}$ is a chlorine atom, and $R^1$ is any of the substituents shown in [Table L1] to [Table L3] (hereinafter, referred to as a compound group SX414).

In the compound (L-1), a compound in which Q is a group represented by Q55, $R^{3b}$ is a hydrogen atom, $R^{3c}$ is a bromine atom, and $R^1$ is any of the substituents shown in [Table L1] to [Table L3] (hereinafter, referred to as a compound group SX415).

In the compound (L-1), a compound in which Q is a group represented by Q55, $R^{3b}$ is a hydrogen atom, $R^{3c}$ is an iodine atom, and $R^1$ is any of the substituents shown in [Table L1] to [Table L3] (hereinafter, referred to as a compound group SX416).

In the compound (L-1), a compound in which Q is a group represented by Q55, $R^{3b}$ is a hydrogen atom, $R^{3c}$ is $CF_3$, and $R^1$ is any of the substituents shown in [Table L1] to [Table L3] (hereinafter, referred to as a compound group SX417).

In the compound (L-1), a compound in which Q is a group represented by Q56, $R^{3b}$ and $R^{3c}$ are hydrogen atoms, and $R^1$ is any of the substituents shown in [Table L1] to [Table L3] (hereinafter, referred to as a compound group SX418).

In the compound (L-1), a compound in which Q is a group represented by Q56, $R^{3b}$ is a chlorine atom, $R^{3c}$ is a hydrogen atom, and $R^1$ is any of the substituents shown in [Table L1] to [Table L3] (hereinafter, referred to as a compound group SX419).

In the compound (L-1), a compound in which Q is a group represented by Q56, $R^{3b}$ is a bromine atom, $R^{3c}$ is a hydrogen atom, and $R^1$ is any of the substituents shown in [Table L1] to [Table L3] (hereinafter, referred to as a compound group SX420).

In the compound (L-1), a compound in which Q is a group represented by Q56, $R^{3b}$ is an iodine atom, $R^{3c}$ is a hydrogen atom, and $R^1$ is any of the substituents shown in [Table L1] to [Table L3] (hereinafter, referred to as a compound group SX421).

In the compound (L-1), a compound in which Q is a group represented by Q56, $R^{3b}$ is $CF_3$, $R^{3c}$ is a hydrogen atom, and $R^1$ is any of the substituents shown in [Table L1] to [Table L3] (hereinafter, referred to as a compound group SX422).

In the compound (L-1), a compound in which Q is a group represented by Q56, $R^{3b}$ is a hydrogen atom, $R^{3c}$ is a chlorine atom, and $R^1$ is any of the substituents shown in [Table L1] to [Table L3] (hereinafter, referred to as a compound group SX423).

In the compound (L-1), a compound in which Q is a group represented by Q56, $R^{3b}$ is a hydrogen atom, $R^{3c}$ is a bromine atom, and $R^1$ is any of the substituents shown in [Table L1] to [Table L3] (hereinafter, referred to as a compound group SX424).

In the compound (L-1), a compound in which Q is a group represented by Q56, $R^{3b}$ is a hydrogen atom, $R^{3c}$ is an iodine atom, and $R^1$ is any of the substituents shown in [Table L1] to [Table L3] (hereinafter, referred to as a compound group SX425).

In the compound (L-1), a compound in which Q is a group represented by Q56, $R^{3b}$ is a hydrogen atom, $R^{3c}$ is $CF_3$, and $R^1$ is any of the substituents shown in [Table L1] to [Table L3] (hereinafter, referred to as a compound group SX426).

In the compound (L-1), a compound in which Q is a group represented by Q57, $R^{3b}$ and $R^{3c}$ are hydrogen atoms, and $R^1$ is any of the substituents shown in [Table L1] to [Table L3] (hereinafter, referred to as a compound group SX427).

In the compound (L-1), a compound in which Q is a group represented by Q57, $R^{3b}$ is a chlorine atom, $R^{3c}$ is a hydrogen atom, and $R^1$ is any of the substituents shown in [Table L1] to [Table L3] (hereinafter, referred to as a compound group SX428).

In the compound (L-1), a compound in which Q is a group represented by Q57, $R^{3b}$ is a bromine atom, $R^{3c}$ is a hydrogen atom, and $R^1$ is any of the substituents shown in [Table L1] to [Table L3] (hereinafter, referred to as a compound group SX429).

In the compound (L-1), a compound in which Q is a group represented by Q57, $R^{3b}$ is an iodine atom, $R^{3c}$ is a hydrogen atom, and $R^1$ is any of the substituents shown in [Table L1] to [Table L3] (hereinafter, referred to as a compound group SX430).

In the compound (L-1), a compound in which Q is a group represented by Q57, $R^{3b}$ is $CF_3$, $R^{3c}$ is a hydrogen atom, and $R^1$ is any of the substituents shown in [Table L1] to [Table L3] (hereinafter, referred to as a compound group SX431).

In the compound (L-1), a compound in which Q is a group represented by Q57, $R^{3b}$ is a hydrogen atom, $R^{3c}$ is a chlorine atom, and $R^1$ is any of the substituents shown in [Table L1] to [Table L3] (hereinafter, referred to as a compound group SX432).

In the compound (L-1), a compound in which Q is a group represented by Q57, $R^{3b}$ is a hydrogen atom, $R^{3c}$ is a bromine atom, and $R^1$ is any of the substituents shown in [Table L1] to [Table L3] (hereinafter, referred to as a compound group SX433).

In the compound (L-1), a compound in which Q is a group represented by Q57, $R^{3b}$ is a hydrogen atom, $R^{3c}$ is an iodine atom, and $R^1$ is any of the substituents shown in [Table L1] to [Table L3] (hereinafter, referred to as a compound group SX434).

In the compound (L-1), a compound in which Q is a group represented by Q57, $R^{3b}$ is a hydrogen atom, $R^{3c}$ is $CF_3$, and $R^1$ is any of the substituents shown in [Table L1] to [Table L3] (hereinafter, referred to as a compound group SX435).

[Chem. 92]

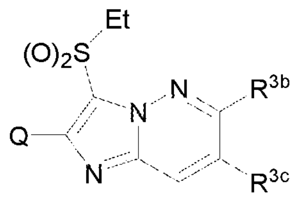

(L-2)

In a compound (hereinafter, referred to as a compound (L-2)) represented by Formula (L-2), a compound in which Q is a group represented by Q11, $R^{3b}$ and $R^{3c}$ are hydrogen atoms, and $R^1$ is any of the substituents shown in [Table L1] to [Table L3] (hereinafter, referred to as a compound group SX28).

In the compound (L-2), a compound in which Q is a group represented by Q12, $R^{3b}$ and $R^{3c}$ are hydrogen atoms, and $R^1$ is any of the substituents shown in [Table L1] to [Table L3] (hereinafter, referred to as a compound group SX29) .

In the compound (L-2), a compound in which Q is a group represented by Q13, $R^{3b}$ and $R^{3c}$ are hydrogen atoms, and $R^1$ is any of the substituents shown in [Table L1] to [Table L3] (hereinafter, referred to as a compound group SX30) .

In the compound (L-2), a compound in which Q is a group represented by Q11, $R^{3b}$ is a chlorine atom, $R^{3c}$ is a hydrogen atom, and $R^1$ is any of the substituents shown in [Table L1] to [Table L3] (hereinafter, referred to as a compound group SX31).

In the compound (L-2), a compound in which Q is a group represented by Q12, $R^{3b}$ is a chlorine atom, $R^{3c}$ is a hydrogen atom, and $R^1$ is any of the substituents shown in [Table L1] to [Table L3] (hereinafter, referred to as a compound group SX32).

In the compound (L-2), a compound in which Q is a group represented by Q13, $R^{3b}$ is a chlorine atom, $R^{3c}$ is a hydrogen atom, and $R^1$ is any of the substituents shown in [Table L1] to [Table L3] (hereinafter, referred to as a compound group SX33).

In the compound (L-2), a compound in which Q is a group represented by Q11, $R^{3b}$ is a bromine atom, $R^{3c}$ is a hydrogen atom, and $R^1$ is any of the substituents shown in [Table L1] to [Table L3] (hereinafter, referred to as a compound group SX34).

In the compound (L-2), a compound in which Q is a group represented by Q12, $R^{3b}$ is a bromine atom, $R^{3c}$ is a hydrogen atom, and $R^1$ is any of the substituents shown in [Table L1] to [Table L3] (hereinafter, referred to as a compound group SX35).

In the compound (L-2), a compound in which Q is a group represented by Q13, $R^{3b}$ is a bromine atom, $R^{3c}$ is

a hydrogen atom, and $R^1$ is any of the substituents shown in [Table L1] to [Table L3] (hereinafter, referred to as a compound group SX36).

In the compound (L-2), a compound in which Q is a group represented by Q11, $R^{3b}$ is an iodine atom, $R^{3c}$ is a hydrogen atom, and $R^1$ is any of the substituents shown in [Table L1] to [Table L3] (hereinafter, referred to as a compound group SX37).

In the compound (L-2), a compound in which Q is a group represented by Q12, $R^{3b}$ is an iodine atom, $R^{3c}$ is a hydrogen atom, and $R^1$ is any of the substituents shown in [Table L1] to [Table L3] (hereinafter, referred to as a compound group SX38).

In the compound (L-2), a compound in which Q is a group represented by Q13, $R^{3b}$ is an iodine atom, $R^{3c}$ is a hydrogen atom, and $R^1$ is any of the substituents shown in [Table L1] to [Table L3] (hereinafter, referred to as a compound group SX39).

In the compound (L-2), a compound in which Q is a group represented by Q11, $R^{3b}$ is $CF_3$, $R^{3c}$ is a hydrogen atom, and $R^1$ is any of the substituents shown in [Table L1] to [Table L3] (hereinafter, referred to as a compound group SX40).

In the compound (L-2), a compound in which Q is a group represented by Q12, $R^{3b}$ is $CF_3$, $R^{3c}$ is a hydrogen atom, and $R^1$ is any of the substituents shown in [Table L1] to [Table L3] (hereinafter, referred to as a compound group SX41).

In the compound (L-2), a compound in which Q is a group represented by Q13, $R^{3b}$ is $CF_3$, $R^{3c}$ is a hydrogen atom, and $R^1$ is any of the substituents shown in [Table L1] to [Table L3] (hereinafter, referred to as a compound group SX42) .

In the compound (L-2), a compound in which Q is a group represented by Q11, $R^{3b}$ is a hydrogen atom, $R^{3c}$ is a chlorine atom, and $R^1$ is any of the substituents shown in [Table L1] to [Table L3] (hereinafter, referred to as a compound group SX43).

In the compound (L-2), a compound in which Q is a group represented by Q12, $R^{3b}$ is a hydrogen atom, $R^{3c}$ is a chlorine atom, and $R^1$ is any of the substituents shown in [Table L1] to [Table L3] (hereinafter, referred to as a compound group SX44).

In the compound (L-2), a compound in which Q is a group represented by Q13, $R^{3b}$ is a hydrogen atom, $R^{3c}$ is a chlorine atom, and $R^1$ is any of the substituents shown in [Table L1] to [Table L3] (hereinafter, referred to as a compound group SX45).

In the compound (L-2), a compound in which Q is a group represented by Q11, $R^{3b}$ is a hydrogen atom, $R^{3c}$ is a bromine atom, and $R^1$ is any of the substituents shown in [Table L1] to [Table L3] (hereinafter, referred to as a compound group SX46).

In the compound (L-2), a compound in which Q is a group represented by Q12, $R^{3b}$ is a hydrogen atom, $R^{3c}$ is a bromine atom, and $R^1$ is any of the substituents shown in [Table L1] to [Table L3] (hereinafter, referred to as a compound group SX47).

In the compound (L-2), a compound in which Q is a group represented by Q13, $R^{3b}$ is a hydrogen atom, $R^{3c}$ is a bromine atom, and $R^1$ is any of the substituents shown in [Table L1] to [Table L3] (hereinafter, referred to as a compound group SX48).

In the compound (L-2), a compound in which Q is a group represented by Q11, $R^{3b}$ is a hydrogen atom, $R^{3c}$ is an iodine atom, and $R^1$ is any of the substituents shown in [Table L1] to [Table L3] (hereinafter, referred to as a compound group SX49).

In the compound (L-2), a compound in which Q is a group represented by Q12, $R^{3b}$ is a hydrogen atom, $R^{3c}$ is an iodine atom, and $R^1$ is any of the substituents shown in [Table L1] to [Table L3] (hereinafter, referred to as a compound group SX50).

In the compound (L-2), a compound in which Q is a group represented by Q13, $R^{3b}$ is a hydrogen atom, $R^{3c}$ is an iodine atom, and $R^1$ is any of the substituents shown in [Table L1] to [Table L3] (hereinafter, referred to as a compound group SX51).

In the compound (L-2), a compound in which Q is a group represented by Q11, $R^{3b}$ is a hydrogen atom, $R^{3c}$ is $CF_3$, and $R^1$ is any of the substituents shown in [Table L1] to [Table L3] (hereinafter, referred to as a compound group SX52) .

In the compound (L-2), a compound in which Q is a group represented by Q12, $R^{3b}$ is a hydrogen atom, $R^{3c}$ is $CF_3$, and $R^1$ is any of the substituents shown in [Table L1] to [Table L3] (hereinafter, referred to as a compound group SX53) .

In the compound (L-2), a compound in which Q is a group represented by Q13, $R^{3b}$ is a hydrogen atom, $R^{3c}$ is $CF_3$, and $R^1$ is any of the substituents shown in [Table L1] to [Table L3] (hereinafter, referred to as a compound group SX54) .

In the compound (L-2), a compound in which Q is a group represented by Q34, $R^{3b}$ and $R^{3c}$ are hydrogen atoms, and $R^1$ is any of the substituents shown in [Table L1] to [Table L3] (hereinafter, referred to as a compound group

SX436) .

In the compound (L-2), a compound in which Q is a group represented by Q34, $R^{3b}$ is a chlorine atom, $R^{3c}$ is a hydrogen atom, and $R^1$ is any of the substituents shown in [Table L1] to [Table L3] (hereinafter, referred to as a compound group SX437).

In the compound (L-2), a compound in which Q is a group represented by Q34, $R^{3b}$ is a bromine atom, $R^{3c}$ is a hydrogen atom, and $R^1$ is any of the substituents shown in [Table L1] to [Table L3] (hereinafter, referred to as a compound group SX438).

In the compound (L-2), a compound in which Q is a group represented by Q34, $R^{3b}$ is an iodine atom, $R^{3c}$ is a hydrogen atom, and $R^1$ is any of the substituents shown in [Table L1] to [Table L3] (hereinafter, referred to as a compound group SX439).

In the compound (L-2), a compound in which Q is a group represented by Q34, $R^{3b}$ is $CF_3$, $R^{3c}$ is a hydrogen atom, and $R^1$ is any of the substituents shown in [Table L1] to [Table L3] (hereinafter, referred to as a compound group SX440).

In the compound (L-2), a compound in which Q is a group represented by Q34, $R^{3b}$ is a hydrogen atom, $R^{3c}$ is a chlorine atom, and $R^1$ is any of the substituents shown in [Table L1] to [Table L3] (hereinafter, referred to as a compound group SX441).

In the compound (L-2), a compound in which Q is a group represented by Q34, $R^{3b}$ is a hydrogen atom, $R^{3c}$ is a bromine atom, and $R^1$ is any of the substituents shown in [Table L1] to [Table L3] (hereinafter, referred to as a compound group SX442).

In the compound (L-2), a compound in which Q is a group represented by Q34, $R^{3b}$ is a hydrogen atom, $R^{3c}$ is an iodine atom, and $R^1$ is any of the substituents shown in [Table L1] to [Table L3] (hereinafter, referred to as a compound group SX443).

In the compound (L-2), a compound in which Q is a group represented by Q34, $R^{3b}$ is a hydrogen atom, $R^{3c}$ is $CF_3$, and $R^1$ is any of the substituents shown in [Table L1] to [Table L3] (hereinafter, referred to as a compound group SX444).

In the compound (L-2), a compound in which Q is a group represented by Q35, $R^{3b}$ and $R^{3c}$ are hydrogen atoms, and $R^1$ is any of the substituents shown in [Table L1] to [Table L3] (hereinafter, referred to as a compound group SX445.

In the compound (L-2), a compound in which Q is a group represented by Q35, $R^{3b}$ is a chlorine atom, $R^{3c}$ is a hydrogen atom, and $R^1$ is any of the substituents shown in [Table L1] to [Table L3] (hereinafter, referred to as a compound group SX446).

In the compound (L-2), a compound in which Q is a group represented by Q35, $R^{3b}$ is a bromine atom, $R^{3c}$ is a hydrogen atom, and $R^1$ is any of the substituents shown in [Table L1] to [Table L3] (hereinafter, referred to as a compound group SX447).

In the compound (L-2), a compound in which Q is a group represented by Q35, $R^{3b}$ is an iodine atom, $R^{3c}$ is a hydrogen atom, and $R^1$ is any of the substituents shown in [Table L1] to [Table L3] (hereinafter, referred to as a compound group SX448).

In the compound (L-2), a compound in which Q is a group represented by Q35, $R^{3b}$ is $CF_3$, $R^{3c}$ is a hydrogen atom, and $R^1$ is any of the substituents shown in [Table L1] to [Table L3] (hereinafter, referred to as a compound group SX449).

In the compound (L-2), a compound in which Q is a group represented by Q35, $R^{3b}$ is a hydrogen atom, $R^{3c}$ is a chlorine atom, and $R^1$ is any of the substituents shown in [Table L1] to [Table L3] (hereinafter, referred to as a compound group SX450).

In the compound (L-2), a compound in which Q is a group represented by Q35, $R^{3b}$ is a hydrogen atom, $R^{3c}$ is a bromine atom, and $R^1$ is any of the substituents shown in [Table L1] to [Table L3] (hereinafter, referred to as a compound group SX451).

In the compound (L-2), a compound in which Q is a group represented by Q35, $R^{3b}$ is a hydrogen atom, $R^{3c}$ is an iodine atom, and $R^1$ is any of the substituents shown in [Table L1] to [Table L3] (hereinafter, referred to as a compound group SX452).

In the compound (L-2), a compound in which Q is a group represented by Q35, $R^{3b}$ is a hydrogen atom, $R^{3c}$ is $CF_3$, and $R^1$ is any of the substituents shown in [Table L1] to [Table L3] (hereinafter, referred to as a compound group SX453) .

In the compound (L-2), a compound in which Q is a group represented by Q36, $R^{3b}$ and $R^{3c}$ are hydrogen atoms, and $R^1$ is any of the substituents shown in [Table L1] to [Table L3] (hereinafter, referred to as a compound group SX454.

In the compound (L-2), a compound in which Q is a group represented by Q36, $R^{3b}$ is a chlorine atom, $R^{3c}$ is a hydrogen atom, and $R^1$ is any of the substituents shown in [Table L1] to [Table L3] (hereinafter, referred to as a compound group SX455).

In the compound (L-2), a compound in which Q is a group represented by Q36, $R^{3b}$ is a bromine atom, $R^{3c}$ is a hydrogen atom, and $R^1$ is any of the substituents shown in [Table L1] to [Table L3] (hereinafter, referred to as a compound group SX456).

In the compound (L-2), a compound in which Q is a group represented by Q36, $R^{3b}$ is an iodine atom, $R^{3c}$ is a hydrogen atom, and $R^1$ is any of the substituents shown in [Table L1] to [Table L3] (hereinafter, referred to as a compound group SX457).

In the compound (L-2), a compound in which Q is a group represented by Q36, $R^{3b}$ is $CF_3$, $R^{3c}$ is a hydrogen atom, and $R^1$ is any of the substituents shown in [Table L1] to [Table L3] (hereinafter, referred to as a compound group SX458) .

In the compound (L-2), a compound in which Q is a group represented by Q36, $R^{3b}$ is a hydrogen atom, $R^{3c}$ is a chlorine atom, and $R^1$ is any of the substituents shown in [Table L1] to [Table L3] (hereinafter, referred to as a compound group SX459).

In the compound (L-2), a compound in which Q is a group represented by Q36, $R^{3b}$ is a hydrogen atom, $R^{3c}$ is a bromine atom, and $R^1$ is any of the substituents shown in [Table L1] to [Table L3] (hereinafter, referred to as a compound group SX460).

In the compound (L-2), a compound in which Q is a group represented by Q36, $R^{3b}$ is a hydrogen atom, $R^{3c}$ is an iodine atom, and $R^1$ is any of the substituents shown in [Table L1] to [Table L3] (hereinafter, referred to as a compound group SX461).

In the compound (L-2), a compound in which Q is a group represented by Q36, $R^{3b}$ is a hydrogen atom, $R^{3c}$ is $CF_3$, and $R^1$ is any of the substituents shown in [Table L1] to [Table L3] (hereinafter, referred to as a compound group SX462) .

In the compound (L-2), a compound in which Q is a group represented by Q37, $R^{3b}$ and $R^{3c}$ are hydrogen atoms, and $R^1$ is any of the substituents shown in [Table L1] to [Table L3] (hereinafter, referred to as a compound group SX463) .

In the compound (L-2), a compound in which Q is a group represented by Q37, $R^{3b}$ is a chlorine atom, $R^{3c}$ is a hydrogen atom, and $R^1$ is any of the substituents shown in [Table L1] to [Table L3] (hereinafter, referred to as a compound group SX464).

In the compound (L-2), a compound in which Q is a group represented by Q37, $R^{3b}$ is a bromine atom, $R^{3c}$ is a hydrogen atom, and $R^1$ is any of the substituents shown in [Table L1] to [Table L3] (hereinafter, referred to as a compound group SX465).

In the compound (L-2), a compound in which Q is a group represented by Q37, $R^{3b}$ is an iodine atom, $R^{3c}$ is a hydrogen atom, and $R^1$ is any of the substituents shown in [Table L1] to [Table L3] (hereinafter, referred to as a compound group SX466).

In the compound (L-2), a compound in which Q is a group represented by Q37, $R^{3b}$ is $CF_3$, $R^{3c}$ is a hydrogen atom, and $R^1$ is any of the substituents shown in [Table L1] to [Table L3] (hereinafter, referred to as a compound group SX467) .

In the compound (L-2), a compound in which Q is a group represented by Q37, $R^{3b}$ is a hydrogen atom, $R^{3c}$ is a chlorine atom, and $R^1$ is any of the substituents shown in [Table L1] to [Table L3] (hereinafter, referred to as a compound group SX468).

In the compound (L-2), a compound in which Q is a group represented by Q37, $R^{3b}$ is a hydrogen atom, $R^{3c}$ is a bromine atom, and $R^1$ is any of the substituents shown in [Table L1] to [Table L3] (hereinafter, referred to as a compound group SX469).

In the compound (L-2), a compound in which Q is a group represented by Q37, $R^{3b}$ is a hydrogen atom, $R^{3c}$ is an iodine atom, and $R^1$ is any of the substituents shown in [Table L1] to [Table L3] (hereinafter, referred to as a compound group SX470).

In the compound (L-2), a compound in which Q is a group represented by Q37, $R^{3b}$ is a hydrogen atom, $R^{3c}$ is $CF_3$, and $R^1$ is any of the substituents shown in [Table L1] to [Table L3] (hereinafter, referred to as a compound group SX471) .

In the compound (L-2), a compound in which Q is a group represented by Q38, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX472).

In the compound (L-2), a compound in which Q is a group represented by Q38, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX473).

In the compound (L-2), a compound in which Q is a group represented by Q38, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX474).

In the compound (L-2), a compound in which Q is a group represented by Q38, $R^{3b}$ is an iodine atom, $R^{3c}$ is

a hydrogen atom, $R^{6a}$ is a hydrogen atom, and $R^1$ is any of the substituents shown in [Table L1] to [Table L3] (hereinafter, referred to as a compound group SX475).

In the compound (L-2), a compound in which Q is a group represented by Q38, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX476).

In the compound (L-2), a compound in which Q is a group represented by Q38, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX477).

In the compound (L-2), a compound in which Q is a group represented by Q38, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX478).

In the compound (L-2), a compound in which Q is a group represented by Q38, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX479).

In the compound (L-2), a compound in which Q is a group represented by Q38, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX480).

In the compound (L-2), a compound in which Q is a group represented by Q38, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX481).

In the compound (L-2), a compound in which Q is a group represented by Q38, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX482).

In the compound (L-2), a compound in which Q is a group represented by Q38, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX483).

In the compound (L-2), a compound in which Q is a group represented by Q38, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX484).

In the compound (L-2), a compound in which Q is a group represented by Q38, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX485).

In the compound (L-2), a compound in which Q is a group represented by Q38, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX486).

In the compound (L-2), a compound in which Q is a group represented by Q38, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX487).

In the compound (L-2), a compound in which Q is a group represented by Q38, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX488).

In the compound (L-2), a compound in which Q is a group represented by Q38, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX489).

In the compound (L-2), a compound in which Q is a group represented by Q39, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX490).

In the compound (L-2), a compound in which Q is a group represented by Q39, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX491).

In the compound (L-2), a compound in which Q is a group represented by Q39, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX492).

In the compound (L-2), a compound in which Q is a group represented by Q39, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX493).

In the compound (L-2), a compound in which Q is a group represented by Q39, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred

to as a compound group SX494).

In the compound (L-2), a compound in which Q is a group represented by Q39, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX495).

In the compound (L-2), a compound in which Q is a group represented by Q39, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX496).

In the compound (L-2), a compound in which Q is a group represented by Q39, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX497).

In the compound (L-2), a compound in which Q is a group represented by Q39, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX498).

In the compound (L-2), a compound in which Q is a group represented by Q39, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX499).

In the compound (L-2), a compound in which Q is a group represented by Q39, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX500).

In the compound (L-2), a compound in which Q is a group represented by Q39, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX501).

In the compound (L-2), a compound in which Q is a group represented by Q39, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX502).

In the compound (L-2), a compound in which Q is a group represented by Q39, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX503).

In the compound (L-2), a compound in which Q is a group represented by Q39, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX504).

In the compound (L-2), a compound in which Q is a group represented by Q39, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX505).

In the compound (L-2), a compound in which Q is a group represented by Q39, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX506).

In the compound (L-2), a compound in which Q is a group represented by Q39, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX507).

In the compound (L-2), a compound in which Q is a group represented by Q40, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX508).

In the compound (L-2), a compound in which Q is a group represented by Q40, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX509).

In the compound (L-2), a compound in which Q is a group represented by Q40, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX510).

In the compound (L-2), a compound in which Q is a group represented by Q40, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX511).

In the compound (L-2), a compound in which Q is a group represented by Q40, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX512).

In the compound (L-2), a compound in which Q is a group represented by Q40, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX513).

In the compound (L-2), a compound in which Q is a group represented by Q40, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX514).

In the compound (L-2), a compound in which Q is a group represented by Q40, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX515).

In the compound (L-2), a compound in which Q is a group represented by Q40, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX516).

In the compound (L-2), a compound in which Q is a group represented by Q40, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX517).

In the compound (L-2), a compound in which Q is a group represented by Q40, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX518).

In the compound (L-2), a compound in which Q is a group represented by Q40, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX519).

In the compound (L-2), a compound in which Q is a group represented by Q40, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX520).

In the compound (L-2), a compound in which Q is a group represented by Q40, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX521).

In the compound (L-2), a compound in which Q is a group represented by Q40, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX522).

In the compound (L-2), a compound in which Q is a group represented by Q40, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX523).

In the compound (L-2), a compound in which Q is a group represented by Q40, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX524).

In the compound (L-2), a compound in which Q is a group represented by Q40, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX525).

In the compound (L-2), a compound in which Q is a group represented by Q41, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX526).

In the compound (L-2), a compound in which Q is a group represented by Q41, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX527).

In the compound (L-2), a compound in which Q is a group represented by Q41, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX528).

In the compound (L-2), a compound in which Q is a group represented by Q41, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX529).

In the compound (L-2), a compound in which Q is a group represented by Q41, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX530).

In the compound (L-2), a compound in which Q is a group represented by Q41, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX531).

In the compound (L-2), a compound in which Q is a group represented by Q41, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX532).

In the compound (L-2), a compound in which Q is a group represented by Q41, $R^{3b}$ is a hydrogen atom, $R^{3c}$

is an iodine atom, $R^{6a}$ is a hydrogen atom, and $R^1$ is any of the substituents shown in [Table L1] to [Table L3] (hereinafter, referred to as a compound group SX533).

In the compound (L-2), a compound in which Q is a group represented by Q41, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX534).

In the compound (L-2), a compound in which Q is a group represented by Q41, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX535).

In the compound (L-2), a compound in which Q is a group represented by Q41, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX536).

In the compound (L-2), a compound in which Q is a group represented by Q41, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX537).

In the compound (L-2), a compound in which Q is a group represented by Q41, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX538).

In the compound (L-2), a compound in which Q is a group represented by Q41, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX539).

In the compound (L-2), a compound in which Q is a group represented by Q41, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX540).

In the compound (L-2), a compound in which Q is a group represented by Q41, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX541).

In the compound (L-2), a compound in which Q is a group represented by Q41, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX542).

In the compound (L-2), a compound in which Q is a group represented by Q41, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX543).

In the compound (L-2), a compound in which Q is a group represented by Q42, $R^{3b}$ and $R^{3c}$ are hydrogen atoms, and $R^1$ is any of the substituents shown in [Table L1] to [Table L3] (hereinafter, referred to as a compound group SX544) .

In the compound (L-2), a compound in which Q is a group represented by Q42, $R^{3b}$ is a chlorine atom, $R^{3c}$ is a hydrogen atom, and $R^1$ is any of the substituents shown in [Table L1] to [Table L3] (hereinafter, referred to as a compound group SX545).

In the compound (L-2), a compound in which Q is a group represented by Q42, $R^{3b}$ is a bromine atom, $R^{3c}$ is a hydrogen atom, and $R^1$ is any of the substituents shown in [Table L1] to [Table L3] (hereinafter, referred to as a compound group SX546).

In the compound (L-2), a compound in which Q is a group represented by Q42, $R^{3b}$ is an iodine atom, $R^{3c}$ is a hydrogen atom, and $R^1$ is any of the substituents shown in [Table L1] to [Table L3] (hereinafter, referred to as a compound group SX547).

In the compound (L-2), a compound in which Q is a group represented by Q42, $R^{3b}$ is $CF_3$, $R^{3c}$ is a hydrogen atom, and $R^1$ is any of the substituents shown in [Table L1] to [Table L3] (hereinafter, referred to as a compound group SX548).

In the compound (L-2), a compound in which Q is a group represented by Q42, $R^{3b}$ is a hydrogen atom, $R^{3c}$ is a chlorine atom, and $R^1$ is any of the substituents shown in [Table L1] to [Table L3] (hereinafter, referred to as a compound group SX549).

In the compound (L-2), a compound in which Q is a group represented by Q42, $R^{3b}$ is a hydrogen atom, $R^{3c}$ is a bromine atom, and $R^1$ is any of the substituents shown in [Table L1] to [Table L3] (hereinafter, referred to as a compound group SX550).

In the compound (L-2), a compound in which Q is a group represented by Q42, $R^{3b}$ is a hydrogen atom, $R^{3c}$ is an iodine atom, and $R^1$ is any of the substituents shown in [Table L1] to [Table L3] (hereinafter, referred to as a compound group SX551).

In the compound (L-2), a compound in which Q is a group represented by Q42, $R^{3b}$ is a hydrogen atom, $R^{3c}$ is $CF_3$, and $R^1$ is any of the substituents shown in [Table L1] to [Table L3] (hereinafter, referred to as a compound group

SX552).

In the compound (L-2), a compound in which Q is a group represented by Q43, $R^{3b}$ and $R^{3c}$ are hydrogen atoms, and $R^1$ is any of the substituents shown in [Table L1] to [Table L3] (hereinafter, referred to as a compound group SX553).

In the compound (L-2), a compound in which Q is a group represented by Q43, $R^{3b}$ is a chlorine atom, $R^{3c}$ is a hydrogen atom, and $R^1$ is any of the substituents shown in [Table L1] to [Table L3] (hereinafter, referred to as a compound group SX554).

In the compound (L-2), a compound in which Q is a group represented by Q43, $R^{3b}$ is a bromine atom, $R^{3c}$ is a hydrogen atom, and $R^1$ is any of the substituents shown in [Table L1] to [Table L3] (hereinafter, referred to as a compound group SX555).

In the compound (L-2), a compound in which Q is a group represented by Q43, $R^{3b}$ is an iodine atom, $R^{3c}$ is a hydrogen atom, and $R^1$ is any of the substituents shown in [Table L1] to [Table L3] (hereinafter, referred to as a compound group SX556).

In the compound (L-2), a compound in which Q is a group represented by Q43, $R^{3b}$ is $CF_3$, $R^{3c}$ is a hydrogen atom, and $R^1$ is any of the substituents shown in [Table L1] to [Table L3] (hereinafter, referred to as a compound group SX557).

In the compound (L-2), a compound in which Q is a group represented by Q43, $R^{3b}$ is a hydrogen atom, $R^{3c}$ is a chlorine atom, and $R^1$ is any of the substituents shown in [Table L1] to [Table L3] (hereinafter, referred to as a compound group SX558).

In the compound (L-2), a compound in which Q is a group represented by Q43, $R^{3b}$ is a hydrogen atom, $R^{3c}$ is a bromine atom, and $R^1$ is any of the substituents shown in [Table L1] to [Table L3] (hereinafter, referred to as a compound group SX559).

In the compound (L-2), a compound in which Q is a group represented by Q43, $R^{3b}$ is a hydrogen atom, $R^{3c}$ is an iodine atom, and $R^1$ is any of the substituents shown in [Table L1] to [Table L3] (hereinafter, referred to as a compound group SX560).

In the compound (L-2), a compound in which Q is a group represented by Q43, $R^{3b}$ is a hydrogen atom, $R^{3c}$ is $CF_3$, and $R^1$ is any of the substituents shown in [Table L1] to [Table L3] (hereinafter, referred to as a compound group SX561).

In the compound (L-2), a compound in which Q is a group represented by Q44, $R^{3b}$ and $R^{3c}$ are hydrogen atoms, and $R^1$ is any of the substituents shown in [Table L1] to [Table L3] (hereinafter, referred to as a compound group SX562).

In the compound (L-2), a compound in which Q is a group represented by Q44, $R^{3b}$ is a chlorine atom, $R^{3c}$ is a hydrogen atom, and $R^1$ is any of the substituents shown in [Table L1] to [Table L3] (hereinafter, referred to as a compound group SX563).

In the compound (L-2), a compound in which Q is a group represented by Q44, $R^{3b}$ is a bromine atom, $R^{3c}$ is a hydrogen atom, and $R^1$ is any of the substituents shown in [Table L1] to [Table L3] (hereinafter, referred to as a compound group SX564).

In the compound (L-2), a compound in which Q is a group represented by Q44, $R^{3b}$ is an iodine atom, $R^{3c}$ is a hydrogen atom, and $R^1$ is any of the substituents shown in [Table L1] to [Table L3] (hereinafter, referred to as a compound group SX565).

In the compound (L-2), a compound in which Q is a group represented by Q44, $R^{3b}$ is $CF_3$, $R^{3c}$ is a hydrogen atom, and $R^1$ is any of the substituents shown in [Table L1] to [Table L3] (hereinafter, referred to as a compound group SX566).

In the compound (L-2), a compound in which Q is a group represented by Q44, $R^{3b}$ is a hydrogen atom, $R^{3c}$ is a chlorine atom, and $R^1$ is any of the substituents shown in [Table L1] to [Table L3] (hereinafter, referred to as a compound group SX567).

In the compound (L-2), a compound in which Q is a group represented by Q44, $R^{3b}$ is a hydrogen atom, $R^{3c}$ is a bromine atom, and $R^1$ is any of the substituents shown in [Table L1] to [Table L3] (hereinafter, referred to as a compound group SX568).

In the compound (L-2), a compound in which Q is a group represented by Q44, $R^{3b}$ is a hydrogen atom, $R^{3c}$ is an iodine atom, and $R^1$ is any of the substituents shown in [Table L1] to [Table L3] (hereinafter, referred to as a compound group SX569).

In the compound (L-2), a compound in which Q is a group represented by Q44, $R^{3b}$ is a hydrogen atom, $R^{3c}$ is $CF_3$, and $R^1$ is any of the substituents shown in [Table L1] to [Table L3] (hereinafter, referred to as a compound group SX570).

In the compound (L-2), a compound in which Q is a group represented by Q45, $R^{3b}$ and $R^{3c}$ are hydrogen atoms, and $R^1$ is any of the substituents shown in [Table L1] to [Table L3] (hereinafter, referred to as a compound group SX571).

In the compound (L-2), a compound in which Q is a group represented by Q45, $R^{3b}$ is a chlorine atom, $R^{3c}$ is a hydrogen atom, and $R^1$ is any of the substituents shown in [Table L1] to [Table L3] (hereinafter, referred to as a compound group SX572).

In the compound (L-2), a compound in which Q is a group represented by Q45, $R^{3b}$ is a bromine atom, $R^{3c}$ is a hydrogen atom, and $R^1$ is any of the substituents shown in [Table L1] to [Table L3] (hereinafter, referred to as a compound group SX573).

In the compound (L-2), a compound in which Q is a group represented by Q45, $R^{3b}$ is an iodine atom, $R^{3c}$ is a hydrogen atom, and $R^1$ is any of the substituents shown in [Table L1] to [Table L3] (hereinafter, referred to as a compound group SX574).

In the compound (L-2), a compound in which Q is a group represented by Q45, $R^{3b}$ is $CF_3$, $R^{3c}$ is a hydrogen atom, and $R^1$ is any of the substituents shown in [Table L1] to [Table L3] (hereinafter, referred to as a compound group SX575).

In the compound (L-2), a compound in which Q is a group represented by Q45, $R^{3b}$ is a hydrogen atom, $R^{3c}$ is a chlorine atom, and $R^1$ is any of the substituents shown in [Table L1] to [Table L3] (hereinafter, referred to as a compound group SX576).

In the compound (L-2), a compound in which Q is a group represented by Q45, $R^{3b}$ is a hydrogen atom, $R^{3c}$ is a bromine atom, and $R^1$ is any of the substituents shown in [Table L1] to [Table L3] (hereinafter, referred to as a compound group SX577).

In the compound (L-2), a compound in which Q is a group represented by Q45, $R^{3b}$ is a hydrogen atom, $R^{3c}$ is an iodine atom, and $R^1$ is any of the substituents shown in [Table L1] to [Table L3] (hereinafter, referred to as a compound group SX578).

In the compound (L-2), a compound in which Q is a group represented by Q45, $R^{3b}$ is a hydrogen atom, $R^{3c}$ is $CF_3$, and $R^1$ is any of the substituents shown in [Table L1] to [Table L3] (hereinafter, referred to as a compound group SX579).

In the compound (L-2), a compound in which Q is a group represented by Q46, $R^{3b}$ and $R^{3c}$ are hydrogen atoms, and $R^1$ is any of the substituents shown in [Table L1] to [Table L3] (hereinafter, referred to as a compound group SX580).

In the compound (L-2), a compound in which Q is a group represented by Q46, $R^{3b}$ is a chlorine atom, $R^{3c}$ is a hydrogen atom, and $R^1$ is any of the substituents shown in [Table L1] to [Table L3] (hereinafter, referred to as a compound group SX581).

In the compound (L-2), a compound in which Q is a group represented by Q46, $R^{3b}$ is a bromine atom, $R^{3c}$ is a hydrogen atom, and $R^1$ is any of the substituents shown in [Table L1] to [Table L3] (hereinafter, referred to as a compound group SX582).

In the compound (L-2), a compound in which Q is a group represented by Q46, $R^{3b}$ is an iodine atom, $R^{3c}$ is a hydrogen atom, and $R^1$ is any of the substituents shown in [Table L1] to [Table L3] (hereinafter, referred to as a compound group SX583).

In the compound (L-2), a compound in which Q is a group represented by Q46, $R^{3b}$ is $CF_3$, $R^{3c}$ is a hydrogen atom, and $R^1$ is any of the substituents shown in [Table L1] to [Table L3] (hereinafter, referred to as a compound group SX584).

In the compound (L-2), a compound in which Q is a group represented by Q46, $R^{3b}$ is a hydrogen atom, $R^{3c}$ is a chlorine atom, and $R^1$ is any of the substituents shown in [Table L1] to [Table L3] (hereinafter, referred to as a compound group SX585).

In the compound (L-2), a compound in which Q is a group represented by Q46, $R^{3b}$ is a hydrogen atom, $R^{3c}$ is a bromine atom, and $R^1$ is any of the substituents shown in [Table L1] to [Table L3] (hereinafter, referred to as a compound group SX586).

In the compound (L-2), a compound in which Q is a group represented by Q46, $R^{3b}$ is a hydrogen atom, $R^{3c}$ is an iodine atom, and $R^1$ is any of the substituents shown in [Table L1] to [Table L3] (hereinafter, referred to as a compound group SX587).

In the compound (L-2), a compound in which Q is a group represented by Q46, $R^{3b}$ is a hydrogen atom, $R^{3c}$ is $CF_3$, and $R^1$ is any of the substituents shown in [Table L1] to [Table L3] (hereinafter, referred to as a compound group SX588).

In the compound (L-2), a compound in which Q is a group represented by Q47, $R^{3b}$ and $R^{3c}$ are hydrogen atoms, and $R^1$ is any of the substituents shown in [Table L1] to [Table L3] (hereinafter, referred to as a compound group SX589).

In the compound (L-2), a compound in which Q is a group represented by Q47, $R^{3b}$ is a chlorine atom, $R^{3c}$ is a hydrogen atom, and $R^1$ is any of the substituents shown in [Table L1] to [Table L3] (hereinafter, referred to as a compound group SX590).

In the compound (L-2), a compound in which Q is a group represented by Q47, $R^{3b}$ is a bromine atom, $R^{3c}$ is

a hydrogen atom, and $R^1$ is any of the substituents shown in [Table L1] to [Table L3] (hereinafter, referred to as a compound group SX591).

In the compound (L-2), a compound in which Q is a group represented by Q47, $R^{3b}$ is an iodine atom, $R^{3c}$ is a hydrogen atom, and $R^1$ is any of the substituents shown in [Table L1] to [Table L3] (hereinafter, referred to as a compound group SX592).

In the compound (L-2), a compound in which Q is a group represented by Q47, $R^{3b}$ is $CF_3$, $R^{3c}$ is a hydrogen atom, and $R^1$ is any of the substituents shown in [Table L1] to [Table L3] (hereinafter, referred to as a compound group SX593) .

In the compound (L-2), a compound in which Q is a group represented by Q47, $R^{3b}$ is a hydrogen atom, $R^{3c}$ is a chlorine atom, and $R^1$ is any of the substituents shown in [Table L1] to [Table L3] (hereinafter, referred to as a compound group SX594).

In the compound (L-2), a compound in which Q is a group represented by Q47, $R^{3b}$ is a hydrogen atom, $R^{3c}$ is a bromine atom, and $R^1$ is any of the substituents shown in [Table L1] to [Table L3] (hereinafter, referred to as a compound group SX595).

In the compound (L-2), a compound in which Q is a group represented by Q47, $R^{3b}$ is a hydrogen atom, $R^{3c}$ is an iodine atom, and $R^1$ is any of the substituents shown in [Table L1] to [Table L3] (hereinafter, referred to as a compound group SX596).

In the compound (L-2), a compound in which Q is a group represented by Q47, $R^{3b}$ is a hydrogen atom, $R^{3c}$ is $CF_3$, and $R^1$ is any of the substituents shown in [Table L1] to [Table L3] (hereinafter, referred to as a compound group SX597).

In the compound (L-2), a compound in which Q is a group represented by Q48, $R^{3b}$ and $R^{3c}$ are hydrogen atoms, and $R^1$ is any of the substituents shown in [Table L1] to [Table L3] (hereinafter, referred to as a compound group SX598).

In the compound (L-2), a compound in which Q is a group represented by Q48, $R^{3b}$ is a chlorine atom, $R^{3c}$ is a hydrogen atom, and $R^1$ is any of the substituents shown in [Table L1] to [Table L3] (hereinafter, referred to as a compound group SX599).

In the compound (L-2), a compound in which Q is a group represented by Q48, $R^{3b}$ is a bromine atom, $R^{3c}$ is a hydrogen atom, and $R^1$ is any of the substituents shown in [Table L1] to [Table L3] (hereinafter, referred to as a compound group SX600).

In the compound (L-2), a compound in which Q is a group represented by Q48, $R^{3b}$ is an iodine atom, $R^{3c}$ is a hydrogen atom, and $R^1$ is any of the substituents shown in [Table L1] to [Table L3] (hereinafter, referred to as a compound group SX601).

In the compound (L-2), a compound in which Q is a group represented by Q48, $R^{3b}$ is $CF_3$, $R^{3c}$ is a hydrogen atom, and $R^1$ is any of the substituents shown in [Table L1] to [Table L3] (hereinafter, referred to as a compound group SX602) .

In the compound (L-2), a compound in which Q is a group represented by Q48, $R^{3b}$ is a hydrogen atom, $R^{3c}$ is a chlorine atom, and $R^1$ is any of the substituents shown in [Table L1] to [Table L3] (hereinafter, referred to as a compound group SX603).

In the compound (L-2), a compound in which Q is a group represented by Q48, $R^{3b}$ is a hydrogen atom, $R^{3c}$ is a bromine atom, and $R^1$ is any of the substituents shown in [Table L1] to [Table L3] (hereinafter, referred to as a compound group SX604).

In the compound (L-2), a compound in which Q is a group represented by Q48, $R^{3b}$ is a hydrogen atom, $R^{3c}$ is an iodine atom, and $R^1$ is any of the substituents shown in [Table L1] to [Table L3] (hereinafter, referred to as a compound group SX605).

In the compound (L-2), a compound in which Q is a group represented by Q48, $R^{3b}$ is a hydrogen atom, $R^{3c}$ is $CF_3$, and $R^1$ is any of the substituents shown in [Table L1] to [Table L3] (hereinafter, referred to as a compound group SX606) .

In the compound (L-2), a compound in which Q is a group represented by Q49, $R^{3b}$ and $R^{3c}$ are hydrogen atoms, and $R^1$ is any of the substituents shown in [Table L1] to [Table L3] (hereinafter, referred to as a compound group SX607) .

In the compound (L-2), a compound in which Q is a group represented by Q49, $R^{3b}$ is a chlorine atom, $R^{3c}$ is a hydrogen atom, and $R^1$ is any of the substituents shown in [Table L1] to [Table L3] (hereinafter, referred to as a compound group SX608).

In the compound (L-2), a compound in which Q is a group represented by Q49, $R^{3b}$ is a bromine atom, $R^{3c}$ is a hydrogen atom, and $R^1$ is any of the substituents shown in [Table L1] to [Table L3] (hereinafter, referred to as a compound group SX609).

In the compound (L-2), a compound in which Q is a group represented by Q49, $R^{3b}$ is an iodine atom, $R^{3c}$ is a hydrogen atom, and $R^1$ is any of the substituents shown in [Table L1] to [Table L3] (hereinafter, referred to as a

compound group SX610).

In the compound (L-2), a compound in which Q is a group represented by Q49, $R^{3b}$ is $CF_3$, $R^{3c}$ is a hydrogen atom, and $R^1$ is any of the substituents shown in [Table L1] to [Table L3] (hereinafter, referred to as a compound group SX611).

In the compound (L-2), a compound in which Q is a group represented by Q49, $R^{3b}$ is a hydrogen atom, $R^{3c}$ is a chlorine atom, and $R^1$ is any of the substituents shown in [Table L1] to [Table L3] (hereinafter, referred to as a compound group SX612).

In the compound (L-2), a compound in which Q is a group represented by Q49, $R^{3b}$ is a hydrogen atom, $R^{3c}$ is a bromine atom, and $R^1$ is any of the substituents shown in [Table L1] to [Table L3] (hereinafter, referred to as a compound group SX613).

In the compound (L-2), a compound in which Q is a group represented by Q49, $R^{3b}$ is a hydrogen atom, $R^{3c}$ is an iodine atom, and $R^1$ is any of the substituents shown in [Table L1] to [Table L3] (hereinafter, referred to as a compound group SX614).

In the compound (L-2), a compound in which Q is a group represented by Q49, $R^{3b}$ is a hydrogen atom, $R^{3c}$ is $CF_3$, and $R^1$ is any of the substituents shown in [Table L1] to [Table L3] (hereinafter, referred to as a compound group SX615) .

In the compound (L-2), a compound in which Q is a group represented by Q50, $R^{3b}$ and $R^{3c}$ are hydrogen atoms, and $R^1$ is any of the substituents shown in [Table L1] to [Table L3] (hereinafter, referred to as a compound group SX616) .

In the compound (L-2), a compound in which Q is a group represented by Q50, $R^{3b}$ is a chlorine atom, $R^{3c}$ is a hydrogen atom, and $R^1$ is any of the substituents shown in [Table L1] to [Table L3] (hereinafter, referred to as a compound group SX617).

In the compound (L-2), a compound in which Q is a group represented by Q50, $R^{3b}$ is a bromine atom, $R^{3c}$ is a hydrogen atom, and $R^1$ is any of the substituents shown in [Table L1] to [Table L3] (hereinafter, referred to as a compound group SX618).

In the compound (L-2), a compound in which Q is a group represented by Q50, $R^{3b}$ is an iodine atom, $R^{3c}$ is a hydrogen atom, and $R^1$ is any of the substituents shown in [Table L1] to [Table L3] (hereinafter, referred to as a compound group SX619).

In the compound (L-2), a compound in which Q is a group represented by Q50, $R^{3b}$ is $CF_3$, $R^{3c}$ is a hydrogen atom, and $R^1$ is any of the substituents shown in [Table L1] to [Table L3] (hereinafter, referred to as a compound group SX620).

In the compound (L-2), a compound in which Q is a group represented by Q50, $R^{3b}$ is a hydrogen atom, $R^{3c}$ is a chlorine atom, and $R^1$ is any of the substituents shown in [Table L1] to [Table L3] (hereinafter, referred to as a compound group SX621).

In the compound (L-2), a compound in which Q is a group represented by Q50, $R^{3b}$ is a hydrogen atom, $R^{3c}$ is a bromine atom, and $R^1$ is any of the substituents shown in [Table L1] to [Table L3] (hereinafter, referred to as a compound group SX622).

In the compound (L-2), a compound in which Q is a group represented by Q50, $R^{3b}$ is a hydrogen atom, $R^{3c}$ is an iodine atom, and $R^1$ is any of the substituents shown in [Table L1] to [Table L3] (hereinafter, referred to as a compound group SX623).

In the compound (L-2), a compound in which Q is a group represented by Q50, $R^{3b}$ is a hydrogen atom, $R^{3c}$ is $CF_3$, and $R^1$ is any of the substituents shown in [Table L1] to [Table L3] (hereinafter, referred to as a compound group SX624).

In the compound (L-2), a compound in which Q is a group represented by Q51, $R^{3b}$ and $R^{3c}$ are hydrogen atoms, and $R^1$ is any of the substituents shown in [Table L1] to [Table L3] (hereinafter, referred to as a compound group SX625).

In the compound (L-2), a compound in which Q is a group represented by Q51, $R^{3b}$ is a chlorine atom, $R^{3c}$ is a hydrogen atom, and $R^1$ is any of the substituents shown in [Table L1] to [Table L3] (hereinafter, referred to as a compound group SX626).

In the compound (L-2), a compound in which Q is a group represented by Q51, $R^{3b}$ is a bromine atom, $R^{3c}$ is a hydrogen atom, and $R^1$ is any of the substituents shown in [Table L1] to [Table L3] (hereinafter, referred to as a compound group SX627).

In the compound (L-2), a compound in which Q is a group represented by Q51, $R^{3b}$ is an iodine atom, $R^{3c}$ is a hydrogen atom, and $R^1$ is any of the substituents shown in [Table L1] to [Table L3] (hereinafter, referred to as a compound group SX628).

In the compound (L-2), a compound in which Q is a group represented by Q51, $R^{3b}$ is $CF_3$, $R^{3c}$ is a hydrogen atom, and $R^1$ is any of the substituents shown in [Table L1] to [Table L3] (hereinafter, referred to as a compound group SX629) .

In the compound (L-2), a compound in which Q is a group represented by Q51, $R^{3b}$ is a hydrogen atom, $R^{3c}$ is a chlorine atom, and $R^1$ is any of the substituents shown in [Table L1] to [Table L3] (hereinafter, referred to as a compound group SX630).

In the compound (L-2), a compound in which Q is a group represented by Q51, $R^{3b}$ is a hydrogen atom, $R^{3c}$ is a bromine atom, and $R^1$ is any of the substituents shown in [Table L1] to [Table L3] (hereinafter, referred to as a compound group SX631).

In the compound (L-2), a compound in which Q is a group represented by Q51, $R^{3b}$ is a hydrogen atom, $R^{3c}$ is an iodine atom, and $R^1$ is any of the substituents shown in [Table L1] to [Table L3] (hereinafter, referred to as a compound group SX632).

In the compound (L-2), a compound in which Q is a group represented by Q51, $R^{3b}$ is a hydrogen atom, $R^{3c}$ is $CF_3$, and $R^1$ is any of the substituents shown in [Table L1] to [Table L3] (hereinafter, referred to as a compound group SX633).

In the compound (L-2), a compound in which Q is a group represented by Q52, $R^{3b}$ and $R^{3c}$ are hydrogen atoms, and $R^1$ is any of the substituents shown in [Table L1] to [Table L3] (hereinafter, referred to as a compound group SX634).

In the compound (L-2), a compound in which Q is a group represented by Q52, $R^{3b}$ is a chlorine atom, $R^{3c}$ is a hydrogen atom, and $R^1$ is any of the substituents shown in [Table L1] to [Table L3] (hereinafter, referred to as a compound group SX635).

In the compound (L-2), a compound in which Q is a group represented by Q52, $R^{3b}$ is a bromine atom, $R^{3c}$ is a hydrogen atom, and $R^1$ is any of the substituents shown in [Table L1] to [Table L3] (hereinafter, referred to as a compound group SX636).

In the compound (L-2), a compound in which Q is a group represented by Q52, $R^{3b}$ is an iodine atom, $R^{3c}$ is a hydrogen atom, and $R^1$ is any of the substituents shown in [Table L1] to [Table L3] (hereinafter, referred to as a compound group SX637).

In the compound (L-2), a compound in which Q is a group represented by Q52, $R^{3b}$ is $CF_3$, $R^{3c}$ is a hydrogen atom, and $R^1$ is any of the substituents shown in [Table L1] to [Table L3] (hereinafter, referred to as a compound group SX638) .

In the compound (L-2), a compound in which Q is a group represented by Q52, $R^{3b}$ is a hydrogen atom, $R^{3c}$ is a chlorine atom, and $R^1$ is any of the substituents shown in [Table L1] to [Table L3] (hereinafter, referred to as a compound group SX639).

In the compound (L-2), a compound in which Q is a group represented by Q52, $R^{3b}$ is a hydrogen atom, $R^{3c}$ is a bromine atom, and $R^1$ is any of the substituents shown in [Table L1] to [Table L3] (hereinafter, referred to as a compound group SX640).

In the compound (L-2), a compound in which Q is a group represented by Q52, $R^{3b}$ is a hydrogen atom, $R^{3c}$ is an iodine atom, and $R^1$ is any of the substituents shown in [Table L1] to [Table L3] (hereinafter, referred to as a compound group SX641).

In the compound (L-2), a compound in which Q is a group represented by Q52, $R^{3b}$ is a hydrogen atom, $R^{3c}$ is $CF_3$, and $R^1$ is any of the substituents shown in [Table L1] to [Table L3] (hereinafter, referred to as a compound group SX642) .

In the compound (L-2), a compound in which Q is a group represented by Q53, $R^{3b}$ and $R^{3c}$ are hydrogen atoms, and $R^1$ is any of the substituents shown in [Table L1] to [Table L3] (hereinafter, referred to as a compound group SX643).

In the compound (L-2), a compound in which Q is a group represented by Q53, $R^{3b}$ is a chlorine atom, $R^{3c}$ is a hydrogen atom, and $R^1$ is any of the substituents shown in [Table L1] to [Table L3] (hereinafter, referred to as a compound group SX644).

In the compound (L-2), a compound in which Q is a group represented by Q53, $R^{3b}$ is a bromine atom, $R^{3c}$ is a hydrogen atom, and $R^1$ is any of the substituents shown in [Table L1] to [Table L3] (hereinafter, referred to as a compound group SX645).

In the compound (L-2), a compound in which Q is a group represented by Q53, $R^{3b}$ is an iodine atom, $R^{3c}$ is a hydrogen atom, and $R^1$ is any of the substituents shown in [Table L1] to [Table L3] (hereinafter, referred to as a compound group SX646).

In the compound (L-2), a compound in which Q is a group represented by Q53, $R^{3b}$ is $CF_3$, $R^{3c}$ is a hydrogen atom, and $R^1$ is any of the substituents shown in [Table L1] to [Table L3] (hereinafter, referred to as a compound group SX647).

In the compound (L-2), a compound in which Q is a group represented by Q53, $R^{3b}$ is a hydrogen atom, $R^{3c}$ is a chlorine atom, and $R^1$ is any of the substituents shown in [Table L1] to [Table L3] (hereinafter, referred to as a compound group SX648).

In the compound (L-2), a compound in which Q is a group represented by Q53, $R^{3b}$ is a hydrogen atom, $R^{3c}$

is a bromine atom, and $R^1$ is any of the substituents shown in [Table L1] to [Table L3] (hereinafter, referred to as a compound group SX649).

In the compound (L-2), a compound in which Q is a group represented by Q53, $R^{3b}$ is a hydrogen atom, $R^{3c}$ is an iodine atom, and $R^1$ is any of the substituents shown in [Table L1] to [Table L3] (hereinafter, referred to as a compound group SX650).

In the compound (L-2), a compound in which Q is a group represented by Q53, $R^{3b}$ is a hydrogen atom, $R^{3c}$ is $CF_3$, and $R^1$ is any of the substituents shown in [Table L1] to [Table L3] (hereinafter, referred to as a compound group SX651) .

In the compound (L-2), a compound in which Q is a group represented by Q54, $R^{3b}$ and $R^{3c}$ are hydrogen atoms, and $R^1$ is any of the substituents shown in [Table L1] to [Table L3] (hereinafter, referred to as a compound group SX652) .

In the compound (L-2), a compound in which Q is a group represented by Q54, $R^{3b}$ is a chlorine atom, $R^{3c}$ is a hydrogen atom, and $R^1$ is any of the substituents shown in [Table L1] to [Table L3] (hereinafter, referred to as a compound group SX653).

In the compound (L-2), a compound in which Q is a group represented by Q54, $R^{3b}$ is a bromine atom, $R^{3c}$ is a hydrogen atom, and $R^1$ is any of the substituents shown in [Table L1] to [Table L3] (hereinafter, referred to as a compound group SX654).

In the compound (L-2), a compound in which Q is a group represented by Q54, $R^{3b}$ is an iodine atom, $R^{3c}$ is a hydrogen atom, and $R^1$ is any of the substituents shown in [Table L1] to [Table L3] (hereinafter, referred to as a compound group SX655).

In the compound (L-2), a compound in which Q is a group represented by Q54, $R^{3b}$ is $CF_3$, $R^{3c}$ is a hydrogen atom, and $R^1$ is any of the substituents shown in [Table L1] to [Table L3] (hereinafter, referred to as a compound group SX656).

In the compound (L-2), a compound in which Q is a group represented by Q54, $R^{3b}$ is a hydrogen atom, $R^{3c}$ is a chlorine atom, and $R^1$ is any of the substituents shown in [Table L1] to [Table L3] (hereinafter, referred to as a compound group SX657).

In the compound (L-2), a compound in which Q is a group represented by Q54, $R^{3b}$ is a hydrogen atom, $R^{3c}$ is a bromine atom, and $R^1$ is any of the substituents shown in [Table L1] to [Table L3] (hereinafter, referred to as a compound group SX658).

In the compound (L-2), a compound in which Q is a group represented by Q54, $R^{3b}$ is a hydrogen atom, $R^{3c}$ is an iodine atom, and $R^1$ is any of the substituents shown in [Table L1] to [Table L3] (hereinafter, referred to as a compound group SX659).

In the compound (L-2), a compound in which Q is a group represented by Q54, $R^{3b}$ is a hydrogen atom, $R^{3c}$ is $CF_3$, and $R^1$ is any of the substituents shown in [Table L1] to [Table L3] (hereinafter, referred to as a compound group SX660).

In the compound (L-2), a compound in which Q is a group represented by Q55, $R^{3b}$ and $R^{3c}$ are hydrogen atoms, and $R^1$ is any of the substituents shown in [Table L1] to [Table L3] (hereinafter, referred to as a compound group SX661).

In the compound (L-2), a compound in which Q is a group represented by Q55, $R^{3b}$ is a chlorine atom, $R^{3c}$ is a hydrogen atom, and $R^1$ is any of the substituents shown in [Table L1] to [Table L3] (hereinafter, referred to as a compound group SX662).

In the compound (L-2), a compound in which Q is a group represented by Q55, $R^{3b}$ is a bromine atom, $R^{3c}$ is a hydrogen atom, and $R^1$ is any of the substituents shown in [Table L1] to [Table L3] (hereinafter, referred to as a compound group SX663).

In the compound (L-2), a compound in which Q is a group represented by Q55, $R^{3b}$ is an iodine atom, $R^{3c}$ is a hydrogen atom, and $R^1$ is any of the substituents shown in [Table L1] to [Table L3] (hereinafter, referred to as a compound group SX664).

In the compound (L-2), a compound in which Q is a group represented by Q55, $R^{3b}$ is $CF_3$, $R^{3c}$ is a hydrogen atom, and $R^1$ is any of the substituents shown in [Table L1] to [Table L3] (hereinafter, referred to as a compound group SX665).

In the compound (L-2), a compound in which Q is a group represented by Q55, $R^{3b}$ is a hydrogen atom, $R^{3c}$ is a chlorine atom, and $R^1$ is any of the substituents shown in [Table L1] to [Table L3] (hereinafter, referred to as a compound group SX666).

In the compound (L-2), a compound in which Q is a group represented by Q55, $R^{3b}$ is a hydrogen atom, $R^{3c}$ is a bromine atom, and $R^1$ is any of the substituents shown in [Table L1] to [Table L3] (hereinafter, referred to as a compound group SX667).

In the compound (L-2), a compound in which Q is a group represented by Q55, $R^{3b}$ is a hydrogen atom, $R^{3c}$ is an iodine atom, and $R^1$ is any of the substituents shown in [Table L1] to [Table L3] (hereinafter, referred to as a

compound group SX668).

In the compound (L-2), a compound in which Q is a group represented by Q55, $R^{3b}$ is a hydrogen atom, $R^{3c}$ is $CF_3$, and $R^1$ is any of the substituents shown in [Table L1] to [Table L3] (hereinafter, referred to as a compound group SX669).

In the compound (L-2), a compound in which Q is a group represented by Q56, $R^{3b}$ and $R^{3c}$ are hydrogen atoms, and $R^1$ is any of the substituents shown in [Table L1] to [Table L3] (hereinafter, referred to as a compound group SX670).

In the compound (L-2), a compound in which Q is a group represented by Q56, $R^{3b}$ is a chlorine atom, $R^{3c}$ is a hydrogen atom, and $R^1$ is any of the substituents shown in [Table L1] to [Table L3] (hereinafter, referred to as a compound group SX671).

In the compound (L-2), a compound in which Q is a group represented by Q56, $R^{3b}$ is a bromine atom, $R^{3c}$ is a hydrogen atom, and $R^1$ is any of the substituents shown in [Table L1] to [Table L3] (hereinafter, referred to as a compound group SX672).

In the compound (L-2), a compound in which Q is a group represented by Q56, $R^{3b}$ is an iodine atom, $R^{3c}$ is a hydrogen atom, and $R^1$ is any of the substituents shown in [Table L1] to [Table L3] (hereinafter, referred to as a compound group SX673).

In the compound (L-2), a compound in which Q is a group represented by Q56, $R^{3b}$ is $CF_3$, $R^{3c}$ is a hydrogen atom, and $R^1$ is any of the substituents shown in [Table L1] to [Table L3] (hereinafter, referred to as a compound group SX674).

In the compound (L-2), a compound in which Q is a group represented by Q56, $R^{3b}$ is a hydrogen atom, $R^{3c}$ is a chlorine atom, and $R^1$ is any of the substituents shown in [Table L1] to [Table L3] (hereinafter, referred to as a compound group SX675).

In the compound (L-2), a compound in which Q is a group represented by Q56, $R^{3b}$ is a hydrogen atom, $R^{3c}$ is a bromine atom, and $R^1$ is any of the substituents shown in [Table L1] to [Table L3] (hereinafter, referred to as a compound group SX676).

In the compound (L-2), a compound in which Q is a group represented by Q56, $R^{3b}$ is a hydrogen atom, $R^{3c}$ is an iodine atom, and $R^1$ is any of the substituents shown in [Table L1] to [Table L3] (hereinafter, referred to as a compound group SX677).

In the compound (L-2), a compound in which Q is a group represented by Q56, $R^{3b}$ is a hydrogen atom, $R^{3c}$ is $CF_3$, and $R^1$ is any of the substituents shown in [Table L1] to [Table L3] (hereinafter, referred to as a compound group SX678).

In the compound (L-2), a compound in which Q is a group represented by Q57, $R^{3b}$ and $R^{3c}$ are hydrogen atoms, and $R^1$ is any of the substituents shown in [Table L1] to [Table L3] (hereinafter, referred to as a compound group SX679).

In the compound (L-2), a compound in which Q is a group represented by Q57, $R^{3b}$ is a chlorine atom, $R^{3c}$ is a hydrogen atom, and $R^1$ is any of the substituents shown in [Table L1] to [Table L3] (hereinafter, referred to as a compound group SX680).

In the compound (L-2), a compound in which Q is a group represented by Q57, $R^{3b}$ is a bromine atom, $R^{3c}$ is a hydrogen atom, and $R^1$ is any of the substituents shown in [Table L1] to [Table L3] (hereinafter, referred to as a compound group SX681).

In the compound (L-2), a compound in which Q is a group represented by Q57, $R^{3b}$ is an iodine atom, $R^{3c}$ is a hydrogen atom, and $R^1$ is any of the substituents shown in [Table L1] to [Table L3] (hereinafter, referred to as a compound group SX682).

In the compound (L-2), a compound in which Q is a group represented by Q57, $R^{3b}$ is $CF_3$, $R^{3c}$ is a hydrogen atom, and $R^1$ is any of the substituents shown in [Table L1] to [Table L3] (hereinafter, referred to as a compound group SX683) .

In the compound (L-2), a compound in which Q is a group represented by Q57, $R^{3b}$ is a hydrogen atom, $R^{3c}$ is a chlorine atom, and $R^1$ is any of the substituents shown in [Table L1] to [Table L3] (hereinafter, referred to as a compound group SX684).

In the compound (L-2), a compound in which Q is a group represented by Q57, $R^{3b}$ is a hydrogen atom, $R^{3c}$ is a bromine atom, and $R^1$ is any of the substituents shown in [Table L1] to [Table L3] (hereinafter, referred to as a compound group SX685).

In the compound (L-2), a compound in which Q is a group represented by Q57, $R^{3b}$ is a hydrogen atom, $R^{3c}$ is an iodine atom, and $R^1$ is any of the substituents shown in [Table L1] to [Table L3] (hereinafter, referred to as a compound group SX686).

In the compound (L-2), a compound in which Q is a group represented by Q57, $R^{3b}$ is a hydrogen atom, $R^{3c}$ is $CF_3$, and $R^1$ is any of the substituents shown in [Table L1] to [Table L3] (hereinafter, referred to as a compound group SX687) .

[Chem. 93]

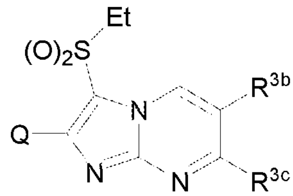

(L-3)

In a compound (hereinafter, referred to as a compound (L-3)) represented by Formula (L-3), a compound in which Q is a group represented by Q11, $R^{3b}$ and $R^{3c}$ are hydrogen atoms, and $R^1$ is any of the substituents shown in [Table L1] to [Table L3] (hereinafter, referred to as a compound group SX55).

In the compound (L-3), a compound in which Q is a group represented by Q12, $R^{3b}$ and $R^{3c}$ are hydrogen atoms, and $R^1$ is any of the substituents shown in [Table L1] to [Table L3] (hereinafter, referred to as a compound group SX56).

In the compound (L-3), a compound in which Q is a group represented by Q13, $R^{3b}$ and $R^{3c}$ are hydrogen atoms, and $R^1$ is any of the substituents shown in [Table L1] to [Table L3] (hereinafter, referred to as a compound group SX57).

In the compound (L-3), a compound in which Q is a group represented by Q11, $R^{3b}$ is a chlorine atom, $R^{3c}$ is a hydrogen atom, and $R^1$ is any of the substituents shown in [Table L1] to [Table L3] (hereinafter, referred to as a compound group SX58).

In the compound (L-3), a compound in which Q is a group represented by Q12, $R^{3b}$ is a chlorine atom, $R^{3c}$ is a hydrogen atom, and $R^1$ is any of the substituents shown in [Table L1] to [Table L3] (hereinafter, referred to as a compound group SX59).

In the compound (L-3), a compound in which Q is a group represented by Q13, $R^{3b}$ is a chlorine atom, $R^{3c}$ is a hydrogen atom, and $R^1$ is any of the substituents shown in [Table L1] to [Table L3] (hereinafter, referred to as a compound group SX60).

In the compound (L-3), a compound in which Q is a group represented by Q11, $R^{3b}$ is a bromine atom, $R^{3c}$ is a hydrogen atom, and $R^1$ is any of the substituents shown in [Table L1] to [Table L3] (hereinafter, referred to as a compound group SX61).

In the compound (L-3), a compound in which Q is a group represented by Q12, $R^{3b}$ is a bromine atom, $R^{3c}$ is a hydrogen atom, and $R^1$ is any of the substituents shown in [Table L1] to [Table L3] (hereinafter, referred to as a compound group SX62).

In the compound (L-3), a compound in which Q is a group represented by Q13, $R^{3b}$ is a bromine atom, $R^{3c}$ is a hydrogen atom, and $R^1$ is any of the substituents shown in [Table L1] to [Table L3] (hereinafter, referred to as a compound group SX63).

In the compound (L-3), a compound in which Q is a group represented by Q11, $R^{3b}$ is an iodine atom, $R^{3c}$ is a hydrogen atom, and $R^1$ is any of the substituents shown in [Table L1] to [Table L3] (hereinafter, referred to as a compound group SX64).

In the compound (L-3), a compound in which Q is a group represented by Q12, $R^{3b}$ is an iodine atom, $R^{3c}$ is a hydrogen atom, and $R^1$ is any of the substituents shown in [Table L1] to [Table L3] (hereinafter, referred to as a compound group SX65).

In the compound (L-3), a compound in which Q is a group represented by Q13, $R^{3b}$ is an iodine atom, $R^{3c}$ is a hydrogen atom, and $R^1$ is any of the substituents shown in [Table L1] to [Table L3] (hereinafter, referred to as a compound group SX66).

In the compound (L-3), a compound in which Q is a group represented by Q11, $R^{3b}$ is $CF_3$, $R^{3c}$ is a hydrogen atom, and $R^1$ is any of the substituents shown in [Table L1] to [Table L3] (hereinafter, referred to as a compound group SX67) .

In the compound (L-3), a compound in which Q is a group represented by Q12, $R^{3b}$ is $CF_3$, $R^{3c}$ is a hydrogen atom, and $R^1$ is any of the substituents shown in [Table L1] to [Table L3] (hereinafter, referred to as a compound group SX68) .

In the compound (L-3), a compound in which Q is a group represented by Q13, $R^{3b}$ is $CF_3$, $R^{3c}$ is a hydrogen atom, and $R^1$ is any of the substituents shown in [Table L1] to [Table L3] (hereinafter, referred to as a compound group SX69) .

In the compound (L-3), a compound in which Q is a group represented by Q11, $R^{3b}$ is a hydrogen atom, $R^{3c}$ is a chlorine atom, and $R^1$ is any of the substituents shown in [Table L1] to [Table L3] (hereinafter, referred to as a compound group SX70).

In the compound (L-3), a compound in which Q is a group represented by Q12, $R^{3b}$ is a hydrogen atom, $R^{3c}$ is a chlorine atom, and $R^1$ is any of the substituents shown in [Table L1] to [Table L3] (hereinafter, referred to as a compound group SX71).

In the compound (L-3), a compound in which Q is a group represented by Q13, $R^{3b}$ is a hydrogen atom, $R^{3c}$ is a chlorine atom, and $R^1$ is any of the substituents shown in [Table L1] to [Table L3] (hereinafter, referred to as a compound group SX72).

In the compound (L-3), a compound in which Q is a group represented by Q11, $R^{3b}$ is a hydrogen atom, $R^{3c}$ is a bromine atom, and $R^1$ is any of the substituents shown in [Table L1] to [Table L3] (hereinafter, referred to as a compound group SX73).

In the compound (L-3), a compound in which Q is a group represented by Q12, $R^{3b}$ is a hydrogen atom, $R^{3c}$ is a bromine atom, and $R^1$ is any of the substituents shown in [Table L1] to [Table L3] (hereinafter, referred to as a compound group SX74).

In the compound (L-3), a compound in which Q is a group represented by Q13, $R^{3b}$ is a hydrogen atom, $R^{3c}$ is a bromine atom, and $R^1$ is any of the substituents shown in [Table L1] to [Table L3] (hereinafter, referred to as a compound group SX75).

In the compound (L-3), a compound in which Q is a group represented by Q11, $R^{3b}$ is a hydrogen atom, $R^{3c}$ is an iodine atom, and $R^1$ is any of the substituents shown in [Table L1] to [Table L3] (hereinafter, referred to as a compound group SX76).

In the compound (L-3), a compound in which Q is a group represented by Q12, $R^{3b}$ is a hydrogen atom, $R^{3c}$ is an iodine atom, and $R^1$ is any of the substituents shown in [Table L1] to [Table L3] (hereinafter, referred to as a compound group SX77).

In the compound (L-3), a compound in which Q is a group represented by Q13, $R^{3b}$ is a hydrogen atom, $R^{3c}$ is an iodine atom, and $R^1$ is any of the substituents shown in [Table L1] to [Table L3] (hereinafter, referred to as a compound group SX78).

In the compound (L-3), a compound in which Q is a group represented by Q11, $R^{3b}$ is a hydrogen atom, $R^{3c}$ is $CF_3$, and $R^1$ is any of the substituents shown in [Table L1] to [Table L3] (hereinafter, referred to as a compound group SX79) .

In the compound (L-3), a compound in which Q is a group represented by Q12, $R^{3b}$ is a hydrogen atom, $R^{3c}$ is $CF_3$, and $R^1$ is any of the substituents shown in [Table L1] to [Table L3] (hereinafter, referred to as a compound group SX80) .

In the compound (L-3), a compound in which Q is a group represented by Q13, $R^{3b}$ is a hydrogen atom, $R^{3c}$ is $CF_3$, and $R^1$ is any of the substituents shown in [Table L1] to [Table L3] (hereinafter, referred to as a compound group SX81) .

In the compound (L-3), a compound in which Q is a group represented by Q34, $R^{3b}$ and $R^{3c}$ are hydrogen atoms, and $R^1$ is any of the substituents shown in [Table L1] to [Table L3] (hereinafter, referred to as a compound group SX688).

In the compound (L-3), a compound in which Q is a group represented by Q34, $R^{3b}$ is a chlorine atom, $R^{3c}$ is a hydrogen atom, and $R^1$ is any of the substituents shown in [Table L1] to [Table L3] (hereinafter, referred to as a compound group SX689).

In the compound (L-3), a compound in which Q is a group represented by Q34, $R^{3b}$ is a bromine atom, $R^{3c}$ is a hydrogen atom, and $R^1$ is any of the substituents shown in [Table L1] to [Table L3] (hereinafter, referred to as a compound group SX690).

In the compound (L-3), a compound in which Q is a group represented by Q34, $R^{3b}$ is an iodine atom, $R^{3c}$ is a hydrogen atom, and $R^1$ is any of the substituents shown in [Table L1] to [Table L3] (hereinafter, referred to as a compound group SX691).

In the compound (L-3), a compound in which Q is a group represented by Q34, $R^{3b}$ is $CF_3$, $R^{3c}$ is a hydrogen atom, and $R^1$ is any of the substituents shown in [Table L1] to [Table L3] (hereinafter, referred to as a compound group SX692).

In the compound (L-3), a compound in which Q is a group represented by Q34, $R^{3b}$ is a hydrogen atom, $R^{3c}$ is a chlorine atom, and $R^1$ is any of the substituents shown in [Table L1] to [Table L3] (hereinafter, referred to as a compound group SX693).

In the compound (L-3), a compound in which Q is a group represented by Q34, $R^{3b}$ is a hydrogen atom, $R^{3c}$ is a bromine atom, and $R^1$ is any of the substituents shown in [Table L1] to [Table L3] (hereinafter, referred to as a compound group SX694).

In the compound (L-3), a compound in which Q is a group represented by Q34, $R^{3b}$ is a hydrogen atom, $R^{3c}$ is an iodine atom, and $R^1$ is any of the substituents shown in [Table L1] to [Table L3] (hereinafter, referred to as a compound group SX695).

In the compound (L-3), a compound in which Q is a group represented by Q34, $R^{3b}$ is a hydrogen atom, $R^{3c}$

is $CF_3$, and $R^1$ is any of the substituents shown in [Table L1] to [Table L3] (hereinafter, referred to as a compound group SX696).

In the compound (L-3), a compound in which Q is a group represented by Q35, $R^{3b}$ and $R^{3c}$ are hydrogen atoms, and $R^1$ is any of the substituents shown in [Table L1] to [Table L3] (hereinafter, referred to as a compound group SX697).

In the compound (L-3), a compound in which Q is a group represented by Q35, $R^{3b}$ is a chlorine atom, $R^{3c}$ is a hydrogen atom, and $R^1$ is any of the substituents shown in [Table L1] to [Table L3] (hereinafter, referred to as a compound group SX698).

In the compound (L-3), a compound in which Q is a group represented by Q35, $R^{3b}$ is a bromine atom, $R^{3c}$ is a hydrogen atom, and $R^1$ is any of the substituents shown in [Table L1] to [Table L3] (hereinafter, referred to as a compound group SX699).

In the compound (L-3), a compound in which Q is a group represented by Q35, $R^{3b}$ is an iodine atom, $R^{3c}$ is a hydrogen atom, and $R^1$ is any of the substituents shown in [Table L1] to [Table L3] (hereinafter, referred to as a compound group SX700).

In the compound (L-3), a compound in which Q is a group represented by Q35, $R^{3b}$ is $CF_3$, $R^{3c}$ is a hydrogen atom, and $R^1$ is any of the substituents shown in [Table L1] to [Table L3] (hereinafter, referred to as a compound group SX701).

In the compound (L-3), a compound in which Q is a group represented by Q35, $R^{3b}$ is a hydrogen atom, $R^{3c}$ is a chlorine atom, and $R^1$ is any of the substituents shown in [Table L1] to [Table L3] (hereinafter, referred to as a compound group SX702).

In the compound (L-3), a compound in which Q is a group represented by Q35, $R^{3b}$ is a hydrogen atom, $R^{3c}$ is a bromine atom, and $R^1$ is any of the substituents shown in [Table L1] to [Table L3] (hereinafter, referred to as a compound group SX703).

In the compound (L-3), a compound in which Q is a group represented by Q35, $R^{3b}$ is a hydrogen atom, $R^{3c}$ is an iodine atom, and $R^1$ is any of the substituents shown in [Table L1] to [Table L3] (hereinafter, referred to as a compound group SX704).

In the compound (L-3), a compound in which Q is a group represented by Q35, $R^{3b}$ is a hydrogen atom, $R^{3c}$ is $CF_3$, and $R^1$ is any of the substituents shown in [Table L1] to [Table L3] (hereinafter, referred to as a compound group SX705).

In the compound (L-3), a compound in which Q is a group represented by Q36, $R^{3b}$ and $R^{3c}$ are hydrogen atoms, and $R^1$ is any of the substituents shown in [Table L1] to [Table L3] (hereinafter, referred to as a compound group SX706).

In the compound (L-3), a compound in which Q is a group represented by Q36, $R^{3b}$ is a chlorine atom, $R^{3c}$ is a hydrogen atom, and $R^1$ is any of the substituents shown in [Table L1] to [Table L3] (hereinafter, referred to as a compound group SX707).

In the compound (L-3), a compound in which Q is a group represented by Q36, $R^{3b}$ is a bromine atom, $R^{3c}$ is a hydrogen atom, and $R^1$ is any of the substituents shown in [Table L1] to [Table L3] (hereinafter, referred to as a compound group SX708).

In the compound (L-3), a compound in which Q is a group represented by Q36, $R^{3b}$ is an iodine atom, $R^{3c}$ is a hydrogen atom, and $R^1$ is any of the substituents shown in [Table L1] to [Table L3] (hereinafter, referred to as a compound group SX709).

In the compound (L-3), a compound in which Q is a group represented by Q36, $R^{3b}$ is $CF_3$, $R^{3c}$ is a hydrogen atom, and $R^1$ is any of the substituents shown in [Table L1] to [Table L3] (hereinafter, referred to as a compound group SX710).

In the compound (L-3), a compound in which Q is a group represented by Q36, $R^{3b}$ is a hydrogen atom, $R^{3c}$ is a chlorine atom, and $R^1$ is any of the substituents shown in [Table L1] to [Table L3] (hereinafter, referred to as a compound group SX711).

In the compound (L-3), a compound in which Q is a group represented by Q36, $R^{3b}$ is a hydrogen atom, $R^{3c}$ is a bromine atom, and $R^1$ is any of the substituents shown in [Table L1] to [Table L3] (hereinafter, referred to as a compound group SX712).

In the compound (L-3), a compound in which Q is a group represented by Q36, $R^{3b}$ is a hydrogen atom, $R^{3c}$ is an iodine atom, and $R^1$ is any of the substituents shown in [Table L1] to [Table L3] (hereinafter, referred to as a compound group SX713).

In the compound (L-3), a compound in which Q is a group represented by Q36, $R^{3b}$ is a hydrogen atom, $R^{3c}$ is $CF_3$, and $R^1$ is any of the substituents shown in [Table L1] to [Table L3] (hereinafter, referred to as a compound group SX714).

In the compound (L-3), a compound in which Q is a group represented by Q37, $R^{3b}$ and $R^{3c}$ are hydrogen atoms, and $R^1$ is any of the substituents shown in [Table L1] to [Table L3] (hereinafter, referred to as a compound group

SX715).

In the compound (L-3), a compound in which Q is a group represented by Q37, $R^{3b}$ is a chlorine atom, $R^{3c}$ is a hydrogen atom, and $R^1$ is any of the substituents shown in [Table L1] to [Table L3] (hereinafter, referred to as a compound group SX716).

In the compound (L-3), a compound in which Q is a group represented by Q37, $R^{3b}$ is a bromine atom, $R^{3c}$ is a hydrogen atom, and $R^1$ is any of the substituents shown in [Table L1] to [Table L3] (hereinafter, referred to as a compound group SX717).

In the compound (L-3), a compound in which Q is a group represented by Q37, $R^{3b}$ is an iodine atom, $R^{3c}$ is a hydrogen atom, and $R^1$ is any of the substituents shown in [Table L1] to [Table L3] (hereinafter, referred to as a compound group SX718).

In the compound (L-3), a compound in which Q is a group represented by Q37, $R^{3b}$ is $CF_3$, $R^{3c}$ is a hydrogen atom, and $R^1$ is any of the substituents shown in [Table L1] to [Table L3] (hereinafter, referred to as a compound group SX719).

In the compound (L-3), a compound in which Q is a group represented by Q37, $R^{3b}$ is a hydrogen atom, $R^{3c}$ is a chlorine atom, and $R^1$ is any of the substituents shown in [Table L1] to [Table L3] (hereinafter, referred to as a compound group SX720).

In the compound (L-3), a compound in which Q is a group represented by Q37, $R^{3b}$ is a hydrogen atom, $R^{3c}$ is a bromine atom, and $R^1$ is any of the substituents shown in [Table L1] to [Table L3] (hereinafter, referred to as a compound group SX721).

In the compound (L-3), a compound in which Q is a group represented by Q37, $R^{3b}$ is a hydrogen atom, $R^{3c}$ is an iodine atom, and $R^1$ is any of the substituents shown in [Table L1] to [Table L3] (hereinafter, referred to as a compound group SX722).

In the compound (L-3), a compound in which Q is a group represented by Q37, $R^{3b}$ is a hydrogen atom, $R^{3c}$ is $CF_3$, and $R^1$ is any of the substituents shown in [Table L1] to [Table L3] (hereinafter, referred to as a compound group SX723).

In the compound (L-3), a compound in which Q is a group represented by Q38, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX724).

In the compound (L-3), a compound in which Q is a group represented by Q38, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX725).

In the compound (L-3), a compound in which Q is a group represented by Q38, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX726).

In the compound (L-3), a compound in which Q is a group represented by Q38, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX727).

In the compound (L-3), a compound in which Q is a group represented by Q38, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX728).

In the compound (L-3), a compound in which Q is a group represented by Q38, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX729).

In the compound (L-3), a compound in which Q is a group represented by Q38, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX730).

In the compound (L-3), a compound in which Q is a group represented by Q38, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX731).

In the compound (L-3), a compound in which Q is a group represented by Q38, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX732).

In the compound (L-3), a compound in which Q is a group represented by Q38, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX733).

In the compound (L-3), a compound in which Q is a group represented by Q38, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX734).

In the compound (L-3), a compound in which Q is a group represented by Q38, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX735).

In the compound (L-3), a compound in which Q is a group represented by Q38, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX736).

In the compound (L-3), a compound in which Q is a group represented by Q38, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX737).

In the compound (L-3), a compound in which Q is a group represented by Q38, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX738).

In the compound (L-3), a compound in which Q is a group represented by Q38, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX739).

In the compound (L-3), a compound in which Q is a group represented by Q38, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX740).

In the compound (L-3), a compound in which Q is a group represented by Q38, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX741).

In the compound (L-3), a compound in which Q is a group represented by Q39, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX742).

In the compound (L-3), a compound in which Q is a group represented by Q39, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX743).

In the compound (L-3), a compound in which Q is a group represented by Q39, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX744).

In the compound (L-3), a compound in which Q is a group represented by Q39, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX745).

In the compound (L-3), a compound in which Q is a group represented by Q39, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX746).

In the compound (L-3), a compound in which Q is a group represented by Q39, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX747).

In the compound (L-3), a compound in which Q is a group represented by Q39, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX748).

In the compound (L-3), a compound in which Q is a group represented by Q39, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX749).

In the compound (L-3), a compound in which Q is a group represented by Q39, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX750).

In the compound (L-3), a compound in which Q is a group represented by Q39, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX751).

In the compound (L-3), a compound in which Q is a group represented by Q39, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX752).

In the compound (L-3), a compound in which Q is a group represented by Q39, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX753).

In the compound (L-3), a compound in which Q is a group represented by Q39, $R^{3b}$ is an iodine atom, $R^{3c}$ is

a hydrogen atom, $R^{6a}$ is a methyl group, and $R^1$ is any of the substituents shown in [Table L1] to [Table L3] (hereinafter, referred to as a compound group SX754).

In the compound (L-3), a compound in which Q is a group represented by Q39, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX755).

In the compound (L-3), a compound in which Q is a group represented by Q39, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX756).

In the compound (L-3), a compound in which Q is a group represented by Q39, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX757).

In the compound (L-3), a compound in which Q is a group represented by Q39, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX758).

In the compound (L-3), a compound in which Q is a group represented by Q39, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX759).

In the compound (L-3), a compound in which Q is a group represented by Q40, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX760).

In the compound (L-3), a compound in which Q is a group represented by Q40, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX761).

In the compound (L-3), a compound in which Q is a group represented by Q40, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX762).

In the compound (L-3), a compound in which Q is a group represented by Q40, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX763).

In the compound (L-3), a compound in which Q is a group represented by Q40, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX764).

In the compound (L-3), a compound in which Q is a group represented by Q40, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX765).

In the compound (L-3), a compound in which Q is a group represented by Q40, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX766).

In the compound (L-3), a compound in which Q is a group represented by Q40, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX767).

In the compound (L-3), a compound in which Q is a group represented by Q40, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX768).

In the compound (L-3), a compound in which Q is a group represented by Q40, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX769).

In the compound (L-3), a compound in which Q is a group represented by Q40, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX770).

In the compound (L-3), a compound in which Q is a group represented by Q40, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX771).

In the compound (L-3), a compound in which Q is a group represented by Q40, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX772).

In the compound (L-3), a compound in which Q is a group represented by Q40, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to

as a compound group SX773).

In the compound (L-3), a compound in which Q is a group represented by Q40, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX774).

In the compound (L-3), a compound in which Q is a group represented by Q40, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX775).

In the compound (L-3), a compound in which Q is a group represented by Q40, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX776).

In the compound (L-3), a compound in which Q is a group represented by Q40, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX777).

In the compound (L-3), a compound in which Q is a group represented by Q41, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX778).

In the compound (L-3), a compound in which Q is a group represented by Q41, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX779).

In the compound (L-3), a compound in which Q is a group represented by Q41, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX780).

In the compound (L-3), a compound in which Q is a group represented by Q41, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX781).

In the compound (L-3), a compound in which Q is a group represented by Q41, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX782).

In the compound (L-3), a compound in which Q is a group represented by Q41, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX783).

In the compound (L-3), a compound in which Q is a group represented by Q41, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX784).

In the compound (L-3), a compound in which Q is a group represented by Q41, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX785).

In the compound (L-3), a compound in which Q is a group represented by Q41, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX786).

In the compound (L-3), a compound in which Q is a group represented by Q41, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX787).

In the compound (L-3), a compound in which Q is a group represented by Q41, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX788).

In the compound (L-3), a compound in which Q is a group represented by Q41, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX789).

In the compound (L-3), a compound in which Q is a group represented by Q41, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX790).

In the compound (L-3), a compound in which Q is a group represented by Q41, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX791).

In the compound (L-3), a compound in which Q is a group represented by Q41, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX792).

In the compound (L-3), a compound in which Q is a group represented by Q41, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX793).

In the compound (L-3), a compound in which Q is a group represented by Q41, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX794).

In the compound (L-3), a compound in which Q is a group represented by Q41, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX795).

In the compound (L-3), a compound in which Q is a group represented by Q42, $R^{3b}$ and $R^{3c}$ are hydrogen atoms, and $R^1$ is any of the substituents shown in [Table L1] to [Table L3] (hereinafter, referred to as a compound group SX796).

In the compound (L-3), a compound in which Q is a group represented by Q42, $R^{3b}$ is a chlorine atom, $R^{3c}$ is a hydrogen atom, and $R^1$ is any of the substituents shown in [Table L1] to [Table L3] (hereinafter, referred to as a compound group SX797).

In the compound (L-3), a compound in which Q is a group represented by Q42, $R^{3b}$ is a bromine atom, $R^{3c}$ is a hydrogen atom, and $R^1$ is any of the substituents shown in [Table L1] to [Table L3] (hereinafter, referred to as a compound group SX798).

In the compound (L-3), a compound in which Q is a group represented by Q42, $R^{3b}$ is an iodine atom, $R^{3c}$ is a hydrogen atom, and $R^1$ is any of the substituents shown in [Table L1] to [Table L3] (hereinafter, referred to as a compound group SX799).

In the compound (L-3), a compound in which Q is a group represented by Q42, $R^{3b}$ is $CF_3$, $R^{3c}$ is a hydrogen atom, and $R^1$ is any of the substituents shown in [Table L1] to [Table L3] (hereinafter, referred to as a compound group SX800).

In the compound (L-3), a compound in which Q is a group represented by Q42, $R^{3b}$ is a hydrogen atom, $R^{3c}$ is a chlorine atom, and $R^1$ is any of the substituents shown in [Table L1] to [Table L3] (hereinafter, referred to as a compound group SX801).

In the compound (L-3), a compound in which Q is a group represented by Q42, $R^{3b}$ is a hydrogen atom, $R^{3c}$ is a bromine atom, and $R^1$ is any of the substituents shown in [Table L1] to [Table L3] (hereinafter, referred to as a compound group SX802).

In the compound (L-3), a compound in which Q is a group represented by Q42, $R^{3b}$ is a hydrogen atom, $R^{3c}$ is an iodine atom, and $R^1$ is any of the substituents shown in [Table L1] to [Table L3] (hereinafter, referred to as a compound group SX803).

In the compound (L-3), a compound in which Q is a group represented by Q42, $R^{3b}$ is a hydrogen atom, $R^{3c}$ is $CF_3$, and $R^1$ is any of the substituents shown in [Table L1] to [Table L3] (hereinafter, referred to as a compound group SX804).

In the compound (L-3), a compound in which Q is a group represented by Q43, $R^{3b}$ and $R^{3c}$ are hydrogen atoms, and $R^1$ is any of the substituents shown in [Table L1] to [Table L3] (hereinafter, referred to as a compound group SX805).

In the compound (L-3), a compound in which Q is a group represented by Q43, $R^{3b}$ is a chlorine atom, $R^{3c}$ is a hydrogen atom, and $R^1$ is any of the substituents shown in [Table L1] to [Table L3] (hereinafter, referred to as a compound group SX806).

In the compound (L-3), a compound in which Q is a group represented by Q43, $R^{3b}$ is a bromine atom, $R^{3c}$ is a hydrogen atom, and $R^1$ is any of the substituents shown in [Table L1] to [Table L3] (hereinafter, referred to as a compound group SX807).

In the compound (L-3), a compound in which Q is a group represented by Q43, $R^{3b}$ is an iodine atom, $R^{3c}$ is a hydrogen atom, and $R^1$ is any of the substituents shown in [Table L1] to [Table L3] (hereinafter, referred to as a compound group SX808).

In the compound (L-3), a compound in which Q is a group represented by Q43, $R^{3b}$ is $CF_3$, $R^{3c}$ is a hydrogen atom, and $R^1$ is any of the substituents shown in [Table L1] to [Table L3] (hereinafter, referred to as a compound group SX809).

In the compound (L-3), a compound in which Q is a group represented by Q43, $R^{3b}$ is a hydrogen atom, $R^{3c}$ is a chlorine atom, and $R^1$ is any of the substituents shown in [Table L1] to [Table L3] (hereinafter, referred to as a compound group SX810).

In the compound (L-3), a compound in which Q is a group represented by Q43, $R^{3b}$ is a hydrogen atom, $R^{3c}$ is a bromine atom, and $R^1$ is any of the substituents shown in [Table L1] to [Table L3] (hereinafter, referred to as a compound group SX811).

In the compound (L-3), a compound in which Q is a group represented by Q43, $R^{3b}$ is a hydrogen atom, $R^{3c}$

is an iodine atom, and $R^1$ is any of the substituents shown in [Table L1] to [Table L3] (hereinafter, referred to as a compound group SX812).

In the compound (L-3), a compound in which Q is a group represented by Q43, $R^{3b}$ is a hydrogen atom, $R^{3c}$ is $CF_3$, and $R^1$ is any of the substituents shown in [Table L1] to [Table L3] (hereinafter, referred to as a compound group SX813).

In the compound (L-3), a compound in which Q is a group represented by Q44, $R^{3b}$ and $R^{3c}$ are hydrogen atoms, and $R^1$ is any of the substituents shown in [Table L1] to [Table L3] (hereinafter, referred to as a compound group SX814).

In the compound (L-3), a compound in which Q is a group represented by Q44, $R^{3b}$ is a chlorine atom, $R^{3c}$ is a hydrogen atom, and $R^1$ is any of the substituents shown in [Table L1] to [Table L3] (hereinafter, referred to as a compound group SX815).

In the compound (L-3), a compound in which Q is a group represented by Q44, $R^{3b}$ is a bromine atom, $R^{3c}$ is a hydrogen atom, and $R^1$ is any of the substituents shown in [Table L1] to [Table L3] (hereinafter, referred to as a compound group SX816).

In the compound (L-3), a compound in which Q is a group represented by Q44, $R^{3b}$ is an iodine atom, $R^{3c}$ is a hydrogen atom, and $R^1$ is any of the substituents shown in [Table L1] to [Table L3] (hereinafter, referred to as a compound group SX817).

In the compound (L-3), a compound in which Q is a group represented by Q44, $R^{3b}$ is $CF_3$, $R^{3c}$ is a hydrogen atom, and $R^1$ is any of the substituents shown in [Table L1] to [Table L3] (hereinafter, referred to as a compound group SX818).

In the compound (L-3), a compound in which Q is a group represented by Q44, $R^{3b}$ is a hydrogen atom, $R^{3c}$ is a chlorine atom, and $R^1$ is any of the substituents shown in [Table L1] to [Table L3] (hereinafter, referred to as a compound group SX819).

In the compound (L-3), a compound in which Q is a group represented by Q44, $R^{3b}$ is a hydrogen atom, $R^{3c}$ is a bromine atom, and $R^1$ is any of the substituents shown in [Table L1] to [Table L3] (hereinafter, referred to as a compound group SX820).

In the compound (L-3), a compound in which Q is a group represented by Q44, $R^{3b}$ is a hydrogen atom, $R^{3c}$ is an iodine atom, and $R^1$ is any of the substituents shown in [Table L1] to [Table L3] (hereinafter, referred to as a compound group SX821).

In the compound (L-3), a compound in which Q is a group represented by Q44, $R^{3b}$ is a hydrogen atom, $R^{3c}$ is $CF_3$, and $R^1$ is any of the substituents shown in [Table L1] to [Table L3] (hereinafter, referred to as a compound group SX822).

In the compound (L-3), a compound in which Q is a group represented by Q45, $R^{3b}$ and $R^{3c}$ are hydrogen atoms, and $R^1$ is any of the substituents shown in [Table L1] to [Table L3] (hereinafter, referred to as a compound group SX823).

In the compound (L-3), a compound in which Q is a group represented by Q45, $R^{3b}$ is a chlorine atom, $R^{3c}$ is a hydrogen atom, and $R^1$ is any of the substituents shown in [Table L1] to [Table L3] (hereinafter, referred to as a compound group SX824).

In the compound (L-3), a compound in which Q is a group represented by Q45, $R^{3b}$ is a bromine atom, $R^{3c}$ is a hydrogen atom, and $R^1$ is any of the substituents shown in [Table L1] to [Table L3] (hereinafter, referred to as a compound group SX825).

In the compound (L-3), a compound in which Q is a group represented by Q45, $R^{3b}$ is an iodine atom, $R^{3c}$ is a hydrogen atom, and $R^1$ is any of the substituents shown in [Table L1] to [Table L3] (hereinafter, referred to as a compound group SX826).

In the compound (L-3), a compound in which Q is a group represented by Q45, $R^{3b}$ is $CF_3$, $R^{3c}$ is a hydrogen atom, and $R^1$ is any of the substituents shown in [Table L1] to [Table L3] (hereinafter, referred to as a compound group SX827).

In the compound (L-3), a compound in which Q is a group represented by Q45, $R^{3b}$ is a hydrogen atom, $R^{3c}$ is a chlorine atom, and $R^1$ is any of the substituents shown in [Table L1] to [Table L3] (hereinafter, referred to as a compound group SX828).

In the compound (L-3), a compound in which Q is a group represented by Q45, $R^{3b}$ is a hydrogen atom, $R^{3c}$ is a bromine atom, and $R^1$ is any of the substituents shown in [Table L1] to [Table L3] (hereinafter, referred to as a compound group SX829).

In the compound (L-3), a compound in which Q is a group represented by Q45, $R^{3b}$ is a hydrogen atom, $R^{3c}$ is an iodine atom, and $R^1$ is any of the substituents shown in [Table L1] to [Table L3] (hereinafter, referred to as a compound group SX830).

In the compound (L-3), a compound in which Q is a group represented by Q45, $R^{3b}$ is a hydrogen atom, $R^{3c}$ is $CF_3$, and $R^1$ is any of the substituents shown in [Table L1] to [Table L3] (hereinafter, referred to as a compound group

SX831).

In the compound (L-3), a compound in which Q is a group represented by Q46, $R^{3b}$ and $R^{3c}$ are hydrogen atoms, and $R^1$ is any of the substituents shown in [Table L1] to [Table L3] (hereinafter, referred to as a compound group SX832).

In the compound (L-3), a compound in which Q is a group represented by Q46, $R^{3b}$ is a chlorine atom, $R^{3c}$ is a hydrogen atom, and $R^1$ is any of the substituents shown in [Table L1] to [Table L3] (hereinafter, referred to as a compound group SX833).

In the compound (L-3), a compound in which Q is a group represented by Q46, $R^{3b}$ is a bromine atom, $R^{3c}$ is a hydrogen atom, and $R^1$ is any of the substituents shown in [Table L1] to [Table L3] (hereinafter, referred to as a compound group SX834).

In the compound (L-3), a compound in which Q is a group represented by Q46, $R^{3b}$ is an iodine atom, $R^{3c}$ is a hydrogen atom, and $R^1$ is any of the substituents shown in [Table L1] to [Table L3] (hereinafter, referred to as a compound group SX835).

In the compound (L-3), a compound in which Q is a group represented by Q46, $R^{3b}$ is $CF_3$, $R^{3c}$ is a hydrogen atom, and $R^1$ is any of the substituents shown in [Table L1] to [Table L3] (hereinafter, referred to as a compound group SX836).

In the compound (L-3), a compound in which Q is a group represented by Q46, $R^{3b}$ is a hydrogen atom, $R^{3c}$ is a chlorine atom, and $R^1$ is any of the substituents shown in [Table L1] to [Table L3] (hereinafter, referred to as a compound group SX837).

In the compound (L-3), a compound in which Q is a group represented by Q46, $R^{3b}$ is a hydrogen atom, $R^{3c}$ is a bromine atom, and $R^1$ is any of the substituents shown in [Table L1] to [Table L3] (hereinafter, referred to as a compound group SX838).

In the compound (L-3), a compound in which Q is a group represented by Q46, $R^{3b}$ is a hydrogen atom, $R^{3c}$ is an iodine atom, and $R^1$ is any of the substituents shown in [Table L1] to [Table L3] (hereinafter, referred to as a compound group SX839).

In the compound (L-3), a compound in which Q is a group represented by Q46, $R^{3b}$ is a hydrogen atom, $R^{3c}$ is $CF_3$, and $R^1$ is any of the substituents shown in [Table L1] to [Table L3] (hereinafter, referred to as a compound group SX840).

In the compound (L-3), a compound in which Q is a group represented by Q47, $R^{3b}$ and $R^{3c}$ are hydrogen atoms, and $R^1$ is any of the substituents shown in [Table L1] to [Table L3] (hereinafter, referred to as a compound group SX841).

In the compound (L-3), a compound in which Q is a group represented by Q47, $R^{3b}$ is a chlorine atom, $R^{3c}$ is a hydrogen atom, and $R^1$ is any of the substituents shown in [Table L1] to [Table L3] (hereinafter, referred to as a compound group SX842).

In the compound (L-3), a compound in which Q is a group represented by Q47, $R^{3b}$ is a bromine atom, $R^{3c}$ is a hydrogen atom, and $R^1$ is any of the substituents shown in [Table L1] to [Table L3] (hereinafter, referred to as a compound group SX843).

In the compound (L-3), a compound in which Q is a group represented by Q47, $R^{3b}$ is an iodine atom, $R^{3c}$ is a hydrogen atom, and $R^1$ is any of the substituents shown in [Table L1] to [Table L3] (hereinafter, referred to as a compound group SX844).

In the compound (L-3), a compound in which Q is a group represented by Q47, $R^{3b}$ is $CF_3$, $R^{3c}$ is a hydrogen atom, and $R^1$ is any of the substituents shown in [Table L1] to [Table L3] (hereinafter, referred to as a compound group SX845).

In the compound (L-3), a compound in which Q is a group represented by Q47, $R^{3b}$ is a hydrogen atom, $R^{3c}$ is a chlorine atom, and $R^1$ is any of the substituents shown in [Table L1] to [Table L3] (hereinafter, referred to as a compound group SX846).

In the compound (L-3), a compound in which Q is a group represented by Q47, $R^{3b}$ is a hydrogen atom, $R^{3c}$ is a bromine atom, and $R^1$ is any of the substituents shown in [Table L1] to [Table L3] (hereinafter, referred to as a compound group SX847).

In the compound (L-3), a compound in which Q is a group represented by Q47, $R^{3b}$ is a hydrogen atom, $R^{3c}$ is an iodine atom, and $R^1$ is any of the substituents shown in [Table L1] to [Table L3] (hereinafter, referred to as a compound group SX848).

In the compound (L-3), a compound in which Q is a group represented by Q47, $R^{3b}$ is a hydrogen atom, $R^{3c}$ is $CF_3$, and $R^1$ is any of the substituents shown in [Table L1] to [Table L3] (hereinafter, referred to as a compound group SX849).

In the compound (L-3), a compound in which Q is a group represented by Q48, $R^{3b}$ and $R^{3c}$ are hydrogen atoms, and $R^1$ is any of the substituents shown in [Table L1] to [Table L3] (hereinafter, referred to as a compound group SX850).

In the compound (L-3), a compound in which Q is a group represented by Q48, $R^{3b}$ is a chlorine atom, $R^{3c}$ is a hydrogen atom, and $R^1$ is any of the substituents shown in [Table L1] to [Table L3] (hereinafter, referred to as a compound group SX851).

In the compound (L-3), a compound in which Q is a group represented by Q48, $R^{3b}$ is a bromine atom, $R^{3c}$ is a hydrogen atom, and $R^1$ is any of the substituents shown in [Table L1] to [Table L3] (hereinafter, referred to as a compound group SX852).

In the compound (L-3), a compound in which Q is a group represented by Q48, $R^{3b}$ is an iodine atom, $R^{3c}$ is a hydrogen atom, and $R^1$ is any of the substituents shown in [Table L1] to [Table L3] (hereinafter, referred to as a compound group SX853).

In the compound (L-3), a compound in which Q is a group represented by Q48, $R^{3b}$ is $CF_3$, $R^{3c}$ is a hydrogen atom, and $R^1$ is any of the substituents shown in [Table L1] to [Table L3] (hereinafter, referred to as a compound group SX854).

In the compound (L-3), a compound in which Q is a group represented by Q48, $R^{3b}$ is a hydrogen atom, $R^{3c}$ is a chlorine atom, and $R^1$ is any of the substituents shown in [Table L1] to [Table L3] (hereinafter, referred to as a compound group SX855).

In the compound (L-3), a compound in which Q is a group represented by Q48, $R^{3b}$ is a hydrogen atom, $R^{3c}$ is a bromine atom, and $R^1$ is any of the substituents shown in [Table L1] to [Table L3] (hereinafter, referred to as a compound group SX856).

In the compound (L-3), a compound in which Q is a group represented by Q48, $R^{3b}$ is a hydrogen atom, $R^{3c}$ is an iodine atom, and $R^1$ is any of the substituents shown in [Table L1] to [Table L3] (hereinafter, referred to as a compound group SX857).

In the compound (L-3), a compound in which Q is a group represented by Q48, $R^{3b}$ is a hydrogen atom, $R^{3c}$ is $CF_3$, and $R^1$ is any of the substituents shown in [Table L1] to [Table L3] (hereinafter, referred to as a compound group SX858).

In the compound (L-3), a compound in which Q is a group represented by Q49, $R^{3b}$ and $R^{3c}$ are hydrogen atoms, and $R^1$ is any of the substituents shown in [Table L1] to [Table L3] (hereinafter, referred to as a compound group SX859).

In the compound (L-3), a compound in which Q is a group represented by Q49, $R^{3b}$ is a chlorine atom, $R^{3c}$ is a hydrogen atom, and $R^1$ is any of the substituents shown in [Table L1] to [Table L3] (hereinafter, referred to as a compound group SX860).

In the compound (L-3), a compound in which Q is a group represented by Q49, $R^{3b}$ is a bromine atom, $R^{3c}$ is a hydrogen atom, and $R^1$ is any of the substituents shown in [Table L1] to [Table L3] (hereinafter, referred to as a compound group SX861).

In the compound (L-3), a compound in which Q is a group represented by Q49, $R^{3b}$ is an iodine atom, $R^{3c}$ is a hydrogen atom, and $R^1$ is any of the substituents shown in [Table L1] to [Table L3] (hereinafter, referred to as a compound group SX862).

In the compound (L-3), a compound in which Q is a group represented by Q49, $R^{3b}$ is $CF_3$, $R^{3c}$ is a hydrogen atom, and $R^1$ is any of the substituents shown in [Table L1] to [Table L3] (hereinafter, referred to as a compound group SX863).

In the compound (L-3), a compound in which Q is a group represented by Q49, $R^{3b}$ is a hydrogen atom, $R^{3c}$ is a chlorine atom, and $R^1$ is any of the substituents shown in [Table L1] to [Table L3] (hereinafter, referred to as a compound group SX864).

In the compound (L-3), a compound in which Q is a group represented by Q49, $R^{3b}$ is a hydrogen atom, $R^{3c}$ is a bromine atom, and $R^1$ is any of the substituents shown in [Table L1] to [Table L3] (hereinafter, referred to as a compound group SX865).

In the compound (L-3), a compound in which Q is a group represented by Q49, $R^{3b}$ is a hydrogen atom, $R^{3c}$ is an iodine atom, and $R^1$ is any of the substituents shown in [Table L1] to [Table L3] (hereinafter, referred to as a compound group SX866).

In the compound (L-3), a compound in which Q is a group represented by Q49, $R^{3b}$ is a hydrogen atom, $R^{3c}$ is $CF_3$, and $R^1$ is any of the substituents shown in [Table L1] to [Table L3] (hereinafter, referred to as a compound group SX867).

In the compound (L-3), a compound in which Q is a group represented by Q50, $R^{3b}$ and $R^{3c}$ are hydrogen atoms, and $R^1$ is any of the substituents shown in [Table L1] to [Table L3] (hereinafter, referred to as a compound group SX868).

In the compound (L-3), a compound in which Q is a group represented by Q50, $R^{3b}$ is a chlorine atom, $R^{3c}$ is a hydrogen atom, and $R^1$ is any of the substituents shown in [Table L1] to [Table L3] (hereinafter, referred to as a compound group SX869).

In the compound (L-3), a compound in which Q is a group represented by Q50, $R^{3b}$ is a bromine atom, $R^{3c}$ is

a hydrogen atom, and $R^1$ is any of the substituents shown in [Table L1] to [Table L3] (hereinafter, referred to as a compound group SX870).

In the compound (L-3), a compound in which Q is a group represented by Q50, $R^{3b}$ is an iodine atom, $R^{3c}$ is a hydrogen atom, and $R^1$ is any of the substituents shown in [Table L1] to [Table L3] (hereinafter, referred to as a compound group SX871).

In the compound (L-3), a compound in which Q is a group represented by Q50, $R^{3b}$ is $CF_3$, $R^{3c}$ is a hydrogen atom, and $R^1$ is any of the substituents shown in [Table L1] to [Table L3] (hereinafter, referred to as a compound group SX872) .

In the compound (L-3), a compound in which Q is a group represented by Q50, $R^{3b}$ is a hydrogen atom, $R^{3c}$ is a chlorine atom, and $R^1$ is any of the substituents shown in [Table L1] to [Table L3] (hereinafter, referred to as a compound group SX873).

In the compound (L-3), a compound in which Q is a group represented by Q50, $R^{3b}$ is a hydrogen atom, $R^{3c}$ is a bromine atom, and $R^1$ is any of the substituents shown in [Table L1] to [Table L3] (hereinafter, referred to as a compound group SX874).

In the compound (L-3), a compound in which Q is a group represented by Q50, $R^{3b}$ is a hydrogen atom, $R^{3c}$ is an iodine atom, and $R^1$ is any of the substituents shown in [Table L1] to [Table L3] (hereinafter, referred to as a compound group SX875).

In the compound (L-3), a compound in which Q is a group represented by Q50, $R^{3b}$ is a hydrogen atom, $R^{3c}$ is $CF_3$, and $R^1$ is any of the substituents shown in [Table L1] to [Table L3] (hereinafter, referred to as a compound group SX876).

In the compound (L-3), a compound in which Q is a group represented by Q51, $R^{3b}$ and $R^{3c}$ are hydrogen atoms, and $R^1$ is any of the substituents shown in [Table L1] to [Table L3] (hereinafter, referred to as a compound group SX877).

In the compound (L-3), a compound in which Q is a group represented by Q51, $R^{3b}$ is a chlorine atom, $R^{3c}$ is a hydrogen atom, and $R^1$ is any of the substituents shown in [Table L1] to [Table L3] (hereinafter, referred to as a compound group SX878).

In the compound (L-3), a compound in which Q is a group represented by Q51, $R^{3b}$ is a bromine atom, $R^{3c}$ is a hydrogen atom, and $R^1$ is any of the substituents shown in [Table L1] to [Table L3] (hereinafter, referred to as a compound group SX879).

In the compound (L-3), a compound in which Q is a group represented by Q51, $R^{3b}$ is an iodine atom, $R^{3c}$ is a hydrogen atom, and $R^1$ is any of the substituents shown in [Table L1] to [Table L3] (hereinafter, referred to as a compound group SX880).

In the compound (L-3), a compound in which Q is a group represented by Q51, $R^{3b}$ is $CF_3$, $R^{3c}$ is a hydrogen atom, and $R^1$ is any of the substituents shown in [Table L1] to [Table L3] (hereinafter, referred to as a compound group SX881).

In the compound (L-3), a compound in which Q is a group represented by Q51, $R^{3b}$ is a hydrogen atom, $R^{3c}$ is a chlorine atom, and $R^1$ is any of the substituents shown in [Table L1] to [Table L3] (hereinafter, referred to as a compound group SX882).

In the compound (L-3), a compound in which Q is a group represented by Q51, $R^{3b}$ is a hydrogen atom, $R^{3c}$ is a bromine atom, and $R^1$ is any of the substituents shown in [Table L1] to [Table L3] (hereinafter, referred to as a compound group SX883).

In the compound (L-3), a compound in which Q is a group represented by Q51, $R^{3b}$ is a hydrogen atom, $R^{3c}$ is an iodine atom, and $R^1$ is any of the substituents shown in [Table L1] to [Table L3] (hereinafter, referred to as a compound group SX884).

In the compound (L-3), a compound in which Q is a group represented by Q51, $R^{3b}$ is a hydrogen atom, $R^{3c}$ is $CF_3$, and $R^1$ is any of the substituents shown in [Table L1] to [Table L3] (hereinafter, referred to as a compound group SX885).

In the compound (L-3), a compound in which Q is a group represented by Q52, $R^{3b}$ and $R^{3c}$ are hydrogen atoms, and $R^1$ is any of the substituents shown in [Table L1] to [Table L3] (hereinafter, referred to as a compound group SX886).

In the compound (L-3), a compound in which Q is a group represented by Q52, $R^{3b}$ is a chlorine atom, $R^{3c}$ is a hydrogen atom, and $R^1$ is any of the substituents shown in [Table L1] to [Table L3] (hereinafter, referred to as a compound group SX887).

In the compound (L-3), a compound in which Q is a group represented by Q52, $R^{3b}$ is a bromine atom, $R^{3c}$ is a hydrogen atom, and $R^1$ is any of the substituents shown in [Table L1] to [Table L3] (hereinafter, referred to as a compound group SX888).

In the compound (L-3), a compound in which Q is a group represented by Q52, $R^{3b}$ is an iodine atom, $R^{3c}$ is a hydrogen atom, and $R^1$ is any of the substituents shown in [Table L1] to [Table L3] (hereinafter, referred to as a

compound group SX889).

In the compound (L-3), a compound in which Q is a group represented by Q52, $R^{3b}$ is $CF_3$, $R^{3c}$ is a hydrogen atom, and $R^1$ is any of the substituents shown in [Table L1] to [Table L3] (hereinafter, referred to as a compound group SX890).

In the compound (L-3), a compound in which Q is a group represented by Q52, $R^{3b}$ is a hydrogen atom, $R^{3c}$ is a chlorine atom, and $R^1$ is any of the substituents shown in [Table L1] to [Table L3] (hereinafter, referred to as a compound group SX891).

In the compound (L-3), a compound in which Q is a group represented by Q52, $R^{3b}$ is a hydrogen atom, $R^{3c}$ is a bromine atom, and $R^1$ is any of the substituents shown in [Table L1] to [Table L3] (hereinafter, referred to as a compound group SX892).

In the compound (L-3), a compound in which Q is a group represented by Q52, $R^{3b}$ is a hydrogen atom, $R^{3c}$ is an iodine atom, and $R^1$ is any of the substituents shown in [Table L1] to [Table L3] (hereinafter, referred to as a compound group SX893).

In the compound (L-3), a compound in which Q is a group represented by Q52, $R^{3b}$ is a hydrogen atom, $R^{3c}$ is $CF_3$, and $R^1$ is any of the substituents shown in [Table L1] to [Table L3] (hereinafter, referred to as a compound group SX894).

In the compound (L-3), a compound in which Q is a group represented by Q53, $R^{3b}$ and $R^{3c}$ are hydrogen atoms, and $R^1$ is any of the substituents shown in [Table L1] to [Table L3] (hereinafter, referred to as a compound group SX895).

In the compound (L-3), a compound in which Q is a group represented by Q53, $R^{3b}$ is a chlorine atom, $R^{3c}$ is a hydrogen atom, and $R^1$ is any of the substituents shown in [Table L1] to [Table L3] (hereinafter, referred to as a compound group SX896).

In the compound (L-3), a compound in which Q is a group represented by Q53, $R^{3b}$ is a bromine atom, $R^{3c}$ is a hydrogen atom, and $R^1$ is any of the substituents shown in [Table L1] to [Table L3] (hereinafter, referred to as a compound group SX897).

In the compound (L-3), a compound in which Q is a group represented by Q53, $R^{3b}$ is an iodine atom, $R^{3c}$ is a hydrogen atom, and $R^1$ is any of the substituents shown in [Table L1] to [Table L3] (hereinafter, referred to as a compound group SX898).

In the compound (L-3), a compound in which Q is a group represented by Q53, $R^{3b}$ is $CF_3$, $R^{3c}$ is a hydrogen atom, and $R^1$ is any of the substituents shown in [Table L1] to [Table L3] (hereinafter, referred to as a compound group SX899).

In the compound (L-3), a compound in which Q is a group represented by Q53, $R^{3b}$ is a hydrogen atom, $R^{3c}$ is a chlorine atom, and $R^1$ is any of the substituents shown in [Table L1] to [Table L3] (hereinafter, referred to as a compound group SX900).

In the compound (L-3), a compound in which Q is a group represented by Q53, $R^{3b}$ is a hydrogen atom, $R^{3c}$ is a bromine atom, and $R^1$ is any of the substituents shown in [Table L1] to [Table L3] (hereinafter, referred to as a compound group SX901).

In the compound (L-3), a compound in which Q is a group represented by Q53, $R^{3b}$ is a hydrogen atom, $R^{3c}$ is an iodine atom, and $R^1$ is any of the substituents shown in [Table L1] to [Table L3] (hereinafter, referred to as a compound group SX902).

In the compound (L-3), a compound in which Q is a group represented by Q53, $R^{3b}$ is a hydrogen atom, $R^{3c}$ is $CF_3$, and $R^1$ is any of the substituents shown in [Table L1] to [Table L3] (hereinafter, referred to as a compound group SX903).

In the compound (L-3), a compound in which Q is a group represented by Q54, $R^{3b}$ and $R^{3c}$ are hydrogen atoms, and $R^1$ is any of the substituents shown in [Table L1] to [Table L3] (hereinafter, referred to as a compound group SX904).

In the compound (L-3), a compound in which Q is a group represented by Q54, $R^{3b}$ is a chlorine atom, $R^{3c}$ is a hydrogen atom, and $R^1$ is any of the substituents shown in [Table L1] to [Table L3] (hereinafter, referred to as a compound group SX905).

In the compound (L-3), a compound in which Q is a group represented by Q54, $R^{3b}$ is a bromine atom, $R^{3c}$ is a hydrogen atom, and $R^1$ is any of the substituents shown in [Table L1] to [Table L3] (hereinafter, referred to as a compound group SX906).

In the compound (L-3), a compound in which Q is a group represented by Q54, $R^{3b}$ is an iodine atom, $R^{3c}$ is a hydrogen atom, and $R^1$ is any of the substituents shown in [Table L1] to [Table L3] (hereinafter, referred to as a compound group SX907).

In the compound (L-3), a compound in which Q is a group represented by Q54, $R^{3b}$ is $CF_3$, $R^{3c}$ is a hydrogen atom, and $R^1$ is any of the substituents shown in [Table L1] to [Table L3] (hereinafter, referred to as a compound group SX908).

In the compound (L-3), a compound in which Q is a group represented by Q54, $R^{3b}$ is a hydrogen atom, $R^{3c}$ is a chlorine atom, and $R^1$ is any of the substituents shown in [Table L1] to [Table L3] (hereinafter, referred to as a compound group SX909).

In the compound (L-3), a compound in which Q is a group represented by Q54, $R^{3b}$ is a hydrogen atom, $R^{3c}$ is a bromine atom, and $R^1$ is any of the substituents shown in [Table L1] to [Table L3] (hereinafter, referred to as a compound group SX910).

In the compound (L-3), a compound in which Q is a group represented by Q54, $R^{3b}$ is a hydrogen atom, $R^{3c}$ is an iodine atom, and $R^1$ is any of the substituents shown in [Table L1] to [Table L3] (hereinafter, referred to as a compound group SX911).

In the compound (L-3), a compound in which Q is a group represented by Q54, $R^{3b}$ is a hydrogen atom, $R^{3c}$ is $CF_3$, and $R^1$ is any of the substituents shown in [Table L1] to [Table L3] (hereinafter, referred to as a compound group SX912).

In the compound (L-3), a compound in which Q is a group represented by Q55, $R^{3b}$ and $R^{3c}$ are hydrogen atoms, and $R^1$ is any of the substituents shown in [Table L1] to [Table L3] (hereinafter, referred to as a compound group SX913).

In the compound (L-3), a compound in which Q is a group represented by Q55, $R^{3b}$ is a chlorine atom, $R^{3c}$ is a hydrogen atom, and $R^1$ is any of the substituents shown in [Table L1] to [Table L3] (hereinafter, referred to as a compound group SX914).

In the compound (L-3), a compound in which Q is a group represented by Q55, $R^{3b}$ is a bromine atom, $R^{3c}$ is a hydrogen atom, and $R^1$ is any of the substituents shown in [Table L1] to [Table L3] (hereinafter, referred to as a compound group SX915).

In the compound (L-3), a compound in which Q is a group represented by Q55, $R^{3b}$ is an iodine atom, $R^{3c}$ is a hydrogen atom, and $R^1$ is any of the substituents shown in [Table L1] to [Table L3] (hereinafter, referred to as a compound group SX916).

In the compound (L-3), a compound in which Q is a group represented by Q55, $R^{3b}$ is $CF_3$, $R^{3c}$ is a hydrogen atom, and $R^1$ is any of the substituents shown in [Table L1] to [Table L3] (hereinafter, referred to as a compound group SX917) .

In the compound (L-3), a compound in which Q is a group represented by Q55, $R^{3b}$ is a hydrogen atom, $R^{3c}$ is a chlorine atom, and $R^1$ is any of the substituents shown in [Table L1] to [Table L3] (hereinafter, referred to as a compound group SX918).

In the compound (L-3), a compound in which Q is a group represented by Q55, $R^{3b}$ is a hydrogen atom, $R^{3c}$ is a bromine atom, and $R^1$ is any of the substituents shown in [Table L1] to [Table L3] (hereinafter, referred to as a compound group SX919).

In the compound (L-3), a compound in which Q is a group represented by Q55, $R^{3b}$ is a hydrogen atom, $R^{3c}$ is an iodine atom, and $R^1$ is any of the substituents shown in [Table L1] to [Table L3] (hereinafter, referred to as a compound group SX920).

In the compound (L-3), a compound in which Q is a group represented by Q55, $R^{3b}$ is a hydrogen atom, $R^{3c}$ is $CF_3$, and $R^1$ is any of the substituents shown in [Table L1] to [Table L3] (hereinafter, referred to as a compound group SX921).

In the compound (L-3), a compound in which Q is a group represented by Q56, $R^{3b}$ and $R^{3c}$ are hydrogen atoms, and $R^1$ is any of the substituents shown in [Table L1] to [Table L3] (hereinafter, referred to as a compound group SX922).

In the compound (L-3), a compound in which Q is a group represented by Q56, $R^{3b}$ is a chlorine atom, $R^{3c}$ is a hydrogen atom, and $R^1$ is any of the substituents shown in [Table L1] to [Table L3] (hereinafter, referred to as a compound group SX923).

In the compound (L-3), a compound in which Q is a group represented by Q56, $R^{3b}$ is a bromine atom, $R^{3c}$ is a hydrogen atom, and $R^1$ is any of the substituents shown in [Table L1] to [Table L3] (hereinafter, referred to as a compound group SX924).

In the compound (L-3), a compound in which Q is a group represented by Q56, $R^{3b}$ is an iodine atom, $R^{3c}$ is a hydrogen atom, and $R^1$ is any of the substituents shown in [Table L1] to [Table L3] (hereinafter, referred to as a compound group SX925).

In the compound (L-3), a compound in which Q is a group represented by Q56, $R^{3b}$ is $CF_3$, $R^{3c}$ is a hydrogen atom, and $R^1$ is any of the substituents shown in [Table L1] to [Table L3] (hereinafter, referred to as a compound group SX926) .

In the compound (L-3), a compound in which Q is a group represented by Q56, $R^{3b}$ is a hydrogen atom, $R^{3c}$ is a chlorine atom, and $R^1$ is any of the substituents shown in [Table L1] to [Table L3] (hereinafter, referred to as a compound group SX927).

In the compound (L-3), a compound in which Q is a group represented by Q56, $R^{3b}$ is a hydrogen atom, $R^{3c}$

is a bromine atom, and $R^1$ is any of the substituents shown in [Table L1] to [Table L3] (hereinafter, referred to as a compound group SX928).

In the compound (L-3), a compound in which Q is a group represented by Q56, $R^{3b}$ is a hydrogen atom, $R^{3c}$ is an iodine atom, and $R^1$ is any of the substituents shown in [Table L1] to [Table L3] (hereinafter, referred to as a compound group SX929).

In the compound (L-3), a compound in which Q is a group represented by Q56, $R^{3b}$ is a hydrogen atom, $R^{3c}$ is $CF_3$, and $R^1$ is any of the substituents shown in [Table L1] to [Table L3] (hereinafter, referred to as a compound group SX930).

In the compound (L-3), a compound in which Q is a group represented by Q57, $R^{3b}$ and $R^{3c}$ are hydrogen atoms, and $R^1$ is any of the substituents shown in [Table L1] to [Table L3] (hereinafter, referred to as a compound group SX931).

In the compound (L-3), a compound in which Q is a group represented by Q57, $R^{3b}$ is a chlorine atom, $R^{3c}$ is a hydrogen atom, and $R^1$ is any of the substituents shown in [Table L1] to [Table L3] (hereinafter, referred to as a compound group SX932).

In the compound (L-3), a compound in which Q is a group represented by Q57, $R^{3b}$ is a bromine atom, $R^{3c}$ is a hydrogen atom, and $R^1$ is any of the substituents shown in [Table L1] to [Table L3] (hereinafter, referred to as a compound group SX933).

In the compound (L-3), a compound in which Q is a group represented by Q57, $R^{3b}$ is an iodine atom, $R^{3c}$ is a hydrogen atom, and $R^1$ is any of the substituents shown in [Table L1] to [Table L3] (hereinafter, referred to as a compound group SX934).

In the compound (L-3), a compound in which Q is a group represented by Q57, $R^{3b}$ is $CF_3$, $R^{3c}$ is a hydrogen atom, and $R^1$ is any of the substituents shown in [Table L1] to [Table L3] (hereinafter, referred to as a compound group SX935).

In the compound (L-3), a compound in which Q is a group represented by Q57, $R^{3b}$ is a hydrogen atom, $R^{3c}$ is a chlorine atom, and $R^1$ is any of the substituents shown in [Table L1] to [Table L3] (hereinafter, referred to as a compound group SX936).

In the compound (L-3), a compound in which Q is a group represented by Q57, $R^{3b}$ is a hydrogen atom, $R^{3c}$ is a bromine atom, and $R^1$ is any of the substituents shown in [Table L1] to [Table L3] (hereinafter, referred to as a compound group SX937).

In the compound (L-3), a compound in which Q is a group represented by Q57, $R^{3b}$ is a hydrogen atom, $R^{3c}$ is an iodine atom, and $R^1$ is any of the substituents shown in [Table L1] to [Table L3] (hereinafter, referred to as a compound group SX938).

In the compound (L-3), a compound in which Q is a group represented by Q57, $R^{3b}$ is a hydrogen atom, $R^{3c}$ is $CF_3$, and $R^1$ is any of the substituents shown in [Table L1] to [Table L3] (hereinafter, referred to as a compound group SX939).

[Chem. 94]

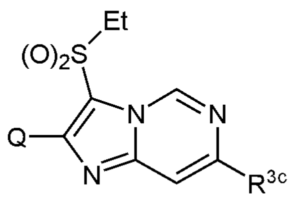

(L-4)

In a compound (hereinafter, referred to as a compound (L-4)) represented by Formula (L-4), a compound in which Q is a group represented by Q11, $R^{3c}$ is a hydrogen atom, and $R^1$ is any of the substituents shown in [Table L1] to [Table L3] (hereinafter, referred to as a compound group SX82).

In the compound (L-4), a compound in which Q is a group represented by Q12, $R^{3b}$ and $R^{3c}$ are hydrogen atoms, and $R^1$ is any of the substituents shown in [Table L1] to [Table L3] (hereinafter, referred to as a compound group SX83).

In the compound (L-4), a compound in which Q is a group represented by Q13, $R^{3b}$ and $R^{3c}$ are hydrogen atoms, and $R^1$ is any of the substituents shown in [Table L1] to [Table L3] (hereinafter, referred to as a compound group SX84).

In the compound (L-4), a compound in which Q is a group represented by Q11, $R^{3b}$ is a chlorine atom, $R^{3c}$ is a hydrogen atom, and $R^1$ is any of the substituents shown in [Table L1] to [Table L3] (hereinafter, referred to as a compound group SX85).

In the compound (L-4), a compound in which Q is a group represented by Q12, $R^{3b}$ is a chlorine atom, $R^{3c}$ is a hydrogen atom, and $R^1$ is any of the substituents shown in [Table L1] to [Table L3] (hereinafter, referred to as a compound group SX86).

In the compound (L-4), a compound in which Q is a group represented by Q13, $R^{3b}$ is a chlorine atom, $R^{3c}$ is a hydrogen atom, and $R^1$ is any of the substituents shown in [Table L1] to [Table L3] (hereinafter, referred to as a compound group SX87).

In the compound (L-4), a compound in which Q is a group represented by Q11, $R^{3b}$ is a bromine atom, $R^{3c}$ is a hydrogen atom, and $R^1$ is any of the substituents shown in [Table L1] to [Table L3] (hereinafter, referred to as a compound group SX88).

In the compound (L-4), a compound in which Q is a group represented by Q12, $R^{3b}$ is a bromine atom, $R^{3c}$ is a hydrogen atom, and $R^1$ is any of the substituents shown in [Table L1] to [Table L3] (hereinafter, referred to as a compound group SX89).

In the compound (L-4), a compound in which Q is a group represented by Q13, $R^{3b}$ is a bromine atom, $R^{3c}$ is a hydrogen atom, and $R^1$ is any of the substituents shown in [Table L1] to [Table L3] (hereinafter, referred to as a compound group SX90).

In the compound (L-4), a compound in which Q is a group represented by Q11, $R^{3b}$ is an iodine atom, $R^{3c}$ is a hydrogen atom, and $R^1$ is any of the substituents shown in [Table L1] to [Table L3] (hereinafter, referred to as a compound group SX91).

In the compound (L-4), a compound in which Q is a group represented by Q12, $R^{3b}$ is an iodine atom, $R^{3c}$ is a hydrogen atom, and $R^1$ is any of the substituents shown in [Table L1] to [Table L3] (hereinafter, referred to as a compound group SX92).

In the compound (L-4), a compound in which Q is a group represented by Q13, $R^{3b}$ is an iodine atom, $R^{3c}$ is a hydrogen atom, and $R^1$ is any of the substituents shown in [Table L1] to [Table L3] (hereinafter, referred to as a compound group SX93).

In the compound (L-4), a compound in which Q is a group represented by Q11, $R^{3b}$ is $CF_3$, $R^{3c}$ is a hydrogen atom, and $R^1$ is any of the substituents shown in [Table L1] to [Table L3] (hereinafter, referred to as a compound group SX94).

In the compound (L-4), a compound in which Q is a group represented by Q12, $R^{3b}$ is $CF_3$, $R^{3c}$ is a hydrogen atom, and $R^1$ is any of the substituents shown in [Table L1] to [Table L3] (hereinafter, referred to as a compound group SX95).

In the compound (L-4), a compound in which Q is a group represented by Q13, $R^{3b}$ is $CF_3$, $R^{3c}$ is a hydrogen atom, and $R^1$ is any of the substituents shown in [Table L1] to [Table L3] (hereinafter, referred to as a compound group SX96).

In the compound (L-4), a compound in which Q is a group represented by Q11, $R^{3b}$ is a hydrogen atom, $R^{3c}$ is a chlorine atom, and $R^1$ is any of the substituents shown in [Table L1] to [Table L3] (hereinafter, referred to as a compound group SX97).

In the compound (L-4), a compound in which Q is a group represented by Q12, $R^{3b}$ is a hydrogen atom, $R^{3c}$ is a chlorine atom, and $R^1$ is any of the substituents shown in [Table L1] to [Table L3] (hereinafter, referred to as a compound group SX98).

In the compound (L-4), a compound in which Q is a group represented by Q13, $R^{3b}$ is a hydrogen atom, $R^{3c}$ is a chlorine atom, and $R^1$ is any of the substituents shown in [Table L1] to [Table L3] (hereinafter, referred to as a compound group SX99).

In the compound (L-4), a compound in which Q is a group represented by Q11, $R^{3b}$ is a hydrogen atom, $R^{3c}$ is a bromine atom, and $R^1$ is any of the substituents shown in [Table L1] to [Table L3] (hereinafter, referred to as a compound group SX100).

In the compound (L-4), a compound in which Q is a group represented by Q12, $R^{3b}$ is a hydrogen atom, $R^{3c}$ is a bromine atom, and $R^1$ is any of the substituents shown in [Table L1] to [Table L3] (hereinafter, referred to as a compound group SX101).

In the compound (L-4), a compound in which Q is a group represented by Q13, $R^{3b}$ is a hydrogen atom, $R^{3c}$ is a bromine atom, and $R^1$ is any of the substituents shown in [Table L1] to [Table L3] (hereinafter, referred to as a compound group SX102).

In the compound (L-4), a compound in which Q is a group represented by Q11, $R^{3b}$ is a hydrogen atom, $R^{3c}$ is an iodine atom, and $R^1$ is any of the substituents shown in [Table L1] to [Table L3] (hereinafter, referred to as a compound group SX103).

In the compound (L-4), a compound in which Q is a group represented by Q12, $R^{3b}$ is a hydrogen atom, $R^{3c}$ is an iodine atom, and $R^1$ is any of the substituents shown in [Table L1] to [Table L3] (hereinafter, referred to as a compound group SX104).

In the compound (L-4), a compound in which Q is a group represented by Q13, $R^{3b}$ is a hydrogen atom, $R^{3c}$

is an iodine atom, and $R^1$ is any of the substituents shown in [Table L1] to [Table L3] (hereinafter, referred to as a compound group SX105).

In the compound (L-4), a compound in which Q is a group represented by Q11, $R^{3b}$ is a hydrogen atom, $R^{3c}$ is $CF_3$, and $R^1$ is any of the substituents shown in [Table L1] to [Table L3] (hereinafter, referred to as a compound group SX106).

In the compound (L-4), a compound in which Q is a group represented by Q12, $R^{3b}$ is a hydrogen atom, $R^{3c}$ is $CF_3$, and $R^1$ is any of the substituents shown in [Table L1] to [Table L3] (hereinafter, referred to as a compound group SX107).

In the compound (L-4), a compound in which Q is a group represented by Q13, $R^{3b}$ is a hydrogen atom, $R^{3c}$ is $CF_3$, and $R^1$ is any of the substituents shown in [Table L1] to [Table L3] (hereinafter, referred to as a compound group SX108).

In the compound (L-4), a compound in which Q is a group represented by Q34, $R^{3b}$ and $R^{3c}$ are hydrogen atoms, and $R^1$ is any of the substituents shown in [Table L1] to [Table L3] (hereinafter, referred to as a compound group SX940).

In the compound (L-4), a compound in which Q is a group represented by Q34, $R^{3b}$ is a chlorine atom, $R^{3c}$ is a hydrogen atom, and $R^1$ is any of the substituents shown in [Table L1] to [Table L3] (hereinafter, referred to as a compound group SX941).

In the compound (L-4), a compound in which Q is a group represented by Q34, $R^{3b}$ is a bromine atom, $R^{3c}$ is a hydrogen atom, and $R^1$ is any of the substituents shown in [Table L1] to [Table L3] (hereinafter, referred to as a compound group SX942).

In the compound (L-4), a compound in which Q is a group represented by Q34, $R^{3b}$ is an iodine atom, $R^{3c}$ is a hydrogen atom, and $R^1$ is any of the substituents shown in [Table L1] to [Table L3] (hereinafter, referred to as a compound group SX943).

In the compound (L-4), a compound in which Q is a group represented by Q34, $R^{3b}$ is $CF_3$, $R^{3c}$ is a hydrogen atom, and $R^1$ is any of the substituents shown in [Table L1] to [Table L3] (hereinafter, referred to as a compound group SX944).

In the compound (L-4), a compound in which Q is a group represented by Q34, $R^{3b}$ is a hydrogen atom, $R^{3c}$ is a chlorine atom, and $R^1$ is any of the substituents shown in [Table L1] to [Table L3] (hereinafter, referred to as a compound group SX945).

In the compound (L-4), a compound in which Q is a group represented by Q34, $R^{3b}$ is a hydrogen atom, $R^{3c}$ is a bromine atom, and $R^1$ is any of the substituents shown in [Table L1] to [Table L3] (hereinafter, referred to as a compound group SX946).

In the compound (L-4), a compound in which Q is a group represented by Q34, $R^{3b}$ is a hydrogen atom, $R^{3c}$ is an iodine atom, and $R^1$ is any of the substituents shown in [Table L1] to [Table L3] (hereinafter, referred to as a compound group SX947).

In the compound (L-4), a compound in which Q is a group represented by Q34, $R^{3b}$ is a hydrogen atom, $R^{3c}$ is $CF_3$, and $R^1$ is any of the substituents shown in [Table L1] to [Table L3] (hereinafter, referred to as a compound group SX948).

In the compound (L-4), a compound in which Q is a group represented by Q35, $R^{3b}$ and $R^{3c}$ are hydrogen atoms, and $R^1$ is any of the substituents shown in [Table L1] to [Table L3] (hereinafter, referred to as a compound group SX949).

In the compound (L-4), a compound in which Q is a group represented by Q35, $R^{3b}$ is a chlorine atom, $R^{3c}$ is a hydrogen atom, and $R^1$ is any of the substituents shown in [Table L1] to [Table L3] (hereinafter, referred to as a compound group SX950).

In the compound (L-4), a compound in which Q is a group represented by Q35, $R^{3b}$ is a bromine atom, $R^{3c}$ is a hydrogen atom, and $R^1$ is any of the substituents shown in [Table L1] to [Table L3] (hereinafter, referred to as a compound group SX951).

In the compound (L-4), a compound in which Q is a group represented by Q35, $R^{3b}$ is an iodine atom, $R^{3c}$ is a hydrogen atom, and $R^1$ is any of the substituents shown in [Table L1] to [Table L3] (hereinafter, referred to as a compound group SX952).

In the compound (L-4), a compound in which Q is a group represented by Q35, $R^{3b}$ is $CF_3$, $R^{3c}$ is a hydrogen atom, and $R^1$ is any of the substituents shown in [Table L1] to [Table L3] (hereinafter, referred to as a compound group SX953).

In the compound (L-4), a compound in which Q is a group represented by Q35, $R^{3b}$ is a hydrogen atom, $R^{3c}$ is a chlorine atom, and $R^1$ is any of the substituents shown in [Table L1] to [Table L3] (hereinafter, referred to as a compound group SX954).

In the compound (L-4), a compound in which Q is a group represented by Q35, $R^{3b}$ is a hydrogen atom, $R^{3c}$ is a bromine atom, and $R^1$ is any of the substituents shown in [Table L1] to [Table L3] (hereinafter, referred to as a

compound group SX955).

In the compound (L-4), a compound in which Q is a group represented by Q35, $R^{3b}$ is a hydrogen atom, $R^{3c}$ is an iodine atom, and $R^1$ is any of the substituents shown in [Table L1] to [Table L3] (hereinafter, referred to as a compound group SX956).

In the compound (L-4), a compound in which Q is a group represented by Q35, $R^{3b}$ is a hydrogen atom, $R^{3c}$ is $CF_3$, and $R^1$ is any of the substituents shown in [Table L1] to [Table L3] (hereinafter, referred to as a compound group SX957).

In the compound (L-4), a compound in which Q is a group represented by Q36, $R^{3b}$ and $R^{3c}$ are hydrogen atoms, and $R^1$ is any of the substituents shown in [Table L1] to [Table L3] (hereinafter, referred to as a compound group SX958).

In the compound (L-4), a compound in which Q is a group represented by Q36, $R^{3b}$ is a chlorine atom, $R^{3c}$ is a hydrogen atom, and $R^1$ is any of the substituents shown in [Table L1] to [Table L3] (hereinafter, referred to as a compound group SX959).

In the compound (L-4), a compound in which Q is a group represented by Q36, $R^{3b}$ is a bromine atom, $R^{3c}$ is a hydrogen atom, and $R^1$ is any of the substituents shown in [Table L1] to [Table L3] (hereinafter, referred to as a compound group SX960).

In the compound (L-4), a compound in which Q is a group represented by Q36, $R^{3b}$ is an iodine atom, $R^{3c}$ is a hydrogen atom, and $R^1$ is any of the substituents shown in [Table L1] to [Table L3] (hereinafter, referred to as a compound group SX961).

In the compound (L-4), a compound in which Q is a group represented by Q36, $R^{3b}$ is $CF_3$, $R^{3c}$ is a hydrogen atom, and $R^1$ is any of the substituents shown in [Table L1] to [Table L3] (hereinafter, referred to as a compound group SX962).

In the compound (L-4), a compound in which Q is a group represented by Q36, $R^{3b}$ is a hydrogen atom, $R^{3c}$ is a chlorine atom, and $R^1$ is any of the substituents shown in [Table L1] to [Table L3] (hereinafter, referred to as a compound group SX963).

In the compound (L-4), a compound in which Q is a group represented by Q36, $R^{3b}$ is a hydrogen atom, $R^{3c}$ is a bromine atom, and $R^1$ is any of the substituents shown in [Table L1] to [Table L3] (hereinafter, referred to as a compound group SX964).

In the compound (L-4), a compound in which Q is a group represented by Q36, $R^{3b}$ is a hydrogen atom, $R^{3c}$ is an iodine atom, and $R^1$ is any of the substituents shown in [Table L1] to [Table L3] (hereinafter, referred to as a compound group SX965).

In the compound (L-4), a compound in which Q is a group represented by Q36, $R^{3b}$ is a hydrogen atom, $R^{3c}$ is $CF_3$, and $R^1$ is any of the substituents shown in [Table L1] to [Table L3] (hereinafter, referred to as a compound group SX966).

In the compound (L-4), a compound in which Q is a group represented by Q37, $R^{3b}$ and $R^{3c}$ are hydrogen atoms, and $R^1$ is any of the substituents shown in [Table L1] to [Table L3] (hereinafter, referred to as a compound group SX967).

In the compound (L-4), a compound in which Q is a group represented by Q37, $R^{3b}$ is a chlorine atom, $R^{3c}$ is a hydrogen atom, and $R^1$ is any of the substituents shown in [Table L1] to [Table L3] (hereinafter, referred to as a compound group SX968).

In the compound (L-4), a compound in which Q is a group represented by Q37, $R^{3b}$ is a bromine atom, $R^{3c}$ is a hydrogen atom, and $R^1$ is any of the substituents shown in [Table L1] to [Table L3] (hereinafter, referred to as a compound group SX969).

In the compound (L-4), a compound in which Q is a group represented by Q37, $R^{3b}$ is an iodine atom, $R^{3c}$ is a hydrogen atom, and $R^1$ is any of the substituents shown in [Table L1] to [Table L3] (hereinafter, referred to as a compound group SX970).

In the compound (L-4), a compound in which Q is a group represented by Q37, $R^{3b}$ is $CF_3$, $R^{3c}$ is a hydrogen atom, and $R^1$ is any of the substituents shown in [Table L1] to [Table L3] (hereinafter, referred to as a compound group SX971).

In the compound (L-4), a compound in which Q is a group represented by Q37, $R^{3b}$ is a hydrogen atom, $R^{3c}$ is a chlorine atom, and $R^1$ is any of the substituents shown in [Table L1] to [Table L3] (hereinafter, referred to as a compound group SX972).

In the compound (L-4), a compound in which Q is a group represented by Q37, $R^{3b}$ is a hydrogen atom, $R^{3c}$ is a bromine atom, and $R^1$ is any of the substituents shown in [Table L1] to [Table L3] (hereinafter, referred to as a compound group SX973).

In the compound (L-4), a compound in which Q is a group represented by Q37, $R^{3b}$ is a hydrogen atom, $R^{3c}$ is an iodine atom, and $R^1$ is any of the substituents shown in [Table L1] to [Table L3] (hereinafter, referred to as a compound group SX974).

In the compound (L-4), a compound in which Q is a group represented by Q37, $R^{3b}$ is a hydrogen atom, $R^{3c}$ is $CF_3$, and $R^1$ is any of the substituents shown in [Table L1] to [Table L3] (hereinafter, referred to as a compound group SX975).

In the compound (L-4), a compound in which Q is a group represented by Q38, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX976).

In the compound (L-4), a compound in which Q is a group represented by Q38, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX977).

In the compound (L-4), a compound in which Q is a group represented by Q38, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX978).

In the compound (L-4), a compound in which Q is a group represented by Q38, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX979).

In the compound (L-4), a compound in which Q is a group represented by Q38, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX980).

In the compound (L-4), a compound in which Q is a group represented by Q38, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX981).

In the compound (L-4), a compound in which Q is a group represented by Q38, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX982).

In the compound (L-4), a compound in which Q is a group represented by Q38, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX983).

In the compound (L-4), a compound in which Q is a group represented by Q38, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX984).

In the compound (L-4), a compound in which Q is a group represented by Q38, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX985).

In the compound (L-4), a compound in which Q is a group represented by Q38, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX986).

In the compound (L-4), a compound in which Q is a group represented by Q38, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX987).

In the compound (L-4), a compound in which Q is a group represented by Q38, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX988).

In the compound (L-4), a compound in which Q is a group represented by Q38, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX989).

In the compound (L-4), a compound in which Q is a group represented by Q38, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX990).

In the compound (L-4), a compound in which Q is a group represented by Q38, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX991).

In the compound (L-4), a compound in which Q is a group represented by Q38, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX992).

In the compound (L-4), a compound in which Q is a group represented by Q38, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX993).

In the compound (L-4), a compound in which Q is a group represented by Q39, $R^{3b}$ and $R^{3c}$ are hydrogen

atoms, $R^{6a}$ is a hydrogen atom, and $R^1$ is any of the substituents shown in [Table L1] to [Table L3] (hereinafter, referred to as a compound group SX994).

In the compound (L-4), a compound in which Q is a group represented by Q39, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX995).

In the compound (L-4), a compound in which Q is a group represented by Q39, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX996).

In the compound (L-4), a compound in which Q is a group represented by Q39, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX997).

In the compound (L-4), a compound in which Q is a group represented by Q39, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX998).

In the compound (L-4), a compound in which Q is a group represented by Q39, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX999).

In the compound (L-4), a compound in which Q is a group represented by Q39, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX1000).

In the compound (L-4), a compound in which Q is a group represented by Q39, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX1001).

In the compound (L-4), a compound in which Q is a group represented by Q39, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX1002).

In the compound (L-4), a compound in which Q is a group represented by Q39, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX1003).

In the compound (L-4), a compound in which Q is a group represented by Q39, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX1004).

In the compound (L-4), a compound in which Q is a group represented by Q39, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX1005).

In the compound (L-4), a compound in which Q is a group represented by Q39, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX1006).

In the compound (L-4), a compound in which Q is a group represented by Q39, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX1007).

In the compound (L-4), a compound in which Q is a group represented by Q39, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX1008).

In the compound (L-4), a compound in which Q is a group represented by Q39, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX1009).

In the compound (L-4), a compound in which Q is a group represented by Q39, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX1010).

In the compound (L-4), a compound in which Q is a group represented by Q39, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX1011).

In the compound (L-4), a compound in which Q is a group represented by Q40, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX1012).

In the compound (L-4), a compound in which Q is a group represented by Q40, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter,

referred to as a compound group SX1013).

In the compound (L-4), a compound in which Q is a group represented by Q40, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1014).

In the compound (L-4), a compound in which Q is a group represented by Q40, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1015).

In the compound (L-4), a compound in which Q is a group represented by Q40, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX1016).

In the compound (L-4), a compound in which Q is a group represented by Q40, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX1017).

In the compound (L-4), a compound in which Q is a group represented by Q40, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX1018).

In the compound (L-4), a compound in which Q is a group represented by Q40, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX1019).

In the compound (L-4), a compound in which Q is a group represented by Q40, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX1020).

In the compound (L-4), a compound in which Q is a group represented by Q40, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX1021).

In the compound (L-4), a compound in which Q is a group represented by Q40, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX1022).

In the compound (L-4), a compound in which Q is a group represented by Q40, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX1023).

In the compound (L-4), a compound in which Q is a group represented by Q40, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX1024).

In the compound (L-4), a compound in which Q is a group represented by Q40, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX1025).

In the compound (L-4), a compound in which Q is a group represented by Q40, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX1026).

In the compound (L-4), a compound in which Q is a group represented by Q40, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX1027).

In the compound (L-4), a compound in which Q is a group represented by Q40, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX1028).

In the compound (L-4), a compound in which Q is a group represented by Q40, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX1029).

In the compound (L-4), a compound in which Q is a group represented by Q41, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX1030).

In the compound (L-4), a compound in which Q is a group represented by Q41, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1031).

In the compound (L-4), a compound in which Q is a group represented by Q41, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1032).

In the compound (L-4), a compound in which Q is a group represented by Q41, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1033).

In the compound (L-4), a compound in which Q is a group represented by Q41, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX1034).

In the compound (L-4), a compound in which Q is a group represented by Q41, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX1035).

In the compound (L-4), a compound in which Q is a group represented by Q41, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX1036).

In the compound (L-4), a compound in which Q is a group represented by Q41, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX1037).

In the compound (L-4), a compound in which Q is a group represented by Q41, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX1038).

In the compound (L-4), a compound in which Q is a group represented by Q41, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX1039).

In the compound (L-4), a compound in which Q is a group represented by Q41, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX1040).

In the compound (L-4), a compound in which Q is a group represented by Q41, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX1041).

In the compound (L-4), a compound in which Q is a group represented by Q41, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX1042).

In the compound (L-4), a compound in which Q is a group represented by Q41, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX1043).

In the compound (L-4), a compound in which Q is a group represented by Q41, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX1044).

In the compound (L-4), a compound in which Q is a group represented by Q41, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX1045).

In the compound (L-4), a compound in which Q is a group represented by Q41, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX1046).

In the compound (L-4), a compound in which Q is a group represented by Q41, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX1047).

In the compound (L-4), a compound in which Q is a group represented by Q42, $R^{3b}$ and $R^{3c}$ are hydrogen atoms, and $R^1$ is any of the substituents shown in [Table L1] to [Table L3] (hereinafter, referred to as a compound group SX1048).

In the compound (L-4), a compound in which Q is a group represented by Q42, $R^{3b}$ is a chlorine atom, $R^{3c}$ is a hydrogen atom, and $R^1$ is any of the substituents shown in [Table L1] to [Table L3] (hereinafter, referred to as a compound group SX1049).

In the compound (L-4), a compound in which Q is a group represented by Q42, $R^{3b}$ is a bromine atom, $R^{3c}$ is a hydrogen atom, and $R^1$ is any of the substituents shown in [Table L1] to [Table L3] (hereinafter, referred to as a compound group SX1050).

In the compound (L-4), a compound in which Q is a group represented by Q42, $R^{3b}$ is an iodine atom, $R^{3c}$ is a hydrogen atom, and $R^1$ is any of the substituents shown in [Table L1] to [Table L3] (hereinafter, referred to as a compound group SX1051).

In the compound (L-4), a compound in which Q is a group represented by Q42, $R^{3b}$ is $CF_3$, $R^{3c}$ is a hydrogen

atom, and $R^1$ is any of the substituents shown in [Table L1] to [Table L3] (hereinafter, referred to as a compound group SX1052).

In the compound (L-4), a compound in which Q is a group represented by Q42, $R^{3b}$ is a hydrogen atom, $R^{3c}$ is a chlorine atom, and $R^1$ is any of the substituents shown in [Table L1] to [Table L3] (hereinafter, referred to as a compound group SX1053).

In the compound (L-4), a compound in which Q is a group represented by Q42, $R^{3b}$ is a hydrogen atom, $R^{3c}$ is a bromine atom, and $R^1$ is any of the substituents shown in [Table L1] to [Table L3] (hereinafter, referred to as a compound group SX1054).

In the compound (L-4), a compound in which Q is a group represented by Q42, $R^{3b}$ is a hydrogen atom, $R^{3c}$ is an iodine atom, and $R^1$ is any of the substituents shown in [Table L1] to [Table L3] (hereinafter, referred to as a compound group SX1055).

In the compound (L-4), a compound in which Q is a group represented by Q42, $R^{3b}$ is a hydrogen atom, $R^{3c}$ is $CF_3$, and $R^1$ is any of the substituents shown in [Table L1] to [Table L3] (hereinafter, referred to as a compound group SX1056).

In the compound (L-4), a compound in which Q is a group represented by Q43, $R^{3b}$ and $R^{3c}$ are hydrogen atoms, and $R^1$ is any of the substituents shown in [Table L1] to [Table L3] (hereinafter, referred to as a compound group SX1057).

In the compound (L-4), a compound in which Q is a group represented by Q43, $R^{3b}$ is a chlorine atom, $R^{3c}$ is a hydrogen atom, and $R^1$ is any of the substituents shown in [Table L1] to [Table L3] (hereinafter, referred to as a compound group SX1058).

In the compound (L-4), a compound in which Q is a group represented by Q43, $R^{3b}$ is a bromine atom, $R^{3c}$ is a hydrogen atom, and $R^1$ is any of the substituents shown in [Table L1] to [Table L3] (hereinafter, referred to as a compound group SX1059).

In the compound (L-4), a compound in which Q is a group represented by Q43, $R^{3b}$ is an iodine atom, $R^{3c}$ is a hydrogen atom, and $R^1$ is any of the substituents shown in [Table L1] to [Table L3] (hereinafter, referred to as a compound group SX1060).

In the compound (L-4), a compound in which Q is a group represented by Q43, $R^{3b}$ is $CF_3$, $R^{3c}$ is a hydrogen atom, and $R^1$ is any of the substituents shown in [Table L1] to [Table L3] (hereinafter, referred to as a compound group SX1061).

In the compound (L-4), a compound in which Q is a group represented by Q43, $R^{3b}$ is a hydrogen atom, $R^{3c}$ is a chlorine atom, and $R^1$ is any of the substituents shown in [Table L1] to [Table L3] (hereinafter, referred to as a compound group SX1062).

In the compound (L-4), a compound in which Q is a group represented by Q43, $R^{3b}$ is a hydrogen atom, $R^{3c}$ is a bromine atom, and $R^1$ is any of the substituents shown in [Table L1] to [Table L3] (hereinafter, referred to as a compound group SX1063).

In the compound (L-4), a compound in which Q is a group represented by Q43, $R^{3b}$ is a hydrogen atom, $R^{3c}$ is an iodine atom, and $R^1$ is any of the substituents shown in [Table L1] to [Table L3] (hereinafter, referred to as a compound group SX1064).

In the compound (L-4), a compound in which Q is a group represented by Q43, $R^{3b}$ is a hydrogen atom, $R^{3c}$ is $CF_3$, and $R^1$ is any of the substituents shown in [Table L1] to [Table L3] (hereinafter, referred to as a compound group SX1065).

In the compound (L-4), a compound in which Q is a group represented by Q44, $R^{3b}$ and $R^{3c}$ are hydrogen atoms, and $R^1$ is any of the substituents shown in [Table L1] to [Table L3] (hereinafter, referred to as a compound group SX1066).

In the compound (L-4), a compound in which Q is a group represented by Q44, $R^{3b}$ is a chlorine atom, $R^{3c}$ is a hydrogen atom, and $R^1$ is any of the substituents shown in [Table L1] to [Table L3] (hereinafter, referred to as a compound group SX1067).

In the compound (L-4), a compound in which Q is a group represented by Q44, $R^{3b}$ is a bromine atom, $R^{3c}$ is a hydrogen atom, and $R^1$ is any of the substituents shown in [Table L1] to [Table L3] (hereinafter, referred to as a compound group SX1068).

In the compound (L-4), a compound in which Q is a group represented by Q44, $R^{3b}$ is an iodine atom, $R^{3c}$ is a hydrogen atom, and $R^1$ is any of the substituents shown in [Table L1] to [Table L3] (hereinafter, referred to as a compound group SX1069).

In the compound (L-4), a compound in which Q is a group represented by Q44, $R^{3b}$ is $CF_3$, $R^{3c}$ is a hydrogen atom, and $R^1$ is any of the substituents shown in [Table L1] to [Table L3] (hereinafter, referred to as a compound group SX1070).

In the compound (L-4), a compound in which Q is a group represented by Q44, $R^{3b}$ is a hydrogen atom, $R^{3c}$ is a chlorine atom, and $R^1$ is any of the substituents shown in [Table L1] to [Table L3] (hereinafter, referred to as a

compound group SX1071).

In the compound (L-4), a compound in which Q is a group represented by Q44, $R^{3b}$ is a hydrogen atom, $R^{3c}$ is a bromine atom, and $R^1$ is any of the substituents shown in [Table L1] to [Table L3] (hereinafter, referred to as a compound group SX1072).

In the compound (L-4), a compound in which Q is a group represented by Q44, $R^{3b}$ is a hydrogen atom, $R^{3c}$ is an iodine atom, and $R^1$ is any of the substituents shown in [Table L1] to [Table L3] (hereinafter, referred to as a compound group SX1073).

In the compound (L-4), a compound in which Q is a group represented by Q44, $R^{3b}$ is a hydrogen atom, $R^{3c}$ is $CF_3$, and $R^1$ is any of the substituents shown in [Table L1] to [Table L3] (hereinafter, referred to as a compound group SX1074).

In the compound (L-4), a compound in which Q is a group represented by Q45, $R^{3b}$ and $R^{3c}$ are hydrogen atoms, and $R^1$ is any of the substituents shown in [Table L1] to [Table L3] (hereinafter, referred to as a compound group SX1075).

In the compound (L-4), a compound in which Q is a group represented by Q45, $R^{3b}$ is a chlorine atom, $R^{3c}$ is a hydrogen atom, and $R^1$ is any of the substituents shown in [Table L1] to [Table L3] (hereinafter, referred to as a compound group SX1076).

In the compound (L-4), a compound in which Q is a group represented by Q45, $R^{3b}$ is a bromine atom, $R^{3c}$ is a hydrogen atom, and $R^1$ is any of the substituents shown in [Table L1] to [Table L3] (hereinafter, referred to as a compound group SX1077).

In the compound (L-4), a compound in which Q is a group represented by Q45, $R^{3b}$ is an iodine atom, $R^{3c}$ is a hydrogen atom, and $R^1$ is any of the substituents shown in [Table L1] to [Table L3] (hereinafter, referred to as a compound group SX1078).

In the compound (L-4), a compound in which Q is a group represented by Q45, $R^{3b}$ is $CF_3$, $R^{3c}$ is a hydrogen atom, and $R^1$ is any of the substituents shown in [Table L1] to [Table L3] (hereinafter, referred to as a compound group SX1079).

In the compound (L-4), a compound in which Q is a group represented by Q45, $R^{3b}$ is a hydrogen atom, $R^{3c}$ is a chlorine atom, and $R^1$ is any of the substituents shown in [Table L1] to [Table L3] (hereinafter, referred to as a compound group SX1080).

In the compound (L-4), a compound in which Q is a group represented by Q45, $R^{3b}$ is a hydrogen atom, $R^{3c}$ is a bromine atom, and $R^1$ is any of the substituents shown in [Table L1] to [Table L3] (hereinafter, referred to as a compound group SX1081).

In the compound (L-4), a compound in which Q is a group represented by Q45, $R^{3b}$ is a hydrogen atom, $R^{3c}$ is an iodine atom, and $R^1$ is any of the substituents shown in [Table L1] to [Table L3] (hereinafter, referred to as a compound group SX1082).

In the compound (L-4), a compound in which Q is a group represented by Q45, $R^{3b}$ is a hydrogen atom, $R^{3c}$ is $CF_3$, and $R^1$ is any of the substituents shown in [Table L1] to [Table L3] (hereinafter, referred to as a compound group SX1083).

In the compound (L-4), a compound in which Q is a group represented by Q46, $R^{3b}$ and $R^{3c}$ are hydrogen atoms, and $R^1$ is any of the substituents shown in [Table L1] to [Table L3] (hereinafter, referred to as a compound group SX1084).

In the compound (L-4), a compound in which Q is a group represented by Q46, $R^{3b}$ is a chlorine atom, $R^{3c}$ is a hydrogen atom, and $R^1$ is any of the substituents shown in [Table L1] to [Table L3] (hereinafter, referred to as a compound group SX1085).

In the compound (L-4), a compound in which Q is a group represented by Q46, $R^{3b}$ is a bromine atom, $R^{3c}$ is a hydrogen atom, and $R^1$ is any of the substituents shown in [Table L1] to [Table L3] (hereinafter, referred to as a compound group SX1086).

In the compound (L-4), a compound in which Q is a group represented by Q46, $R^{3b}$ is an iodine atom, $R^{3c}$ is a hydrogen atom, and $R^1$ is any of the substituents shown in [Table L1] to [Table L3] (hereinafter, referred to as a compound group SX1087).

In the compound (L-4), a compound in which Q is a group represented by Q46, $R^{3b}$ is $CF_3$, $R^{3c}$ is a hydrogen atom, and $R^1$ is any of the substituents shown in [Table L1] to [Table L3] (hereinafter, referred to as a compound group SX1088).

In the compound (L-4), a compound in which Q is a group represented by Q46, $R^{3b}$ is a hydrogen atom, $R^{3c}$ is a chlorine atom, and $R^1$ is any of the substituents shown in [Table L1] to [Table L3] (hereinafter, referred to as a compound group SX1089).

In the compound (L-4), a compound in which Q is a group represented by Q46, $R^{3b}$ is a hydrogen atom, $R^{3c}$ is a bromine atom, and $R^1$ is any of the substituents shown in [Table L1] to [Table L3] (hereinafter, referred to as a compound group SX1090).

In the compound (L-4), a compound in which Q is a group represented by Q46, $R^{3b}$ is a hydrogen atom, $R^{3c}$ is an iodine atom, and $R^1$ is any of the substituents shown in [Table L1] to [Table L3] (hereinafter, referred to as a compound group SX1091).

In the compound (L-4), a compound in which Q is a group represented by Q46, $R^{3b}$ is a hydrogen atom, $R^{3c}$ is $CF_3$, and $R^1$ is any of the substituents shown in [Table L1] to [Table L3] (hereinafter, referred to as a compound group SX1092).

In the compound (L-4), a compound in which Q is a group represented by Q47, $R^{3b}$ and $R^{3c}$ are hydrogen atoms, and $R^1$ is any of the substituents shown in [Table L1] to [Table L3] (hereinafter, referred to as a compound group SX1093).

In the compound (L-4), a compound in which Q is a group represented by Q47, $R^{3b}$ is a chlorine atom, $R^{3c}$ is a hydrogen atom, and $R^1$ is any of the substituents shown in [Table L1] to [Table L3] (hereinafter, referred to as a compound group SX1094).

In the compound (L-4), a compound in which Q is a group represented by Q47, $R^{3b}$ is a bromine atom, $R^{3c}$ is a hydrogen atom, and $R^1$ is any of the substituents shown in [Table L1] to [Table L3] (hereinafter, referred to as a compound group SX1095).

In the compound (L-4), a compound in which Q is a group represented by Q47, $R^{3b}$ is an iodine atom, $R^{3c}$ is a hydrogen atom, and $R^1$ is any of the substituents shown in [Table L1] to [Table L3] (hereinafter, referred to as a compound group SX1096).

In the compound (L-4), a compound in which Q is a group represented by Q47, $R^{3b}$ is $CF_3$, $R^{3c}$ is a hydrogen atom, and $R^1$ is any of the substituents shown in [Table L1] to [Table L3] (hereinafter, referred to as a compound group SX1097).

In the compound (L-4), a compound in which Q is a group represented by Q47, $R^{3b}$ is a hydrogen atom, $R^{3c}$ is a chlorine atom, and $R^1$ is any of the substituents shown in [Table L1] to [Table L3] (hereinafter, referred to as a compound group SX1098).

In the compound (L-4), a compound in which Q is a group represented by Q47, $R^{3b}$ is a hydrogen atom, $R^{3c}$ is a bromine atom, and $R^1$ is any of the substituents shown in [Table L1] to [Table L3] (hereinafter, referred to as a compound group SX1099).

In the compound (L-4), a compound in which Q is a group represented by Q47, $R^{3b}$ is a hydrogen atom, $R^{3c}$ is an iodine atom, and $R^1$ is any of the substituents shown in [Table L1] to [Table L3] (hereinafter, referred to as a compound group SX1100).

In the compound (L-4), a compound in which Q is a group represented by Q47, $R^{3b}$ is a hydrogen atom, $R^{3c}$ is $CF_3$, and $R^1$ is any of the substituents shown in [Table L1] to [Table L3] (hereinafter, referred to as a compound group SX1101).

In the compound (L-4), a compound in which Q is a group represented by Q48, $R^{3b}$ and $R^{3c}$ are hydrogen atoms, and $R^1$ is any of the substituents shown in [Table L1] to [Table L3] (hereinafter, referred to as a compound group SX1102).

In the compound (L-4), a compound in which Q is a group represented by Q48, $R^{3b}$ is a chlorine atom, $R^{3c}$ is a hydrogen atom, and $R^1$ is any of the substituents shown in [Table L1] to [Table L3] (hereinafter, referred to as a compound group SX1103).

In the compound (L-4), a compound in which Q is a group represented by Q48, $R^{3b}$ is a bromine atom, $R^{3c}$ is a hydrogen atom, and $R^1$ is any of the substituents shown in [Table L1] to [Table L3] (hereinafter, referred to as a compound group SX1104).

In the compound (L-4), a compound in which Q is a group represented by Q48, $R^{3b}$ is an iodine atom, $R^{3c}$ is a hydrogen atom, and $R^1$ is any of the substituents shown in [Table L1] to [Table L3] (hereinafter, referred to as a compound group SX1105).

In the compound (L-4), a compound in which Q is a group represented by Q48, $R^{3b}$ is $CF_3$, $R^{3c}$ is a hydrogen atom, and $R^1$ is any of the substituents shown in [Table L1] to [Table L3] (hereinafter, referred to as a compound group SX1106).

In the compound (L-4), a compound in which Q is a group represented by Q48, $R^{3b}$ is a hydrogen atom, $R^{3c}$ is a chlorine atom, and $R^1$ is any of the substituents shown in [Table L1] to [Table L3] (hereinafter, referred to as a compound group SX1107).

In the compound (L-4), a compound in which Q is a group represented by Q48, $R^{3b}$ is a hydrogen atom, $R^{3c}$ is a bromine atom, and $R^1$ is any of the substituents shown in [Table L1] to [Table L3] (hereinafter, referred to as a compound group SX1108).

In the compound (L-4), a compound in which Q is a group represented by Q48, $R^{3b}$ is a hydrogen atom, $R^{3c}$ is an iodine atom, and $R^1$ is any of the substituents shown in [Table L1] to [Table L3] (hereinafter, referred to as a compound group SX1109).

In the compound (L-4), a compound in which Q is a group represented by Q48, $R^{3b}$ is a hydrogen atom, $R^{3c}$

is $CF_3$, and $R^1$ is any of the substituents shown in [Table L1] to [Table L3] (hereinafter, referred to as a compound group SX1110).

In the compound (L-4), a compound in which Q is a group represented by Q49, $R^{3b}$ and $R^{3c}$ are hydrogen atoms, and $R^1$ is any of the substituents shown in [Table L1] to [Table L3] (hereinafter, referred to as a compound group SX1111).

In the compound (L-4), a compound in which Q is a group represented by Q49, $R^{3b}$ is a chlorine atom, $R^{3c}$ is a hydrogen atom, and $R^1$ is any of the substituents shown in [Table L1] to [Table L3] (hereinafter, referred to as a compound group SX1112).

In the compound (L-4), a compound in which Q is a group represented by Q49, $R^{3b}$ is a bromine atom, $R^{3c}$ is a hydrogen atom, and $R^1$ is any of the substituents shown in [Table L1] to [Table L3] (hereinafter, referred to as a compound group SX1113).

In the compound (L-4), a compound in which Q is a group represented by Q49, $R^{3b}$ is an iodine atom, $R^{3c}$ is a hydrogen atom, and $R^1$ is any of the substituents shown in [Table L1] to [Table L3] (hereinafter, referred to as a compound group SX1114).

In the compound (L-4), a compound in which Q is a group represented by Q49, $R^{3b}$ is $CF_3$, $R^{3c}$ is a hydrogen atom, and $R^1$ is any of the substituents shown in [Table L1] to [Table L3] (hereinafter, referred to as a compound group SX1115).

In the compound (L-4), a compound in which Q is a group represented by Q49, $R^{3b}$ is a hydrogen atom, $R^{3c}$ is a chlorine atom, and $R^1$ is any of the substituents shown in [Table L1] to [Table L3] (hereinafter, referred to as a compound group SX1116).

In the compound (L-4), a compound in which Q is a group represented by Q49, $R^{3b}$ is a hydrogen atom, $R^{3c}$ is a bromine atom, and $R^1$ is any of the substituents shown in [Table L1] to [Table L3] (hereinafter, referred to as a compound group SX1117).

In the compound (L-4), a compound in which Q is a group represented by Q49, $R^{3b}$ is a hydrogen atom, $R^{3c}$ is an iodine atom, and $R^1$ is any of the substituents shown in [Table L1] to [Table L3] (hereinafter, referred to as a compound group SX1118).

In the compound (L-4), a compound in which Q is a group represented by Q49, $R^{3b}$ is a hydrogen atom, $R^{3c}$ is $CF_3$, and $R^1$ is any of the substituents shown in [Table L1] to [Table L3] (hereinafter, referred to as a compound group SX1119).

In the compound (L-4), a compound in which Q is a group represented by Q50, $R^{3b}$ and $R^{3c}$ are hydrogen atoms, and $R^1$ is any of the substituents shown in [Table L1] to [Table L3] (hereinafter, referred to as a compound group SX1120).

In the compound (L-4), a compound in which Q is a group represented by Q50, $R^{3b}$ is a chlorine atom, $R^{3c}$ is a hydrogen atom, and $R^1$ is any of the substituents shown in [Table L1] to [Table L3] (hereinafter, referred to as a compound group SX1121).

In the compound (L-4), a compound in which Q is a group represented by Q50, $R^{3b}$ is a bromine atom, $R^{3c}$ is a hydrogen atom, and $R^1$ is any of the substituents shown in [Table L1] to [Table L3] (hereinafter, referred to as a compound group SX1122).

In the compound (L-4), a compound in which Q is a group represented by Q50, $R^{3b}$ is an iodine atom, $R^{3c}$ is a hydrogen atom, and $R^1$ is any of the substituents shown in [Table L1] to [Table L3] (hereinafter, referred to as a compound group SX1123).

In the compound (L-4), a compound in which Q is a group represented by Q50, $R^{3b}$ is $CF_3$, $R^{3c}$ is a hydrogen atom, and $R^1$ is any of the substituents shown in [Table L1] to [Table L3] (hereinafter, referred to as a compound group SX1124).

In the compound (L-4), a compound in which Q is a group represented by Q50, $R^{3b}$ is a hydrogen atom, $R^{3c}$ is a chlorine atom, and $R^1$ is any of the substituents shown in [Table L1] to [Table L3] (hereinafter, referred to as a compound group SX1125).

In the compound (L-4), a compound in which Q is a group represented by Q50, $R^{3b}$ is a hydrogen atom, $R^{3c}$ is a bromine atom, and $R^1$ is any of the substituents shown in [Table L1] to [Table L3] (hereinafter, referred to as a compound group SX1126).

In the compound (L-4), a compound in which Q is a group represented by Q50, $R^{3b}$ is a hydrogen atom, $R^{3c}$ is an iodine atom, and $R^1$ is any of the substituents shown in [Table L1] to [Table L3] (hereinafter, referred to as a compound group SX1127).

In the compound (L-4), a compound in which Q is a group represented by Q50, $R^{3b}$ is a hydrogen atom, $R^{3c}$ is $CF_3$, and $R^1$ is any of the substituents shown in [Table L1] to [Table L3] (hereinafter, referred to as a compound group SX1128).

In the compound (L-4), a compound in which Q is a group represented by Q51, $R^{3b}$ and $R^{3c}$ are hydrogen atoms, and $R^1$ is any of the substituents shown in [Table L1] to [Table L3] (hereinafter, referred to as a compound group

SX1129).

In the compound (L-4), a compound in which Q is a group represented by Q51, $R^{3b}$ is a chlorine atom, $R^{3c}$ is a hydrogen atom, and $R^1$ is any of the substituents shown in [Table L1] to [Table L3] (hereinafter, referred to as a compound group SX1130).

In the compound (L-4), a compound in which Q is a group represented by Q51, $R^{3b}$ is a bromine atom, $R^{3c}$ is a hydrogen atom, and $R^1$ is any of the substituents shown in [Table L1] to [Table L3] (hereinafter, referred to as a compound group SX1131).

In the compound (L-4), a compound in which Q is a group represented by Q51, $R^{3b}$ is an iodine atom, $R^{3c}$ is a hydrogen atom, and $R^1$ is any of the substituents shown in [Table L1] to [Table L3] (hereinafter, referred to as a compound group SX1132).

In the compound (L-4), a compound in which Q is a group represented by Q51, $R^{3b}$ is $CF_3$, $R^{3c}$ is a hydrogen atom, and $R^1$ is any of the substituents shown in [Table L1] to [Table L3] (hereinafter, referred to as a compound group SX1133).

In the compound (L-4), a compound in which Q is a group represented by Q51, $R^{3b}$ is a hydrogen atom, $R^{3c}$ is a chlorine atom, and $R^1$ is any of the substituents shown in [Table L1] to [Table L3] (hereinafter, referred to as a compound group SX1134).

In the compound (L-4), a compound in which Q is a group represented by Q51, $R^{3b}$ is a hydrogen atom, $R^{3c}$ is a bromine atom, and $R^1$ is any of the substituents shown in [Table L1] to [Table L3] (hereinafter, referred to as a compound group SX1135).

In the compound (L-4), a compound in which Q is a group represented by Q51, $R^{3b}$ is a hydrogen atom, $R^{3c}$ is an iodine atom, and $R^1$ is any of the substituents shown in [Table L1] to [Table L3] (hereinafter, referred to as a compound group SX1136).

In the compound (L-4), a compound in which Q is a group represented by Q51, $R^{3b}$ is a hydrogen atom, $R^{3c}$ is $CF_3$, and $R^1$ is any of the substituents shown in [Table L1] to [Table L3] (hereinafter, referred to as a compound group SX1137).

In the compound (L-4), a compound in which Q is a group represented by Q52, $R^{3b}$ and $R^{3c}$ are hydrogen atoms, and $R^1$ is any of the substituents shown in [Table L1] to [Table L3] (hereinafter, referred to as a compound group SX1138).

In the compound (L-4), a compound in which Q is a group represented by Q52, $R^{3b}$ is a chlorine atom, $R^{3c}$ is a hydrogen atom, and $R^1$ is any of the substituents shown in [Table L1] to [Table L3] (hereinafter, referred to as a compound group SX1139).

In the compound (L-4), a compound in which Q is a group represented by Q52, $R^{3b}$ is a bromine atom, $R^{3c}$ is a hydrogen atom, and $R^1$ is any of the substituents shown in [Table L1] to [Table L3] (hereinafter, referred to as a compound group SX1140).

In the compound (L-4), a compound in which Q is a group represented by Q52, $R^{3b}$ is an iodine atom, $R^{3c}$ is a hydrogen atom, and $R^1$ is any of the substituents shown in [Table L1] to [Table L3] (hereinafter, referred to as a compound group SX1141).

In the compound (L-4), a compound in which Q is a group represented by Q52, $R^{3b}$ is $CF_3$, $R^{3c}$ is a hydrogen atom, and $R^1$ is any of the substituents shown in [Table L1] to [Table L3] (hereinafter, referred to as a compound group SX1142).

In the compound (L-4), a compound in which Q is a group represented by Q52, $R^{3b}$ is a hydrogen atom, $R^{3c}$ is a chlorine atom, and $R^1$ is any of the substituents shown in [Table L1] to [Table L3] (hereinafter, referred to as a compound group SX1143).

In the compound (L-4), a compound in which Q is a group represented by Q52, $R^{3b}$ is a hydrogen atom, $R^{3c}$ is a bromine atom, and $R^1$ is any of the substituents shown in [Table L1] to [Table L3] (hereinafter, referred to as a compound group SX1144).

In the compound (L-4), a compound in which Q is a group represented by Q52, $R^{3b}$ is a hydrogen atom, $R^{3c}$ is an iodine atom, and $R^1$ is any of the substituents shown in [Table L1] to [Table L3] (hereinafter, referred to as a compound group SX1145).

In the compound (L-4), a compound in which Q is a group represented by Q52, $R^{3b}$ is a hydrogen atom, $R^{3c}$ is $CF_3$, and $R^1$ is any of the substituents shown in [Table L1] to [Table L3] (hereinafter, referred to as a compound group SX1146).

In the compound (L-4), a compound in which Q is a group represented by Q53, $R^{3b}$ and $R^{3c}$ are hydrogen atoms, and $R^1$ is any of the substituents shown in [Table L1] to [Table L3] (hereinafter, referred to as a compound group SX1147).

In the compound (L-4), a compound in which Q is a group represented by Q53, $R^{3b}$ is a chlorine atom, $R^{3c}$ is a hydrogen atom, and $R^1$ is any of the substituents shown in [Table L1] to [Table L3] (hereinafter, referred to as a compound group SX1148).

In the compound (L-4), a compound in which Q is a group represented by Q53, $R^{3b}$ is a bromine atom, $R^{3c}$ is a hydrogen atom, and $R^1$ is any of the substituents shown in [Table L1] to [Table L3] (hereinafter, referred to as a compound group SX1149).

In the compound (L-4), a compound in which Q is a group represented by Q53, $R^{3b}$ is an iodine atom, $R^{3c}$ is a hydrogen atom, and $R^1$ is any of the substituents shown in [Table L1] to [Table L3] (hereinafter, referred to as a compound group SX1150).

In the compound (L-4), a compound in which Q is a group represented by Q53, $R^{3b}$ is $CF_3$, $R^{3c}$ is a hydrogen atom, and $R^1$ is any of the substituents shown in [Table L1] to [Table L3] (hereinafter, referred to as a compound group SX1151).

In the compound (L-4), a compound in which Q is a group represented by Q53, $R^{3b}$ is a hydrogen atom, $R^{3c}$ is a chlorine atom, and $R^1$ is any of the substituents shown in [Table L1] to [Table L3] (hereinafter, referred to as a compound group SX1152).

In the compound (L-4), a compound in which Q is a group represented by Q53, $R^{3b}$ is a hydrogen atom, $R^{3c}$ is a bromine atom, and $R^1$ is any of the substituents shown in [Table L1] to [Table L3] (hereinafter, referred to as a compound group SX1153).

In the compound (L-4), a compound in which Q is a group represented by Q53, $R^{3b}$ is a hydrogen atom, $R^{3c}$ is an iodine atom, and $R^1$ is any of the substituents shown in [Table L1] to [Table L3] (hereinafter, referred to as a compound group SX1154).

In the compound (L-4), a compound in which Q is a group represented by Q53, $R^{3b}$ is a hydrogen atom, $R^{3c}$ is $CF_3$, and $R^1$ is any of the substituents shown in [Table L1] to [Table L3] (hereinafter, referred to as a compound group SX1155).

In the compound (L-4), a compound in which Q is a group represented by Q54, $R^{3b}$ and $R^{3c}$ are hydrogen atoms, and $R^1$ is any of the substituents shown in [Table L1] to [Table L3] (hereinafter, referred to as a compound group SX1156).

In the compound (L-4), a compound in which Q is a group represented by Q54, $R^{3b}$ is a chlorine atom, $R^{3c}$ is a hydrogen atom, and $R^1$ is any of the substituents shown in [Table L1] to [Table L3] (hereinafter, referred to as a compound group SX1157).

In the compound (L-4), a compound in which Q is a group represented by Q54, $R^{3b}$ is a bromine atom, $R^{3c}$ is a hydrogen atom, and $R^1$ is any of the substituents shown in [Table L1] to [Table L3] (hereinafter, referred to as a compound group SX1158).

In the compound (L-4), a compound in which Q is a group represented by Q54, $R^{3b}$ is an iodine atom, $R^{3c}$ is a hydrogen atom, and $R^1$ is any of the substituents shown in [Table L1] to [Table L3] (hereinafter, referred to as a compound group SX1159).

In the compound (L-4), a compound in which Q is a group represented by Q54, $R^{3b}$ is $CF_3$, $R^{3c}$ is a hydrogen atom, and $R^1$ is any of the substituents shown in [Table L1] to [Table L3] (hereinafter, referred to as a compound group SX1160).

In the compound (L-4), a compound in which Q is a group represented by Q54, $R^{3b}$ is a hydrogen atom, $R^{3c}$ is a chlorine atom, and $R^1$ is any of the substituents shown in [Table L1] to [Table L3] (hereinafter, referred to as a compound group SX1161).

In the compound (L-4), a compound in which Q is a group represented by Q54, $R^{3b}$ is a hydrogen atom, $R^{3c}$ is a bromine atom, and $R^1$ is any of the substituents shown in [Table L1] to [Table L3] (hereinafter, referred to as a compound group SX1162).

In the compound (L-4), a compound in which Q is a group represented by Q54, $R^{3b}$ is a hydrogen atom, $R^{3c}$ is an iodine atom, and $R^1$ is any of the substituents shown in [Table L1] to [Table L3] (hereinafter, referred to as a compound group SX1163).

In the compound (L-4), a compound in which Q is a group represented by Q54, $R^{3b}$ is a hydrogen atom, $R^{3c}$ is $CF_3$, and $R^1$ is any of the substituents shown in [Table L1] to [Table L3] (hereinafter, referred to as a compound group SX1164).

In the compound (L-4), a compound in which Q is a group represented by Q55, $R^{3b}$ and $R^{3c}$ are hydrogen atoms, and $R^1$ is any of the substituents shown in [Table L1] to [Table L3] (hereinafter, referred to as a compound group SX1165).

In the compound (L-4), a compound in which Q is a group represented by Q55, $R^{3b}$ is a chlorine atom, $R^{3c}$ is a hydrogen atom, and $R^1$ is any of the substituents shown in [Table L1] to [Table L3] (hereinafter, referred to as a compound group SX1166).

In the compound (L-4), a compound in which Q is a group represented by Q55, $R^{3b}$ is a bromine atom, $R^{3c}$ is a hydrogen atom, and $R^1$ is any of the substituents shown in [Table L1] to [Table L3] (hereinafter, referred to as a compound group SX1167).

In the compound (L-4), a compound in which Q is a group represented by Q55, $R^{3b}$ is an iodine atom, $R^{3c}$ is

a hydrogen atom, and $R^1$ is any of the substituents shown in [Table L1] to [Table L3] (hereinafter, referred to as a compound group SX1168).

In the compound (L-4), a compound in which Q is a group represented by Q55, $R^{3b}$ is $CF_3$, $R^{3c}$ is a hydrogen atom, and $R^1$ is any of the substituents shown in [Table L1] to [Table L3] (hereinafter, referred to as a compound group SX1169).

In the compound (L-4), a compound in which Q is a group represented by Q55, $R^{3b}$ is a hydrogen atom, $R^{3c}$ is a chlorine atom, and $R^1$ is any of the substituents shown in [Table L1] to [Table L3] (hereinafter, referred to as a compound group SX1170).

In the compound (L-4), a compound in which Q is a group represented by Q55, $R^{3b}$ is a hydrogen atom, $R^{3c}$ is a bromine atom, and $R^1$ is any of the substituents shown in [Table L1] to [Table L3] (hereinafter, referred to as a compound group SX1171).

In the compound (L-4), a compound in which Q is a group represented by Q55, $R^{3b}$ is a hydrogen atom, $R^{3c}$ is an iodine atom, and $R^1$ is any of the substituents shown in [Table L1] to [Table L3] (hereinafter, referred to as a compound group SX1172).

In the compound (L-4), a compound in which Q is a group represented by Q55, $R^{3b}$ is a hydrogen atom, $R^{3c}$ is $CF_3$, and $R^1$ is any of the substituents shown in [Table L1] to [Table L3] (hereinafter, referred to as a compound group SX1173) .

In the compound (L-4), a compound in which Q is a group represented by Q56, $R^{3b}$ and $R^{3c}$ are hydrogen atoms, and $R^1$ is any of the substituents shown in [Table L1] to [Table L3] (hereinafter, referred to as a compound group SX1174).

In the compound (L-4), a compound in which Q is a group represented by Q56, $R^{3b}$ is a chlorine atom, $R^{3c}$ is a hydrogen atom, and $R^1$ is any of the substituents shown in [Table L1] to [Table L3] (hereinafter, referred to as a compound group SX1175).

In the compound (L-4), a compound in which Q is a group represented by Q56, $R^{3b}$ is a bromine atom, $R^{3c}$ is a hydrogen atom, and $R^1$ is any of the substituents shown in [Table L1] to [Table L3] (hereinafter, referred to as a compound group SX1176).

In the compound (L-4), a compound in which Q is a group represented by Q56, $R^{3b}$ is an iodine atom, $R^{3c}$ is a hydrogen atom, and $R^1$ is any of the substituents shown in [Table L1] to [Table L3] (hereinafter, referred to as a compound group SX1177).

In the compound (L-4), a compound in which Q is a group represented by Q56, $R^{3b}$ is $CF_3$, $R^{3c}$ is a hydrogen atom, and $R^1$ is any of the substituents shown in [Table L1] to [Table L3] (hereinafter, referred to as a compound group SX1178).

In the compound (L-4), a compound in which Q is a group represented by Q56, $R^{3b}$ is a hydrogen atom, $R^{3c}$ is a chlorine atom, and $R^1$ is any of the substituents shown in [Table L1] to [Table L3] (hereinafter, referred to as a compound group SX1179).

In the compound (L-4), a compound in which Q is a group represented by Q56, $R^{3b}$ is a hydrogen atom, $R^{3c}$ is a bromine atom, and $R^1$ is any of the substituents shown in [Table L1] to [Table L3] (hereinafter, referred to as a compound group SX1180).

In the compound (L-4), a compound in which Q is a group represented by Q56, $R^{3b}$ is a hydrogen atom, $R^{3c}$ is an iodine atom, and $R^1$ is any of the substituents shown in [Table L1] to [Table L3] (hereinafter, referred to as a compound group SX1181).

In the compound (L-4), a compound in which Q is a group represented by Q56, $R^{3b}$ is a hydrogen atom, $R^{3c}$ is $CF_3$, and $R^1$ is any of the substituents shown in [Table L1] to [Table L3] (hereinafter, referred to as a compound group SX1182).

In the compound (L-4), a compound in which Q is a group represented by Q57, $R^{3b}$ and $R^{3c}$ are hydrogen atoms, and $R^1$ is any of the substituents shown in [Table L1] to [Table L3] (hereinafter, referred to as a compound group SX1183).

In the compound (L-4), a compound in which Q is a group represented by Q57, $R^{3b}$ is a chlorine atom, $R^{3c}$ is a hydrogen atom, and $R^1$ is any of the substituents shown in [Table L1] to [Table L3] (hereinafter, referred to as a compound group SX1184).

In the compound (L-4), a compound in which Q is a group represented by Q57, $R^{3b}$ is a bromine atom, $R^{3c}$ is a hydrogen atom, and $R^1$ is any of the substituents shown in [Table L1] to [Table L3] (hereinafter, referred to as a compound group SX1185).

In the compound (L-4), a compound in which Q is a group represented by Q57, $R^{3b}$ is an iodine atom, $R^{3c}$ is a hydrogen atom, and $R^1$ is any of the substituents shown in [Table L1] to [Table L3] (hereinafter, referred to as a compound group SX1186).

In the compound (L-4), a compound in which Q is a group represented by Q57, $R^{3b}$ is $CF_3$, $R^{3c}$ is a hydrogen atom, and $R^1$ is any of the substituents shown in [Table L1] to [Table L3] (hereinafter, referred to as a compound group

SX1187).

In the compound (L-4), a compound in which Q is a group represented by Q57, $R^{3b}$ is a hydrogen atom, $R^{3c}$ is a chlorine atom, and $R^1$ is any of the substituents shown in [Table L1] to [Table L3] (hereinafter, referred to as a compound group SX1188).

In the compound (L-4), a compound in which Q is a group represented by Q57, $R^{3b}$ is a hydrogen atom, $R^{3c}$ is a bromine atom, and $R^1$ is any of the substituents shown in [Table L1] to [Table L3] (hereinafter, referred to as a compound group SX1189).

In the compound (L-4), a compound in which Q is a group represented by Q57, $R^{3b}$ is a hydrogen atom, $R^{3c}$ is an iodine atom, and $R^1$ is any of the substituents shown in [Table L1] to [Table L3] (hereinafter, referred to as a compound group SX1190).

In the compound (L-4), a compound in which Q is a group represented by Q57, $R^{3b}$ is a hydrogen atom, $R^{3c}$ is $CF_3$, and $R^1$ is any of the substituents shown in [Table L1] to [Table L3] (hereinafter, referred to as a compound group SX1191).

[Chem. 95]

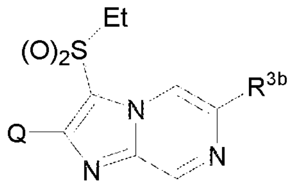

(L-5)

In a compound (hereinafter, referred to as a compound (L-5)) represented by Formula (L-5), a compound in which Q is a group represented by Q11, $R^{3b}$ is a hydrogen atom, and $R^1$ is any of the substituents shown in [Table L1] to [Table L3] (hereinafter, referred to as a compound group SX109).

In the compound (L-5), a compound in which Q is a group represented by Q12, $R^{3b}$ and $R^{3c}$ are hydrogen atoms, and $R^1$ is any of the substituents shown in [Table L1] to [Table L3] (hereinafter, referred to as a compound group SX110).

In the compound (L-5), a compound in which Q is a group represented by Q13, $R^{3b}$ and $R^{3c}$ are hydrogen atoms, and $R^1$ is any of the substituents shown in [Table L1] to [Table L3] (hereinafter, referred to as a compound group SX111).

In the compound (L-5), a compound in which Q is a group represented by Q11, $R^{3b}$ is a chlorine atom, $R^{3c}$ is a hydrogen atom, and $R^1$ is any of the substituents shown in [Table L1] to [Table L3] (hereinafter, referred to as a compound group SX112).

In the compound (L-5), a compound in which Q is a group represented by Q12, $R^{3b}$ is a chlorine atom, $R^{3c}$ is a hydrogen atom, and $R^1$ is any of the substituents shown in [Table L1] to [Table L3] (hereinafter, referred to as a compound group SX113).

In the compound (L-5), a compound in which Q is a group represented by Q13, $R^{3b}$ is a chlorine atom, $R^{3c}$ is a hydrogen atom, and $R^1$ is any of the substituents shown in [Table L1] to [Table L3] (hereinafter, referred to as a compound group SX114).

In the compound (L-5), a compound in which Q is a group represented by Q11, $R^{3b}$ is a bromine atom, $R^{3c}$ is a hydrogen atom, and $R^1$ is any of the substituents shown in [Table L1] to [Table L3] (hereinafter, referred to as a compound group SX115).

In the compound (L-5), a compound in which Q is a group represented by Q12, $R^{3b}$ is a bromine atom, $R^{3c}$ is a hydrogen atom, and $R^1$ is any of the substituents shown in [Table L1] to [Table L3] (hereinafter, referred to as a compound group SX116).

In the compound (L-5), a compound in which Q is a group represented by Q13, $R^{3b}$ is a bromine atom, $R^{3c}$ is a hydrogen atom, and $R^1$ is any of the substituents shown in [Table L1] to [Table L3] (hereinafter, referred to as a compound group SX117).

In the compound (L-5), a compound in which Q is a group represented by Q11, $R^{3b}$ is an iodine atom, $R^{3c}$ is a hydrogen atom, and $R^1$ is any of the substituents shown in [Table L1] to [Table L3] (hereinafter, referred to as a compound group SX118).

In the compound (L-5), a compound in which Q is a group represented by Q12, $R^{3b}$ is an iodine atom, $R^{3c}$ is a hydrogen atom, and $R^1$ is any of the substituents shown in [Table L1] to [Table L3] (hereinafter, referred to as a compound group SX119).

In the compound (L-5), a compound in which Q is a group represented by Q13, $R^{3b}$ is an iodine atom, $R^{3c}$ is a hydrogen atom, and $R^1$ is any of the substituents shown in [Table L1] to [Table L3] (hereinafter, referred to as a

compound group SX120).

In the compound (L-5), a compound in which Q is a group represented by Q11, $R^{3b}$ is $CF_3$, $R^{3c}$ is a hydrogen atom, and $R^1$ is any of the substituents shown in [Table L1] to [Table L3] (hereinafter, referred to as a compound group SX121).

In the compound (L-5), a compound in which Q is a group represented by Q12, $R^{3b}$ is $CF_3$, $R^{3c}$ is a hydrogen atom, and $R^1$ is any of the substituents shown in [Table L1] to [Table L3] (hereinafter, referred to as a compound group SX122).

In the compound (L-5), a compound in which Q is a group represented by Q13, $R^{3b}$ is $CF_3$, $R^{3c}$ is a hydrogen atom, and $R^1$ is any of the substituents shown in [Table L1] to [Table L3] (hereinafter, referred to as a compound group SX123).

In the compound (L-5), a compound in which Q is a group represented by Q11, $R^{3b}$ is a hydrogen atom, $R^{3c}$ is a chlorine atom, and $R^1$ is any of the substituents shown in [Table L1] to [Table L3] (hereinafter, referred to as a compound group SX124).

In the compound (L-5), a compound in which Q is a group represented by Q12, $R^{3b}$ is a hydrogen atom, $R^{3c}$ is a chlorine atom, and $R^1$ is any of the substituents shown in [Table L1] to [Table L3] (hereinafter, referred to as a compound group SX125).

In the compound (L-5), a compound in which Q is a group represented by Q13, $R^{3b}$ is a hydrogen atom, $R^{3c}$ is a chlorine atom, and $R^1$ is any of the substituents shown in [Table L1] to [Table L3] (hereinafter, referred to as a compound group SX126).

In the compound (L-5), a compound in which Q is a group represented by Q11, $R^{3b}$ is a hydrogen atom, $R^{3c}$ is a bromine atom, and $R^1$ is any of the substituents shown in [Table L1] to [Table L3] (hereinafter, referred to as a compound group SX127).

In the compound (L-5), a compound in which Q is a group represented by Q12, $R^{3b}$ is a hydrogen atom, $R^{3c}$ is a bromine atom, and $R^1$ is any of the substituents shown in [Table L1] to [Table L3] (hereinafter, referred to as a compound group SX128).

In the compound (L-5), a compound in which Q is a group represented by Q13, $R^{3b}$ is a hydrogen atom, $R^{3c}$ is a bromine atom, and $R^1$ is any of the substituents shown in [Table L1] to [Table L3] (hereinafter, referred to as a compound group SX129).

In the compound (L-5), a compound in which Q is a group represented by Q11, $R^{3b}$ is a hydrogen atom, $R^{3c}$ is an iodine atom, and $R^1$ is any of the substituents shown in [Table L1] to [Table L3] (hereinafter, referred to as a compound group SX130).

In the compound (L-5), a compound in which Q is a group represented by Q12, $R^{3b}$ is a hydrogen atom, $R^{3c}$ is an iodine atom, and $R^1$ is any of the substituents shown in [Table L1] to [Table L3] (hereinafter, referred to as a compound group SX131).

In the compound (L-5), a compound in which Q is a group represented by Q13, $R^{3b}$ is a hydrogen atom, $R^{3c}$ is an iodine atom, and $R^1$ is any of the substituents shown in [Table L1] to [Table L3] (hereinafter, referred to as a compound group SX132).

In the compound (L-5), a compound in which Q is a group represented by Q11, $R^{3b}$ is a hydrogen atom, $R^{3c}$ is $CF_3$, and $R^1$ is any of the substituents shown in [Table L1] to [Table L3] (hereinafter, referred to as a compound group SX133).

In the compound (L-5), a compound in which Q is a group represented by Q12, $R^{3b}$ is a hydrogen atom, $R^{3c}$ is $CF_3$, and $R^1$ is any of the substituents shown in [Table L1] to [Table L3] (hereinafter, referred to as a compound group SX134).

In the compound (L-5), a compound in which Q is a group represented by Q13, $R^{3b}$ is a hydrogen atom, $R^{3c}$ is $CF_3$, and $R^1$ is any of the substituents shown in [Table L1] to [Table L3] (hereinafter, referred to as a compound group SX135).

In the compound (L-5), a compound in which Q is a group represented by Q34, $R^{3b}$ and $R^{3c}$ are hydrogen atoms, and $R^1$ is any of the substituents shown in [Table L1] to [Table L3] (hereinafter, referred to as a compound group SX1192).

In the compound (L-5), a compound in which Q is a group represented by Q34, $R^{3b}$ is a chlorine atom, $R^{3c}$ is a hydrogen atom, and $R^1$ is any of the substituents shown in [Table L1] to [Table L3] (hereinafter, referred to as a compound group SX1193).

In the compound (L-5), a compound in which Q is a group represented by Q34, $R^{3b}$ is a bromine atom, $R^{3c}$ is a hydrogen atom, and $R^1$ is any of the substituents shown in [Table L1] to [Table L3] (hereinafter, referred to as a compound group SX1194).

In the compound (L-5), a compound in which Q is a group represented by Q34, $R^{3b}$ is an iodine atom, $R^{3c}$ is a hydrogen atom, and $R^1$ is any of the substituents shown in [Table L1] to [Table L3] (hereinafter, referred to as a compound group SX1195).

In the compound (L-5), a compound in which Q is a group represented by Q34, $R^{3b}$ is $CF_3$, $R^{3c}$ is a hydrogen atom, and $R^1$ is any of the substituents shown in [Table L1] to [Table L3] (hereinafter, referred to as a compound group SX1196).

In the compound (L-5), a compound in which Q is a group represented by Q34, $R^{3b}$ is a hydrogen atom, $R^{3c}$ is a chlorine atom, and $R^1$ is any of the substituents shown in [Table L1] to [Table L3] (hereinafter, referred to as a compound group SX1197).

In the compound (L-5), a compound in which Q is a group represented by Q34, $R^{3b}$ is a hydrogen atom, $R^{3c}$ is a bromine atom, and $R^1$ is any of the substituents shown in [Table Ll] to [Table L3] (hereinafter, referred to as a compound group SX1198).

In the compound (L-5), a compound in which Q is a group represented by Q34, $R^{3b}$ is a hydrogen atom, $R^{3c}$ is an iodine atom, and $R^1$ is any of the substituents shown in [Table Ll] to [Table L3] (hereinafter, referred to as a compound group SX1199).

In the compound (L-5), a compound in which Q is a group represented by Q34, $R^{3b}$ is a hydrogen atom, $R^{3c}$ is $CF_3$, and $R^1$ is any of the substituents shown in [Table L1] to [Table L3] (hereinafter, referred to as a compound group SX1200).

In the compound (L-5), a compound in which Q is a group represented by Q35, $R^{3b}$ and $R^{3c}$ are hydrogen atoms, and $R^1$ is any of the substituents shown in [Table L1] to [Table L3] (hereinafter, referred to as a compound group SX1201).

In the compound (L-5), a compound in which Q is a group represented by Q35, $R^{3b}$ is a chlorine atom, $R^{3c}$ is a hydrogen atom, and $R^1$ is any of the substituents shown in [Table Ll] to [Table L3] (hereinafter, referred to as a compound group SX1202).

In the compound (L-5), a compound in which Q is a group represented by Q35, $R^{3b}$ is a bromine atom, $R^{3c}$ is a hydrogen atom, and $R^1$ is any of the substituents shown in [Table Ll] to [Table L3] (hereinafter, referred to as a compound group SX1203).

In the compound (L-5), a compound in which Q is a group represented by Q35, $R^{3b}$ is an iodine atom, $R^{3c}$ is a hydrogen atom, and $R^1$ is any of the substituents shown in [Table L1] to [Table L3] (hereinafter, referred to as a compound group SX1204).

In the compound (L-5), a compound in which Q is a group represented by Q35, $R^{3b}$ is $CF_3$, $R^{3c}$ is a hydrogen atom, and $R^1$ is any of the substituents shown in [Table L1] to [Table L3] (hereinafter, referred to as a compound group SX1205).

In the compound (L-5), a compound in which Q is a group represented by Q35, $R^{3b}$ is a hydrogen atom, $R^{3c}$ is a chlorine atom, and $R^1$ is any of the substituents shown in [Table L1] to [Table L3] (hereinafter, referred to as a compound group SX1206).

In the compound (L-5), a compound in which Q is a group represented by Q35, $R^{3b}$ is a hydrogen atom, $R^{3c}$ is a bromine atom, and $R^1$ is any of the substituents shown in [Table L1] to [Table L3] (hereinafter, referred to as a compound group SX1207).

In the compound (L-5), a compound in which Q is a group represented by Q35, $R^{3b}$ is a hydrogen atom, $R^{3c}$ is an iodine atom, and $R^1$ is any of the substituents shown in [Table Ll] to [Table L3] (hereinafter, referred to as a compound group SX1208).

In the compound (L-5), a compound in which Q is a group represented by Q35, $R^{3b}$ is a hydrogen atom, $R^{3c}$ is $CF_3$, and $R^1$ is any of the substituents shown in [Table L1] to [Table L3] (hereinafter, referred to as a compound group SX1209).

In the compound (L-5), a compound in which Q is a group represented by Q36, $R^{3b}$ and $R^{3c}$ are hydrogen atoms, and $R^1$ is any of the substituents shown in [Table L1] to [Table L3] (hereinafter, referred to as a compound group SX1210).

In the compound (L-5), a compound in which Q is a group represented by Q36, $R^{3b}$ is a chlorine atom, $R^{3c}$ is a hydrogen atom, and $R^1$ is any of the substituents shown in [Table L1] to [Table L3] (hereinafter, referred to as a compound group SX1211).

In the compound (L-5), a compound in which Q is a group represented by Q36, $R^{3b}$ is a bromine atom, $R^{3c}$ is a hydrogen atom, and $R^1$ is any of the substituents shown in [Table L1] to [Table L3] (hereinafter, referred to as a compound group SX1212).

In the compound (L-5), a compound in which Q is a group represented by Q36, $R^{3b}$ is an iodine atom, $R^{3c}$ is a hydrogen atom, and $R^1$ is any of the substituents shown in [Table L1] to [Table L3] (hereinafter, referred to as a compound group SX1213).

In the compound (L-5), a compound in which Q is a group represented by Q36, $R^{3b}$ is $CF_3$, $R^{3c}$ is a hydrogen atom, and $R^1$ is any of the substituents shown in [Table L1] to [Table L3] (hereinafter, referred to as a compound group SX1214).

In the compound (L-5), a compound in which Q is a group represented by Q36, $R^{3b}$ is a hydrogen atom, $R^{3c}$

is a chlorine atom, and $R^1$ is any of the substituents shown in [Table L1] to [Table L3] (hereinafter, referred to as a compound group SX1215).

In the compound (L-5), a compound in which Q is a group represented by Q36, $R^{3b}$ is a hydrogen atom, $R^{3c}$ is a bromine atom, and $R^1$ is any of the substituents shown in [Table L1] to [Table L3] (hereinafter, referred to as a compound group SX1216).

In the compound (L-5), a compound in which Q is a group represented by Q36, $R^{3b}$ is a hydrogen atom, $R^{3c}$ is an iodine atom, and $R^1$ is any of the substituents shown in [Table L1] to [Table L3] (hereinafter, referred to as a compound group SX1217).

In the compound (L-5), a compound in which Q is a group represented by Q36, $R^{3b}$ is a hydrogen atom, $R^{3c}$ is $CF_3$, and $R^1$ is any of the substituents shown in [Table L1] to [Table L3] (hereinafter, referred to as a compound group SX1218).

In the compound (L-5), a compound in which Q is a group represented by Q37, $R^{3b}$ and $R^{3c}$ are hydrogen atoms, and $R^1$ is any of the substituents shown in [Table L1] to [Table L3] (hereinafter, referred to as a compound group SX1219).

In the compound (L-5), a compound in which Q is a group represented by Q37, $R^{3b}$ is a chlorine atom, $R^{3c}$ is a hydrogen atom, and $R^1$ is any of the substituents shown in [Table L1] to [Table L3] (hereinafter, referred to as a compound group SX1220).

In the compound (L-5), a compound in which Q is a group represented by Q37, $R^{3b}$ is a bromine atom, $R^{3c}$ is a hydrogen atom, and $R^1$ is any of the substituents shown in [Table L1] to [Table L3] (hereinafter, referred to as a compound group SX1221).

In the compound (L-5), a compound in which Q is a group represented by Q37, $R^{3b}$ is an iodine atom, $R^{3c}$ is a hydrogen atom, and $R^1$ is any of the substituents shown in [Table L1] to [Table L3] (hereinafter, referred to as a compound group SX1222).

In the compound (L-5), a compound in which Q is a group represented by Q37, $R^{3b}$ is $CF_3$, $R^{3c}$ is a hydrogen atom, and $R^1$ is any of the substituents shown in [Table L1] to [Table L3] (hereinafter, referred to as a compound group SX1223).

In the compound (L-5), a compound in which Q is a group represented by Q37, $R^{3b}$ is a hydrogen atom, $R^{3c}$ is a chlorine atom, and $R^1$ is any of the substituents shown in [Table L1] to [Table L3] (hereinafter, referred to as a compound group SX1224).

In the compound (L-5), a compound in which Q is a group represented by Q37, $R^{3b}$ is a hydrogen atom, $R^{3c}$ is a bromine atom, and $R^1$ is any of the substituents shown in [Table L1] to [Table L3] (hereinafter, referred to as a compound group SX1225).

In the compound (L-5), a compound in which Q is a group represented by Q37, $R^{3b}$ is a hydrogen atom, $R^{3c}$ is an iodine atom, and $R^1$ is any of the substituents shown in [Table L1] to [Table L3] (hereinafter, referred to as a compound group SX1226).

In the compound (L-5), a compound in which Q is a group represented by Q37, $R^{3b}$ is a hydrogen atom, $R^{3c}$ is $CF_3$, and $R^1$ is any of the substituents shown in [Table L1] to [Table L3] (hereinafter, referred to as a compound group SX1227).

In the compound (L-5), a compound in which Q is a group represented by Q38, $R^{3b}$ and $R^{3c}$ are hydrogen atoms, $R^{6a}$ is a hydrogen atom, and $R^1$ is any of the substituents shown in [Table Ll] to [Table L3] (hereinafter, referred to as a compound group SX1228).

In the compound (L-5), a compound in which Q is a group represented by Q38, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1229).

In the compound (L-5), a compound in which Q is a group represented by Q38, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1230).

In the compound (L-5), a compound in which Q is a group represented by Q38, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1231).

In the compound (L-5), a compound in which Q is a group represented by Q38, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX1232).

In the compound (L-5), a compound in which Q is a group represented by Q38, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX1233).

In the compound (L-5), a compound in which Q is a group represented by Q38, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter,

referred to as a compound group SX1234).

In the compound (L-5), a compound in which Q is a group represented by Q38, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX1235).

In the compound (L-5), a compound in which Q is a group represented by Q38, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX1236).

In the compound (L-5), a compound in which Q is a group represented by Q38, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX1237).

In the compound (L-5), a compound in which Q is a group represented by Q38, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX1238).

In the compound (L-5), a compound in which Q is a group represented by Q38, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX1239).

In the compound (L-5), a compound in which Q is a group represented by Q38, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX1240).

In the compound (L-5), a compound in which Q is a group represented by Q38, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX1241).

In the compound (L-5), a compound in which Q is a group represented by Q38, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX1242).

In the compound (L-5), a compound in which Q is a group represented by Q38, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX1243).

In the compound (L-5), a compound in which Q is a group represented by Q38, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX1244).

In the compound (L-5), a compound in which Q is a group represented by Q38, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX1245).

In the compound (L-5), a compound in which Q is a group represented by Q39, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX1246).

In the compound (L-5), a compound in which Q is a group represented by Q39, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1247).

In the compound (L-5), a compound in which Q is a group represented by Q39, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1248).

In the compound (L-5), a compound in which Q is a group represented by Q39, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1249).

In the compound (L-5), a compound in which Q is a group represented by Q39, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX1250).

In the compound (L-5), a compound in which Q is a group represented by Q39, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX1251).

In the compound (L-5), a compound in which Q is a group represented by Q39, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX1252).

In the compound (L-5), a compound in which Q is a group represented by Q39, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX1253).

In the compound (L-5), a compound in which Q is a group represented by Q39, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX1254).

In the compound (L-5), a compound in which Q is a group represented by Q39, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX1255).

In the compound (L-5), a compound in which Q is a group represented by Q39, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX1256).

In the compound (L-5), a compound in which Q is a group represented by Q39, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX1257).

In the compound (L-5), a compound in which Q is a group represented by Q39, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX1258).

In the compound (L-5), a compound in which Q is a group represented by Q39, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX1259).

In the compound (L-5), a compound in which Q is a group represented by Q39, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX1260).

In the compound (L-5), a compound in which Q is a group represented by Q39, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX1261).

In the compound (L-5), a compound in which Q is a group represented by Q39, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX1262).

In the compound (L-5), a compound in which Q is a group represented by Q39, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX1263).

In the compound (L-5), a compound in which Q is a group represented by Q40, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX1264).

In the compound (L-5), a compound in which Q is a group represented by Q40, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1265).

In the compound (L-5), a compound in which Q is a group represented by Q40, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1266).

In the compound (L-5), a compound in which Q is a group represented by Q40, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1267).

In the compound (L-5), a compound in which Q is a group represented by Q40, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX1268).

In the compound (L-5), a compound in which Q is a group represented by Q40, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX1269).

In the compound (L-5), a compound in which Q is a group represented by Q40, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX1270).

In the compound (L-5), a compound in which Q is a group represented by Q40, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX1271).

In the compound (L-5), a compound in which Q is a group represented by Q40, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX1272).

In the compound (L-5), a compound in which Q is a group represented by Q40, $R^{3b}$ and $R^{3c}$ are hydrogen

atoms, $R^{6a}$ is a methyl group, and $R^1$ is any of the substituents shown in [Table L1] to [Table L3] (hereinafter, referred to as a compound group SX1273).

In the compound (L-5), a compound in which Q is a group represented by Q40, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter, referred to as a compound group SX1274).

In the compound (L-5), a compound in which Q is a group represented by Q40, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX1275).

In the compound (L-5), a compound in which Q is a group represented by Q40, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX1276).

In the compound (L-5), a compound in which Q is a group represented by Q40, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX1277).

In the compound (L-5), a compound in which Q is a group represented by Q40, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX1278).

In the compound (L-5), a compound in which Q is a group represented by Q40, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX1279).

In the compound (L-5), a compound in which Q is a group represented by Q40, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX1280).

In the compound (L-5), a compound in which Q is a group represented by Q40, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX1281).

In the compound (L-5), a compound in which Q is a group represented by Q41, $R^{3b}$ and $R^{3c}$ are hydrogen atoms, $R^{6a}$ is a hydrogen atom, and $R^1$ is any of the substituents shown in [Table L1] to [Table L3] (hereinafter, referred to as a compound group SX1282).

In the compound (L-5), a compound in which Q is a group represented by Q41, $R^{3b}$ is a chlorine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1283).

In the compound (L-5), a compound in which Q is a group represented by Q41, $R^{3b}$ is a bromine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1284).

In the compound (L-5), a compound in which Q is a group represented by Q41, $R^{3b}$ is an iodine atom, $R^{3c}$ is a hydrogen atom, $R^{6a}$ is a hydrogen atom, and $R^1$ is any of the substituents shown in [Table L1] to [Table L3] (hereinafter, referred to as a compound group SX1285).

In the compound (L-5), a compound in which Q is a group represented by Q41, $R^{3b}$ is $CF_3$, $R^{3c}$ is a hydrogen atom, $R^{6a}$ is a hydrogen atom, and $R^1$ is any of the substituents shown in [Table L1] to [Table L3] (hereinafter, referred to as a compound group SX1286).

In the compound (L-5), a compound in which Q is a group represented by Q41, $R^{3b}$ is a hydrogen atom, $R^{3c}$ is a chlorine atom, $R^{6a}$ is a hydrogen atom, and $R^1$ is any of the substituents shown in [Table L1] to [Table L3] (hereinafter, referred to as a compound group SX1287).

In the compound (L-5), a compound in which Q is a group represented by Q41, $R^{3b}$ is a hydrogen atom, $R^{3c}$ is a bromine atom, $R^{6a}$ is a hydrogen atom, and $R^1$ is any of the substituents shown in [Table L1] to [Table L3] (hereinafter, referred to as a compound group SX1288).

In the compound (L-5), a compound in which Q is a group represented by Q41, $R^{3b}$ is a hydrogen atom, $R^{3c}$ is an iodine atom, $R^{6a}$ is a hydrogen atom, and $R^1$ is any of the substituents shown in [Table L1] to [Table L3] (hereinafter, referred to as a compound group SX1289).

In the compound (L-5), a compound in which Q is a group represented by Q41, $R^{3b}$ is a hydrogen atom, $R^{3c}$ is $CF_3$, $R^{6a}$ is a hydrogen atom, and $R^1$ is any of the substituents shown in [Table L1] to [Table L3] (hereinafter, referred to as a compound group SX1290).

In the compound (L-5), a compound in which Q is a group represented by Q41, $R^{3b}$ and $R^{3c}$ are hydrogen atoms, $R^{6a}$ is a methyl group, and $R^1$ is any of the substituents shown in [Table L1] to [Table L3] (hereinafter, referred to as a compound group SX1291).

In the compound (L-5), a compound in which Q is a group represented by Q41, $R^{3b}$ is a chlorine atom, $R^{3c}$ is a hydrogen atom, $R^{6a}$ is a methyl group, and $R^1$ is any of the substituents shown in [Table L1] to [Table L3] (hereinafter,

referred to as a compound group SX1292).

In the compound (L-5), a compound in which Q is a group represented by Q41, $R^{3b}$ is a bromine atom, $R^{3c}$ is a hydrogen atom, $R^{6a}$ is a methyl group, and $R^1$ is any of the substituents shown in [Table L1] to [Table L3] (hereinafter, referred to as a compound group SX1293).

In the compound (L-5), a compound in which Q is a group represented by Q41, $R^{3b}$ is an iodine atom, $R^{3c}$ is a hydrogen atom, $R^{6a}$ is a methyl group, and $R^1$ is any of the substituents shown in [Table L1] to [Table L3] (hereinafter, referred to as a compound group SX1294).

In the compound (L-5), a compound in which Q is a group represented by Q41, $R^{3b}$ is $CF_3$, $R^{3c}$ is a hydrogen atom, $R^{6a}$ is a methyl group, and $R^1$ is any of the substituents shown in [Table L1] to [Table L3] (hereinafter, referred to as a compound group SX1295).

In the compound (L-5), a compound in which Q is a group represented by Q41, $R^{3b}$ is a hydrogen atom, $R^{3c}$ is a chlorine atom, $R^{6a}$ is a methyl group, and $R^1$ is any of the substituents shown in [Table L1] to [Table L3] (hereinafter, referred to as a compound group SX1296).

In the compound (L-5), a compound in which Q is a group represented by Q41, $R^{3b}$ is a hydrogen atom, $R^{3c}$ is a bromine atom, $R^{6a}$ is a methyl group, and $R^1$ is any of the substituents shown in [Table L1] to [Table L3] (hereinafter, referred to as a compound group SX1297).

In the compound (L-5), a compound in which Q is a group represented by Q41, $R^{3b}$ is a hydrogen atom, $R^{3c}$ is an iodine atom, $R^{6a}$ is a methyl group, and $R^1$ is any of the substituents shown in [Table L1] to [Table L3] (hereinafter, referred to as a compound group SX1298).

In the compound (L-5), a compound in which Q is a group represented by Q41, $R^{3b}$ is a hydrogen atom, $R^{3c}$ is $CF_3$, $R^{6a}$ is a methyl group, and $R^1$ is any of the substituents shown in [Table L1] to [Table L3] (hereinafter, referred to as a compound group SX1299).

In the compound (L-5), a compound in which Q is a group represented by Q42, $R^{3b}$ and $R^{3c}$ are hydrogen atoms, and $R^1$ is any of the substituents shown in [Table L1] to [Table L3] (hereinafter, referred to as a compound group SX1300).

In the compound (L-5), a compound in which Q is a group represented by Q42, $R^{3b}$ is a chlorine atom, $R^{3c}$ is a hydrogen atom, and $R^1$ is any of the substituents shown in [Table L1] to [Table L3] (hereinafter, referred to as a compound group SX1301).

In the compound (L-5), a compound in which Q is a group represented by Q42, $R^{3b}$ is a bromine atom, $R^{3c}$ is a hydrogen atom, and $R^1$ is any of the substituents shown in [Table L1] to [Table L3] (hereinafter, referred to as a compound group SX1302).

In the compound (L-5), a compound in which Q is a group represented by Q42, $R^{3b}$ is an iodine atom, $R^{3c}$ is a hydrogen atom, and $R^1$ is any of the substituents shown in [Table Ll] to [Table L3] (hereinafter, referred to as a compound group SX1303).

In the compound (L-5), a compound in which Q is a group represented by Q42, $R^{3b}$ is $CF_3$, $R^{3c}$ is a hydrogen atom, and $R^1$ is any of the substituents shown in [Table L1] to [Table L3] (hereinafter, referred to as a compound group SX1304).

In the compound (L-5), a compound in which Q is a group represented by Q42, $R^{3b}$ is a hydrogen atom, $R^{3c}$ is a chlorine atom, and $R^1$ is any of the substituents shown in [Table L1] to [Table L3] (hereinafter, referred to as a compound group SX1305).

In the compound (L-5), a compound in which Q is a group represented by Q42, $R^{3b}$ is a hydrogen atom, $R^{3c}$ is a bromine atom, and $R^1$ is any of the substituents shown in [Table L1] to [Table L3] (hereinafter, referred to as a compound group SX1306).

In the compound (L-5), a compound in which Q is a group represented by Q42, $R^{3b}$ is a hydrogen atom, $R^{3c}$ is an iodine atom, and $R^1$ is any of the substituents shown in [Table L1] to [Table L3] (hereinafter, referred to as a compound group SX1307).

In the compound (L-5), a compound in which Q is a group represented by Q42, $R^{3b}$ is a hydrogen atom, $R^{3c}$ is $CF_3$, and $R^1$ is any of the substituents shown in [Table L1] to [Table L3] (hereinafter, referred to as a compound group SX1308).

In the compound (L-5), a compound in which Q is a group represented by Q43, $R^{3b}$ and $R^{3c}$ are hydrogen atoms, and $R^1$ is any of the substituents shown in [Table L1] to [Table L3] (hereinafter, referred to as a compound group SX1309).

In the compound (L-5), a compound in which Q is a group represented by Q43, $R^{3b}$ is a chlorine atom, $R^{3c}$ is a hydrogen atom, and $R^1$ is any of the substituents shown in [Table L1] to [Table L3] (hereinafter, referred to as a compound group SX1310).

In the compound (L-5), a compound in which Q is a group represented by Q43, $R^{3b}$ is a bromine atom, $R^{3c}$ is a hydrogen atom, and $R^1$ is any of the substituents shown in [Table L1] to [Table L3] (hereinafter, referred to as a compound group SX1311).

In the compound (L-5), a compound in which Q is a group represented by Q43, $R^{3b}$ is an iodine atom, $R^{3c}$ is a hydrogen atom, and $R^1$ is any of the substituents shown in [Table L1] to [Table L3] (hereinafter, referred to as a compound group SX1312).

In the compound (L-5), a compound in which Q is a group represented by Q43, $R^{3b}$ is $CF_3$, $R^{3c}$ is a hydrogen atom, and $R^1$ is any of the substituents shown in [Table L1] to [Table L3] (hereinafter, referred to as a compound group SX1313).

In the compound (L-5), a compound in which Q is a group represented by Q43, $R^{3b}$ is a hydrogen atom, $R^{3c}$ is a chlorine atom, and $R^1$ is any of the substituents shown in [Table L1] to [Table L3] (hereinafter, referred to as a compound group SX1314).

In the compound (L-5), a compound in which Q is a group represented by Q43, $R^{3b}$ is a hydrogen atom, $R^{3c}$ is a bromine atom, and $R^1$ is any of the substituents shown in [Table L1] to [Table L3] (hereinafter, referred to as a compound group SX1315).

In the compound (L-5), a compound in which Q is a group represented by Q43, $R^{3b}$ is a hydrogen atom, $R^{3c}$ is an iodine atom, and $R^1$ is any of the substituents shown in [Table L1] to [Table L3] (hereinafter, referred to as a compound group SX1316).

In the compound (L-5), a compound in which Q is a group represented by Q43, $R^{3b}$ is a hydrogen atom, $R^{3c}$ is $CF_3$, and $R^1$ is any of the substituents shown in [Table L1] to [Table L3] (hereinafter, referred to as a compound group SX1317).

In the compound (L-5), a compound in which Q is a group represented by Q44, $R^{3b}$ and $R^{3c}$ are hydrogen atoms, and $R^1$ is any of the substituents shown in [Table L1] to [Table L3] (hereinafter, referred to as a compound group SX1318).

In the compound (L-5), a compound in which Q is a group represented by Q44, $R^{3b}$ is a chlorine atom, $R^{3c}$ is a hydrogen atom, and $R^1$ is any of the substituents shown in [Table L1] to [Table L3] (hereinafter, referred to as a compound group SX1319).

In the compound (L-5), a compound in which Q is a group represented by Q44, $R^{3b}$ is a bromine atom, $R^{3c}$ is a hydrogen atom, and $R^1$ is any of the substituents shown in [Table L1] to [Table L3] (hereinafter, referred to as a compound group SX1320).

In the compound (L-5), a compound in which Q is a group represented by Q44, $R^{3b}$ is an iodine atom, $R^{3c}$ is a hydrogen atom, and $R^1$ is any of the substituents shown in [Table L1] to [Table L3] (hereinafter, referred to as a compound group SX1321).

In the compound (L-5), a compound in which Q is a group represented by Q44, $R^{3b}$ is $CF_3$, $R^{3c}$ is a hydrogen atom, and $R^1$ is any of the substituents shown in [Table L1] to [Table L3] (hereinafter, referred to as a compound group SX1322).

In the compound (L-5), a compound in which Q is a group represented by Q44, $R^{3b}$ is a hydrogen atom, $R^{3c}$ is a chlorine atom, and $R^1$ is any of the substituents shown in [Table L1] to [Table L3] (hereinafter, referred to as a compound group SX1323).

In the compound (L-5), a compound in which Q is a group represented by Q44, $R^{3b}$ is a hydrogen atom, $R^{3c}$ is a bromine atom, and $R^1$ is any of the substituents shown in [Table L1] to [Table L3] (hereinafter, referred to as a compound group SX1324).

In the compound (L-5), a compound in which Q is a group represented by Q44, $R^{3b}$ is a hydrogen atom, $R^{3c}$ is an iodine atom, and $R^1$ is any of the substituents shown in [Table L1] to [Table L3] (hereinafter, referred to as a compound group SX1325).

In the compound (L-5), a compound in which Q is a group represented by Q44, $R^{3b}$ is a hydrogen atom, $R^{3c}$ is $CF_3$, and $R^1$ is any of the substituents shown in [Table L1] to [Table L3] (hereinafter, referred to as a compound group SX1326).

In the compound (L-5), a compound in which Q is a group represented by Q45, $R^{3b}$ and $R^{3c}$ are hydrogen atoms, and $R^1$ is any of the substituents shown in [Table L1] to [Table L3] (hereinafter, referred to as a compound group SX1327).

In the compound (L-5), a compound in which Q is a group represented by Q45, $R^{3b}$ is a chlorine atom, $R^{3c}$ is a hydrogen atom, and $R^1$ is any of the substituents shown in [Table L1] to [Table L3] (hereinafter, referred to as a compound group SX1328).

In the compound (L-5), a compound in which Q is a group represented by Q45, $R^{3b}$ is a bromine atom, $R^{3c}$ is a hydrogen atom, and $R^1$ is any of the substituents shown in [Table L1] to [Table L3] (hereinafter, referred to as a compound group SX1329).

In the compound (L-5), a compound in which Q is a group represented by Q45, $R^{3b}$ is an iodine atom, $R^{3c}$ is a hydrogen atom, and $R^1$ is any of the substituents shown in [Table L1] to [Table L3] (hereinafter, referred to as a compound group SX1330).

In the compound (L-5), a compound in which Q is a group represented by Q45, $R^{3b}$ is $CF_3$, $R^{3c}$ is a hydrogen

atom, and $R^1$ is any of the substituents shown in [Table L1] to [Table L3] (hereinafter, referred to as a compound group SX1331).

In the compound (L-5), a compound in which Q is a group represented by Q45, $R^{3b}$ is a hydrogen atom, $R^{3c}$ is a chlorine atom, and $R^1$ is any of the substituents shown in [Table L1] to [Table L3] (hereinafter, referred to as a compound group SX1332).

In the compound (L-5), a compound in which Q is a group represented by Q45, $R^{3b}$ is a hydrogen atom, $R^{3c}$ is a bromine atom, and $R^1$ is any of the substituents shown in [Table L1] to [Table L3] (hereinafter, referred to as a compound group SX1333).

In the compound (L-5), a compound in which Q is a group represented by Q45, $R^{3b}$ is a hydrogen atom, $R^{3c}$ is an iodine atom, and $R^1$ is any of the substituents shown in [Table L1] to [Table L3] (hereinafter, referred to as a compound group SX1334).

In the compound (L-5), a compound in which Q is a group represented by Q45, $R^{3b}$ is a hydrogen atom, $R^{3c}$ is $CF_3$, and $R^1$ is any of the substituents shown in [Table L1] to [Table L3] (hereinafter, referred to as a compound group SX1335).

In the compound (L-5), a compound in which Q is a group represented by Q46, $R^{3b}$ and $R^{3c}$ are hydrogen atoms, and $R^1$ is any of the substituents shown in [Table L1] to [Table L3] (hereinafter, referred to as a compound group SX1336).

In the compound (L-5), a compound in which Q is a group represented by Q46, $R^{3b}$ is a chlorine atom, $R^{3c}$ is a hydrogen atom, and $R^1$ is any of the substituents shown in [Table L1] to [Table L3] (hereinafter, referred to as a compound group SX1337).

In the compound (L-5), a compound in which Q is a group represented by Q46, $R^{3b}$ is a bromine atom, $R^{3c}$ is a hydrogen atom, and $R^1$ is any of the substituents shown in [Table L1] to [Table L3] (hereinafter, referred to as a compound group SX1338).

In the compound (L-5), a compound in which Q is a group represented by Q46, $R^{3b}$ is an iodine atom, $R^{3c}$ is a hydrogen atom, and $R^1$ is any of the substituents shown in [Table L1] to [Table L3] (hereinafter, referred to as a compound group SX1339).

In the compound (L-5), a compound in which Q is a group represented by Q46, $R^{3b}$ is $CF_3$, $R^{3c}$ is a hydrogen atom, and $R^1$ is any of the substituents shown in [Table L1] to [Table L3] (hereinafter, referred to as a compound group SX1340).

In the compound (L-5), a compound in which Q is a group represented by Q46, $R^{3b}$ is a hydrogen atom, $R^{3c}$ is a chlorine atom, and $R^1$ is any of the substituents shown in [Table L1] to [Table L3] (hereinafter, referred to as a compound group SX1341).

In the compound (L-5), a compound in which Q is a group represented by Q46, $R^{3b}$ is a hydrogen atom, $R^{3c}$ is a bromine atom, and $R^1$ is any of the substituents shown in [Table L1] to [Table L3] (hereinafter, referred to as a compound group SX1342).

In the compound (L-5), a compound in which Q is a group represented by Q46, $R^{3b}$ is a hydrogen atom, $R^{3c}$ is an iodine atom, and $R^1$ is any of the substituents shown in [Table Ll] to [Table L3] (hereinafter, referred to as a compound group SX1343).

In the compound (L-5), a compound in which Q is a group represented by Q46, $R^{3b}$ is a hydrogen atom, $R^{3c}$ is $CF_3$, and $R^1$ is any of the substituents shown in [Table L1] to [Table L3] (hereinafter, referred to as a compound group SX1344).

In the compound (L-5), a compound in which Q is a group represented by Q47, $R^{3b}$ and $R^{3c}$ are hydrogen atoms, and $R^1$ is any of the substituents shown in [Table L1] to [Table L3] (hereinafter, referred to as a compound group SX1345).

In the compound (L-5), a compound in which Q is a group represented by Q47, $R^{3b}$ is a chlorine atom, $R^{3c}$ is a hydrogen atom, and $R^1$ is any of the substituents shown in [Table L1] to [Table L3] (hereinafter, referred to as a compound group SX1346).

In the compound (L-5), a compound in which Q is a group represented by Q47, $R^{3b}$ is a bromine atom, $R^{3c}$ is a hydrogen atom, and $R^1$ is any of the substituents shown in [Table L1] to [Table L3] (hereinafter, referred to as a compound group SX1347).

In the compound (L-5), a compound in which Q is a group represented by Q47, $R^{3b}$ is an iodine atom, $R^{3c}$ is a hydrogen atom, and $R^1$ is any of the substituents shown in [Table Ll] to [Table L3] (hereinafter, referred to as a compound group SX1348).

In the compound (L-5), a compound in which Q is a group represented by Q47, $R^{3b}$ is $CF_3$, $R^{3c}$ is a hydrogen atom, and $R^1$ is any of the substituents shown in [Table L1] to [Table L3] (hereinafter, referred to as a compound group SX1349).

In the compound (L-5), a compound in which Q is a group represented by Q47, $R^{3b}$ is a hydrogen atom, $R^{3c}$ is a chlorine atom, and $R^1$ is any of the substituents shown in [Table L1] to [Table L3] (hereinafter, referred to as a

compound group SX1350).

In the compound (L-5), a compound in which Q is a group represented by Q47, $R^{3b}$ is a hydrogen atom, $R^{3c}$ is a bromine atom, and $R^1$ is any of the substituents shown in [Table L1] to [Table L3] (hereinafter, referred to as a compound group SX1351).

In the compound (L-5), a compound in which Q is a group represented by Q47, $R^{3b}$ is a hydrogen atom, $R^{3c}$ is an iodine atom, and $R^1$ is any of the substituents shown in [Table L1] to [Table L3] (hereinafter, referred to as a compound group SX1352).

In the compound (L-5), a compound in which Q is a group represented by Q47, $R^{3b}$ is a hydrogen atom, $R^{3c}$ is $CF_3$, and $R^1$ is any of the substituents shown in [Table L1] to [Table L3] (hereinafter, referred to as a compound group SX1353).

In the compound (L-5), a compound in which Q is a group represented by Q48, $R^{3b}$ and $R^{3c}$ are hydrogen atoms, and $R^1$ is any of the substituents shown in [Table L1] to [Table L3] (hereinafter, referred to as a compound group SX1354).

In the compound (L-5), a compound in which Q is a group represented by Q48, $R^{3b}$ is a chlorine atom, $R^{3c}$ is a hydrogen atom, and $R^1$ is any of the substituents shown in [Table L1] to [Table L3] (hereinafter, referred to as a compound group SX1355).

In the compound (L-5), a compound in which Q is a group represented by Q48, $R^{3b}$ is a bromine atom, $R^{3c}$ is a hydrogen atom, and $R^1$ is any of the substituents shown in [Table L1] to [Table L3] (hereinafter, referred to as a compound group SX1356).

In the compound (L-5), a compound in which Q is a group represented by Q48, $R^{3b}$ is an iodine atom, $R^{3c}$ is a hydrogen atom, and $R^1$ is any of the substituents shown in [Table L1] to [Table L3] (hereinafter, referred to as a compound group SX1357).

In the compound (L-5), a compound in which Q is a group represented by Q48, $R^{3b}$ is $CF_3$, $R^{3c}$ is a hydrogen atom, and $R^1$ is any of the substituents shown in [Table L1] to [Table L3] (hereinafter, referred to as a compound group SX1358).

In the compound (L-5), a compound in which Q is a group represented by Q48, $R^{3b}$ is a hydrogen atom, $R^{3c}$ is a chlorine atom, and $R^1$ is any of the substituents shown in [Table L1] to [Table L3] (hereinafter, referred to as a compound group SX1359).

In the compound (L-5), a compound in which Q is a group represented by Q48, $R^{3b}$ is a hydrogen atom, $R^{3c}$ is a bromine atom, and $R^1$ is any of the substituents shown in [Table L1] to [Table L3] (hereinafter, referred to as a compound group SX1360).

In the compound (L-5), a compound in which Q is a group represented by Q48, $R^{3b}$ is a hydrogen atom, $R^{3c}$ is an iodine atom, and $R^1$ is any of the substituents shown in [Table L1] to [Table L3] (hereinafter, referred to as a compound group SX1361).

In the compound (L-5), a compound in which Q is a group represented by Q48, $R^{3b}$ is a hydrogen atom, $R^{3c}$ is $CF_3$, and $R^1$ is any of the substituents shown in [Table L1] to [Table L3] (hereinafter, referred to as a compound group SX1362).

In the compound (L-5), a compound in which Q is a group represented by Q49, $R^{3b}$ and $R^{3c}$ are hydrogen atoms, and $R^1$ is any of the substituents shown in [Table L1] to [Table L3] (hereinafter, referred to as a compound group SX1363).

In the compound (L-5), a compound in which Q is a group represented by Q49, $R^{3b}$ is a chlorine atom, $R^{3c}$ is a hydrogen atom, and $R^1$ is any of the substituents shown in [Table L1] to [Table L3] (hereinafter, referred to as a compound group SX1364).

In the compound (L-5), a compound in which Q is a group represented by Q49, $R^{3b}$ is a bromine atom, $R^{3c}$ is a hydrogen atom, and $R^1$ is any of the substituents shown in [Table L1] to [Table L3] (hereinafter, referred to as a compound group SX1365).

In the compound (L-5), a compound in which Q is a group represented by Q49, $R^{3b}$ is an iodine atom, $R^{3c}$ is a hydrogen atom, and $R^1$ is any of the substituents shown in [Table L1] to [Table L3] (hereinafter, referred to as a compound group SX1366).

In the compound (L-5), a compound in which Q is a group represented by Q49, $R^{3b}$ is $CF_3$, $R^{3c}$ is a hydrogen atom, and $R^1$ is any of the substituents shown in [Table L1] to [Table L3] (hereinafter, referred to as a compound group SX1367).

In the compound (L-5), a compound in which Q is a group represented by Q49, $R^{3b}$ is a hydrogen atom, $R^{3c}$ is a chlorine atom, and $R^1$ is any of the substituents shown in [Table L1] to [Table L3] (hereinafter, referred to as a compound group SX1368).

In the compound (L-5), a compound in which Q is a group represented by Q49, $R^{3b}$ is a hydrogen atom, $R^{3c}$ is a bromine atom, and $R^1$ is any of the substituents shown in [Table L1] to [Table L3] (hereinafter, referred to as a compound group SX1369).

In the compound (L-5), a compound in which Q is a group represented by Q49, $R^{3b}$ is a hydrogen atom, $R^{3c}$ is an iodine atom, and $R^1$ is any of the substituents shown in [Table L1] to [Table L3] (hereinafter, referred to as a compound group SX1370).

In the compound (L-5), a compound in which Q is a group represented by Q49, $R^{3b}$ is a hydrogen atom, $R^{3c}$ is $CF_3$, and $R^1$ is any of the substituents shown in [Table L1] to [Table L3] (hereinafter, referred to as a compound group SX1371).

In the compound (L-5), a compound in which Q is a group represented by Q50, $R^{3b}$ and $R^{3c}$ are hydrogen atoms, and $R^1$ is any of the substituents shown in [Table L1] to [Table L3] (hereinafter, referred to as a compound group SX1372).

In the compound (L-5), a compound in which Q is a group represented by Q50, $R^{3b}$ is a chlorine atom, $R^{3c}$ is a hydrogen atom, and $R^1$ is any of the substituents shown in [Table L1] to [Table L3] (hereinafter, referred to as a compound group SX1373).

In the compound (L-5), a compound in which Q is a group represented by Q50, $R^{3b}$ is a bromine atom, $R^{3c}$ is a hydrogen atom, and $R^1$ is any of the substituents shown in [Table L1] to [Table L3] (hereinafter, referred to as a compound group SX1374).

In the compound (L-5), a compound in which Q is a group represented by Q50, $R^{3b}$ is an iodine atom, $R^{3c}$ is a hydrogen atom, and $R^1$ is any of the substituents shown in [Table L1] to [Table L3] (hereinafter, referred to as a compound group SX1375).

In the compound (L-5), a compound in which Q is a group represented by Q50, $R^{3b}$ is $CF_3$, $R^{3c}$ is a hydrogen atom, and $R^1$ is any of the substituents shown in [Table L1] to [Table L3] (hereinafter, referred to as a compound group SX1376).

In the compound (L-5), a compound in which Q is a group represented by Q50, $R^{3b}$ is a hydrogen atom, $R^{3c}$ is a chlorine atom, and $R^1$ is any of the substituents shown in [Table L1] to [Table L3] (hereinafter, referred to as a compound group SX1377).

In the compound (L-5), a compound in which Q is a group represented by Q50, $R^{3b}$ is a hydrogen atom, $R^{3c}$ is a bromine atom, and $R^1$ is any of the substituents shown in [Table L1] to [Table L3] (hereinafter, referred to as a compound group SX1378).

In the compound (L-5), a compound in which Q is a group represented by Q50, $R^{3b}$ is a hydrogen atom, $R^{3c}$ is an iodine atom, and $R^1$ is any of the substituents shown in [Table L1] to [Table L3] (hereinafter, referred to as a compound group SX1379).

In the compound (L-5), a compound in which Q is a group represented by Q50, $R^{3b}$ is a hydrogen atom, $R^{3c}$ is $CF_3$, and $R^1$ is any of the substituents shown in [Table L1] to [Table L3] (hereinafter, referred to as a compound group SX1380).

In the compound (L-5), a compound in which Q is a group represented by Q51, $R^{3b}$ and $R^{3c}$ are hydrogen atoms, and $R^1$ is any of the substituents shown in [Table L1] to [Table L3] (hereinafter, referred to as a compound group SX1381).

In the compound (L-5), a compound in which Q is a group represented by Q51, $R^{3b}$ is a chlorine atom, $R^{3c}$ is a hydrogen atom, and $R^1$ is any of the substituents shown in [Table L1] to [Table L3] (hereinafter, referred to as a compound group SX1382).

In the compound (L-5), a compound in which Q is a group represented by Q51, $R^{3b}$ is a bromine atom, $R^{3c}$ is a hydrogen atom, and $R^1$ is any of the substituents shown in [Table L1] to [Table L3] (hereinafter, referred to as a compound group SX1383).

In the compound (L-5), a compound in which Q is a group represented by Q51, $R^{3b}$ is an iodine atom, $R^{3c}$ is a hydrogen atom, and $R^1$ is any of the substituents shown in [Table L1] to [Table L3] (hereinafter, referred to as a compound group SX1384).

In the compound (L-5), a compound in which Q is a group represented by Q51, $R^{3b}$ is $CF_3$, $R^{3c}$ is a hydrogen atom, and $R^1$ is any of the substituents shown in [Table L1] to [Table L3] (hereinafter, referred to as a compound group SX1385).

In the compound (L-5), a compound in which Q is a group represented by Q51, $R^{3b}$ is a hydrogen atom, $R^{3c}$ is a chlorine atom, and $R^1$ is any of the substituents shown in [Table L1] to [Table L3] (hereinafter, referred to as a compound group SX1386).

In the compound (L-5), a compound in which Q is a group represented by Q51, $R^{3b}$ is a hydrogen atom, $R^{3c}$ is a bromine atom, and $R^1$ is any of the substituents shown in [Table L1] to [Table L3] (hereinafter, referred to as a compound group SX1387).

In the compound (L-5), a compound in which Q is a group represented by Q51, $R^{3b}$ is a hydrogen atom, $R^{3c}$ is an iodine atom, and $R^1$ is any of the substituents shown in [Table L1] to [Table L3] (hereinafter, referred to as a compound group SX1388).

In the compound (L-5), a compound in which Q is a group represented by Q51, $R^{3b}$ is a hydrogen atom, $R^{3c}$

is $CF_3$, and $R^1$ is any of the substituents shown in [Table L1] to [Table L3] (hereinafter, referred to as a compound group SX1389).

In the compound (L-5), a compound in which Q is a group represented by Q52, $R^{3b}$ and $R^{3c}$ are hydrogen atoms, and $R^1$ is any of the substituents shown in [Table L1] to [Table L3] (hereinafter, referred to as a compound group SX1390).

In the compound (L-5), a compound in which Q is a group represented by Q52, $R^{3b}$ is a chlorine atom, $R^{3c}$ is a hydrogen atom, and $R^1$ is any of the substituents shown in [Table L1] to [Table L3] (hereinafter, referred to as a compound group SX1391).

In the compound (L-5), a compound in which Q is a group represented by Q52, $R^{3b}$ is a bromine atom, $R^{3c}$ is a hydrogen atom, and $R^1$ is any of the substituents shown in [Table L1] to [Table L3] (hereinafter, referred to as a compound group SX1392).

In the compound (L-5), a compound in which Q is a group represented by Q52, $R^{3b}$ is an iodine atom, $R^{3c}$ is a hydrogen atom, and $R^1$ is any of the substituents shown in [Table L1] to [Table L3] (hereinafter, referred to as a compound group SX1393).

In the compound (L-5), a compound in which Q is a group represented by Q52, $R^{3b}$ is $CF_3$, $R^{3c}$ is a hydrogen atom, and $R^1$ is any of the substituents shown in [Table L1] to [Table L3] (hereinafter, referred to as a compound group SX1394).

In the compound (L-5), a compound in which Q is a group represented by Q52, $R^{3b}$ is a hydrogen atom, $R^{3c}$ is a chlorine atom, and $R^1$ is any of the substituents shown in [Table L1] to [Table L3] (hereinafter, referred to as a compound group SX1395).

In the compound (L-5), a compound in which Q is a group represented by Q52, $R^{3b}$ is a hydrogen atom, $R^{3c}$ is a bromine atom, and $R^1$ is any of the substituents shown in [Table L1] to [Table L3] (hereinafter, referred to as a compound group SX1396).

In the compound (L-5), a compound in which Q is a group represented by Q52, $R^{3b}$ is a hydrogen atom, $R^{3c}$ is an iodine atom, and $R^1$ is any of the substituents shown in [Table L1] to [Table L3] (hereinafter, referred to as a compound group SX1397).

In the compound (L-5), a compound in which Q is a group represented by Q52, $R^{3b}$ is a hydrogen atom, $R^{3c}$ is $CF_3$, and $R^1$ is any of the substituents shown in [Table L1] to [Table L3] (hereinafter, referred to as a compound group SX1398).

In the compound (L-5), a compound in which Q is a group represented by Q53, $R^{3b}$ and $R^{3c}$ are hydrogen atoms, and $R^1$ is any of the substituents shown in [Table L1] to [Table L3] (hereinafter, referred to as a compound group SX1399).

In the compound (L-5), a compound in which Q is a group represented by Q53, $R^{3b}$ is a chlorine atom, $R^{3c}$ is a hydrogen atom, and $R^1$ is any of the substituents shown in [Table L1] to [Table L3] (hereinafter, referred to as a compound group SX1400).

In the compound (L-5), a compound in which Q is a group represented by Q53, $R^{3b}$ is a bromine atom, $R^{3c}$ is a hydrogen atom, and $R^1$ is any of the substituents shown in [Table L1] to [Table L3] (hereinafter, referred to as a compound group SX1401).

In the compound (L-5), a compound in which Q is a group represented by Q53, $R^{3b}$ is an iodine atom, $R^{3c}$ is a hydrogen atom, and $R^1$ is any of the substituents shown in [Table L1] to [Table L3] (hereinafter, referred to as a compound group SX1402).

In the compound (L-5), a compound in which Q is a group represented by Q53, $R^{3b}$ is $CF_3$, $R^{3c}$ is a hydrogen atom, and $R^1$ is any of the substituents shown in [Table L1] to [Table L3] (hereinafter, referred to as a compound group SX1403).

In the compound (L-5), a compound in which Q is a group represented by Q53, $R^{3b}$ is a hydrogen atom, $R^{3c}$ is a chlorine atom, and $R^1$ is any of the substituents shown in [Table L1] to [Table L3] (hereinafter, referred to as a compound group SX1404).

In the compound (L-5), a compound in which Q is a group represented by Q53, $R^{3b}$ is a hydrogen atom, $R^{3c}$ is a bromine atom, and $R^1$ is any of the substituents shown in [Table L1] to [Table L3] (hereinafter, referred to as a compound group SX1405).

In the compound (L-5), a compound in which Q is a group represented by Q53, $R^{3b}$ is a hydrogen atom, $R^{3c}$ is an iodine atom, and $R^1$ is any of the substituents shown in [Table L1] to [Table L3] (hereinafter, referred to as a compound group SX1406).

In the compound (L-5), a compound in which Q is a group represented by Q53, $R^{3b}$ is a hydrogen atom, $R^{3c}$ is $CF_3$, and $R^1$ is any of the substituents shown in [Table L1] to [Table L3] (hereinafter, referred to as a compound group SX1407).

In the compound (L-5), a compound in which Q is a group represented by Q54, $R^{3b}$ and $R^{3c}$ are hydrogen atoms, and $R^1$ is any of the substituents shown in [Table L1] to [Table L3] (hereinafter, referred to as a compound group

SX1408).

In the compound (L-5), a compound in which Q is a group represented by Q54, $R^{3b}$ is a chlorine atom, $R^{3c}$ is a hydrogen atom, and $R^1$ is any of the substituents shown in [Table L1] to [Table L3] (hereinafter, referred to as a compound group SX1409).

In the compound (L-5), a compound in which Q is a group represented by Q54, $R^{3b}$ is a bromine atom, $R^{3c}$ is a hydrogen atom, and $R^1$ is any of the substituents shown in [Table L1] to [Table L3] (hereinafter, referred to as a compound group SX1410).

In the compound (L-5), a compound in which Q is a group represented by Q54, $R^{3b}$ is an iodine atom, $R^{3c}$ is a hydrogen atom, and $R^1$ is any of the substituents shown in [Table L1] to [Table L3] (hereinafter, referred to as a compound group SX1411).

In the compound (L-5), a compound in which Q is a group represented by Q54, $R^{3b}$ is $CF_3$, $R^{3c}$ is a hydrogen atom, and $R^1$ is any of the substituents shown in [Table L1] to [Table L3] (hereinafter, referred to as a compound group SX1412).

In the compound (L-5), a compound in which Q is a group represented by Q54, $R^{3b}$ is a hydrogen atom, $R^{3c}$ is a chlorine atom, and $R^1$ is any of the substituents shown in [Table L1] to [Table L3] (hereinafter, referred to as a compound group SX1413).

In the compound (L-5), a compound in which Q is a group represented by Q54, $R^{3b}$ is a hydrogen atom, $R^{3c}$ is a bromine atom, and $R^1$ is any of the substituents shown in [Table L1] to [Table L3] (hereinafter, referred to as a compound group SX1414).

In the compound (L-5), a compound in which Q is a group represented by Q54, $R^{3b}$ is a hydrogen atom, $R^{3c}$ is an iodine atom, and $R^1$ is any of the substituents shown in [Table L1] to [Table L3] (hereinafter, referred to as a compound group SX1415).

In the compound (L-5), a compound in which Q is a group represented by Q54, $R^{3b}$ is a hydrogen atom, $R^{3c}$ is $CF_3$, and $R^1$ is any of the substituents shown in [Table L1] to [Table L3] (hereinafter, referred to as a compound group SX1416).

In the compound (L-5), a compound in which Q is a group represented by Q55, $R^{3b}$ and $R^{3c}$ are hydrogen atoms, and $R^1$ is any of the substituents shown in [Table L1] to [Table L3] (hereinafter, referred to as a compound group SX1417).

In the compound (L-5), a compound in which Q is a group represented by Q55, $R^{3b}$ is a chlorine atom, $R^{3c}$ is a hydrogen atom, and $R^1$ is any of the substituents shown in [Table L1] to [Table L3] (hereinafter, referred to as a compound group SX1418).

In the compound (L-5), a compound in which Q is a group represented by Q55, $R^{3b}$ is a bromine atom, $R^{3c}$ is a hydrogen atom, and $R^1$ is any of the substituents shown in [Table L1] to [Table L3] (hereinafter, referred to as a compound group SX1419).

In the compound (L-5), a compound in which Q is a group represented by Q55, $R^{3b}$ is an iodine atom, $R^{3c}$ is a hydrogen atom, and $R^1$ is any of the substituents shown in [Table L1] to [Table L3] (hereinafter, referred to as a compound group SX1420).

In the compound (L-5), a compound in which Q is a group represented by Q55, $R^{3b}$ is $CF_3$, $R^{3c}$ is a hydrogen atom, and $R^1$ is any of the substituents shown in [Table L1] to [Table L3] (hereinafter, referred to as a compound group SX1421).

In the compound (L-5), a compound in which Q is a group represented by Q55, $R^{3b}$ is a hydrogen atom, $R^{3c}$ is a chlorine atom, and $R^1$ is any of the substituents shown in [Table L1] to [Table L3] (hereinafter, referred to as a compound group SX1422).

In the compound (L-5), a compound in which Q is a group represented by Q55, $R^{3b}$ is a hydrogen atom, $R^{3c}$ is a bromine atom, and $R^1$ is any of the substituents shown in [Table L1] to [Table L3] (hereinafter, referred to as a compound group SX1423).

In the compound (L-5), a compound in which Q is a group represented by Q55, $R^{3b}$ is a hydrogen atom, $R^{3c}$ is an iodine atom, and $R^1$ is any of the substituents shown in [Table L1] to [Table L3] (hereinafter, referred to as a compound group SX1424).

In the compound (L-5), a compound in which Q is a group represented by Q55, $R^{3b}$ is a hydrogen atom, $R^{3c}$ is $CF_3$, and $R^1$ is any of the substituents shown in [Table L1] to [Table L3] (hereinafter, referred to as a compound group SX1425).

In the compound (L-5), a compound in which Q is a group represented by Q56, $R^{3b}$ and $R^{3c}$ are hydrogen atoms, and $R^1$ is any of the substituents shown in [Table L1] to [Table L3] (hereinafter, referred to as a compound group SX1426).

In the compound (L-5), a compound in which Q is a group represented by Q56, $R^{3b}$ is a chlorine atom, $R^{3c}$ is a hydrogen atom, and $R^1$ is any of the substituents shown in [Table L1] to [Table L3] (hereinafter, referred to as a compound group SX1427).

In the compound (L-5), a compound in which Q is a group represented by Q56, $R^{3b}$ is a bromine atom, $R^{3c}$ is a hydrogen atom, and $R^1$ is any of the substituents shown in [Table L1] to [Table L3] (hereinafter, referred to as a compound group SX1428).

In the compound (L-5), a compound in which Q is a group represented by Q56, $R^{3b}$ is an iodine atom, $R^{3c}$ is a hydrogen atom, and $R^1$ is any of the substituents shown in [Table L1] to [Table L3] (hereinafter, referred to as a compound group SX1429).

In the compound (L-5), a compound in which Q is a group represented by Q56, $R^{3b}$ is $CF_3$, $R^{3c}$ is a hydrogen atom, and $R^1$ is any of the substituents shown in [Table L1] to [Table L3] (hereinafter, referred to as a compound group SX1430).

In the compound (L-5), a compound in which Q is a group represented by Q56, $R^{3b}$ is a hydrogen atom, $R^{3c}$ is a chlorine atom, and $R^1$ is any of the substituents shown in [Table L1] to [Table L3] (hereinafter, referred to as a compound group SX1431).

In the compound (L-5), a compound in which Q is a group represented by Q56, $R^{3b}$ is a hydrogen atom, $R^{3c}$ is a bromine atom, and $R^1$ is any of the substituents shown in [Table L1] to [Table L3] (hereinafter, referred to as a compound group SX1432).

In the compound (L-5), a compound in which Q is a group represented by Q56, $R^{3b}$ is a hydrogen atom, $R^{3c}$ is an iodine atom, and $R^1$ is any of the substituents shown in [Table L1] to [Table L3] (hereinafter, referred to as a compound group SX1433).

In the compound (L-5), a compound in which Q is a group represented by Q56, $R^{3b}$ is a hydrogen atom, $R^{3c}$ is $CF_3$, and $R^1$ is any of the substituents shown in [Table L1] to [Table L3] (hereinafter, referred to as a compound group SX1434).

In the compound (L-5), a compound in which Q is a group represented by Q57, $R^{3b}$ and $R^{3c}$ are hydrogen atoms, and $R^1$ is any of the substituents shown in [Table L1] to [Table L3] (hereinafter, referred to as a compound group SX1435).

In the compound (L-5), a compound in which Q is a group represented by Q57, $R^{3b}$ is a chlorine atom, $R^{3c}$ is a hydrogen atom, and $R^1$ is any of the substituents shown in [Table L1] to [Table L3] (hereinafter, referred to as a compound group SX1436).

In the compound (L-5), a compound in which Q is a group represented by Q57, $R^{3b}$ is a bromine atom, $R^{3c}$ is a hydrogen atom, and $R^1$ is any of the substituents shown in [Table L1] to [Table L3] (hereinafter, referred to as a compound group SX1437).

In the compound (L-5), a compound in which Q is a group represented by Q57, $R^{3b}$ is an iodine atom, $R^{3c}$ is a hydrogen atom, and $R^1$ is any of the substituents shown in [Table L1] to [Table L3] (hereinafter, referred to as a compound group SX1438).

In the compound (L-5), a compound in which Q is a group represented by Q57, $R^{3b}$ is $CF_3$, $R^{3c}$ is a hydrogen atom, and $R^1$ is any of the substituents shown in [Table L1] to [Table L3] (hereinafter, referred to as a compound group SX1439).

In the compound (L-5), a compound in which Q is a group represented by Q57, $R^{3b}$ is a hydrogen atom, $R^{3c}$ is a chlorine atom, and $R^1$ is any of the substituents shown in [Table L1] to [Table L3] (hereinafter, referred to as a compound group SX1440).

In the compound (L-5), a compound in which Q is a group represented by Q57, $R^{3b}$ is a hydrogen atom, $R^{3c}$ is a bromine atom, and $R^1$ is any of the substituents shown in [Table L1] to [Table L3] (hereinafter, referred to as a compound group SX1441).

In the compound (L-5), a compound in which Q is a group represented by Q57, $R^{3b}$ is a hydrogen atom, $R^{3c}$ is an iodine atom, and $R^1$ is any of the substituents shown in [Table L1] to [Table L3] (hereinafter, referred to as a compound group SX1442).

In the compound (L-5), a compound in which Q is a group represented by Q57, $R^{3b}$ is a hydrogen atom, $R^{3c}$ is $CF_3$, and $R^1$ is any of the substituents shown in [Table L1] to [Table L3] (hereinafter, referred to as a compound group SX1443).

The groups represented by Q21 to Q26 and the groups represented by Q58 to Q61 are groups shown below.

[Chem. 96]

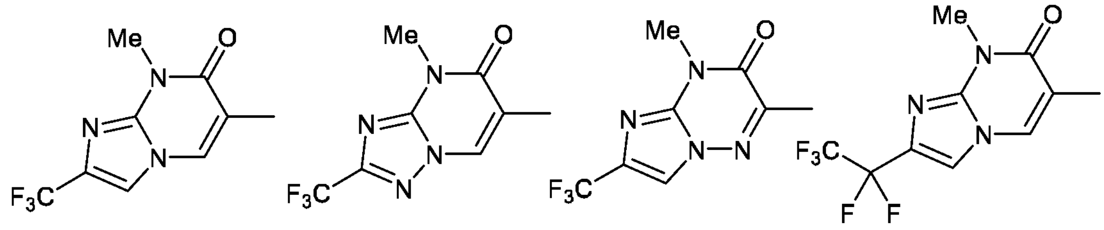

Q21 Q22 Q23 Q24

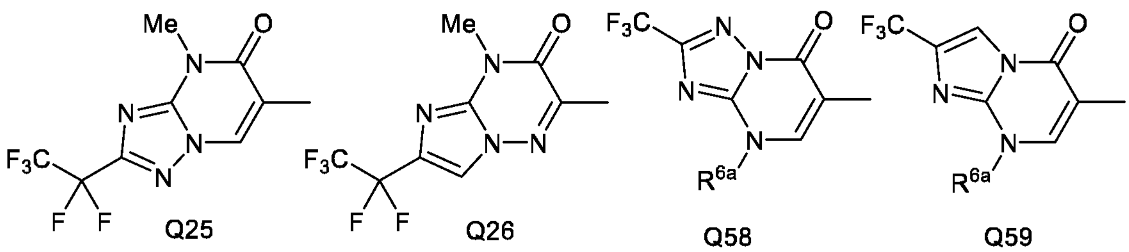

Q25 Q26 Q58 Q59

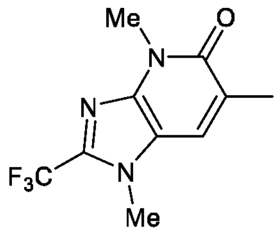

Q60

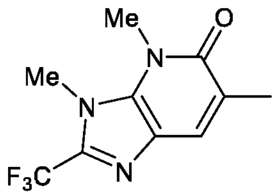

Q61

[Chem. 97]

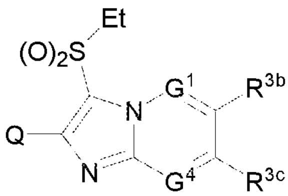

(L-6)

In a compound (hereinafter, referred to as a compound (L-6)) represented by Formula (L-6), a compound in which Q is a group represented by Q21, $G^1$ and $G^4$ are CHs, $R^{3b}$ is any of the substituents shown in [Table L4] to [Table L12], and $R^{3c}$ is a hydrogen atom (hereinafter, referred to as a compound group SX136).

[Table 3]

[Table L4]

| |
|---|
| F |
| Cl |
| Br |
| I |
| Me |
| Et |
| Pr |
| i-Pr |
| $CF_3$ |
| $CHF_2$ |
| CH=$CH_2$ |
| CMe=$CH_2$ |
| 1-F-c-Pr |
| 2,2-$F_2$-c-Pr |
| c-Pr |
| c-Bu |
| c-Pen |
| c-Hex |
| 1-CN-c-Pr |
| 1-CN-c-Bu |
| 1-CN-c-Pen |
| 1-CN-c-Hex |
| CHO |
| C(O)Me |
| C(O)c-Pr |
| C(O)OEt |
| C(O)$NMe_2$ |
| C(O)NHc-Pr |
| CH=N-OH |
| CH=N-OMe |
| CH=N-OEt |
| CH=N-$OCH_2CF_3$ |
| CMe=N-OH |
| CMe=N-OMe |
| CMe=N-OEt |
| CMe=N-$OCH_2CF_3$ |

[Table L5]

| |
|---|
| Ph |
| 3-F-Ph |
| 4-F-Ph |
| 3-Cl-Ph |
| 4-Cl-Ph |
| 3-$CF_3$-Ph |
| 4-$CF_3$-Ph |
| 3-$NMe_2$-Ph |
| 4-$NMe_2$-Ph |
| 3-CN-Ph |
| 4-CN-Ph |
| 4-C(O)$NMe_2$-Ph |
| 4-NHC(O)Me-Ph |
| 3,4-$F_2$-Ph |
| 3,5-$F_2$-Ph |
| 2,4-$F_2$-Ph |
| 3,4,5-$F_3$-Ph |
| 3,4-$Cl_2$-Ph |
| 3,5-$Cl_2$-Ph |
| 3,5-$Cl_2$-4-F-Ph |
| OPh |
| O-2-F-Ph |
| O-3-$CF_3$-Ph |
| O-4-$CF_3$-Ph |
| $NH_2$ |
| $NHCH_2CF_3$ |
| NHc-Pr |
| NH(1-CN-c-Pr) |
| NHOMe |
| $NMe_2$ |
| NHC(O)Me |
| NHC(O)c-Pr |
| NMeC(O)c-Pr |
| CN |
| $NO_2$ |
| $CMe_2CN$ |
| $CMe(CN)_2$ |

[Table L6]

| |
|---|
| Py2 |
| 4-F-Py2 |
| 5-F-Py2 |
| 4-Cl-Py2 |
| 5-Cl-Py2 |
| 4-$CF_3$-Py2 |
| 5-$CF_3$-Py2 |
| 6-$CF_3$-Py2 |
| 3-Me-Py2 |
| 4-Me-Py2 |
| 5-Me-Py2 |
| 6-Me-Py2 |
| 4-CN-Py2 |
| 5-CN-Py2 |
| 5-$OCH_2CF_2CF_3$-Py2 |
| 3,5-$F_2$-Py2 |
| Py3 |
| 6-$CF_3$-Py3 |
| 5-$CF_3$-Py3 |
| 6-F-Py3 |
| 6-Cl-Py3 |
| Py4 |
| OPy2 |
| OPy3 |
| OPy4 |
| O-5-$CF_3$-Py2 |
| O-6-$CF_3$-Py2 |
| OMe |
| OEt |
| OPr |
| Oi-Pr |
| Oc-Pr |
| $OCMe_2CN$ |
| SEt |
| S(O)Et |
| $S(O)_2Et$ |

[Table 4]

| [Table L7] | [Table L8] | [Table L9] |
|---|---|---|
| 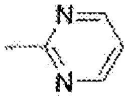 | 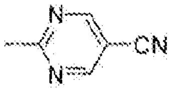 | 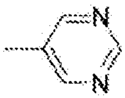 |
| 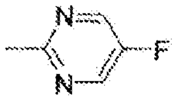 | 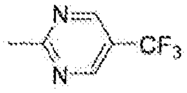 | 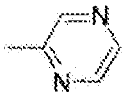 |
| 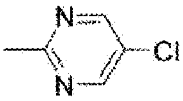 | 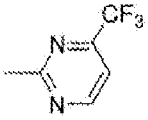 | 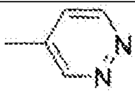 |
| 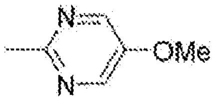 | 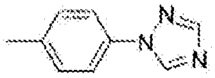 | 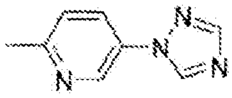 |
| 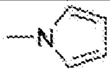 | 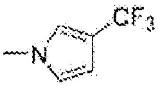 | 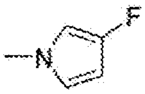 |
| 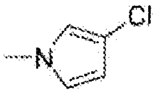 | 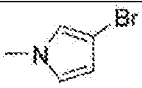 | 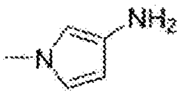 |
| 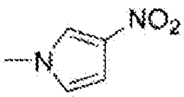 | 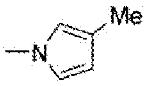 | 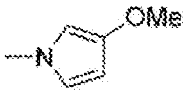 |
| 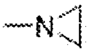 | 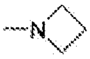 | 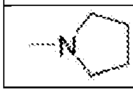 |

[Table 5]

[Table L10]

| |
|---|
| 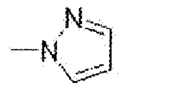 |
| 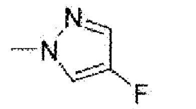 |
| 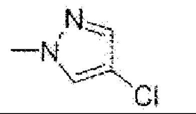 |
| 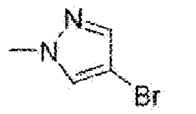 |
| 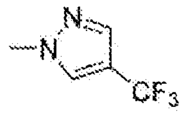 |
| 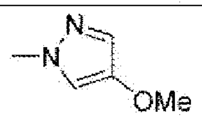 |
| 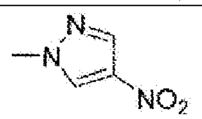 |
| 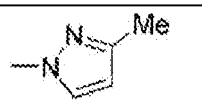 |
| 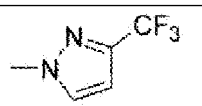 |
| 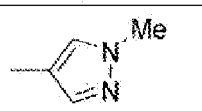 |

[Table L11]

| |
|---|
| 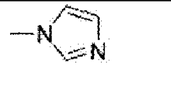 |
| 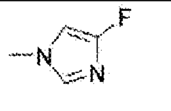 |
| 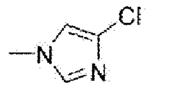 |
| 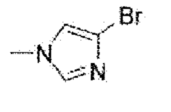 |
| 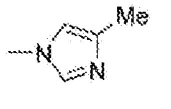 |
| 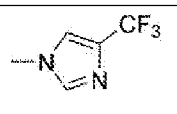 |
| 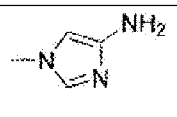 |
| 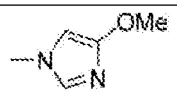 |
| 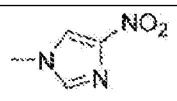 |
| 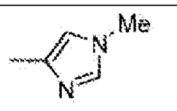 |

[Table L12]

| |
|---|
| 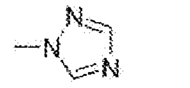 |
| 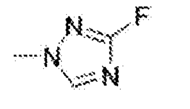 |
| 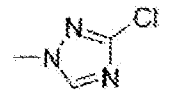 |
| 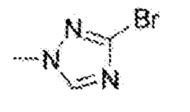 |
| 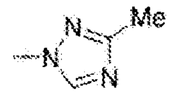 |
| 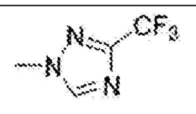 |
| 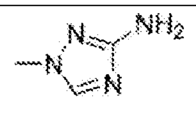 |
| 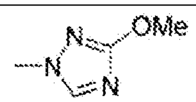 |
| 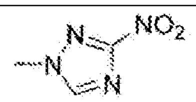 |
| 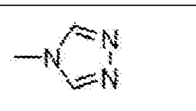 |

In the compound (L-6), a compound in which Q is a group represented by Q22, $G^1$ and $G^4$ are CHs, $R^{3b}$ is any of the substituents shown in [Table L4] to [Table L12], and $R^{3c}$ is a hydrogen atom (hereinafter, referred to as a compound group SX137).

In the compound (L-6), a compound in which Q is a group represented by Q23, $G^1$ and $G^4$ are CHs, $R^{3b}$ is any of the substituents shown in [Table L4] to [Table L12], and $R^{3c}$ is a hydrogen atom (hereinafter, referred to as a compound group SX138).

In the compound (L-6), a compound in which Q is a group represented by Q24, $G^1$ and $G^4$ are CHs, $R^{3b}$ is any of the substituents shown in [Table L4] to [Table L12], and $R^{3c}$ is a hydrogen atom (hereinafter, referred to as a compound group SX139).

In the compound (L-6), a compound in which Q is a group represented by Q25, $G^1$ and $G^4$ are CHs, $R^{3b}$ is any of the substituents shown in [Table L4] to [Table L12], and $R^{3c}$ is a hydrogen atom (hereinafter, referred to as a compound group SX140).

In the compound (L-6), a compound in which Q is a group represented by Q26, $G^1$ and $G^4$ are CHs, $R^{3b}$ is any of the substituents shown in [Table L4] to [Table L12], and $R^{3c}$ is a hydrogen atom (hereinafter, referred to as a compound group SX141).

In the compound (L-6), a compound in which Q is a group represented by Q21, $G^1$ and $G^4$ are CHs, $R^{3b}$ is hydrogen atom, and $R^{3c}$ is any of the substituents shown in [Table L4] to [Table L12] (hereinafter, referred to as a compound group SX142).

In the compound (L-6), a compound in which Q is a group represented by Q22, $G^1$ and $G^4$ are CHs, $R^{3b}$ is hydrogen atom, and $R^{3c}$ is any of the substituents shown in [Table L4] to [Table L12] (hereinafter, referred to as a

compound group SX143).

In the compound (L-6), a compound in which Q is a group represented by Q23, $G^1$ and $G^4$ are CHs, $R^{3b}$ is hydrogen atom, and $R^{3c}$ is any of the substituents shown in [Table L4] to [Table L12] (hereinafter, referred to as a compound group SX144).

In the compound (L-6), a compound in which Q is a group represented by Q24, $G^1$ and $G^4$ are CHs, $R^{3b}$ is hydrogen atom, and $R^{3c}$ is any of the substituents shown in [Table L4] to [Table L12] (hereinafter, referred to as a compound group SX145).

In the compound (L-6), a compound in which Q is a group represented by Q25, $G^1$ and $G^4$ are CHs, $R^{3b}$ is hydrogen atom, and $R^{3c}$ is any of the substituents shown in [Table L4] to [Table L12] (hereinafter, referred to as a compound group SX146).

In the compound (L-6), a compound in which Q is a group represented by Q26, $G^1$ and $G^4$ are CHs, $R^{3b}$ is hydrogen atom, and $R^{3c}$ is any of the substituents shown in [Table L4] to [Table L12] (hereinafter, referred to as a compound group SX147).

In the compound (L-6), a compound in which Q is a group represented by Q21, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX148).

In the compound (L-6), a compound in which Q is a group represented by Q22, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX149).

In the compound (L-6), a compound in which Q is a group represented by Q23, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX150).

In the compound (L-6), a compound in which Q is a group represented by Q24, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX151).

In the compound (L-6), a compound in which Q is a group represented by Q25, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX152).

In the compound (L-6), a compound in which Q is a group represented by Q26, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX153).

In the compound (L-6), a compound in which Q is a group represented by Q21, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX154).

In the compound (L-6), a compound in which Q is a group represented by Q22, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX155).

In the compound (L-6), a compound in which Q is a group represented by Q23, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX156).

In the compound (L-6), a compound in which Q is a group represented by Q24, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX157).

In the compound (L-6), a compound in which Q is a group represented by Q25, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX158).

In the compound (L-6), a compound in which Q is a group represented by Q26, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX159).

In the compound (L-6), a compound in which Q is a group represented by Q21, $G^1$ is CH, $G^4$ is a nitrogen atom, $R^{3b}$ is any of the substituents shown in [Table L4] to [Table L12], and $R^{3c}$ is a hydrogen atom (hereinafter, referred to as a compound group SX160).

In the compound (L-6), a compound in which Q is a group represented by Q22, $G^1$ and $G^4$ are CHs, $R^{3b}$ is any of the substituents shown in [Table L4] to [Table L12], and $R^{3c}$ is a hydrogen atom (hereinafter, referred to as a compound group SX161).

In the compound (L-6), a compound in which Q is a group represented by Q23, $G^1$ and $G^4$ are CHs, $R^{3b}$ is any of the substituents shown in [Table L4] to [Table L12], and $R^{3c}$ is a hydrogen atom (hereinafter, referred to as a compound group SX162).

In the compound (L-6), a compound in which Q is a group represented by Q24, $G^1$ and $G^4$ are CHs, $R^{3b}$ is any of the substituents shown in [Table L4] to [Table L12], and $R^{3c}$ is a hydrogen atom (hereinafter, referred to as a compound group SX163).

In the compound (L-6), a compound in which Q is a group represented by Q25, $G^1$ and $G^4$ are CHs, $R^{3b}$ is any of the substituents shown in [Table L4] to [Table L12], and $R^{3c}$ is a hydrogen atom (hereinafter, referred to as a compound group SX164).

In the compound (L-6), a compound in which Q is a group represented by Q26, $G^1$ and $G^4$ are CHs, $R^{3b}$ is any of the substituents shown in [Table L4] to [Table L12], and $R^{3c}$ is a hydrogen atom (hereinafter, referred to as a compound group SX165).

In the compound (L-6), a compound in which Q is a group represented by Q21, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX166).

In the compound (L-6), a compound in which Q is a group represented by Q22, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX167).

In the compound (L-6), a compound in which Q is a group represented by Q23, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX168).

In the compound (L-6), a compound in which Q is a group represented by Q24, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX169).

In the compound (L-6), a compound in which Q is a group represented by Q25, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX170).

In the compound (L-6), a compound in which Q is a group represented by Q26, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX171).

In the compound (L-6), a compound in which Q is a group represented by Q58, $R^{6a}$ is a hydrogen atom, $G^1$ and $G^4$ are CHs, $R^{3b}$ is any of the substituents shown in [Table L4] to [Table L12], and $R^{3c}$ is a hydrogen atom (hereinafter, referred to as a compound group SX1444).

In the compound (L-6), a compound in which Q is a group represented by Q58, $R^{6a}$ is a hydrogen atom, $G^1$ and $G^4$ are CHs, $R^{3b}$ is a hydrogen atom, and $R^{3c}$ is any of the substituents shown in [Table L4] to [Table L12] (hereinafter, referred to as a compound group SX1445).

In the compound (L-6), a compound in which Q is a group represented by Q58, $R^{6a}$ is a hydrogen atom, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX1446).

In the compound (L-6), a compound in which Q is a group represented by Q58, $R^{6a}$ is a hydrogen atom, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX1447).

In the compound (L-6), a compound in which Q is a group represented by Q58, $R^{6a}$ is a hydrogen atom, $G^1$ is CH, $G^4$ is a nitrogen atom, $R^{3b}$ is any of the substituents shown in [Table L4] to [Table L12], and $R^{3c}$ is a hydrogen atom (hereinafter, referred to as a compound group SX1448).

In the compound (L-6), a compound in which Q is a group represented by Q58, $R^{6a}$ is a hydrogen atom, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX1449).

In the compound (L-6), a compound in which Q is a group represented by Q58, $R^{6a}$ is a methyl group, $G^1$ and $G^4$ are CHs, $R^{3b}$ is any of the substituents shown in [Table L4] to [Table L12], and $R^{3c}$ is a hydrogen atom (hereinafter, referred to as a compound group SX1450).

In the compound (L-6), a compound in which Q is a group represented by Q58, $R^{6a}$ is a methyl group, $G^1$ and $G^4$ are CHs, $R^{3b}$ is a hydrogen atom, and $R^{3c}$ is any of the substituents shown in [Table L4] to [Table L12] (hereinafter, referred to as a compound group SX1451).

In the compound (L-6), a compound in which Q is a group represented by Q58, $R^{6a}$ is a methyl group, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX1452).

In the compound (L-6), a compound in which Q is a group represented by Q58, $R^{6a}$ is a methyl group, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX1453).

In the compound (L-6), a compound in which Q is a group represented by Q58, $R^{6a}$ is a methyl group, $G^1$ is

CH, $G^4$ is a nitrogen atom, $R^{3b}$ is any of the substituents shown in [Table L4] to [Table L12], and $R^{3c}$ is a hydrogen atom (hereinafter, referred to as a compound group SX1454).

In the compound (L-6), a compound in which Q is a group represented by Q58, $R^{6a}$ is a methyl group, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX1455).

In the compound (L-6), a compound in which Q is a group represented by Q59, $R^{6a}$ is a hydrogen atom, $G^1$ and $G^4$ are CHs, $R^{3b}$ is any of the substituents shown in [Table L4] to [Table L12], and $R^{3c}$ is a hydrogen atom (hereinafter, referred to as a compound group SX1456).

In the compound (L-6), a compound in which Q is a group represented by Q59, $R^{6a}$ is a hydrogen atom, $G^1$ and $G^4$ are CHs, $R^{3b}$ is a hydrogen atom, and $R^{3c}$ is any of the substituents shown in [Table L4] to [Table L12] (hereinafter, referred to as a compound group SX1457).

In the compound (L-6), a compound in which Q is a group represented by Q59, $R^{6a}$ is a hydrogen atom, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX1458).

In the compound (L-6), a compound in which Q is a group represented by Q59, $R^{6a}$ is a hydrogen atom, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX1459).

In the compound (L-6), a compound in which Q is a group represented by Q59, $R^{6a}$ is a hydrogen atom, $G^1$ is CH, $G^4$ is a nitrogen atom, $R^{3b}$ is any of the substituents shown in [Table L4] to [Table L12], and $R^{3c}$ is a hydrogen atom (hereinafter, referred to as a compound group SX1460).

In the compound (L-6), a compound in which Q is a group represented by Q59, $R^{6a}$ is a hydrogen atom, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX1461).

In the compound (L-6), a compound in which Q is a group represented by Q59, $R^{6a}$ is a methyl group, $G^1$ and $G^4$ are CHs, $R^{3b}$ is any of the substituents shown in [Table L4] to [Table L12], and $R^{3c}$ is a hydrogen atom (hereinafter, referred to as a compound group SX1462).

In the compound (L-6), a compound in which Q is a group represented by Q59, $R^{6a}$ is a methyl group, $G^1$ and $G^4$ are CHs, $R^{3b}$ is a hydrogen atom, $R^{3c}$ is any of the substituents shown in [Table L4] to [Table L12] (hereinafter, referred to as a compound group SX1463).

In the compound (L-6), a compound in which Q is a group represented by Q59, $R^{6a}$ is a methyl group, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX1464).

In the compound (L-6), a compound in which Q is a group represented by Q59, $R^{6a}$ is a methyl group, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX1465).

In the compound (L-6), a compound in which Q is a group represented by Q59, $R^{6a}$ is a methyl group, $G^1$ is CH, $G^4$ is a nitrogen atom, $R^{3b}$ is any of the substituents shown in [Table L4] to [Table L12], and $R^{3c}$ is a hydrogen atom (hereinafter, referred to as a compound group SX1466).

In the compound (L-6), a compound in which Q is a group represented by Q59, $R^{6a}$ is a methyl group, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX1467).

In the compound (L-6), a compound in which Q is a group represented by Q60, $G^1$ and $G^4$ are CHs, $R^{3b}$ is any of the substituents shown in [Table L4] to [Table L12], and $R^{3c}$ is a hydrogen atom (hereinafter, referred to as a compound group SX1468).

In the compound (L-6), a compound in which Q is a group represented by Q60, $G^1$ and $G^4$ are CHs, $R^{3b}$ is hydrogen atom, and $R^{3c}$ is any of the substituents shown in [Table L4] to [Table L12] (hereinafter, referred to as a compound group SX1469).

In the compound (L-6), a compound in which Q is a group represented by Q60, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX1470).

In the compound (L-6), a compound in which Q is a group represented by Q60, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX1471).

In the compound (L-6), a compound in which Q is a group represented by Q60, $G^1$ is CH, $G^4$ is a nitrogen atom, $R^{3b}$ is any of the substituents shown in [Table L4] to [Table L12], and $R^{3c}$ is a hydrogen atom (hereinafter, referred to as a compound group SX1472).

In the compound (L-6), a compound in which Q is a group represented by Q60, $G^1$ is CH, $G^4$ is a nitrogen atom, $R^{3b}$ is a hydrogen atom, and $R^{3c}$ is any of the substituents shown in [Table L4] to [Table L12] (hereinafter, referred to

as a compound group SX1473).

In the compound (L-6), a compound in which Q is a group represented by Q61, $G^1$ and $G^4$ are CHs, $R^{3b}$ is any of the substituents shown in [Table L4] to [Table L12], and $R^{3c}$ is a hydrogen atom (hereinafter, referred to as a compound group SX1474).

In the compound (L-6), a compound in which Q is a group represented by Q61, $G^1$ and $G^4$ are CHs, $R^{3b}$ is hydrogen atom, and $R^{3c}$ is any of the substituents shown in [Table L4] to [Table L12] (hereinafter, referred to as a compound group SX1475).

In the compound (L-6), a compound in which Q is a group represented by Q61, $G^1$ is a nitrogen atom, $G^4$ is CH, $R^{3b}$ is any of the substituents shown in [Table L4] to [Table L12], and $R^{3c}$ is a hydrogen atom (hereinafter, referred to as a compound group SX1476).

In the compound (L-6), a compound in which Q is a group represented by Q61, $G^1$ is a nitrogen atom, $G^4$ is CH, $R^{3b}$ is a hydrogen atom, and $R^{3c}$ is any of the substituents shown in [Table L4] to [Table L12] (hereinafter, referred to as a compound group SX1477).

In the compound (L-6), a compound in which Q is a group represented by Q61, $G^1$ is CH, $G^4$ is a nitrogen atom, $R^{3b}$ is any of the substituents shown in [Table L4] to [Table L12], and $R^{3c}$ is a hydrogen atom (hereinafter, referred to as a compound group SX1478).

In the compound (L-6), a compound in which Q is a group represented by Q61, $G^1$ is CH, $G^4$ is a nitrogen atom, $R^{3b}$ is a hydrogen atom, and $R^{3c}$ is any of the substituents shown in [Table L4] to [Table L12] (hereinafter, referred to as a compound group SX1479).

[Chem. 98]

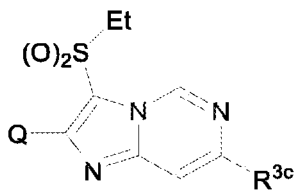

(L-7)

In a compound (hereinafter, referred to as a compound (L-7)) represented by Formula (L-7), a compound in which Q is a group represented by Q21, $R^{3c}$ is any of the substituents shown in [Table L4] to [Table L12] (hereinafter, referred to as a compound group SX172).

In the compound (L-7), a compound in which Q is a group represented by Q22, $R^{3c}$ is any of the substituents shown in [Table L4] to [Table L12] (hereinafter, referred to as a compound group SX173).

In the compound (L-7), a compound in which Q is a group represented by Q23, $R^{3c}$ is any of the substituents shown in [Table L4] to [Table L12] (hereinafter, referred to as a compound group SX174).

In the compound (L-7), a compound in which Q is a group represented by Q24, $R^{3c}$ is any of the substituents shown in [Table L4] to [Table L12] (hereinafter, referred to as a compound group SX175).

In the compound (L-7), a compound in which Q is a group represented by Q25, $R^{3c}$ is any of the substituents shown in [Table L4] to [Table L12] (hereinafter, referred to as a compound group SX176).

In the compound (L-7), a compound in which Q is a group represented by Q26, $R^{3c}$ is any of the substituents shown in [Table L4] to [Table L12] (hereinafter, referred to as a compound group SX177).

In the compound (L-7), a compound in which Q is a group represented by Q58, $R^{6a}$ is a hydrogen atom, and $R^{3c}$ is any of the substituents shown in [Table L4] to [Table L12] (hereinafter, referred to as a compound group SX1480).

In the compound (L-7), a compound in which Q is a group represented by Q58, $R^{6a}$ is a methyl group, and $R^{3c}$ is any of the substituents shown in [Table L4] to [Table L12] (hereinafter, referred to as a compound group SX1481).

In the compound (L-7), a compound in which Q is a group represented by Q59, $R^{6a}$ is a hydrogen atom, and $R^{3c}$ is any of the substituents shown in [Table L4] to [Table L12] (hereinafter, referred to as a compound group SX1482).

In the compound (L-7), a compound in which Q is a group represented by Q59, $R^{6a}$ is a methyl group, and $R^{3c}$ is any of the substituents shown in [Table L4] to [Table L12] (hereinafter, referred to as a compound group SX1483).

In the compound (L-7), a compound in which Q is a group represented by Q60, $R^{3c}$ is any of the substituents shown in [Table L4] to [Table L12] (hereinafter, referred to as a compound group SX1484).

In the compound (L-7), a compound in which Q is a group represented by Q61, $R^{3c}$ is any of the substituents shown in [Table L4] to [Table L12] (hereinafter, referred to as a compound group SX1485).

[Chem. 99]

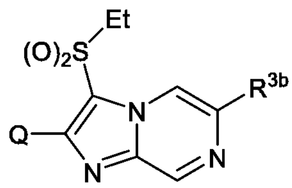

(L-8)

In a compound (hereinafter, referred to as a compound (L-8)) represented by Formula (L-8), a compound in which Q is a group represented by Q21, $R^{3b}$ is any of the substituents shown in [Table L4] to [Table L12] (hereinafter, referred to as a compound group SX178).

In the compound (L-8), a compound in which Q is a group represented by Q22, $R^{3b}$ is any of the substituents shown in [Table L4] to [Table L12] (hereinafter, referred to as a compound group SX179).

In the compound (L-8), a compound in which Q is a group represented by Q23, $R^{3b}$ is any of the substituents shown in [Table L4] to [Table L12] (hereinafter, referred to as a compound group SX180).

In the compound (L-8), a compound in which Q is a group represented by Q24, $R^{3b}$ is any of the substituents shown in [Table L4] to [Table L12] (hereinafter, referred to as a compound group SX181).

In the compound (L-8), a compound in which Q is a group represented by Q25, $R^{3b}$ is any of the substituents shown in [Table L4] to [Table L12] (hereinafter, referred to as a compound group SX182).

In the compound (L-8), a compound in which Q is a group represented by Q26, $R^{3b}$ is any of the substituents shown in [Table L4] to [Table L12] (hereinafter, referred to as a compound group SX183).

In the compound (L-8), a compound in which Q is a group represented by Q58, $R^{6a}$ is a hydrogen atom, and $R^{3b}$ is any of the substituents shown in [Table L4] to [Table L12] (hereinafter, referred to as a compound group SX1486).

In the compound (L-8), a compound in which Q is a group represented by Q58, $R^{6a}$ is a methyl group, and $R^{3b}$ is any of the substituents shown in [Table L4] to [Table L12] (hereinafter, referred to as a compound group SX1487).

In the compound (L-8), a compound in which Q is a group represented by Q59, $R^{6a}$ is a hydrogen atom, and $R^{3b}$ is any of the substituents shown in [Table L4] to [Table L12] (hereinafter, referred to as a compound group SX1488).

In the compound (L-8), a compound in which Q is a group represented by Q59, $R^{6a}$ is a methyl group, and $R^{3b}$ is any of the substituents shown in [Table L4] to [Table L12] (hereinafter, referred to as a compound group SX1489).

In the compound (L-8), a compound in which Q is a group represented by Q60, $R^{3b}$ is any of the substituents shown in [Table L4] to [Table L12] (hereinafter, referred to as a compound group SX1490).

In the compound (L-8), a compound in which Q is a group represented by Q61, $R^{3b}$ is any of the substituents shown in [Table L4] to [Table L12] (hereinafter, referred to as a compound group SX1491).

Next, examples of production examples described in examples and intermediates the compound X of present invention produced in accordance with any of the production methods described in the present specification are shown below.

[Chem. 100]

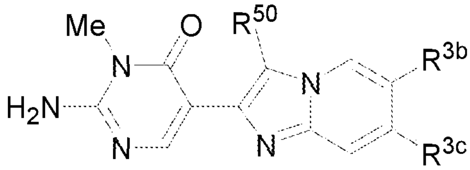

(M-1)

In a compound (hereinafter, referred to as a compound (M-1)) represented by Formula (M-1), a compound in which $R^{50}$ is a hydrogen atom, $R^{3b}$ is any of the substituents shown in [Table L4] to [Table L12], and $R^{3c}$ is a hydrogen atom.

In the compound (M-1), a compound in which $R^{50}$ is SMe, $R^{3b}$ is any of the substituents shown in [Table L4] to [Table L12], and $R^{3c}$ is a hydrogen atom.

In the compound (M-1), a compound in which $R^{50}$ is SEt, $R^{3b}$ is any of the substituents shown in [Table L4] to [Table L12], and $R^{3c}$ is a hydrogen atom.

In the compound (M-1), a compound in which $R^{50}$ is S(O)Me, $R^{3b}$ is any of the substituents shown in [Table L4] to [Table L12], and $R^{3c}$ is a hydrogen atom.

In the compound (M-1), a compound in which $R^{50}$ is S(O)Et, $R^{3b}$ is any of the substituents shown in [Table L4] to [Table L12], and $R^{3c}$ is a hydrogen atom.

In the compound (M-1), a compound in which $R^{50}$ is $S(O)_2Me$, $R^{3b}$ is any of the substituents shown in [Table

L4] to [Table L12], and $R^{3c}$ is a hydrogen atom.

In the compound (M-1), a compound in which $R^{50}$ is $S(O)_2Et$, $R^{3b}$ is any of the substituents shown in [Table L4] to [Table L12], and $R^{3c}$ is a hydrogen atom.

In the compound (M-1), a compound in which $R^{50}$ is a hydrogen atom, $R^{3b}$ is a hydrogen atom, and $R^{3c}$ is any of the substituents shown in [Table L4] to [Table L12].

In the compound (M-1), a compound in which $R^{50}$ is SMe, $R^{3b}$ is a hydrogen atom, and $R^{3c}$ is any of the substituents shown in [Table L4] to [Table L12].

In the compound (M-1), a compound in which $R^{50}$ is SEt, $R^{3b}$ is a hydrogen atom, and $R^{3c}$ is any of the substituents shown in [Table L4] to [Table L12].

In the compound (M-1), a compound in which $R^{50}$ is S(O)Me, $R^{3b}$ is a hydrogen atom, and $R^{3c}$ is any of the substituents shown in [Table L4] to [Table L12].

In the compound (M-1), a compound in which $R^{50}$ is S(O)Et, $R^{3b}$ is a hydrogen atom, and $R^{3c}$ is any of the substituents shown in [Table L4] to [Table L12].

In the compound (M-1), a compound in which $R^{50}$ is $S(O)_2Me$, $R^{3b}$ is a hydrogen atom, and $R^{3c}$ is any of the substituents shown in [Table L4] to [Table L12].

In the compound (M-1), a compound in which $R^{50}$ is $S(O)_2Et$, $R^{3b}$ is a hydrogen atom, and $R^{3c}$ is any of the substituents shown in [Table L4] to [Table L12].

[Chem. 101]

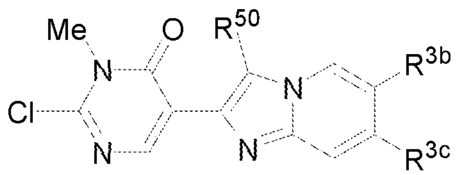

(M-2)

In a compound (hereinafter, referred to as a compound (M-2)) represented by Formula (M-2), a compound in which $R^{50}$ is a hydrogen atom, $R^{3b}$ is any of the substituents shown in [Table L4] to [Table L12], and $R^{3c}$ is a hydrogen atom.

In the compound (M-2), a compound in which $R^{50}$ is SMe, $R^{3b}$ is any of the substituents shown in [Table L4] to [Table L12], and $R^{3c}$ is a hydrogen atom.

In the compound (M-2), a compound in which $R^{50}$ is SEt, $R^{3b}$ is any of the substituents shown in [Table L4] to [Table L12], and $R^{3c}$ is a hydrogen atom.

In the compound (M-2), a compound in which $R^{50}$ is S(O)Me, $R^{3b}$ is any of the substituents shown in [Table L4] to [Table L12], and $R^{3c}$ is a hydrogen atom.

In the compound (M-2), a compound in which $R^{50}$ is S(O)Et, $R^{3b}$ is any of the substituents shown in [Table L4] to [Table L12], and $R^{3c}$ is a hydrogen atom.

In the compound (M-2), a compound in which $R^{50}$ is $S(O)_2Me$, $R^{3b}$ is any of the substituents shown in [Table L4] to [Table L12], and $R^{3c}$ is a hydrogen atom.

In the compound (M-2), a compound in which $R^{50}$ is $S(O)_2Et$, $R^{3b}$ is any of the substituents shown in [Table L4] to [Table L12], and $R^{3c}$ is a hydrogen atom.

In the compound (M-2), a compound in which $R^{50}$ is a hydrogen atom, $R^{3b}$ is a hydrogen atom, and $R^{3c}$ is any of the substituents shown in [Table L4] to [Table L12].

In the compound (M-2), a compound in which $R^{50}$ is SMe, $R^{3b}$ is a hydrogen atom, and $R^{3c}$ is any of the substituents shown in [Table L4] to [Table L12].

In the compound (M-2), a compound in which $R^{50}$ is SEt, $R^{3b}$ is a hydrogen atom, and $R^{3c}$ is any of the substituents shown in [Table L4] to [Table L12].

In the compound (M-2), a compound in which $R^{50}$ is S(O)Me, $R^{3b}$ is a hydrogen atom, and $R^{3c}$ is any of the substituents shown in [Table L4] to [Table L12].

In the compound (M-2), a compound in which $R^{50}$ is S(O)Et, $R^{3b}$ is a hydrogen atom, and $R^{3c}$ is any of the substituents shown in [Table L4] to [Table L12].

In the compound (M-2), a compound in which $R^{50}$ is $S(O)_2Me$, $R^{3b}$ is a hydrogen atom, and $R^{3c}$ is any of the substituents shown in [Table L4] to [Table L12].

In the compound (M-2), a compound in which $R^{50}$ is $S(O)_2Et$, $R^{3b}$ is a hydrogen atom, and $R^{3c}$ is any of the substituents shown in [Table L4] to [Table L12].

[Chem. 102]

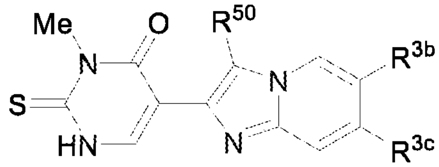

(M-3)

In a compound (hereinafter, referred to as a compound (M-3)) represented by Formula (M-3), a compound in which $R^{50}$ is a hydrogen atom, $R^{3b}$ is any of the substituents shown in [Table L4] to [Table L12], and $R^{3c}$ is a hydrogen atom.

In the compound (M-3), a compound in which $R^{50}$ is SMe, $R^{3b}$ is any of the substituents shown in [Table L4] to [Table L12], and $R^{3c}$ is a hydrogen atom.

In the compound (M-3), a compound in which $R^{50}$ is SEt, $R^{3b}$ is any of the substituents shown in [Table L4] to [Table L12], and $R^{3c}$ is a hydrogen atom.

In the compound (M-3), a compound in which $R^{50}$ is S(O)Me, $R^{3b}$ is any of the substituents shown in [Table L4] to [Table L12], and $R^{3c}$ is a hydrogen atom.

In the compound (M-3), a compound in which $R^{50}$ is S(O)Et, $R^{3b}$ is any of the substituents shown in [Table L4] to [Table L12], and $R^{3c}$ is a hydrogen atom.

In the compound (M-3), a compound in which $R^{50}$ is $S(O)_2Me$, $R^{3b}$ is any of the substituents shown in [Table L4] to [Table L12], and $R^{3c}$ is a hydrogen atom.

In the compound (M-3), a compound in which $R^{50}$ is $S(O)_2Et$, $R^{3b}$ is any of the substituents shown in [Table L4] to [Table L12], and $R^{3c}$ is a hydrogen atom.

In the compound (M-3), a compound in which $R^{50}$ is a hydrogen atom, $R^{3b}$ is a hydrogen atom, and $R^{3c}$ is any of the substituents shown in [Table L4] to [Table L12].

In the compound (M-3), a compound in which $R^{50}$ is SMe, $R^{3b}$ is a hydrogen atom, and $R^{3c}$ is any of the substituents shown in [Table L4] to [Table L12].

In the compound (M-3), a compound in which $R^{50}$ is SEt, $R^{3b}$ is a hydrogen atom, and $R^{3c}$ is any of the substituents shown in [Table L4] to [Table L12].

In the compound (M-3), a compound in which $R^{50}$ is S(O)Me, $R^{3b}$ is a hydrogen atom, and $R^{3c}$ is any of the substituents shown in [Table L4] to [Table L12].

In the compound (M-3), a compound in which $R^{50}$ is S(O)Et, $R^{3b}$ is a hydrogen atom, and $R^{3c}$ is any of the substituents shown in [Table L4] to [Table L12].

In the compound (M-3), a compound in which $R^{50}$ is $S(O)_2Me$, $R^{3b}$ is a hydrogen atom, and $R^{3c}$ is any of the substituents shown in [Table L4] to [Table L12].

In the compound (M-3), a compound in which $R^{50}$ is $S(O)_2Et$, $R^{3b}$ is a hydrogen atom, and $R^{3c}$ is any of the substituents shown in [Table L4] to [Table L12].

[Chem. 103]

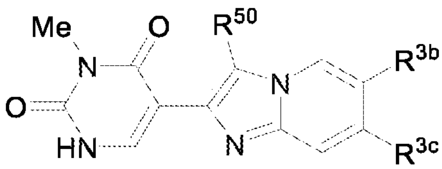

(M-4)

In a compound (hereinafter, referred to as a compound (M-4)) represented by Formula (M-4), a compound in which $R^{50}$ is a hydrogen atom, $R^{3b}$ is any of the substituents shown in [Table L4] to [Table L12], and $R^{3c}$ is a hydrogen atom.

In the compound (M-4), a compound in which $R^{50}$ is SMe, $R^{3b}$ is any of the substituents shown in [Table L4] to [Table L12], and $R^{3c}$ is a hydrogen atom.

In the compound (M-4), a compound in which $R^{50}$ is SEt, $R^{3b}$ is any of the substituents shown in [Table L4] to [Table L12], and $R^{3c}$ is a hydrogen atom.

In the compound (M-4), a compound in which $R^{50}$ is S(O)Me, $R^{3b}$ is any of the substituents shown in [Table L4] to [Table L12], and $R^{3c}$ is a hydrogen atom.

In the compound (M-4), a compound in which $R^{50}$ is S(O)Et, $R^{3b}$ is any of the substituents shown in [Table L4] to [Table L12], and $R^{3c}$ is a hydrogen atom.

In the compound (M-4), a compound in which $R^{50}$ is $S(O)_2Me$, $R^{3b}$ is any of the substituents shown in [Table

L4] to [Table L12], and $R^{3c}$ is a hydrogen atom.

In the compound (M-4), a compound in which $R^{50}$ is $S(O)_2Et$, $R^{3b}$ is any of the substituents shown in [Table L4] to [Table L12], and $R^{3c}$ is a hydrogen atom.

In the compound (M-4), a compound in which $R^{50}$ is a hydrogen atom, $R^{3b}$ is a hydrogen atom, and $R^{3c}$ is any of the substituents shown in [Table L4] to [Table L12].

In the compound (M-4), a compound in which $R^{50}$ is SMe, $R^{3b}$ is a hydrogen atom, and $R^{3c}$ is any of the substituents shown in [Table L4] to [Table L12].

In the compound (M-4), a compound in which $R^{50}$ is SEt, $R^{3b}$ is a hydrogen atom, and $R^{3c}$ is any of the substituents shown in [Table L4] to [Table L12].

In the compound (M-4), a compound in which $R^{50}$ is S(O)Me, $R^{3b}$ is a hydrogen atom, and $R^{3c}$ is any of the substituents shown in [Table L4] to [Table L12].

In the compound (M-4), a compound in which $R^{50}$ is S(O)Et, $R^{3b}$ is a hydrogen atom, and $R^{3c}$ is any of the substituents shown in [Table L4] to [Table L12].

In the compound (M-4), a compound in which $R^{50}$ is $S(O)_2Me$, $R^{3b}$ is a hydrogen atom, and $R^{3c}$ is any of the substituents shown in [Table L4] to [Table L12].

In the compound (M-4), a compound in which $R^{50}$ is $S(O)_2Et$, $R^{3b}$ is a hydrogen atom, and $R^{3c}$ is any of the substituents shown in [Table L4] to [Table L12].

[Chem. 104]

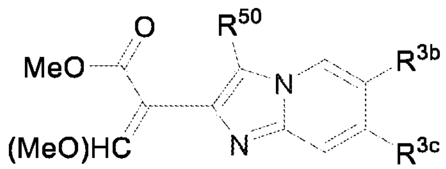

(M-5)

In a compound (hereinafter, referred to as a compound (M-5)) represented by Formula (M-5), a compound in which $R^{50}$ is a hydrogen atom, $R^{3b}$ is any of the substituents shown in [Table L4] to [Table L12], and $R^{3c}$ is a hydrogen atom.

In the compound (M-5), a compound in which $R^{50}$ is SMe, $R^{3b}$ is any of the substituents shown in [Table L4] to [Table L12], and $R^{3c}$ is a hydrogen atom.

In the compound (M-5), a compound in which $R^{50}$ is SEt, $R^{3b}$ is any of the substituents shown in [Table L4] to [Table L12], and $R^{3c}$ is a hydrogen atom.

In the compound (M-5), a compound in which $R^{50}$ is S(O)Me, $R^{3b}$ is any of the substituents shown in [Table L4] to [Table L12], and $R^{3c}$ is a hydrogen atom.

In the compound (M-5), a compound in which $R^{50}$ is S(O)Et, $R^{3b}$ is any of the substituents shown in [Table L4] to [Table L12], and $R^{3c}$ is a hydrogen atom.

In the compound (M-5), a compound in which $R^{50}$ is $S(O)_2Me$, $R^{3b}$ is any of the substituents shown in [Table L4] to [Table L12], and $R^{3c}$ is a hydrogen atom.

In the compound (M-5), a compound in which $R^{50}$ is $S(O)_2Et$, $R^{3b}$ is any of the substituents shown in [Table L4] to [Table L12], and $R^{3c}$ is a hydrogen atom.

In the compound (M-5), a compound in which $R^{50}$ is a hydrogen atom, $R^{3b}$ is a hydrogen atom, and $R^{3c}$ is any of the substituents shown in [Table L4] to [Table L12].

In the compound (M-5), a compound in which $R^{50}$ is SMe, $R^{3b}$ is a hydrogen atom, and $R^{3c}$ is any of the substituents shown in [Table L4] to [Table L12].

In the compound (M-5), a compound in which $R^{50}$ is SEt, $R^{3b}$ is a hydrogen atom, and $R^{3c}$ is any of the substituents shown in [Table L4] to [Table L12].

In the compound (M-5), a compound in which $R^{50}$ is S(O)Me, $R^{3b}$ is a hydrogen atom, and $R^{3c}$ is any of the substituents shown in [Table L4] to [Table L12].

In the compound (M-5), a compound in which $R^{50}$ is S(O)Et, $R^{3b}$ is a hydrogen atom, and $R^{3c}$ is any of the substituents shown in [Table L4] to [Table L12].

In the compound (M-5), a compound in which $R^{50}$ is $S(O)_2Me$, $R^{3b}$ is a hydrogen atom, and $R^{3c}$ is any of the substituents shown in [Table L4] to [Table L12].

In the compound (M-5), a compound in which $R^{50}$ is $S(O)_2Et$, $R^{3b}$ is a hydrogen atom, and $R^{3c}$ is any of the substituents shown in [Table L4] to [Table L12].

[Chem. 105]

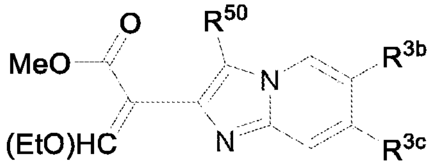

(M-6)

In a compound (hereinafter, referred to as a compound (M-6)) represented by Formula (M-6), a compound in which $R^{50}$ is a hydrogen atom, $R^{3b}$ is any of the substituents shown in [Table L4] to [Table L12], and $R^{3c}$ is a hydrogen atom.

In the compound (M-6), a compound in which $R^{50}$ is SMe, $R^{3b}$ is any of the substituents shown in [Table L4] to [Table L12], and $R^{3c}$ is a hydrogen atom.

In the compound (M-6), a compound in which $R^{50}$ is SEt, $R^{3b}$ is any of the substituents shown in [Table L4] to [Table L12], and $R^{3c}$ is a hydrogen atom.

In the compound (M-6), a compound in which $R^{50}$ is S(O)Me, $R^{3b}$ is any of the substituents shown in [Table L4] to [Table L12], and $R^{3c}$ is a hydrogen atom.

In the compound (M-6), a compound in which $R^{50}$ is S(O)Et, $R^{3b}$ is any of the substituents shown in [Table L4] to [Table L12], and $R^{3c}$ is a hydrogen atom.

In the compound (M-6), a compound in which $R^{50}$ is $S(O)_2Me$, $R^{3b}$ is any of the substituents shown in [Table L4] to [Table L12], and $R^{3c}$ is a hydrogen atom.

In the compound (M-6), a compound in which $R^{50}$ is $S(O)_2Et$, $R^{3b}$ is any of the substituents shown in [Table L4] to [Table L12], and $R^{3c}$ is a hydrogen atom.

In the compound (M-6), a compound in which $R^{50}$ is a hydrogen atom, $R^{3b}$ is a hydrogen atom, and $R^{3c}$ is any of the substituents shown in [Table L4] to [Table L12].

In the compound (M-6), a compound in which $R^{50}$ is SMe, $R^{3b}$ is a hydrogen atom, and $R^{3c}$ is any of the substituents shown in [Table L4] to [Table L12].

In the compound (M-6), a compound in which $R^{50}$ is SEt, $R^{3b}$ is a hydrogen atom, and $R^{3c}$ is any of the substituents shown in [Table L4] to [Table L12].

In the compound (M-6), a compound in which $R^{50}$ is S(O)Me, $R^{3b}$ is a hydrogen atom, and $R^{3c}$ is any of the substituents shown in [Table L4] to [Table L12].

In the compound (M-6), a compound in which $R^{50}$ is S(O)Et, $R^{3b}$ is a hydrogen atom, and $R^{3c}$ is any of the substituents shown in [Table L4] to [Table L12].

In the compound (M-6), a compound in which $R^{50}$ is $S(O)_2Me$, $R^{3b}$ is a hydrogen atom, and $R^{3c}$ is any of the substituents shown in [Table L4] to [Table L12].

In the compound (M-6), a compound in which $R^{50}$ is $S(O)_2Et$, $R^{3b}$ is a hydrogen atom, and $R^{3c}$ is any of the substituents shown in [Table L4] to [Table L12].

[Chem. 106]

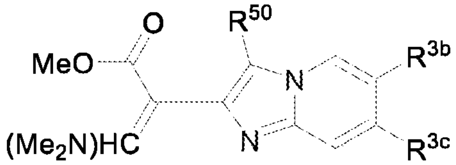

(M-7)

In a compound (hereinafter, referred to as a compound (M-7)) represented by Formula (M-7), a compound in which $R^{50}$ is a hydrogen atom, $R^{3b}$ is any of the substituents shown in [Table L4] to [Table L12], and $R^{3c}$ is a hydrogen atom.

In the compound (M-7), a compound in which $R^{50}$ is SMe, $R^{3b}$ is any of the substituents shown in [Table L4] to [Table L12], and $R^{3c}$ is a hydrogen atom.

In the compound (M-7), a compound in which $R^{50}$ is SEt, $R^{3b}$ is any of the substituents shown in [Table L4] to [Table L12], and $R^{3c}$ is a hydrogen atom.

In the compound (M-7), a compound in which $R^{50}$ is S(O)Me, $R^{3b}$ is any of the substituents shown in [Table L4] to [Table L12], and $R^{3c}$ is a hydrogen atom.

In the compound (M-7), a compound in which $R^{50}$ is S(O)Et, $R^{3b}$ is any of the substituents shown in [Table L4] to [Table L12], and $R^{3c}$ is a hydrogen atom.

In the compound (M-7), a compound in which $R^{50}$ is $S(O)_2Me$, $R^{3b}$ is any of the substituents shown in [Table

L4] to [Table L12], and $R^{3c}$ is a hydrogen atom.

In the compound (M-7), a compound in which $R^{50}$ is $S(O)_2Et$, $R^{3b}$ is any of the substituents shown in [Table L4] to [Table L12], and $R^{3c}$ is a hydrogen atom.

In the compound (M-7), a compound in which $R^{50}$ is a hydrogen atom, $R^{3b}$ is a hydrogen atom, and $R^{3c}$ is any of the substituents shown in [Table L4] to [Table L12].

In the compound (M-7), a compound in which $R^{50}$ is SMe, $R^{3b}$ is a hydrogen atom, and $R^{3c}$ is any of the substituents shown in [Table L4] to [Table L12].

In the compound (M-7), a compound in which $R^{50}$ is SEt, $R^{3b}$ is a hydrogen atom, and $R^{3c}$ is any of the substituents shown in [Table L4] to [Table L12].

In the compound (M-7), a compound in which $R^{50}$ is S(O)Me, $R^{3b}$ is a hydrogen atom, and $R^{3c}$ is any of the substituents shown in [Table L4] to [Table L12].

In the compound (M-7), a compound in which $R^{50}$ is S(O)Et, $R^{3b}$ is a hydrogen atom, and $R^{3c}$ is any of the substituents shown in [Table L4] to [Table L12].

In the compound (M-7), a compound in which $R^{50}$ is $S(O)_2Me$, $R^{3b}$ is a hydrogen atom, and $R^{3c}$ is any of the substituents shown in [Table L4] to [Table L12].

In the compound (M-7), a compound in which $R^{50}$ is $S(O)_2Et$, $R^{3b}$ is a hydrogen atom, and $R^{3c}$ is any of the substituents shown in [Table L4] to [Table L12].

[Chem. 107]

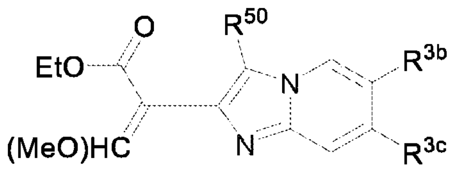

(M-8)

In a compound (hereinafter, referred to as a compound (M-8)) represented by Formula (M-8), a compound in which $R^{50}$ is a hydrogen atom, $R^{3b}$ is any of the substituents shown in [Table L4] to [Table L12], and $R^{3c}$ is a hydrogen atom.

In the compound (M-8), a compound in which $R^{50}$ is SMe, $R^{3b}$ is any of the substituents shown in [Table L4] to [Table L12], and $R^{3c}$ is a hydrogen atom.

In the compound (M-8), a compound in which $R^{50}$ is SEt, $R^{3b}$ is any of the substituents shown in [Table L4] to [Table L12], and $R^{3c}$ is a hydrogen atom.

In the compound (M-8), a compound in which $R^{50}$ is S(O)Me, $R^{3b}$ is any of the substituents shown in [Table L4] to [Table L12], and $R^{3c}$ is a hydrogen atom.

In the compound (M-8), a compound in which $R^{50}$ is S(O)Et, $R^{3b}$ is any of the substituents shown in [Table L4] to [Table L12], and $R^{3c}$ is a hydrogen atom.

In the compound (M-8), a compound in which $R^{50}$ is $S(O)_2Me$, $R^{3b}$ is any of the substituents shown in [Table L4] to [Table L12], and $R^{3c}$ is a hydrogen atom.

In the compound (M-8), a compound in which $R^{50}$ is $S(O)_2Et$, $R^{3b}$ is any of the substituents shown in [Table L4] to [Table L12], and $R^{3c}$ is a hydrogen atom.

In the compound (M-8), a compound in which $R^{50}$ is a hydrogen atom, $R^{3b}$ is a hydrogen atom, and $R^{3c}$ is any of the substituents shown in [Table L4] to [Table L12].

In the compound (M-8), a compound in which $R^{50}$ is SMe, $R^{3b}$ is a hydrogen atom, and $R^{3c}$ is any of the substituents shown in [Table L4] to [Table L12].

In the compound (M-8), a compound in which $R^{50}$ is SEt, $R^{3b}$ is a hydrogen atom, and $R^{3c}$ is any of the substituents shown in [Table L4] to [Table L12].

In the compound (M-8), a compound in which $R^{50}$ is S(O)Me, $R^{3b}$ is a hydrogen atom, and $R^{3c}$ is any of the substituents shown in [Table L4] to [Table L12].

In the compound (M-8), a compound in which $R^{50}$ is S(O)Et, $R^{3b}$ is a hydrogen atom, and $R^{3c}$ is any of the substituents shown in [Table L4] to [Table L12].

In the compound (M-8), a compound in which $R^{50}$ is $S(O)_2Me$, $R^{3b}$ is a hydrogen atom, and $R^{3c}$ is any of the substituents shown in [Table L4] to [Table L12].

In the compound (M-8), a compound in which $R^{50}$ is $S(O)_2Et$, $R^{3b}$ is a hydrogen atom, and $R^{3c}$ is any of the substituents shown in [Table L4] to [Table L12].

[Chem. 108]

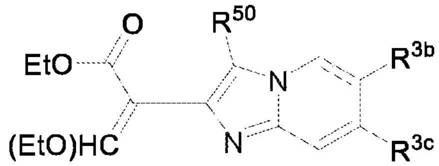

(M-9)

In a compound (hereinafter, referred to as a compound (M-9)) represented by Formula (M-9), a compound in which $R^{50}$ is a hydrogen atom, $R^{3b}$ is any of the substituents shown in [Table L4] to [Table L12], and $R^{3c}$ is a hydrogen atom.

In the compound (M-9), a compound in which $R^{50}$ is SMe, $R^{3b}$ is any of the substituents shown in [Table L4] to [Table L12], and $R^{3c}$ is a hydrogen atom.

In the compound (M-9), a compound in which $R^{50}$ is SEt, $R^{3b}$ is any of the substituents shown in [Table L4] to [Table L12], and $R^{3c}$ is a hydrogen atom.

In the compound (M-9), a compound in which $R^{50}$ is S(O)Me, $R^{3b}$ is any of the substituents shown in [Table L4] to [Table L12], and $R^{3c}$ is a hydrogen atom.

In the compound (M-9), a compound in which $R^{50}$ is S(O)Et, $R^{3b}$ is any of the substituents shown in [Table L4] to [Table L12], and $R^{3c}$ is a hydrogen atom.

In the compound (M-9), a compound in which $R^{50}$ is $S(O)_2Me$, $R^{3b}$ is any of the substituents shown in [Table L4] to [Table L12], and $R^{3c}$ is a hydrogen atom.

In the compound (M-9), a compound in which $R^{50}$ is $S(O)_2Et$, $R^{3b}$ is any of the substituents shown in [Table L4] to [Table L12], and $R^{3c}$ is a hydrogen atom.

In the compound (M-9), a compound in which $R^{50}$ is a hydrogen atom, $R^{3b}$ is a hydrogen atom, and $R^{3c}$ is any of the substituents shown in [Table L4] to [Table L12].

In the compound (M-9), a compound in which $R^{50}$ is SMe, $R^{3b}$ is a hydrogen atom, and $R^{3c}$ is any of the substituents shown in [Table L4] to [Table L12].

In the compound (M-9), a compound in which $R^{50}$ is SEt, $R^{3b}$ is a hydrogen atom, and $R^{3c}$ is any of the substituents shown in [Table L4] to [Table L12].

In the compound (M-9), a compound in which $R^{50}$ is S(O)Me, $R^{3b}$ is a hydrogen atom, and $R^{3c}$ is any of the substituents shown in [Table L4] to [Table L12].

In the compound (M-9), a compound in which $R^{50}$ is S(O)Et, $R^{3b}$ is a hydrogen atom, and $R^{3c}$ is any of the substituents shown in [Table L4] to [Table L12].

In the compound (M-9), a compound in which $R^{50}$ is $S(O)_2Me$, $R^{3b}$ is a hydrogen atom, and $R^{3c}$ is any of the substituents shown in [Table L4] to [Table L12].

In the compound (M-9), a compound in which $R^{50}$ is $S(O)_2Et$, $R^{3b}$ is a hydrogen atom, and $R^{3c}$ is any of the substituents shown in [Table L4] to [Table L12].

[Chem. 109]

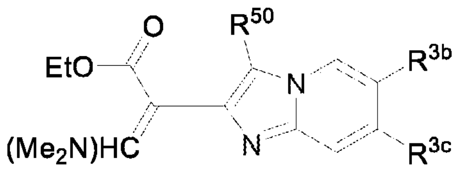

(M-10)

In a compound (hereinafter, referred to as a compound (M-10)) represented by Formula (M-10), a compound in which $R^{50}$ is a hydrogen atom, $R^{3b}$ is any of the substituents shown in [Table L4] to [Table L12], and $R^{3c}$ is a hydrogen atom.

In the compound (M-10), a compound in which $R^{50}$ is SMe, $R^{3b}$ is any of the substituents shown in [Table L4] to [Table L12], and $R^{3c}$ is a hydrogen atom.

In the compound (M-10), a compound in which $R^{50}$ is SEt, $R^{3b}$ is any of the substituents shown in [Table L4] to [Table L12], and $R^{3c}$ is a hydrogen atom.

In the compound (M-10), a compound in which $R^{50}$ is S(O)Me, $R^{3b}$ is any of the substituents shown in [Table L4] to [Table L12], and $R^{3c}$ is a hydrogen atom.

In the compound (M-10), a compound in which $R^{50}$ is S(O)Et, $R^{3b}$ is any of the substituents shown in [Table L4] to [Table L12], and $R^{3c}$ is a hydrogen atom.

In the compound (M-10), a compound in which $R^{50}$ is $S(O)_2Me$, $R^{3b}$ is any of the substituents shown in [Table L4] to [Table L12], and $R^{3c}$ is a hydrogen atom.

In the compound (M-10), a compound in which $R^{50}$ is $S(O)_2Et$, $R^{3b}$ is any of the substituents shown in [Table L4] to [Table L12], and $R^{3c}$ is a hydrogen atom.

In the compound (M-10), a compound in which $R^{50}$ is a hydrogen atom, $R^{3b}$ is a hydrogen atom, and $R^{3c}$ is any of the substituents shown in [Table L4] to [Table L12].

In the compound (M-10), a compound in which $R^{50}$ is SMe, $R^{3b}$ is a hydrogen atom, and $R^{3c}$ is any of the substituents shown in [Table L4] to [Table L12].

In the compound (M-10), a compound in which $R^{50}$ is SEt, $R^{3b}$ is a hydrogen atom, and $R^{3c}$ is any of the substituents shown in [Table L4] to [Table L12].

In the compound (M-10), a compound in which $R^{50}$ is S(O)Me, $R^{3b}$ is a hydrogen atom, and $R^{3c}$ is any of the substituents shown in [Table L4] to [Table L12].

In the compound (M-10), a compound in which $R^{50}$ is S(O)Et, $R^{3b}$ is a hydrogen atom, and $R^{3c}$ is any of the substituents shown in [Table L4] to [Table L12].

In the compound (M-10), a compound in which $R^{50}$ is $S(O)_2Me$, $R^{3b}$ is a hydrogen atom, and $R^{3c}$ is any of the substituents shown in [Table L4] to [Table L12].

In the compound (M-10), a compound in which $R^{50}$ is $S(O)_2Et$, $R^{3b}$ is a hydrogen atom, and $R^{3c}$ is any of the substituents shown in [Table L4] to [Table L12].

The groups represented by Q31 to Q33 are the groups shown below.

[Chem. 110]

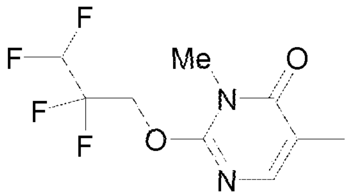
Q31

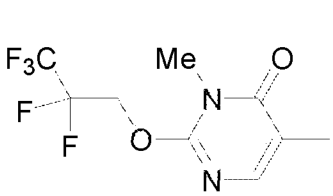
Q32

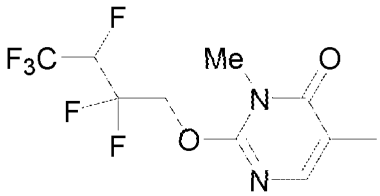
Q33

[Chem. 111]

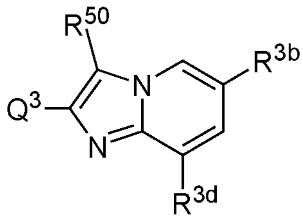
(M-11)

In a compound (hereinafter, referred to as a compound (M-11)) represented by Formula (M-11), a compound in which $Q^3$ is a group represented by Q31, $R^{3b}$ is a chlorine atom, $R^{3d}$ is a hydrogen atom, and $R^{50}$ is a hydrogen atom.

In the compound (M-11), a compound in which $Q^3$ is a group represented by Q31, $R^{3b}$ is a bromine atom, $R^{3d}$ is a hydrogen atom, and $R^{50}$ is a hydrogen atom.

In the compound (M-11), a compound in which $Q^3$ is a group represented by Q31, $R^{3b}$ is an iodine atom, $R^{3d}$ is a hydrogen atom, and $R^{50}$ is a hydrogen atom.

In the compound (M-11), a compound in which $Q^3$ is a group represented by Q31, $R^{3b}$ is a chlorine atom, $R^{3d}$ is a hydrogen atom, and $R^{50}$ is SMe.

In the compound (M-11), a compound in which $Q^3$ is a group represented by Q31, $R^{3b}$ is a bromine atom, $R^{3d}$ is a hydrogen atom, and $R^{50}$ is SMe.

In the compound (M-11), a compound in which $Q^3$ is a group represented by Q31, $R^{3b}$ is an iodine atom, $R^{3d}$ is a hydrogen atom, and $R^{50}$ is SMe.

In the compound (M-11), a compound in which $Q^3$ is a group represented by Q31, $R^{3b}$ is a chlorine atom, $R^{3d}$ is a hydrogen atom, and $R^{50}$ is SEt.

In the compound (M-11), a compound in which $Q^3$ is a group represented by Q31, $R^{3b}$ is a bromine atom, $R^{3d}$ is a hydrogen atom, and $R^{50}$ is SEt.

In the compound (M-11), a compound in which $Q^3$ is a group represented by Q31, $R^{3b}$ is an iodine atom, $R^{3d}$ is a hydrogen atom, and $R^{50}$ is SEt.

In the compound (M-11), a compound in which $Q^3$ is a group represented by Q31, $R^{3b}$ is a chlorine atom, $R^{3d}$ is a hydrogen atom, and $R^{50}$ is S(O)Me.

In the compound (M-11), a compound in which $Q^3$ is a group represented by Q31, $R^{3b}$ is a bromine atom, $R^{3d}$ is a hydrogen atom, and $R^{50}$ is S(O)Me.

In the compound (M-11), a compound in which $Q^3$ is a group represented by Q31, $R^{3b}$ is an iodine atom, $R^{3d}$ is a hydrogen atom, and $R^{50}$ is S(O)Me.

In the compound (M-11), a compound in which $Q^3$ is a group represented by Q31, $R^{3b}$ is a chlorine atom, $R^{3d}$ is a hydrogen atom, and $R^{50}$ is S(O)Et.

In the compound (M-11), a compound in which $Q^3$ is a group represented by Q31, $R^{3b}$ is a bromine atom, $R^{3d}$ is a hydrogen atom, and $R^{50}$ is S(O)Et.

In the compound (M-11), a compound in which $Q^3$ is a group represented by Q31, $R^{3b}$ is an iodine atom, $R^{3d}$ is a hydrogen atom, and $R^{50}$ is S(O)Et.

In the compound (M-11), a compound in which $Q^3$ is a group represented by Q31, $R^{3b}$ is a chlorine atom, $R^{3d}$ is a hydrogen atom, and $R^{50}$ is $S(O)_2Me$.

In the compound (M-11), a compound in which $Q^3$ is a group represented by Q31, $R^{3b}$ is a bromine atom, $R^{3d}$ is a hydrogen atom, and $R^{50}$ is $S(O)_2Me$.

In the compound (M-11), a compound in which $Q^3$ is a group represented by Q31, $R^{3b}$ is an iodine atom, $R^{3d}$ is a hydrogen atom, and $R^{50}$ is $S(O)_2Me$.

In the compound (M-11), a compound in which $Q^3$ is a group represented by Q31, $R^{3b}$ is a chlorine atom, $R^{3d}$ is a hydrogen atom, and $R^{50}$ is $S(O)_2Et$.

In the compound (M-11), a compound in which $Q^3$ is a group represented by Q31, $R^{3b}$ is a bromine atom, $R^{3d}$ is a hydrogen atom, and $R^{50}$ is $S(O)_2Et$.

In the compound (M-11), a compound in which $Q^3$ is a group represented by Q31, $R^{3b}$ is an iodine atom, $R^{3d}$ is a hydrogen atom, and $R^{50}$ is $S(O)_2Et$.

In the compound (M-11), a compound in which $Q^3$ is a group represented by Q31, $R^{3b}$ and $R^{3d}$ are chlorine atoms and $R^{50}$ is a hydrogen atom.

In the compound (M-11), a compound in which $Q^3$ is a group represented by Q31, $R^{3b}$ and $R^{3d}$ are bromine atoms and $R^{50}$ is a hydrogen atom.

In the compound (M-11), a compound in which $Q^3$ is a group represented by Q31, $R^{3b}$ and $R^{3d}$ are iodine atoms and $R^{50}$ is a hydrogen atom.

In the compound (M-11), a compound in which $Q^3$ is a group represented by Q31, $R^{3b}$ and $R^{3d}$ are chlorine atoms and $R^{50}$ is SMe.

In the compound (M-11), a compound in which $Q^3$ is a group represented by Q31, $R^{3b}$ and $R^{3d}$ are bromine atoms and $R^{50}$ is SMe.

In the compound (M-11), a compound in which $Q^3$ is a group represented by Q31, $R^{3b}$ and $R^{3d}$ are iodine atoms and $R^{50}$ is SMe.

In the compound (M-11), a compound in which $Q^3$ is a group represented by Q31, $R^{3b}$ and $R^{3d}$ are chlorine atoms and $R^{50}$ is SEt.

In the compound (M-11), a compound in which $Q^3$ is a group represented by Q31, $R^{3b}$ and $R^{3d}$ are bromine atoms and $R^{50}$ is SEt.

In the compound (M-11), a compound in which $Q^3$ is a group represented by Q31, $R^{3b}$ and $R^{3d}$ are iodine atoms and $R^{50}$ is SEt.

In the compound (M-11), a compound in which $Q^3$ is a group represented by Q31, $R^{3b}$ and $R^{3d}$ are chlorine atoms and $R^{50}$ is S(O)Me.

In the compound (M-11), a compound in which $Q^3$ is a group represented by Q31, $R^{3b}$ and $R^{3d}$ are bromine atoms and $R^{50}$ is S(O)Me.

In the compound (M-11), a compound in which $Q^3$ is a group represented by Q31, $R^{3b}$ and $R^{3d}$ are iodine atoms and $R^{50}$ is S(O)Me.

In the compound (M-11), a compound in which $Q^3$ is a group represented by Q31, $R^{3b}$ and $R^{3d}$ are chlorine atoms and $R^{50}$ is S(O)Et.

In the compound (M-11), a compound in which $Q^3$ is a group represented by Q31, $R^{3b}$ and $R^{3d}$ are bromine atoms and $R^{50}$ is S(O)Et.

In the compound (M-11), a compound in which $Q^3$ is a group represented by Q31, $R^{3b}$ and $R^{3d}$ are iodine atoms and $R^{50}$ is S(O)Et.

In the compound (M-11), a compound in which $Q^3$ is a group represented by Q31, $R^{3b}$ and $R^{3d}$ are chlorine atoms and $R^{50}$ is $S(O)_2Me$ .

In the compound (M-11), a compound in which $Q^3$ is a group represented by Q31, $R^{3b}$ and $R^{3d}$ are bromine atoms and $R^{50}$ is $S(O)_2Me$.
In the compound (M-11), a compound in which $Q^3$ is a group represented by Q31, $R^{3b}$ and $R^{3d}$ are iodine atoms and $R^{50}$ is $S(O)_2Me$.
In the compound (M-11), a compound in which $Q^3$ is a group represented by Q31, $R^{3b}$ and $R^{3d}$ are chlorine atoms and $R^{50}$ is $S(O)_2Et$.
In the compound (M-11), a compound in which $Q^3$ is a group represented by Q31, $R^{3b}$ and $R^{3d}$ are bromine atoms and $R^{50}$ is $S(O)_2Et$.
In the compound (M-11), a compound in which $Q^3$ is a group represented by Q31, $R^{3b}$ and $R^{3d}$ are iodine atoms and $R^{50}$ is $S(O)_2Et$.
In the compound (M-11), a compound in which $Q^3$ is a group represented by Q32, $R^{3b}$ is a chlorine atom, $R^{3d}$ is a hydrogen atom, and $R^{50}$ is a hydrogen atom.
In the compound (M-11), a compound in which $Q^3$ is a group represented by Q32, $R^{3b}$ is a bromine atom, $R^{3d}$ is a hydrogen atom, and $R^{50}$ is a hydrogen atom.
In the compound (M-11), a compound in which $Q^3$ is a group represented by Q32, $R^{3b}$ is an iodine atom, $R^{3d}$ is a hydrogen atom, and $R^{50}$ is a hydrogen atom.
In the compound (M-11), a compound in which $Q^3$ is a group represented by Q32, $R^{3b}$ is a chlorine atom, $R^{3d}$ is a hydrogen atom, and $R^{50}$ is SMe.
In the compound (M-11), a compound in which $Q^3$ is a group represented by Q32, $R^{3b}$ is a bromine atom, $R^{3d}$ is a hydrogen atom, and $R^{50}$ is SMe.
In the compound (M-11), a compound in which $Q^3$ is a group represented by Q32, $R^{3b}$ is an iodine atom, $R^{3d}$ is a hydrogen atom, and $R^{50}$ is SMe.
In the compound (M-11), a compound in which $Q^3$ is a group represented by Q32, $R^{3b}$ is a chlorine atom, $R^{3d}$ is a hydrogen atom, and $R^{50}$ is SEt.
In the compound (M-11), a compound in which $Q^3$ is a group represented by Q32, $R^{3b}$ is a bromine atom, $R^{3d}$ is a hydrogen atom, and $R^{50}$ is SEt.
In the compound (M-11), a compound in which $Q^3$ is a group represented by Q32, $R^{3b}$ is an iodine atom, $R^{3d}$ is a hydrogen atom, and $R^{50}$ is SEt.
In the compound (M-11), a compound in which $Q^3$ is a group represented by Q32, $R^{3b}$ is a chlorine atom, $R^{3d}$ is a hydrogen atom, and $R^{50}$ is S(O)Me.
In the compound (M-11), a compound in which $Q^3$ is a group represented by Q32, $R^{3b}$ is a bromine atom, $R^{3d}$ is a hydrogen atom, and $R^{50}$ is S(O)Me.
In the compound (M-11), a compound in which $Q^3$ is a group represented by Q32, $R^{3b}$ is an iodine atom, $R^{3d}$ is a hydrogen atom, and $R^{50}$ is S(O)Me.
In the compound (M-11), a compound in which $Q^3$ is a group represented by Q32, $R^{3b}$ is a chlorine atom, $R^{3d}$ is a hydrogen atom, and $R^{50}$ is S(O)Et.
In the compound (M-11), a compound in which $Q^3$ is a group represented by Q32, $R^{3b}$ is a bromine atom, $R^{3d}$ is a hydrogen atom, and $R^{50}$ is S(O)Et.
In the compound (M-11), a compound in which $Q^3$ is a group represented by Q32, $R^{3b}$ is an iodine atom, $R^{3d}$ is a hydrogen atom, and $R^{50}$ is S(O)Et.
In the compound (M-11), a compound in which $Q^3$ is a group represented by Q32, $R^{3b}$ is a chlorine atom, $R^{3d}$ is a hydrogen atom, and $R^{50}$ is $S(O)_2Me$.
In the compound (M-11), a compound in which $Q^3$ is a group represented by Q32, $R^{3b}$ is a bromine atom, $R^{3d}$ is a hydrogen atom, and $R^{50}$ is $S(O)_2Me$.
In the compound (M-11), a compound in which $Q^3$ is a group represented by Q32, $R^{3b}$ is an iodine atom, $R^{3d}$ is a hydrogen atom, and $R^{50}$ is $S(O)_2Me$.
In the compound (M-11), a compound in which $Q^3$ is a group represented by Q32, $R^{3b}$ is a chlorine atom, $R^{3d}$ is a hydrogen atom, and $R^{50}$ is $S(O)_2Et$.
In the compound (M-11), a compound in which $Q^3$ is a group represented by Q32, $R^{3b}$ is a bromine atom, $R^{3d}$ is a hydrogen atom, and $R^{50}$ is $S(O)_2Et$.
In the compound (M-11), a compound in which $Q^3$ is a group represented by Q32, $R^{3b}$ is an iodine atom, $R^{3d}$ is a hydrogen atom, and $R^{50}$ is $S(O)_2Et$.
In the compound (M-11), a compound in which $Q^3$ is a group represented by Q32, $R^{3b}$ and $R^{3d}$ are chlorine atoms and $R^{50}$ is a hydrogen atom.
In the compound (M-11), a compound in which $Q^3$ is a group represented by Q32, $R^{3b}$ and $R^{3d}$ are bromine atoms and $R^{50}$ is a hydrogen atom.
In the compound (M-11), a compound in which $Q^3$ is a group represented by Q32, $R^{3b}$ and $R^{3d}$ are iodine atoms and $R^{50}$ is a hydrogen atom.

In the compound (M-11), a compound in which $Q^3$ is a group represented by Q32, $R^{3b}$ and $R^{3d}$ are chlorine atoms and $R^{50}$ is SMe.

In the compound (M-11), a compound in which $Q^3$ is a group represented by Q32, $R^{3b}$ and $R^{3d}$ are bromine atoms and $R^{50}$ is SMe.

In the compound (M-11), a compound in which $Q^3$ is a group represented by Q32, $R^{3b}$ and $R^{3d}$ are iodine atoms and $R^{50}$ is SMe.

In the compound (M-11), a compound in which $Q^3$ is a group represented by Q32, $R^{3b}$ and $R^{3d}$ are chlorine atoms and $R^{50}$ is SEt.

In the compound (M-11), a compound in which $Q^3$ is a group represented by Q32, $R^{3b}$ and $R^{3d}$ are bromine atoms and $R^{50}$ is SEt.

In the compound (M-11), a compound in which $Q^3$ is a group represented by Q32, $R^{3b}$ and $R^{3d}$ are iodine atoms and $R^{50}$ is SEt.

In the compound (M-11), a compound in which $Q^3$ is a group represented by Q32, $R^{3b}$ and $R^{3d}$ are chlorine atoms and $R^{50}$ is S(O)Me.

In the compound (M-11), a compound in which $Q^3$ is a group represented by Q32, $R^{3b}$ and $R^{3d}$ are bromine atoms and $R^{50}$ is S(O)Me.

In the compound (M-11), a compound in which $Q^3$ is a group represented by Q32, $R^{3b}$ and $R^{3d}$ are iodine atoms and $R^{50}$ is S(O)Me.

In the compound (M-11), a compound in which $Q^3$ is a group represented by Q32, $R^{3b}$ and $R^{3d}$ are chlorine atoms and $R^{50}$ is S(O)Et.

In the compound (M-11), a compound in which $Q^3$ is a group represented by Q32, $R^{3b}$ and $R^{3d}$ are bromine atoms and $R^{50}$ is S(O)Et.

In the compound (M-11), a compound in which $Q^3$ is a group represented by Q32, $R^{3b}$ and $R^{3d}$ are iodine atoms and $R^{50}$ is S (O)Et.

In the compound (M-11), a compound in which $Q^3$ is a group represented by Q32, $R^{3b}$ and $R^{3d}$ are chlorine atoms and $R^{50}$ is $S(O)_2Me$.

In the compound (M-11), a compound in which $Q^3$ is a group represented by Q32, $R^{3b}$ and $R^{3d}$ are bromine atoms and $R^{50}$ is $S(O)_2Me$.

In the compound (M-11), a compound in which $Q^3$ is a group represented by Q32, $R^{3b}$ and $R^{3d}$ are iodine atoms and $R^{50}$ is $S(O)_2Me$.

In the compound (M-11), a compound in which $Q^3$ is a group represented by Q32, $R^{3b}$ and $R^{3d}$ are chlorine atoms and $R^{50}$ is $S(O)_2Et$.

In the compound (M-11), a compound in which $Q^3$ is a group represented by Q32, $R^{3b}$ and $R^{3d}$ are bromine atoms and $R^{50}$ is $S(O)_2Et$.

In the compound (M-11), a compound in which $Q^3$ is a group represented by Q32, $R^{3b}$ and $R^{3d}$ are iodine atoms and $R^{50}$ is $S(O)_2Et$.

In the compound (M-11), a compound in which $Q^3$ is a group represented by Q33, $R^{3b}$ is a chlorine atom, $R^{3d}$ is a hydrogen atom, and $R^{50}$ is a hydrogen atom.

In the compound (M-11), a compound in which $Q^3$ is a group represented by Q33, $R^{3b}$ is a bromine atom, $R^{3d}$ is a hydrogen atom, and $R^{50}$ is a hydrogen atom.

In the compound (M-11), a compound in which $Q^3$ is a group represented by Q33, $R^{3b}$ is an iodine atom, $R^{3d}$ is a hydrogen atom, and $R^{50}$ is a hydrogen atom.

In the compound (M-11), a compound in which $Q^3$ is a group represented by Q33, $R^{3b}$ is a chlorine atom, $R^{3d}$ is a hydrogen atom, and $R^{50}$ is SMe.

In the compound (M-11), a compound in which $Q^3$ is a group represented by Q33, $R^{3b}$ is a bromine atom, $R^{3d}$ is a hydrogen atom, and $R^{50}$ is SMe.

In the compound (M-11), a compound in which $Q^3$ is a group represented by Q33, $R^{3b}$ is an iodine atom, $R^{3d}$ is a hydrogen atom, and $R^{50}$ is SMe.

In the compound (M-11), a compound in which $Q^3$ is a group represented by Q33, $R^{3b}$ is a chlorine atom, $R^{3d}$ is a hydrogen atom, and $R^{50}$ is SEt.

In the compound (M-11), a compound in which $Q^3$ is a group represented by Q33, $R^{3b}$ is a bromine atom, $R^{3d}$ is a hydrogen atom, and $R^{50}$ is SEt.

In the compound (M-11), a compound in which $Q^3$ is a group represented by Q33, $R^{3b}$ is an iodine atom, $R^{3d}$ is a hydrogen atom, and $R^{50}$ is SEt.

In the compound (M-11), a compound in which $Q^3$ is a group represented by Q33, $R^{3b}$ is a chlorine atom, $R^{3d}$ is a hydrogen atom, and $R^{50}$ is S(O)Me.

In the compound (M-11), a compound in which $Q^3$ is a group represented by Q33, $R^{3b}$ is a bromine atom, $R^{3d}$ is a hydrogen atom, and $R^{50}$ is S(O)Me.

In the compound (M-11), a compound in which $Q^3$ is a group represented by Q33, $R^{3b}$ is an iodine atom, $R^{3d}$ is a hydrogen atom, and $R^{50}$ is S(O)Me.

In the compound (M-11), a compound in which $Q^3$ is a group represented by Q33, $R^{3b}$ is a chlorine atom, $R^{3d}$ is a hydrogen atom, and $R^{50}$ is S(O)Et.

In the compound (M-11), a compound in which $Q^3$ is a group represented by Q33, $R^{3b}$ is a bromine atom, $R^{3d}$ is a hydrogen atom, and $R^{50}$ is S(O)Et.

In the compound (M-11), a compound in which $Q^3$ is a group represented by Q33, $R^{3b}$ is an iodine atom, $R^{3d}$ is a hydrogen atom, and $R^{50}$ is S(O)Et.

In the compound (M-11), a compound in which $Q^3$ is a group represented by Q33, $R^{3b}$ is a chlorine atom, $R^{3d}$ is a hydrogen atom, and $R^{50}$ is $S(O)_2Me$.

In the compound (M-11), a compound in which $Q^3$ is a group represented by Q33, $R^{3b}$ is a bromine atom, $R^{3d}$ is a hydrogen atom, and $R^{50}$ is $S(O)_2Me$.

In the compound (M-11), a compound in which $Q^3$ is a group represented by Q33, $R^{3b}$ is an iodine atom, $R^{3d}$ is a hydrogen atom, and $R^{50}$ is $S(O)_2Me$.

In the compound (M-11), a compound in which $Q^3$ is a group represented by Q33, $R^{3b}$ is a chlorine atom, $R^{3d}$ is a hydrogen atom, and $R^{50}$ is $S(O)_2Et$.

In the compound (M-11), a compound in which $Q^3$ is a group represented by Q33, $R^{3b}$ is a bromine atom, $R^{3d}$ is a hydrogen atom, and $R^{50}$ is $S(O)_2Et$.

In the compound (M-11), a compound in which $Q^3$ is a group represented by Q33, $R^{3b}$ is an iodine atom, $R^{3d}$ is a hydrogen atom, and $R^{50}$ is $S(O)_2Et$.

In the compound (M-11), a compound in which $Q^3$ is a group represented by Q33, $R^{3b}$ and $R^{3d}$ are chlorine atoms and $R^{50}$ is a hydrogen atom.

In the compound (M-11), a compound in which $Q^3$ is a group represented by Q33, $R^{3b}$ and $R^{3d}$ are bromine atoms and $R^{50}$ is a hydrogen atom.

In the compound (M-11), a compound in which $Q^3$ is a group represented by Q33, $R^{3b}$ and $R^{3d}$ are iodine atoms and $R^{50}$ is a hydrogen atom.

In the compound (M-11), a compound in which $Q^3$ is a group represented by Q33, $R^{3b}$ and $R^{3d}$ are chlorine atoms and $R^{50}$ is SMe.

In the compound (M-11), a compound in which $Q^3$ is a group represented by Q33, $R^{3b}$ and $R^{3d}$ are bromine atoms and $R^{50}$ is SMe.

In the compound (M-11), a compound in which $Q^3$ is a group represented by Q33, $R^{3b}$ and $R^{3d}$ are iodine atoms and $R^{50}$ is SMe.

In the compound (M-11), a compound in which $Q^3$ is a group represented by Q33, $R^{3b}$ and $R^{3d}$ are chlorine atoms and $R^{50}$ is SEt.

In the compound (M-11), a compound in which $Q^3$ is a group represented by Q33, $R^{3b}$ and $R^{3d}$ are bromine atoms and $R^{50}$ is SEt.

In the compound (M-11), a compound in which $Q^3$ is a group represented by Q33, $R^{3b}$ and $R^{3d}$ are iodine atoms and $R^{50}$ is SEt.

In the compound (M-11), a compound in which $Q^3$ is a group represented by Q33, $R^{3b}$ and $R^{3d}$ are chlorine atoms and $R^{50}$ is S(O)Me.

In the compound (M-11), a compound in which $Q^3$ is a group represented by Q33, $R^{3b}$ and $R^{3d}$ are bromine atoms and $R^{50}$ is S(O)Me.

In the compound (M-11), a compound in which $Q^3$ is a group represented by Q33, $R^{3b}$ and $R^{3d}$ are iodine atoms and $R^{50}$ is S(O)Me.

In the compound (M-11), a compound in which $Q^3$ is a group represented by Q33, $R^{3b}$ and $R^{3d}$ are chlorine atoms and $R^{50}$ is S(O)Et.

In the compound (M-11), a compound in which $Q^3$ is a group represented by Q33, $R^{3b}$ and $R^{3d}$ are bromine atoms and $R^{50}$ is S(O)Et.

In the compound (M-11), a compound in which $Q^3$ is a group represented by Q33, $R^{3b}$ and $R^{3d}$ are iodine atoms and $R^{50}$ is S (O)Et.

In the compound (M-11), a compound in which $Q^3$ is a group represented by Q33, $R^{3b}$ and $R^{3d}$ are chlorine atoms and $R^{50}$ is $S(O)_2Me$.

In the compound (M-11), a compound in which $Q^3$ is a group represented by Q33, $R^{3b}$ and $R^{3d}$ are bromine atoms and $R^{50}$ is $S(O)_2Me$.

In the compound (M-11), a compound in which $Q^3$ is a group represented by Q33, $R^{3b}$ and $R^{3d}$ are iodine atoms and $R^{50}$ is $S(O)_2Me$.

In the compound (M-11), a compound in which $Q^3$ is a group represented by Q33, $R^{3b}$ and $R^{3d}$ are chlorine atoms and $R^{50}$ is $S(O)_2Et$.

In the compound (M-11), a compound in which $Q^3$ is a group represented by Q33, $R^{3b}$ and $R^{3d}$ are bromine atoms and $R^{50}$ is $S(O)_2Et$.
In the compound (M-11), a compound in which $Q^3$ is a group represented by Q33, $R^{3b}$ and $R^{3d}$ are iodine atoms and $R^{50}$ is $S(O)_2Et$.
Next, formulation examples of the compound X of the present invention are shown. The part means part by weight. In addition, the compound S of the present invention represents a compound described in the compound groups SX1 to SX1491.

Formulation Example 1

10 parts of any one of the compounds S of the present invention are mixed in a mixture of 35 parts of xylene and 35 parts of DMF, 14 parts of polyoxyethylenestyrylphenyl ether and 6 parts of calcium dodecylbenzenesulfonate are added thereto, and the mixture is mixed to obtain formulation.

Formulation Example 2

4 parts of sodium lauryl sulfate, 2 parts of calcium lignosulfonate, 20 parts of wet silica, and 54 parts of diatomaceous earth are mixed, and 20 parts of any one of the compounds S of the present invention is further added thereto, and the mixture is mixed to obtain formulation.

Formulation Example 3

1 part of wet silica, 2 parts of calcium lignosulfonate, 30 parts of bentonite and 65 parts of kaolin clay are added to 2 parts of any one of the compounds S of the present invention. Subsequently, an appropriate amount of water is added to this mixture, and the mixture is further stirred, granulated with a granulator, and forced-air dried to obtain formulation.

Formulation Example 4

1 part of any one of the compounds S of the present invention is mixed with an appropriate amount of acetone, 5 parts of wet silica, 0.3 parts of isopropyl acid phosphate, and 93.7 parts of kaolin clay are added thereto. The mixture is sufficiently stirred and mixed to evaporate and eliminate acetone to obtain formulation.

Formulation Example 5

35 parts of a mixture of polyoxyethylene alkyl ether sulfate ammonium salt and wet silica (weight ratio 1:1), and 20 parts of any one of the compounds S of the present invention and 45 parts of water are sufficiently mixed to obtain formulation.

Formulation Example 6

10 parts of any one of the compounds S of the present invention and a mixture of 18 parts of benzyl alcohol and 9 parts of DMSO are mixed, 6.3 parts of GERONOL (registered trademark) TE250, 2.7 parts of Ethylan (trademark) NS-500 LQ, and 54 parts of solvent naphtha are added thereto, and the mixture is mixed to obtain a formulation.
Next, the efficacy of the compound X of the present invention against the harmful arthropod is shown by Test Examples. In the following Test Examples, the test was performed at 25°C.

Test Method 1

A test compound is formed into a formulation according to the method described in Formulation Example 5, and water containing 0.03 volume% of SINDAIN (registered trademark) is added thereto to prepare a diluent containing the test compound at a predetermined concentration.
A cucumber (Cucumis sativus) seedling (second true leaf stage) planted in a container is inoculated with about 30 aphis gossypii (whole stage). One day later, the diluent is sprayed at a rate of 10 mL/seedling. After 5 days, the number of surviving insects is examined, and the control value is determined by the following formula.

$$\text{Control value } (\%) = \{1 - (Cb \times Tai)/(Cai \times Tb)\} \times 100$$

wherein the symbols represent as follows:

Cb: the number of test insects in a non-treated section
Cai: the number of surviving insects in a non-treated section on observation
Tb: the number of test insects in a treated section
Tai: the number of surviving insects in a treated section on observation

Here, the non-treated section means a section that performs the same operation as the treated section except that the test compound is not used.

Test Example 1-1

As a result of performing a test according to Test Method 1 using the following compound of the present invention as a test compound at a predetermined concentration of 500 ppm, the following compound of the present invention showed a control value of 90% or more.
Compound of Present Invention: 1
Test Method 2
A test compound is formed into a formulation according to the method described in Formulation Example 6, and water is added thereto to prepare a diluent containing the test compound at a predetermined concentration.
A cucumber seedling (second true leaf stage) planted in a container is irrigated with the diluent at a rate of 5 mL/seedling. After 7 days, the leaf surface of this seedling is inoculated with about 30 aphis gossypii (whole stage). After 6 days, the number of surviving insects is examined, and the control value is determined by the following formula.

$$\text{Control value (\%)} = \{1 - (Cb \times Tai)/(Cai \times Tb)\} \times 100$$

wherein the symbols represent as follows:

Cb: the number of test insects in a non-treated section
Cai: the number of surviving insects in a non-treated section on observation
Tb: the number of test insects in a treated section
Tai: the number of surviving insects in a treated section on observation

Here, the non-treated section means a section that performs the same operation as the treated section except that the test compound is not used.

Test Example 2-1

As a result of performing a test according to Test Method 2 using the following compound of the present invention as a test compound at a predetermined concentration of 1000 ppm, all the following compounds of the present invention showed a control value of 90% or more.
Compounds of Present Invention: 1, 7

Test Example 2-2

As a result of performing a test according to Test Method 2 using the following compound of the present invention as a test compound at a predetermined concentration of 200 ppm, all the following compounds of the present invention showed a control value of 90% or more.
Compounds of Present Invention: 1, 7

Test Method 3

A test compound is formed into a formulation according to the method described in Formulation Example 5, and water containing 0.03 volume% of SINDAIN (registered trademark) is added thereto to prepare a diluent containing the test compound at a predetermined concentration.
A cabbage (Brassicae oleracea) seedling (second to third true leaf stages) planted in a container is sprayed with the diluent at a rate of 20 mL/seedling. Thereafter, a foliage portion of the seedling is cut out and placed in a container on which filter paper is laid. Thereto are released five third-instar larvae of Spodoptera litura. After 5 days, the number

of surviving insects is counted, and the death rate is calculated according to the following equation:

$$\text{Death rate (\%)} = (1 - \text{Surviving insects}/5) \times 100$$

Test Example 3-1

As a result of performing a test according to Test Method 3 using the following compound of the present invention as a test compound at a predetermined concentration of 500 ppm, the following compound of the present invention showed a death rate of 80% or more.

Compound of Present Invention: 1

Test Method 4

A test compound is formed into a formulation according to the method described in Formulation Example 5, and water containing 0.03 volume% of SINDAIN (registered trademark) is added thereto to prepare a diluent containing the test compound at a predetermined concentration.

A cabbage seedling (second to third true leaf stages) planted in a container is sprayed with the diluent at a rate of 20 mL/seedling. Thereafter, a foliage portion of the seedling is cut out and placed in a container on which filter paper is laid. Thereto are released five third-instar larvae of Plutella xylostella. After 5 days, the number of surviving insects is counted, and the death rate is calculated according to the following equation:

$$\text{Death rate (\%)} = (1 - \text{Surviving insects}/5) \times 100$$

Test Example 4-1

As a result of performing a test according to Test Method 4 using the following compound of the present invention as a test compound at a predetermined concentration of 500 ppm, the following compound of the present invention showed a death rate of 80% or more.

Compound of Present Invention: 1

Test Method 5

The test compound is dissolved in 50 μL of a mixed solution of polyoxyethylene sorbitan monococoate : acetone = 5 : 95 (volume ratio) per 1 mg. Water containing 0.03 volume% of SINDAIN (registered trademark) is added thereto to prepare a diluent containing the test compound at a predetermined concentration.

Young seedlings of corn (Zea mays) are immersed in the diluent for 30 seconds. Thereafter, the two fruits are placed in a petri dish (90 mm diameter), and 10 second-instar larvae of Western corn rootworm are released. After 5 days, the number of dead insects is counted, and the death rate is calculated according to the following equation:

$$\text{Death rate (\%)} = (\text{Dead insects}/10) \times 100$$

Test Example 5-1

As a result of performing a test according to Test Method 5 using the following compound of the present invention as a test compound at a predetermined concentration of 500 ppm, the following compound of the present invention showed a death rate of 80% or more.

Compound of Present Invention: 1

Test Example 5-2

As a result of performing a test according to Test Method 5 using the following compound of the present invention as a test compound at a predetermined concentration of 200 ppm, the following compound of the present invention showed a death rate of 80% or more.

Compound of Present Invention: 1

Test Method 6

An acetone solution prepared so that the amount of the test compound is 800 ppm is poured into a container having an internal volume of 50 mL, and an inner surface of the container is uniformly coated so that the amount of the test compound is 40 $mg/m^2$, and then dried.

Five male imagoes of Blattella germanica are placed in the container, and a lid is closed. After a lapse of a predetermined time, the state of Blattella germanica is examined, and the death rate is determined. The death rate is calculated by the following equation.

$$\text{Death rate (\%)} = (\text{Dead insects}/5) \times 100$$

Test Example 6-1

As a result of performing a test according to Test Method 6 using the compound of the present invention as a test compound with a predetermined time set to one day, the following compound of the present invention showed a death rate of 80% or more.

Compound of Present Invention: 1

Test Example 6-2

As a result of performing a test according to Test Method 6 using the compound of the present invention as a test compound with a predetermined time set to three days, the following compound of the present invention showed a death rate of 80% or more.

Compound of Present Invention: 1

Test Method 7

An acetone solution prepared so that the amount of the test compound is 2000 ppm is poured into a container having an internal volume of 20 mL, and an inner surface of the container is uniformly coated so that the amount of the test compound is 100 $mg/m^2$, and then dried.

Five nymphs of Haemaphysalis longicornis are placed in the container, and the lid is closed. After a lapse of a predetermined time, the state of Haemaphysalis longicornis is examined, and the dead + moribund rate is determined. The dead + moribund rate is calculated by the following equation.

$$\text{Dead + moribund rate (\%)} = (\text{dead + moribund insects}/5) \times 100$$

Test Example 7-1

As a result of performing a test according to Test Method 7 using the compound of the present invention as a test compound with a predetermined time set to two days, the following compound of the present invention showed a Dead + moribund of 100%.

Compound of Present Invention: 9

Test Method 8

An acetone solution prepared so that the amount of the test compound is 800 ppm is poured into a container having an internal volume of 20 mL, and an inner surface of the container is uniformly coated so that the amount of the test compound is 40 $mg/m^2$, and then dried.

Five adult female Musca domestica are placed in the container, and the lid is closed. After a lapse of a predetermined time, the state of Musca domestica is examined, and the death rate is determined. The death rate is calculated by the following equation.

$$\text{Death rate (\%)} = (\text{Dead insects}/5) \times 100$$

Test Example 8-1

As a result of performing a test according to Test Method 8 using the following compound of the present invention as a test compound with a predetermined time set to one day, the following compound of the present invention showed a death rate of 80% or more.
Compound of Present Invention: 1

Test Method 9

A test compound is formed into a formulation according to the method described in Formulation Example 6, and water containing 0.03 volume% of SINDAIN (registered trademark) is added thereto to prepare a diluent containing the test compound at a predetermined concentration.
A cucumber (Cucumis sativus) seedling (second true leaf stage) planted in a container is inoculated with about 30 aphis gossypii (whole stage). One day later, the diluent is sprayed at a rate of 10 mL/seedling. After 5 days, the number of surviving insects is examined, and the control value is determined by the following formula.

$$\text{Control value (\%)} = \{1 - (Cb \times Tai)/(Cai \times Tb)\} \times 100$$

wherein the symbols represent as follows:

Cb: the number of test insects in a non-treated section
Cai: the number of surviving insects in a non-treated section on observation
Tb: the number of test insects in a treated section
Tai: the number of surviving insects in a treated section on observation

Here, the non-treated section means a section that performs the same operation as the treated section except that the test compound is not used.

Test Example 9-1

As a result of performing a test according to Test Method 9 using the following compound of the present invention as a test compound at a predetermined concentration of 500 ppm, all the following compounds of the present invention showed a control value of 90% or more.
Compounds of Present Invention: 1, 3, 4, 7, 10

Test Example 9-2

As a result of performing a test according to Test Method 9 using the following compound of the present invention as a test compound at a predetermined concentration of 200 ppm, all the following compounds of the present invention showed a control value of 90% or more.
Compounds of Present Invention: 1, 3, 4, 7, 10

Test Method 10

A test compound is formed into a formulation according to the method described in Formulation Example 6, and water containing 0.03 volume% of SINDAIN (registered trademark) is added thereto to prepare a diluent containing the test compound at a predetermined concentration.
A cabbage (Brassicae oleracea) seedling (third to fourth true leaf stages) planted in a container is sprayed with the diluent at a rate of 20 mL/seedling. Thereafter, ten third-instar larvae of Spodoptera litura are released. After 6 days, the number of surviving insects is counted, and the death rate is calculated according to the following equation:

$$\text{Death rate (\%)} = (1 - \text{Surviving insects}/10) \times 100$$

Test Example 10-1

As a result of performing a test according to Test Method 10 using the following compound of the present

invention as a test compound at a predetermined concentration of 500 ppm, all the following compounds of the present invention showed a death rate of 80% or more.
Compounds of Present Invention: 1, 3, 4, 6, 7

Test Example 10-2

As a result of performing a test according to Test Method 10 using the following compound of the present invention as a test compound at a predetermined concentration of 200 ppm, all the following compounds of the present invention showed a death rate of 80% or more.
Compounds of Present Invention: 1, 3, 4, 6, 7

Test Method 11

A test compound is formed into a formulation according to the method described in Formulation Example 6, and water containing 0.03 volume% of SINDAIN (registered trademark) is added thereto to prepare a diluent containing the test compound at a predetermined concentration.
A cabbage (Brassicae oleracea) seedling (second to third true leaf stages) planted in a container is sprayed with the diluent at a rate of 20 mL/seedling. Thereafter, a foliage portion of the seedling is cut out and placed in a container on which filter paper is laid. Five second-instar larvae of Plutella xylostella are released thereto. After 5 days, the number of surviving insects is counted, and the death rate is calculated according to the following equation:

$$\text{Death rate (\%)} = (1 - \text{Surviving insects}/5) \times 100$$

Test Example 11-1

As a result of performing a test according to Test Method 11 using the following compound of the present invention as a test compound at a predetermined concentration of 500 ppm, all the following compounds of the present invention showed a death rate of 80% or more.
Compounds of Present Invention: 1, 3, 4, 6, 7, 9

Test Example 11-2

As a result of performing a test according to Test Method 11 using the following compound of the present invention as a test compound at a predetermined concentration of 200 ppm, all the following compounds of the present invention showed a death rate of 80% or more.
Compounds of Present Invention: 1, 3, 4, 6, 7, 9

Test Method 12

1 mg of the compound X of the present invention is dissolved in 10 μL of a mixed solution of xylene: DMF: surfactant = 4:4:1 (volume ratio), and diluted with water containing 0.02 volume% of a spreader to prepare a diluent A containing a predetermined concentration of the compound X of the present invention.
1 mg of the present ingredient is dissolved in 10 μL of a mixed solution of xylene: DMF: surfactant = 4:4:1 (volume ratio), and diluted with water containing 0.02 volume% of a spreader to prepare a diluent B containing a predetermined concentration of the present ingredient.
A diluent A and a diluent B are mixed to obtain a diluent C.
Leaf pieces of cucumber cotyledons (length 1.5 cm) are accommodated in each well of a 24 well microplate, 2 aphis gossypii and 8 larvae are released per well, and 20 μL of the diluent C is sprayed per well. This is defined as a treated section.
A well to which 20 μL of water containing 0.02 vol% of a spreader is sprayed instead of the diluent C is defined as a non-treated section.
After the diluent C is dried, an upper portion of the microplate is covered with a film sheet. After 5 days, the number of surviving insects in each well is examined.
The control value is calculated from the following formula.

$$\text{Control value (\%)} = \{1 - (Cb \times Tai)/(Cai \times Tb)\} \times 100$$

Note that, the symbols represent as follows:

Cai: the number of surviving insects in a non-treated section on observation
Tai: the number of surviving insects in a treated section on observation

Specific diluents C whose effects can be confirmed by Test Method 12 are shown in the following 1) to 5).
1) Diluent C having a concentration of the compound of the present invention of 200 ppm and a concentration of the present ingredient of 2000 ppm in the combination shown in the list A. In the list A, Comp X means any one compound selected from the compound 1 of the present invention to the compound 10 of the present invention.

List A:

Comp X + clothianidin; Comp X + thiamethoxam; Comp X + imidacloprid; Comp X + thiacloprid; Comp X + flupyradifuron; Comp X + sulfoxaflor; Comp X + triflumezopyrime; Comp X + dichloromethothiazide; Comp X + beta cyfluthrin; Comp X + tefluthrin; Comp X + fipronil; Comp X + chlorantraniliprole; Comp X + cyantraniliprole; Comp X + tetraniliprole; Comp X + thiodicarb; Comp X + carbofuran; Comp X + fluxamethamide; Comp X + afoxolanel; Comp X + fluraranel; Comp X + brofuranilide; Comp X + abamectin; Comp X + fluopyram; Comp X + fluenesulfone; Comp X + fluazaindolidine; Comp X + thioxazafen; Comp X + flupyrimine; Comp X + mycorrhizal fungi; Comp X + bradyrhizobium japonicum TA-11 strain; Comp X + bacillus film; Comp X + bacillus film 1-1582 strain; Comp X + bacillus amyloliquefaciens; Comp X + bacillus amyloliquefaciens FZB 42 strain; Comp X + pasteuria nishizawae; Comp X + pasteuria nishizawae Pn1 strain; Comp X + pasteuria penetrans; Comp X + tebuconazole; Comp X + prothioconazole; Comp X + metconazole; Comp X + ipconazole; Comp X + triticonazole; Comp X + difenoconazole; Comp X + imazalyl; Comp X + triadimenol; Comp X + tetraconazole; Comp X + flutriafol; Comp X + mandestrobin; Comp X + azoxystrobin; Comp X + pyraclostrobin; Comp X + trifloxystrobin; Comp X + fluoxastrobin; Comp X + picoxystrobin; Comp X + phenamidone; Comp X + metalaxyl; Comp X + metalaxyl M; Comp X + fludioxonil; Comp X + sedaxane; Comp X + penflufen; Comp X + fluxapyroxad; Comp X + benzobindiflupyr; Comp X + boscalid; Comp X + carboxin; Comp X + penthiopyrad; Comp X + flutolanyl; Comp X + captan; Comp X + thiuram; Comp X + tolclophos-methyl; Comp X + thiabendazole; Comp X + ethaboxam; Comp X + mancozeb; Comp X + picarbutrazox; Comp X + oxathiapiprolin; Comp X + silthiofam; and Comp X + inpyrfluxam.
2) Diluent C having a concentration of the compound of the present invention of 200 ppm and a concentration of the present ingredient of 200 ppm in the combination shown in the list A.
3) Diluent C having a concentration of the compound of the present invention of 500 ppm and a concentration of the present ingredient of 50 ppm in the combination shown in the list A.
4) Diluent C having a concentration of the compound of the present invention of 500 ppm and a concentration of the present ingredient of 5 ppm in the combination shown in the list A.
5) Diluent C having a concentration of the compound of the present invention of 500 ppm and a concentration of the present ingredient of 0.5 ppm in the combination shown in the list A.

INDUSTRIAL APPLICABILITY

The compound X of the present invention exhibits an excellent control effect on a harmful arthropod.

**Claims**

**1.** A compound represented by Formula (I),

[Chem. 1]

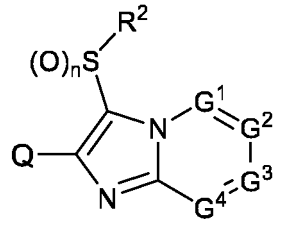

(I)

[wherein

$R^2$ represents a C1-C6 alkyl group that may be substituted with one or more halogen atoms, a cyclopropyl group, or a cyclopropylmethyl group,
n represents 0, 1, or 2,
$G^1$ represents a nitrogen atom or $CR^{3a}$,
$G^2$ represents a nitrogen atom or $CR^{3b}$,
$G^3$ represents a nitrogen atom or $CR^{3c}$,
$G^4$ represents a nitrogen atom or $CR^{3d}$,
$R^{3a}$, $R^{3b}$,$R^{3c}$, and $R^{3d}$ are the same or different and represent a C1-C6 chain hydrocarbon group that may be substituted with one or more substituents selected from a group B, a C3-C7 cycloalkyl group that may be substituted with one or more substituents selected from a group E, a phenyl group that may be substituted with one or more substituents selected from a group H, a 5- or 6-membered aromatic heterocyclic group that may be substituted with one or more substituents selected from a group H, $OR^{12}$, $NR^{11}R^{12}$, $NR^{11a}R^{12a}$, $NR^{24}NR^{11}R^{12}$, $NR^{24}OR^{11}$, $NR^{11}C(O)R^{13}$, $NR^{24}NR^{11}C(O)R^{13}$, $NR^{11}C(O)OR^{14}$, $NR^{24}NR^{11}C(O)OR^{14}$, $NR^{11}C(O)NR^{31}R^{32}$, $NR^{24}NR^{11}C(O)NR^{31}R^{32}$, $N=CHNR^{31}R^{32}$, $N=S(O)_pR^{15}R^{16}$, $C(O)R^{13}$, $C(O)OR^{17}$, $C(O)NR^{31}R^{32}$, $C(O)NR^{11}S(O)_2R^{23}$, $CR^{30}=NOR^{17}$, $NR^{11}CR^{24}=NOR^{17}$, $S(O)_mR^{23}$, a cyano group, a nitro group, a hydrogen atom, or a halogen atom,
p represents 0 or 1,
m represents 0, 1, or 2,
$R^{30}$ represents a C1-C6 chain hydrocarbon group that may be substituted with one or more halogen atoms, a halogen atom, $OR^{35}$, $NR^{36}R^{37}$, or a hydrogen atom,
$R^{35}$ represents a C1-C6 chain hydrocarbon group that may be substituted with one or more halogen atoms,
$R^{17}$ represents a C1-C6 chain hydrocarbon group that may be substituted with one or more halogen atoms, a phenyl group that may be substituted with one or more substituents selected from a group D, or a hydrogen atom,
$R^{11}$, $R^{24}$, $R^{36}$, and $R^{37}$ are the same or different, and represent a C1-C6 chain hydrocarbon group that may be substituted with one or more halogen atoms, or a hydrogen atom,
$R^{12}$ represents a C1-C6 chain hydrocarbon group that may be substituted with one or more substituents selected from a group F, a C3-C7 cycloalkyl group that may be substituted with one or more substituents selected from a group J, a C3-C7 cycloalkenyl group that may be substituted with one or more substituents selected from a group J, a phenyl group that may be substituted with one or more substituents selected from a group D, a 6-membered aromatic heterocyclic group that may be substituted with one or more substituents selected from a group D, a hydrogen atom, or $S(O)_2R^{23}$,
$R^{23}$ represents a C1-C6 chain hydrocarbon group that may be substituted with one or more halogen atoms, or a phenyl group that may be substituted with one or more substituents selected from a group D,
$R^{11a}$ a and $R^{12a}$, together with the nitrogen atom to which they are attached, form a 3- to 7-membered non-aromatic heterocyclic group that may be substituted with one or more substituents selected from a group E,
$R^{13}$ represents a C1-C6 chain hydrocarbon group that may be substituted with one or more halogen atoms, a C3-C7 cycloalkyl group that may be substituted with one or more halogen atoms, a (C3-C6 cycloalkyl) C1-C3 alkyl group that may be substituted with one or more halogen atoms, a phenyl group that may be substituted with one or more substituents selected from a group D, a 5- or 6-membered aromatic heterocyclic group that may be substituted with one or more substituents selected from a group D, or a hydrogen atom,
$R^{14}$ represents a C1-C6 chain hydrocarbon group that may be substituted with one or more halogen atoms, a C3-C7 cycloalkyl group that may be substituted with one or more halogen atoms, a (C3-C6 cycloalkyl) C1-C3 alkyl group that may be substituted with one or more halogen atoms, or a phenyl C1-C3 alkyl group {a phenyl part in the phenyl C1-C3 alkyl group may be substituted with one or more substituents selected from a group D},
$R^{15}$ and $R^{16}$ are the same or different and represent a C1-C6 alkyl group that may be substituted with one or more halogen atoms,
$R^{31}$ represents a C1-C6 alkyl group that may be substituted with one or more halogen atoms or a hydrogen atom, and
$R^{32}$ represents a C1-C6 chain hydrocarbon group that may be substituted with one or more substituents selected from a group F, a C3-C7 cycloalkyl group that may be substituted with one or more substituents selected from a group J, $S(O)_2R^{23}$, or a hydrogen atom,
Q represents a group represented by Q2, a group represented by Q3, a group represented by Q4, a group represented by Q5, a group represented by Q6, or a group represented by Q7,

[Chem. 2]

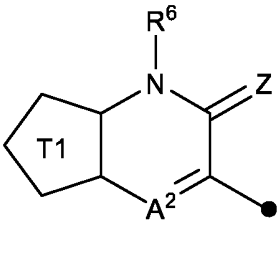
Q2

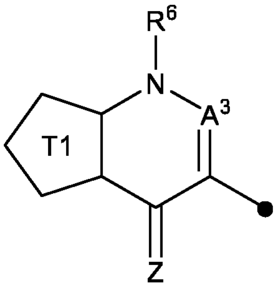
Q3

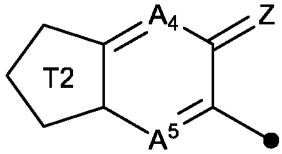
Q4

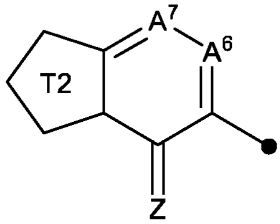
Q5

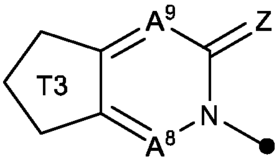
Q6

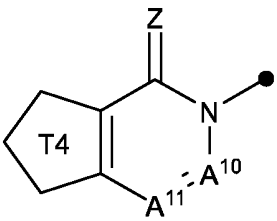
Q7

• represents a site of binding with the rest of molecules,
T1 represents a group represented by T1-1, a group represented by Tl-2, or a group represented by T1-3,

[Chem. 3]

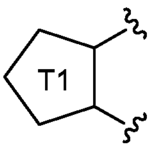
=

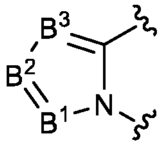
T1-1

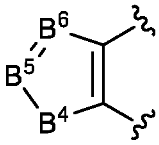
T1-2

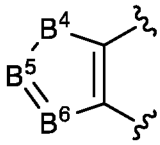
T1-3

T2 represents a group represented by T2-1, a group represented by T2-2, or a group represented by T2-3,

[Chem. 4]

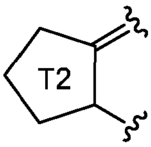
=

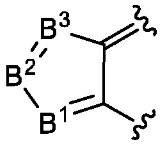
T2-1

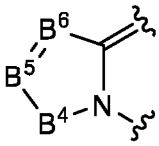
T2-2

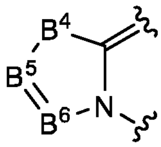
T2-3

T3 represents a group represented by T3-1 or a group represented by T3-2,

[Chem. 5]

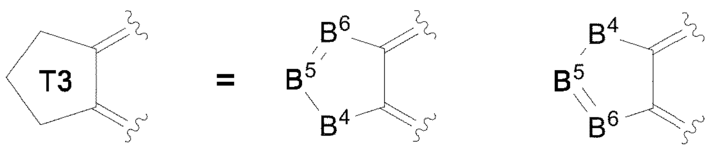

T3-1 T3-2

T4 represents a group represented by T4-1 or a group represented by T4-2,

[Chem. 6]

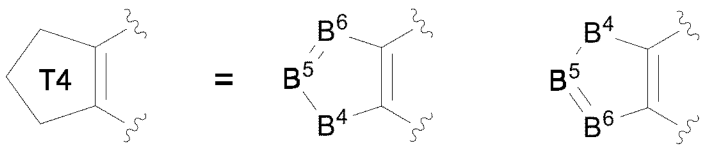

T4-1 T4-2

$A^2$ represents a nitrogen atom or $CR^{4a}$,
$A^3$ represents a nitrogen atom or $CR^{4b}$,
$A^4$ represents a nitrogen atom or $CR^{4c}$,
$A^5$ represents a nitrogen atom or $CR^{4d}$,
$A^6$ represents a nitrogen atom or $CR^{4e}$,
$A^7$ represents a nitrogen atom or $CR^{4f}$,
$A^8$ represents a nitrogen atom or $CR^{4g}$,
$A^9$ represents a nitrogen atom or $CR^{4h}$,
$A^{10}$ represents a nitrogen atom or $CR^{4i}$,
$A^{11}$ represents a nitrogen atom or $CR^{4j}$,
a combination of $B^1$, $B^2$, and $B^3$ represents
a combination in which $B^1$ is $CR^1$, $B^2$ is a nitrogen atom or $CR^{38b}$, and $B^3$ is a nitrogen atom or $CR^{38c}$;
a combination in which $B^1$ is a nitrogen atom or $CR^{38a}$, $B^2$ is $CR^1$, and $B^3$ is a nitrogen atom or $CR^{38c}$; or
a combination in which $B^1$ is a nitrogen atom or $CR^{38a}$, $B^2$ is a nitrogen atom or $CR^{38b}$, and $B^3$ is $CR^1$,
a combination of $B^4$, $B^5$, and $B^6$ represents
a combination in which $B^4$ is an oxygen atom, a sulfur atom, or $NR^5$, $B^5$ is $CR^1$, and $B^6$ is a nitrogen atom or $CR^{38c}$; or
a combination in which $B^4$ is an oxygen atom, a sulfur atom, or $NR^5$, $B^5$ is a nitrogen atom or $CR^{38b}$, and $B^6$ is $CR^1$,
Z represents an oxygen atom or a sulfur atom,
$R^1$ represents a C1-C6 chain hydrocarbon group that is substituted with one or more substituents selected from the group consisting of a cyano group and a halogen atom, a C3-C4 cycloalkyl group that may be substituted with one or more substituents selected from the group consisting of a cyano group and a halogen atom, $SR^8$, $S(O)R^3$, $S(O)_2R^8$, $OR^8$, or $OS(O)_2R^8$,
$R^8$ represents a C1-C6 chain hydrocarbon group that is substituted with one or more substituents selected from the group consisting of a cyano group and a halogen atom or a C3-C4 cycloalkyl group that may be substituted with one or more substituents selected from the group consisting of a cyano group and a halogen atom,
$R^{4a}$, $R^{4b}$, $R^{4c}$, $R^{4d}$, $R^{4e}$, $R^{4f}$, $R^{4g}$, $R^{4h}$, $R^{4i}$, and $R^{4j}$ are the same or different and represent a C1-C6 chain hydrocarbon group that may be substituted with one or more halogen atoms, a nitro group, $OR^{18}$, $NR^{18}R^{19}$, a cyano group, an amino group, a halogen atom, or a hydrogen atom,
$R^{18}$ represents a C1-C6 chain hydrocarbon group that may be substituted with one or more halogen atoms,
$R^{19}$ represents a C1-C6 chain hydrocarbon group that may be substituted with one or more halogen atoms or a hydrogen atom,
$R^{38a}$, $R^{38b}$, and $R^{38c}$ are the same or different, and represent a C1-C3 chain hydrocarbon group that may be substituted with one or more halogen atoms, a halogen atom, or a hydrogen atom,

$R^5$ and $R^6$ are the same or different and represent a C1-C6 chain hydrocarbon group that may be substituted with one or more substituents selected from a group F, a C3-C6 cycloalkyl group that may be substituted with one or more substituents selected from a group J, a phenyl group that may be substituted with one or more substituents selected from a group H, a 5- or 6-membered aromatic heterocyclic group that may be substituted with one or more substituents selected from a group H, or a hydrogen atom,

Group B: a group consisting of a C1-C6 alkoxy group that may be substituted with one or more halogen atoms, a C3-C6 alkenyloxy group that may be substituted with one or more halogen atoms, a C3-C6 alkynyloxy group that may be substituted with one or more halogen atoms, a C1-C6 alkylsulfanyl group that may be substituted with one or more halogen atoms, a C1-C6 alkylsulfinyl group that may be substituted with one or more halogen atoms, a C1-C6 alkylsulfonyl that may be substituted with one or more halogen atoms, a C3-C6 cycloalkyl group that may be substituted with one or more halogen atoms, a cyano group, a hydroxy group, and a halogen atom.

Group C: a group consisting of a C1-C6 chain hydrocarbon groups that may be substituted with one or more halogen atoms, a C1-C6 alkoxy group that may be substituted with one or more halogen atoms, a C3-C6 alkenyloxy group that may be substituted with one or more halogen atoms, a C3-C6 alkynyloxy group that may be substituted with one or more halogen atoms, and a halogen atom.

Group D: a group consisting of a C1-C6 chain hydrocarbon group that may be substituted with one or more halogen atoms, a hydroxy group, a C1-C6 alkoxy group that may be substituted with one or more halogen atoms, a C3-C6 alkenyloxy group that may be substituted with one or more halogen atoms, a C3-C6 alkynyloxy group that may be substituted with one or more halogen atoms, a sulfanyl group, a C1-C6 alkylsulfanyl group that may be substituted with one or more halogen atoms, a C1-C6 alkylsulfinyl group that may be substituted with one or more halogen atoms, a C1-C6 alkylsulfonyl that may be substituted with one or more halogen atoms, an amino group, $NHR^{21}$, $NR^{21}R^{22}$, $C(O)R^{21}$, $OC(O)R^{21}$, $C(O)OR^{21}$, a cyano group, a nitro group, and a halogen atom.

$R^{21}$ and $R^{22}$ are the same or different and represent a C1-C6 alkyl group that may be substituted with one or more halogen atoms.

Group E: a group consisting of a C1-C6 chain hydrocarbon groups that may be substituted with one or more halogen atoms, a C1-C6 alkoxy group that may be substituted with one or more halogen atoms, a C3-C6 alkenyloxy group that may be substituted with one or more halogen atoms, a C3-C6 alkynyloxy group that may be substituted with one or more halogen atoms, a halogen atom, an oxo group, a hydroxy group, a cyano group, and a nitro group.

Group F: a group consisting of a C1-C6 alkoxy group that may be substituted with one or more halogen atoms, a phenyl group that may be substituted with one or more substituents selected from a group D, a 5- or 6-membered aromatic heterocyclic group that may be substituted with one or more substituents selected from a group D, a C3-C7 cycloalkyl group that may be substituted with one or more halogen atoms, a 3- to 7-membered non-aromatic heterocyclic group that may be substituted with one or more substituents selected from a group C, an amino group, $NHR^{21}$, $NR^{21}R^{22}$, a halogen atom, and a cyano group.

Group H: a group consisting of a C1-C6 alkyl group that may be substituted with one or more halogen atoms, 5- or 6-membered aromatic heterocyclic group that may be substituted with one or more substituents selected from a group D, $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, and an amino group.

$R^9$ represents a C1-C6 alkyl group that may be substituted with one or more halogen atoms or a C3-C6 cycloalkyl group that may be substituted with one or more halogen atoms,

$R^{10}$ represents a C1-C6 alkyl group that may be substituted with one or more halogen atoms, a C3-C6 cycloalkyl group that may be substituted with one or more halogen atoms, or a hydrogen atom.

Group J: a group consisting of C1-C6 alkyl group that may be substituted with one or more halogen atoms, a halogen atom, and a cyano group.

or an N-oxide thereof.

**2.** The compound or the N-oxide thereof according to claim 1, wherein

Q is a group represented by Q2, a group represented by Q3, a group represented by Q6, or a group represented by Q7,

$A^2$ is $CR^{4a}$, $A^3$ is $CR^{4b}$, $A^8$ is $CR^{4g}$, $A^9$ is a nitrogen atom or $CR^{4h}$, $A^{10}$ is $CR^{4i}$, and $A^{11}$ is a nitrogen atom or $CR^{4j}$, $B^1$ is $CR^{38a}$, $B^2$ is $CR^1$, $B^3$ is a nitrogen atom, $B^4$ is $NR^5$, $B^5$ is $CR^1$, and $B^6$ is a nitrogen atom.

**3.** The compound or the N-oxide thereof according to claim 1, wherein Q represents a group represented by Q2 or a group represented by Q3.

**4.** The compound or the N-oxide thereof according to claim 3, wherein

$A^2$ is $CR^{4a}$, and $A^3$ is $CR^{4b}$, and
$B^1$ is $CR^{38a}$, $B^2$ is $CR^1$, $B^3$ is a nitrogen atom, $B^4$ is $NR^5$, $B^5$ is $CR^1$, and $B^6$ is a nitrogen atom.

**5.** The compound or the N-oxide thereof according to any one of claims 1 to 4, wherein

$R^1$ is a C1-C6 chain hydrocarbon group substituted with one or more halogen atoms,
$R^{4a}$, $R^{4b}$, $R^{4c}$, $R^{4d}$, $R^{4e}$, $R^{4f}$, $R^{4g}$, $R^{4h}$, $R^{4i}$, and $R^{4j}$ are the same or different and are a C1-C6 chain hydrocarbon group that may be substituted with one or more halogen atoms, a halogen atom, or a hydrogen atom, and
$R^5$ and $R^6$ are the same or different and are a C1-C6 chain hydrocarbon group that may be substituted with one or more halogen atoms, or a hydrogen atom.

**6.** The compound or the N-oxide thereof according to claim 1, wherein
Q is a group represented by Q1,

[Chem. 7]

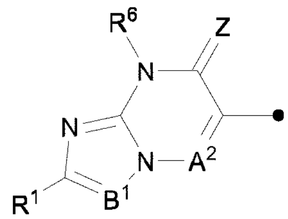

Q1

a combination of $A^2$ and $B^1$ represents
a combination in which $A^2$ is a nitrogen atom and $B^1$ is $CR^{38}$; or
a combination in which $A^2$ is $CR^{4a}$ and $B^1$ is a nitrogen atom or $CR^{38}$,
$R^{38}$ represents a C1-C3 chain hydrocarbon group that may be substituted with one or more halogen atoms, a halogen atom, or a hydrogen atom, and
$R^6$ represents a C1-C6 chain hydrocarbon group that may be substituted with one or more substituents selected from a group F, a C3-C6 cycloalkyl group that may be substituted with one or more substituents selected from a group J, a phenyl group that may be substituted with one or more substituents selected from a group H, or a 5- or 6-membered aromatic heterocyclic group that may be substituted with one or more substituents selected from a group H.

**7.** The compound or the N-oxide thereof according to any one of claims 1 to 6, wherein $G^1$ is CH, $G^2$ is $CR^{3b}$, G3 is $CR^{3c}$, or $G^4$ is CH.

**8.** The compound or the N-oxide thereof according to any one of claims 1 to 7, wherein $R^2$ is an ethyl group.

**9.** The compound or the N-oxide thereof according to any one of claims 1 to 8, wherein Z is an oxygen atom.

**10.** A harmful arthropod-controlling composition containing the compound and the N-oxide thereof according to any one of claims 1 to 9.

**11.** A composition comprising:

one or more ingredients selected from the group consisting of a group (a), a group (b), a group (c), and a group (d); and
the compound or the N-oxide thereof according to any one of claims 1 to 9,
Group (a): a group consisting of an insecticidal active ingredient, an acaricidal active ingredient, and a nematicidal active ingredient;
Group (b): a bactericidal active ingredient;
Group (c): a plant growth regulating ingredient; and

Group (d): a repellent ingredient.

**12.** A method for controlling a harmful arthropod comprising
applying an effective amount of the compound or the N-oxide thereof according to any one of claims 1 to 9 or an effective amount of the composition according to claim 11 to a harmful arthropod or a habitat of the harmful arthropod.

**13.** A seed or a vegetative reproductive organ retaining an effective amount of the compound or the N-oxide thereof according to any one of claims 1 to 9 or an effective amount of the composition according to claim 11.

## INTERNATIONAL SEARCH REPORT

International application No. PCT/JP2021/000444

A. CLASSIFICATION OF SUBJECT MATTER

Int.Cl. C07D519/00(2006.01)i, A01N43/90(2006.01)i, A01P7/02(2006.01)i, A01P7/04(2006.01)i, A61K31/519(2006.01)i, A61K31/53(2006.01)i, A61P33/14(2006.01)i

FI: C07D519/00311, A01N43/90104, A01N43/90105, A01P7/02, A01P7/04, A61K31/519, A61K31/53, A61P33/14, C07D519/00CSP

According to International Patent Classification (IPC) or to both national classification and IPC

B. FIELDS SEARCHED

Minimum documentation searched (classification system followed by classification symbols)

Int.Cl. C07D519/00, A01N43/90, A01P7/02, A01P7/04, A61K31/519, A61K31/53, A61P33/14

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2019/065570 A1 (SUMITOMO CHEMICAL CO., LTD.) 04 April 2019 (2019-04-04), claims, paragraphs [0139]-[0191] | 1-13 |
| A | JP 2017-518309 A (BAYER CROPSCIENCE AG) 06 July 2017 (2017-07-06), claims, paragraphs [0332]-[0534] | 1-13 |
| A | JP 2018-502912 A (SYNGENTA PARTICIPATIONS AG) 01 February 2018 (2018-02-01), claims 8-10 | 1-13 |
| A | WO 2019/131587 A1 (SUMITOMO CHEMICAL CO., LTD.) 04 July 2019 (2019-07-04), claims, paragraphs [0127]-[0238] | 1-13 |
| A | JP 2018-522858 A (UNIV HEALTH NETWORK) 16 August 2018 (2018-08-16), paragraphs [0095]-[0097], [0101], [0106] | 1-13 |

☒ Further documents are listed in the continuation of Box C. ☒ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 05 February 2021 | 16 February 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/000444

C (Continuation). DOCUMENTS CONSIDERED TO BE RELEVANT

| Category* | Citation of document, with indication, where appropriate, of the relevant passages | Relevant to claim No. |
|---|---|---|
| P, A | WO 2020/158889 A1 (SUMITOMO CHEMICAL CO., LTD.) 06 August 2020 (2020-08-06), entire text, all drawings | 1-13 |
| P, A | WO 2020/171077 A1 (SUMITOMO CHEMICAL CO., LTD.) 27 August 2020 (2020-08-27), entire text, all drawings | 1-13 |

Form PCT/ISA/210 (second sheet) (January 2015)

**INTERNATIONAL SEARCH REPORT**
Information on patent family members

International application No.
PCT/JP2021/000444

| | | |
|---|---|---|
| WO 2019/065570 A1 | 04 April 2019 | US 2020/0231587 A1<br>claims, paragraphs [1097]-[1175]<br>EP 3689872 A1<br>CN 111164081 A |
| JP 2017-518309 A | 06 July 2017 | US 2017/0101422 A1<br>claims, paragraphs [0457]-[0675]<br>WO 2015/185531 A1<br>EP 3152216 A1<br>CN 106715424 A |
| JP 2018-502912 A | 01 February 2018 | US 2018/0009813 A1<br>claims 8-10<br>WO 2016/107742 A1<br>EP 3240788 A1<br>CN 107108612 A |
| WO 2019/131587 A1 | 04 July 2019 | US 2020/0399278 A1<br>claims, paragraphs [1091]-[1396]<br>EP 3733672 A1<br>CN 111566108 A |
| JP 2018-522858 A | 16 August 2018 | US 2018/0179221 A1<br>paragraphs [0163]-[0167], [0176], [0184]<br>WO 2016/205942 A1<br>EP 3322711 A1<br>KR 10-2018-0015255 A<br>CN 107922431 A |
| WO 2020/158889 A1 | 06 August 2020 | (Family: none) |
| WO 2020/171077 A1 | 27 August 2020 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020001899 A
- JP 2020132784 A
- WO 2016129684 A
- US 20180009778 A
- WO 2016121970 A
- WO 2019124548 A
- WO 2012020780 A
- WO 2016123253 A
- WO 2013191113 A
- WO 2015086527 A


### Non-patent literature cited in the description


- *Chem. Med. Chem.,* 2018, vol. 13, 988
- *Org. Lett.,* 2016, vol. 18, 6344
- *Tetrahedron,* 2017, vol. 73, 3939
- *Bioorganic & Medicinal Chemistry,* 2008, vol. 16 (6), 3125
- *Tetrahedron,* 2005, vol. 61, 7508
- *Bioorg. Med. Chem. Lett.,* 2013, vol. 23, 6928
- *Bioorg. Med. Chem. Lett.,* 2016, vol. 26, 5290
- *Chem. Eur. J.,* 2016, vol. 22, 11854
- *Chem. Commun.,* 2015, vol. 51, 17744
- *Tetrahedron Lett.,* 2015, vol. 56, 6043
- *Tetrahedron Letters,* 2013, vol. 54, 201